(19) Europäisches Patentamt
European Patent Office
Office européen des brevets

(11) EP 2 406 226 B1

(12) EUROPÄISCHE PATENTSCHRIFT

(45) Veröffentlichungstag und Bekanntmachung des Hinweises auf die Patenterteilung:
**16.09.2015 Patentblatt 2015/38**

(21) Anmeldenummer: **10708139.0**

(22) Anmeldetag: **11.03.2010**

(51) Int Cl.:
*C07D 213/82* (2006.01) *A61K 31/4436* (2006.01)
*A61K 31/465* (2006.01) *C07D 401/12* (2006.01)
*C07D 405/12* (2006.01) *C07D 409/12* (2006.01)
*C07D 413/12* (2006.01) *C07D 417/12* (2006.01)
*C07D 409/14* (2006.01)

(86) Internationale Anmeldenummer:
**PCT/EP2010/001507**

(87) Internationale Veröffentlichungsnummer:
**WO 2010/102809 (16.09.2010 Gazette 2010/37)**

(54) **SUBSTITUIERTE NICOTINAMIDE ALS KCNQ2/3 MODULATOREN**

SUBSTITUTED NICOTINAMIDES AS KCNQ2/3 MODULATORS

NICOTINAMIDES SUBSTITUÉS UTILISÉS COMME MODULATEURS DES CANAUX KCNQ2/3

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **12.03.2009 EP 09003598**

(43) Veröffentlichungstag der Anmeldung:
**18.01.2012 Patentblatt 2012/03**

(73) Patentinhaber: **Grünenthal GmbH**
**52078 Aachen (DE)**

(72) Erfinder:
- **KÜHNERT, Sven**
  **52355 Düren (DE)**
- **MERLA, Beatrix**
  **52078 Aachen (DE)**
- **BAHRENBERG, Gregor**
  **52156 Monschau-Konzen (DE)**
- **SCHRÖDER, Wolfgang**
  **52074 Aachen (DE)**

(56) Entgegenhaltungen:
**EP-A- 0 480 258**
**EP-A- 0 716 077**
**EP-A- 1 449 841**
**WO-A-01/10381**
**WO-A-02/066036**
**WO-A-2005/035514**
**WO-A-2005/105733**
**WO-A-2007/015767**
**WO-A-2009/052078**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 406 226 B1

## Beschreibung

Die Erfindung betrifft substituierte Nicotinamide, Verfahren zu ihrer Herstellung, Arzneimittel enthaltend diese Verbindungen sowie die Verwendung dieser Verbindungen zur Herstellung von Arzneimitteln.

Die Behandlung von Schmerz, insbesondere von neuropathischem Schmerz, hat in der Medizin große Bedeutung. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nociception in letzter Zeit erschienen sind.

Ein pathophysiologisches Merkmal von chronischen Schmerzen besteht in der Übererregbarkeit von Neuronen. Die neuronale Erregbarkeit wird entscheidend von der Aktivität von $K^+$ Kanälen beeinflusst, da diese das Ruhemembranpotential der Zelle und somit die Erregbarkeitsschwelle maßgeblich bestimmen. Heteromere $K^+$ Kanäle vom molekularen Subtyp KCNQ2/3 (Kv7.2/7.3) sind in Neuronen verschiedener Regionen des zentralen (Hippocampus, Amygdala) und peripheren (Hinterwurzelganglien) Nervensystems exprimiert und regulieren deren Erregbarkeit. Die Aktivierung von KCNQ2/3 $K^+$ Kanälen führt zu einer Hyperpolarisation der Zellmembran und damit einhergehend zu einer Abnahme der elektrischen Erregbarkeit dieser Neurone. KCNQ2/3 exprimierende Neurone der Hinterwurzelganglien sind an der Übertragung nociceptiver Erregungen von der Peripherie ins Rückenmark beteiligt (Passmore et al., J Neurosci. 2003; 23(18):7227-36).

Dementsprechend konnte für den KCNQ2/3 Agonisten Retigabine eine analgetische Wirksamkeit in präklinischen Neuropathie- und Entzündungsschmerzmodellen nachgewiesen werden (Blackburn-Munro and Jensen, Eur J Pharmacol. 2003; 460(2-3):109-16; Dost et al., Naunyn Schmiedebergs Arch Pharmacol 2004; 369(4): 382-390).

Der KCNQ2/3 $K^+$ Kanal stellt somit einen geeigneten Ansatzpunkt zur Behandlung von Schmerz; insbesondere von Schmerz ausgewählt aus der Gruppe bestehend aus chronischem Schmerz, neuropathischem Schmerz, inflammatorischem Schmerz und muskulärem Schmerz (Nielsen et al., Eur J Pharmacol. 2004; 487(1-3): 93-103), insbesondere von neuropathischem und inflammatorischem Schmerz dar.

Darüber hinaus ist der KCNQ2/3 $K^+$ Kanal ein geeignetes Target für die Therapie einer Vielzahl weiterer Erkrankungen wie beispielsweise Migräne (US2002/0128277), kognitive Erkrankungen (Gribkoff, Expert Opin Ther Targets 2003; 7(6): 737-748), Angstzuständen (Korsgaard et al., J Pharmacol Exp Ther. 2005, 14(1): 282-92), Epilepsie (Wickenden et al., Expert Opin Ther Pat 2004, 14(4): 457-469; Gribkoff, Expert Opin Ther Targets 2008, 12(5): 565-81; Miceli et al., Curr Opin Pharmacol 2008, 8(1): 65-74), Harninkontinenz (Streng et al., J Urol 2004; 172: 2054-2058), Abhängigkeit (Hansen et al., Eur J Pharmacol 2007, 570(1-3): 77-88), Manie/bipolare Störungen (Dencker et al., Epilepsy Behav 2008, 12(1): 49-53), Dystonie-assoziierte Dyskinesien (Richter et al., Br J Pharmacol 2006, 149(6): 747-53).

Es besteht ein Bedarf an weiteren Verbindungen mit vergleichbaren oder besseren Eigenschaften, nicht nur im Hinblick auf die Affinität an KCNQ2/3 als solche (*potency, efficacy*).

So kann es vorteilhaft sein, die metabolische Stabilität, die Löslichkeit in wässrigen Medien oder die Permeabilität der Verbindungen zu verbessern. Diese Faktoren können sich günstig auf die orale Bioverfügbarkeit auswirken oder können das PK/PD (Pharmakokinetik/Pharmakodynamik)-Profil verändern, was z.B. zu einer günstigeren Wirkdauer führen kann.

Auch eine schwache oder nicht vorhandene Wechselwirkung mit Transportermolekülen, die an der Aufnahme und der Ausscheidung von Arzneistoffen beteiligt sind, ist als Hinweis auf eine verbesserte Bioverfügbarkeit und allenfalls geringe Arzneimittelwechselwirkungen zu werten. Ferner sollten auch die Wechselwirkungen mit den am Abbau und der Ausscheidung von Arzneistoffen beteiligten Enzymen möglichst gering sein, da solche Testergebnisse ebenfalls darauf hindeuten, dass allenfalls geringe oder überhaupt keine Arzneimittelwechselwirkungen zu erwarten sind.

Ferner kann es von Vorteil sein, wenn die Verbindungen eine hohe Selektivität gegenüber anderen Rezeptoren der KCNQ-Familie zeigen (Spezifität), beispielsweise gegenüber KCNQ1, KCNQ3/5 oder KCNQ4. Eine hohe Selektivität kann sich günstig auf das Nebenwirkungsprofil auswirken. So ist beispielsweise bekannt, dass Verbindungen, welche (auch) an KCNQ1 binden, ein hohes Risiko kardialer Nebenwirkungen mit sich bringen, weshalb eine hohe Selektivität gegenüber KCNQ1 wünschenswert sein kann. Eine hohe Selektivität kann jedoch auch gegenüber anderen Rezeptoren von Vorteil sein. Eine geringe Affinität zum hERG-Ionenkanal oder zum L-Typ Calcium-Ionenkanal (Phenylalkylamin-, Benzothiazepin-, Dihydropyridin-Bindungsstellen) kann vorteilhaft sein, da diese Rezeptoren in Zusammenhang mit dem Auftreten kardialer Nebenwirkungen gebracht werden. Insgesamt kann eine verbesserte Selektivität bezüglich der Bindung an andere endogene Proteine (d.h. z.B. Rezeptoren oder Enzyme) zu einer Verbesserung des Nebenwirkungsprofils, und dadurch zu einer verbesserten Verträglichkeit führen.

Eine Aufgabe der Erfindung bestand daher darin, neue Verbindungen zur Verfügung zu stellen, welche Vorteile gegenüber den Verbindungen des Standes der Technik aufweisen. Die Verbindungen sollten sich insbesondere als pharmakologische Wirkstoffe in Arzneimitteln eignen, vorzugsweise in Arzneimitteln zur Behandlung von Störungen oder Krankheiten, welche zumindest teilweise durch KCNQ2/3 $K^+$ Kanäle vermittelt werden.

Diese Aufgabe wird durch den Gegenstand der Patentansprüche gelöst.

Aus dem Stand der Technik sind substituierte Aryl- oder Heteroarylamide bekannt, die sich als Antagonisten des $EP_4$-Rezeptors eignen (WO 2005/105733). Ferner sind Verbindungen bekannt, welche sich als Inhibitoren des DPP-IV Enzyms (WO 2007/015767) sowie des 11-ß-HSD1-Enzyms (WO 2008/012532) eignen.

Es wurde überraschend gefunden, dass sich substituierte Nicotinamide der nachstehend angegebenen allgemeinen Formel (1) zur Behandlung von Schmerzen eignen. Es wurde ferner überraschend gefunden, dass substituierte Nicotinamide der nachstehend angegebenen allgemeinen Formel (1) auch eine ausgezeichnete Affinität zum KCNQ2/3 $K^+$ Kanal aufweisen und sich daher zur Behandlung von Störungen oder Krankheiten eignen, die zumindest teilweise durch KCNQ2/3 $K^+$ Kanäle vermittelt werden. Dabei wirken die substituierte Nicotinamide als Modulatoren, d.h. Agonisten oder Antagonisten, des KCNQ2/3 $K^+$ Kanals.

Ein Gegenstand der Erfindung sind substituierte Nicotinamide der allgemeinen Formel (1)

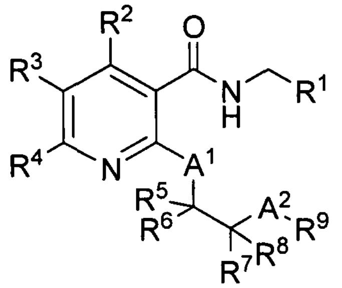

(1)

,

worin

$A^1$ für $CR^{10}R^{11}$ oder S steht;

$A^2$ für $CR^{12}R^{13}$, C(=O), O, S, S(=O) oder $S(=O)_2$ steht;

$R^1$ für $C_{1-10}$-Alkyl oder $C_{2-10}$-Heteroalkyl, jeweils gesättigt oder ungesättigt, verzweigt oder unverzweigt, unsubstituiert oder einfach oder mehrfach substituiert; $C_{3-10}$-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; über $C_{1-8}$-Alkyl oder $C_{2-8}$-Heteroalkyl verbrücktes $C_{3-10}$-Cycloalkyl oder Heterocyclyl, jeweils gesättigt oder ungesättigt, unsubstituiert oder einfach oder mehrfach substituiert, wobei die Alkyl- oder Heteroalkylkette jeweils verzweigt oder unverzweigt, gesättigt oder ungesättigt, unsubstituiert, einfach oder mehrfach substituiert sein kann; oder über $C_{1-8}$-Alkyl oder $C_{2-8}$-Heteroalkyl verbrücktes Aryl oder Heteroaryl, jeweils unsubstituiert oder einfach oder mehrfach substituiert, wobei die Alkyl- oder Heteroalkylkette jeweils verzweigt oder unverzweigt, gesättigt oder ungesättigt, unsubstituiert, einfach oder mehrfach substituiert sein kann; steht;

$R^2$, $R^3$ und $R^4$ jeweils unabhängig voneinander für H; F; Cl; Br; I; $NO_2$; $CF_3$; CN; OH; $OCF_3$; SH; $SCF_3$; Methyl; $CH_2$-O-Methyl; $CH_2$-OH; $C_{2-6}$-Alkyl, O-$C_{1-6}$-Alkyl, S-$C_{1-6}$-Alkyl, jeweils gesättigt oder ungesättigt, verzweigt oder unverzweigt, unsubstituiert oder einfach oder mehrfach substituiert; $C_{3-7}$-Cycloalkyl, gesättigt oder ungesättigt, verzweigt oder unverzweigt, unsubstituiert oder einfach oder mehrfach substituiert; $NR^aR^b$, wobei $R^a$ und $R^b$ jeweils unabhängig voneinander für H oder $C_{1-4}$-Alkyl, gesättigt oder ungesättigt, verzweigt oder unverzweigt, unsubstituiert oder einfach oder mehrfach substituiert, stehen oder $R^a$ und $R^b$ gemeinsam mit dem sie verbindenden Stickstoffatom ein Heterocyclyl, gesättigt oder ungesättigt, verzweigt oder unverzweigt, unsubstituiert oder einfach oder mehrfach substituiert; bilden.

$R^5$, $R^6$, $R^7$, $R^8$, $R^{10}$, $R^{11}$, $R^{12}$ und $R^{13}$ jeweils unabhängig voneinander für H; F; Cl; Br; I; $NO_2$; $CF_3$; CN; OH; $OCF_3$; SH; $SCF_3$; $C_{1-10}$-Alkyl, $C_{2-10}$-Heteroalkyl, O-$C_{1-10}$-Alkyl oder S-$C_{1-10}$-Alkyl, jeweils gesättigt oder ungesättigt, verzweigt oder unverzweigt, unsubstituiert oder einfach oder mehrfach substituiert; $C_{3-10}$-Cycloalkyl, gesättigt oder ungesättigt, verzweigt oder unverzweigt, unsubstituiert oder einfach oder mehrfach substituiert; stehen;
mit der Maßgabe, dass, wenn $R^5$, $R^6$, $R^7$ und $R^8$ jeweils H bedeuten und $A^1$ für S steht, $A^2$ nicht S, S(=O) oder $S(=O)_2$ bedeuten kann;
oder $R^5$ und $R^6$ oder $R^7$ und $R^8$ oder $R^{10}$ und $R^{11}$ oder $R^{12}$ und $R^{13}$ oder $R^5$ und $R^{11}$ oder $R^5$ und $R^{13}$ oder $R^7$ und $R^{13}$ oder $R^7$ und $R^{11}$ oder $R^{11}$ und $R^{13}$ gemeinsam mit dem oder den sie verbindenden Kohlenstoffatom(en) ein $C_{3-8}$-Cycloalkyl oder ein Heterocyclyl mit drei bis acht Ringgliedern bilden, jeweils gesättigt oder ungesättigt, unsubstituiert oder

einfach oder mehrfach substituiert; wobei die jeweiligen verbleibenden Substituenten $R^5$, $R^6$, $R^7$, $R^8$, $R^{10}$, $R^{11}$, $R^{12}$ und $R^{13}$ die vorstehende Bedeutung haben;

oder $R^5$ und $R^7$ gemeinsam mit dem oder den sie verbindenden Kohlenstoffatomen ein $C_{3-8}$-Cycloalkyl bilden, gesättigt oder ungesättigt, unsubstituiert oder einfach oder mehrfach substituiert; oder ein Heterocyclyl mit drei bis acht Ringgliedern bilden, ungesättigt, unsubstituiert oder einfach oder mehrfach substituiert; wobei die jeweiligen verbleibenden Substituenten $R^6$, $R^8$, $R^{10}$, $R^{11}$, $R^{12}$ und $R^{13}$ die vorstehende Bedeutung haben;

$R^9$ für $C_{3-10}$-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; oder für $CR^cR^d$ steht, wobei $R^c$ und $R^d$ jeweils unabhängig voneinander $C_{1-4}$-Alkyl, jeweils gesättigt oder ungesättigt, verzweigt oder unverzweigt, unsubstituiert oder einfach oder mehrfach substituiert; bedeuten;

mit der Maßgabe, dass, wenn $A^2$ für O oder S steht und $R^9$ Heterocyclyl, gesättigt oder ungesättigt, unsubstituiert oder einfach oder mehrfach substituiert; oder Heteroaryl, unsubstituiert oder einfach oder mehrfach substituiert; bedeutet, die Bindung des Heteroaryls oder Heterocyclyls über ein Kohlenstoffatom des Heteroaryls oder Heterocyclyls erfolgt;

worin "Alkyl substituiert", "Heteroalkyl substituiert", "Heterocyclyl substituiert" und "Cycloalkyl substituiert" für die Substitution eines oder mehrerer Wasserstoffatome jeweils unabhängig voneinander durch F; Cl; Br; I; $NO_2$; $CF_3$; CN; =O; $C_{1-8}$-Alkyl; $C_{2-8}$-Heteroalkyl; Aryl; Heteroaryl; $C_{3-10}$-Cycloalkyl; Heterocyclyl; über $C_{1-8}$-Alkyl oder $C_{2-8}$-Heteroalkyl verbrücktes Aryl, Heteroaryl, $C_{3-10}$-Cycloalkyl oder Heterocyclyl; CHO; $C(=O)C_{1-8}$-Alkyl; C(=O)Aryl; C(=O)Heteroaryl; $CO_2H$; $C(=O)O-C_{1-8}$-Alkyl; C(=O)O-Aryl; C(=O)O-Heteroaryl; $CONH_2$; $C(=O)NH-C_{1-8}$-Alkyl; $C(=O)N(C_{1-8}$-Alkyl$)_2$; C(=O)NH-Aryl; $C(=O)N(Aryl)_2$; C(=O)NH-Heteroaryl; $C(=O)N(Heteroaryl)_2$; $C(=O)N(C_{1-8}$-Alkyl)(Aryl); $C(=O)N(C_{1-8}$-Alkyl)(Heteroaryl); C(=O)N(Heteroaryl)(Aryl); OH; $O-C_{1-8}$-Alkyl; $OCF_3$; $O-(C_{1-8}$-Alkyl)-OH; $O-(C_{1-8}$-Alkyl)-$O-C_{1-8}$-Alkyl; O-Benzyl; O-Aryl; O-Heteroaryl; $O-C(=O)C_{1-8}$-Alkyl; O-C(=O)Aryl; O-C(=O)Heteroaryl; $NH_2$; $NH-C_{1-8}$-Alkyl; $N(C_{1-8}$-Alkyl$)_2$; $NH-C(=O)C_{1-8}$-Alkyl; $N(C_{1-8}$-Alkyl)-$C(=O)C_{1-8}$-Alkyl; $N(C(=O)C_{1-8}$-Alkyl$)_2$; NH-C(=O)-Aryl; NH-C(=O)-Heteroaryl; SH; $S-C_{1-8}$-Alkyl; $SCF_3$; S-Benzyl; S-Aryl; S-Heteroaryl; $S(=O)_2C_{1-8}$-Alkyl; $S(=O)_2$Aryl; $S(=O)_2$Heteroaryl; $S(=O)_2OH$; $S(=O)_2O-C_{1-8}$-Alkyl; $S(=O)_2O$-Aryl; $S(=O)_2O$-Heteroaryl; $S(=O)_2-NH-C_{1-8}$-Alkyl; $S(=O)_2$-NH-Aryl; und $S(=O)_2-NH-C_{1-8}$-Heteroaryl; steht;

worin "Aryl substituiert" und "Heteroaryl substituiert" für die Substitution eines oder mehrerer Wasserstoffatome jeweils unabhängig voneinander durch F; Cl; Br; I; $NO_2$; $CF_3$; CN; $C_{1-8}$-Alkyl; $C_{2-8}$-Heteroalkyl; Aryl; Heteroaryl; $C_{3-10}$-Cycloalkyl; Heterocyclyl; $C_{1-8}$-Alkyl oder $C_{2-8}$-Heteroalkyl verbrücktes Aryl, Heteroaryl, $C_{3-10}$-Cycloalkyl oder Heterocyclyl; CHO; $C(=O)C_{1-8}$-Alkyl; C(=O)Aryl; C(=O)Heteroaryl; $CO_2H$; $C(=O)O-C_{1-8}$-Alkyl; C(=O)O-Aryl; C(=O)O-Heteroaryl; $CONH_2$; $C(=O)NH-C_{1-8}$-Alkyl; $C(=O)N(C_{1-8}$-Alkyl$)_2$; C(=O)NH-Aryl; $C(=O)N(Aryl)_2$; C(=O)NH-Heteroaryl; $C(=O)N(Heteroaryl)_2$; $C(=O)N(C_{1-8}$-Alkyl)(Aryl); $C(=O)N(C_{1-8}$-Alkyl)(Heteroaryl); C(=O)N(Heteroaryl)(Aryl); OH; $O-C_{1-8}$-Alkyl; $OCF_3$; $O-(C_{1-8}$-Alkyl)-OH; $O-(C_{1-8}$-Alkyl)-$O-C_{1-8}$-Alkyl; O-Benzyl; O-Aryl; O-Heteroaryl; $O-C(=O)C_{1-8}$-Alkyl; O-C(=O)Aryl; O-C(=O)Heteroaryl; $NH_2$ ; $NH-C_{1-8}$-Alkyl; $N(C_{1-8}$-Alkyl$)_2$; $NH-C(=O)C_{1-8}$-Alkyl; $N(C_{1-8}$-Alkyl)-$C(=O)C_{1-8}$-Alkyl; $N(C(=O)C_{1-8}$-Alkyl$)_2$; NH-C(=O)-Aryl; NH-C(=O)-Heteroaryl; SH; $S-C_{1-8}$-Alkyl; $SCF_3$; S-Benzyl; S-Aryl; S-Heteroaryl; $S(=O)_2C_{1-8}$-Alkyl; $S(=O)_2$Aryl; $S(=O)_2$Heteroaryl; $S(=O)_2OH$; $S(=O)_2O-C_{1-8}$-Alkyl; $S(=O)_2O$-Aryl; $S(=O)_2O$-Heteroaryl; $S(=O)_2-NH-C_{1-8}$-Alkyl; $S(=O)_2$-NH-Aryl; $S(=O)_2-NH-C_{1-8}$-Heteroaryl; steht;

in Form der freien Verbindungen oder Salze physiologisch verträglicher Säuren oder Basen.

Die Ausdrücke "Alkyl" bzw. "$C_{1-10}$-Alkyl", "$C_{1-8}$-Alkyl", "$C_{1-6}$-Alkyl", "$C_{1-4}$-Alkyl", "$C_{1-2}$-Alkyl" und "$C_{2-6}$-Alkyl" umfassen im Sinne dieser Erfindung acyclische gesättigte oder ungesättigte aliphatische Kohlenwasserstoffreste, die verzweigt oder unverzweigt sowie unsubstituiert oder ein- oder mehrfach substituiert sein können, mit 1 bis 10 bzw. 1 bis 8 bzw. 1 bis 6 bzw. 1 bis 4 bzw. 1 bis 2 bzw. 2 bis 6 C-Atomen, d.h. $C_{1-10}$-Alkanyle, $C_{2-10}$-Alkenyle und $C_{2-10}$-Alkinyle bzw. $C_{1-8}$-Alkanyle, $C_{2-8}$-Alkenyle und $C_{2-8}$-Alkinyle bzw. $C_{1-6}$-Alkanyle, $C_{2-6}$-Alkenyle und $C_{2-6}$-Alkinyle bzw. $C_{1-4}$-Alkanyle, $C_{2-4}$-Alkenyle und $C_{2-4}$-Alkinyle bzw. $C_{1-2}$-Alkanyle, $C_2$-Alkenyle und $C_2$-Alkinyle bzw. $C_{2-6}$-Alkanyle, $C_{2-6}$-Alkenyle und $C_{2-6}$-Alkinyle. Dabei weisen Alkenyle mindestens eine C-C-Doppelbindung und Alkinyle mindestens eine C-C-Dreifachbindung auf. Bevorzugt ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, iso-Pentyl, neo-Pentyl, n-Hexyl, n-Heptyl, n-Octyl, n-Nonyl, n-Decyl, Ethenyl (Vinyl), Ethinyl, Propenyl ($-CH_2CH=CH_2$, $-CH=CH-CH_3$, $-C(=CH_2)-CH_3$), Propinyl ($-CH-C\equiv CH$, $-C\equiv C-CH_3$), Butenyl, Butinyl, Pentenyl, Pentinyl, Hexenyl und Hexinyl, Heptenyl, Heptinyl, Octenyl, Octinyl, Nonenyl, Noninyl, Decenyl und Decinyl umfasst.

Die Ausdrücke "Heteroalkyl" bzw. "$C_{2-10}$-Heteroalkyl" und "$C_{2-8}$-Heteroalkyl" umfassen im Sinne dieser Erfindung acyclische aliphatische gesättigte oder ungesättigte Kohlenwasserstoffreste mit 2 bis 10 C-Atomen, d.h. $C_{2-10}$-Heteroalkanyle, $C_{2-10}$-Heteroalkenyle und $C_{2-10}$-Heteroalkinyle bzw. mit 2 bis 8 C-Atomen, d.h. $C_{2-8}$-Heteroalkanyle, $C_{2-8}$-Heteroalkenyle und $C_{2-8}$-Heteroalkinyle, die jeweils verzweigt oder unverzweigt sowie unsubstituiert oder ein- oder mehrfach substituiert sein können und in denen mindestens ein, gegebenenfalls auch zwei oder drei Kohlenstoffatome durch ein Heteroatom oder eine Heteroatomgruppe jeweils unabhängig voneinander ausgewählt aus der Gruppe bestehend aus O, N, NH, und N(C-$_{1-8}$-Alkyl), vorzugsweise $N(CH_3)$ ersetzt sind, wobei das anfangsständige Kohlenstoffatom eines $C_{2-10}$-Heteroalkyls oder eines $C_{2-8}$-Heteroalkyls, über welches das $C_{2-10}$-Heteroalkyl oder das $C_{2-8}$-Heteroalkyl an die jeweilige übergeordnete allgemeine Struktur gebunden wird, nicht durch ein Heteroatom oder eine Heteroatomgruppe ersetzt werden kann und einander benachbarte Kohlenstoffatome nicht gleichzeitig durch ein Heteroatom oder eine Heteroatomgruppe ersetzt werden können. Gegebenenfalls können die Heteroatomgruppen NH und $N(C_{1-8}$-Alkyl) des Heteroalkyls einfach oder mehrfach substituiert sein. $C_{2-10}$-Heteroalkenyle und $C_{2-8}$-Heteroalkenyle weisen mindestens eine C-C- oder eine C-N-Doppelbindung und $C_{2-10}$-Heteroalkinyle und $C_{2-8}$-Heteroalkinyle mindestens eine C-C-Dreifachbindung auf. Bevorzugt ist Heteroalkyl aus der Gruppe ausgewählt, die $-CH_2-O-CH_3$, $-CH_2-CH_2-O-CH_3$, $-CH_2-CH_2-O-CH_2-CH_3$, $-CH_2-CH_2-O-CH_2-CH_2-O-CH_3$, $-CH=CH-O-CH_3$, $-CH=CH-O-CH_2-CH_3$, $=CH-O-CH_3$, $=CH-O-CH_2-CH_3$, $=CH-CH_2-O-CH_2-CH_3$, $=CH-CH_2-O-CH_3$, $-CH_2-NH-CH_3$, $-CH_2-CH_2-NH-CH_3$, $-CH_2-CH_2-NH-CH_2-CH_3$, $-CH_2-CH_2-NH-CH_2-CH_2-NH-CH_3$, $-CH=CH-NH-CH_3$, $-CH=CH-NH-CH_2-CH_3$, $-CH=CH-N(CH_3)-CH_2-CH_3$, $=CH-NH-CH_3$, $=CH-NH-CH_2-CH_3$, $=CH-CH_2-NH-CH_2-CH_3$, $=CH-CH_2-NH-CH_3$, $-CH_2-N(CH_3)-CH_3$, $-CH_2-CH_2-N(CH_3)-CH_3$, $-CH_2-CH_2-N(CH_3)-CH_2-CH_3$, $-CH_2-CH_2-N(CH_3)-CH_2-CH_2-N(CH_3)-CH_3$, $CH_2-CH_2-NH-CH_2-CH_2-O-CH_3$, $CH_2-CH_2-O-CH_2-CH_2-NH-CH_3$, $CH_2-CH_2-N(CH_3)-CH_2-CH_2-O-CH_3$, $CH_2-CH_2-O-CH_2-CH_2-N(CH_3)-CH_3$, $CH_2-NH-CH_2-O-CH_3$, $CH_2$ $-O-CH_2-NH-CH_3$, $CH_2-N(CH_3)-CH_2-O-CH_3$, $CH_2-O-CH_2-N(CH_3)-CH_3$, $-CH=CH-N(CH_3)-CH_3$, $=CH-N(CH_3)-CH_3$, $=CH-N(CH_3)-CH_2-CH_3$, $=CH-CH_2-N(CH_3)-CH_2-CH_3$, $=CH-CH_2-N(CH_3)-CH_3$,$-CH_2-CH_2=N(CH_3)$ und $-CH_2=N(CH_3)$ umfasst.

Die Ausdrücke "Cycloalkyl" bzw. "$C_{3-10}$-Cycloalkyl", "$C_{3-7}$-Cycloalkyl" und "$C_{3-8}$-Cycloalkyl" bedeuten für die Zwecke dieser Erfindung cyclische aliphatische Kohlenwasserstoffe mit 3, 4, 5, 6, 7, 8, 9 oder 10 Kohlenstoffatomen bzw. mit 3, 4, 5, 6 oder 7 Kohlenstoffatomen bzw. mit 3, 4, 5, 6, 7 oder 8 Kohlenstoffatomen, wobei die Kohlenwasserstoffe gesättigt oder ungesättigt (aber nicht aromatisch), unsubstituiert oder ein- oder mehrfach substituiert sein können. Die Bindung des Cycloalkyls an die jeweilige übergeordnete allgemeine Struktur kann über jedes beliebige und mögliche Ringglied des Cycloalkyl-Restes erfolgen. Die Cycloalkyl-Reste können auch mit weiteren gesättigten, (partiell) ungesättigten, (hetero)cyclischen, aromatischen oder heteroaromatischen Ringsystemen , d.h. mit Cycloalkyl, Heterocyclyl, Aryl oder Heteroaryl kondensiert sein, die wiederum unsubstituiert oder einfach oder mehrfach substituiert sein können. Die Cycloalkyl-Reste können weiterhin einfach oder mehrfach verbrückt sein wie bspw. im Fall von Adamantyl, Bicyclo[2.2.1]heptyl oder Bicyclo[2.2.2]octyl. Bevorzugt ist Cycloalkyl aus der Gruppe ausgewählt, die Cyclopropyl, Cyclobutyl, Cyclopentyl, Cyclohexyl, Cycloheptyl, Cyclooctyl, Cyclononyl, Cyclodecyl, Adamantyl,

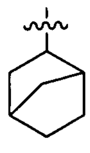 , 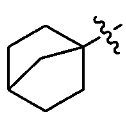 , 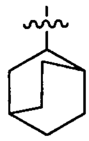 , 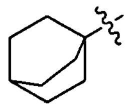 , 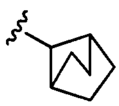 , 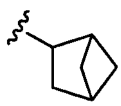 , 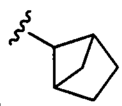 ,

Cyclopentenyl, Cyclohexenyl, Cycloheptenyl und Cyclooctenyl umfasst.

Der Begriff "Heterocyclyl" oder "Heterocycloalkyl" umfasst aliphatische gesättigte oder ungesättigte (aber nicht aromatische) Cycloalkyle mit drei bis zehn, d.h. 3, 4, 5, 6, 7, 8, 9 oder 10 Ringgliedern, in denen mindestens ein, ggf. auch zwei oder drei Kohlenstoffatome durch ein Heteroatom oder eine Heteroatomgruppe jeweils unabhängig voneinander ausgewählt aus der Gruppe bestehend aus O, S, N, NH und $N(C_{1-8}$-Alkyl), vorzugsweise $N(CH_3)$ ersetzt sind, wobei die Ringglieder unsubstituiert oder ein- oder mehrfach substituiert sein können. Die Bindung des Heterocyclyls an die übergeordnete allgemeine Struktur kann über jedes beliebige und mögliche Ringglied des Heterocyclyl-Restes erfolgen. Die Heterocyclyl-Reste können auch mit weiteren gesättigten, (partiell) ungesättigten (hetero)cyclischen oder aromatischen oder heteroaromatischen Ringsystemen, d.h. mit Cycloalkyl, Heterocyclyl, Aryl oder Heteroaryl kondensiert sein, die wiederum unsubstituiert oder einfach oder mehrfach substituiert sein können. Bevorzugt sind Heterocyclyl-Reste aus der Gruppe umfassend Azetidinyl, Aziridinyl, Azepanyl, Azocanyl, Diazepanyl, Dithiolanyl, Dihydrochinolinyl, Dihydropyrrolyl, Dioxanyl, Dioxolanyl, Dihydroindenyl Dihydropyridinyl, Dihydrofuranyl, Dihydroisochinolinyl, Dihydroindolinyl, Dihydroisoindolyl, Imidazolidinyl, Isoxazolidinyl, Morpholinyl, Oxiranyl, Oxetanyl, Pyrrolidinyl, Piperazinyl, Piperidinyl, Pyrazolidinyl, Pyranyl, Tetrahydropyrrolyl, Tetrahydropyranyl, Tetrahydrochinolinyl, Tetrahydroisochinolinyl, Tetrahydroindolinyl, Tetrahydrofuranyl, Tetrahydropyridinyl, Tetrahydrothiophenyl, Tetrahydropyridoindolyl, Tetrahydronaphthyl, Tetrahydrocarbolinyl, Tetrahydroisoxazolopyridinyl, Thiazolidinyl und Thiomorpholinyl.

Der Begriff "Aryl" bedeutet im Sinne dieser Erfindung aromatische Kohlenwasserstoffe mit bis zu 14 Ringgliedern, u.a. Phenyle und Naphthyle. Jeder Aryl-Rest kann unsubstituiert oder einfach oder mehrfach substituiert vorliegen, wobei die ArylSubstituenten gleich oder verschieden und in jeder beliebigen und möglichen Position des Aryls sein können. Die Bindung des Aryls an die übergeordnete allgemeine Struktur kann über jedes beliebige und mögliche Ringglied des ArylRestes erfolgen. Die Aryl-Reste können auch mit weiteren gesättigten, (partiell) ungesättigten, (hetero)cyclischen, aromatischen oder heteroaromatischen Ringsystemen kondensiert sein, d.h. mit Cycloalkyl, Heterocyclyl, Aryl oder Heteroaryl, die wiederum unsubstituiert oder einfach oder mehrfach substituiert sein können. Beispiele für kondensierte Aryl-Reste sind Benzodioxolanyl und Benzodioxanyl. Bevorzugt ist Aryl aus der Gruppe ausgewählt, die Phenyl, 1-Naphthyl und 2-Naphthyl enthält, welche jeweils unsubstituiert oder ein- oder mehrfach substituiert sein können. Ein besonders bevorzugtes Aryl ist Phenyl, unsubstituiert oder einfach oder mehrfach substituiert.

Der Begriff "Heteroaryl" steht für einen 5- oder 6-gliedrigen cyclischen aromatischen Rest, der mindestens 1, ggf. auch 2, 3, 4 oder 5 Heteroatome, enthält, wobei die Heteroatome jeweils unabhängig voneinander ausgewählt sind aus der Gruppe S, N und O und der Heteroaryl-Rest unsubstituiert oder ein- oder mehrfach substituiert sein kann; im Falle der Substitution am Heteroaryl können die Substituenten gleich oder verschieden sein und in jeder beliebigen und möglichen Position des Heteroaryls sein. Die Bindung an die übergeordnete allgemeine Struktur kann über jedes beliebige und mögliche Ringglied des Heteroaryl-Restes erfolgen. Das Heteroaryl kann auch Teil eines bi- oder polycyclischen Systems mit bis zu 14 Ringgliedern sein, wobei das Ringsystem mit weiteren gesättigten, (partiell) ungesättigten, (hetero)cyclischen oder aromatischen oder heteroaromatischen Ringen gebildet werden kann, d.h. mit Cycloalkyl, Heterocyclyl, Aryl oder Heteroaryl, die wiederum unsubstituiert oder einfach oder mehrfach substituiert sein können. Es ist bevorzugt, dass der Heteroaryl-Rest ausgewählt ist aus der Gruppe, die Benzofuranyl, Benzoimidazolyl, Benzothienyl, Benzothiadiazolyl, Benzothiazolyl, Benzotriazolyl, Benzooxazolyl, Benzooxadiazolyl, Chinazolinyl, Chinoxalinyl, Carbazolyl, Chinolinyl, Dibenzofuranyl, Dibenzothienyl, Furyl (Furanyl), Imidazolyl, Imidazothiazolyl, Indazolyl, Indolizinyl, Indolyl, Isochinolinyl, Isoxazoyl, Isothiazolyl, Indolyl, Naphthyridinyl, Oxazolyl, Oxadiazolyl, Phenazinyl, Phenothiazinyl, Phtalazinyl, Pyrazolyl, Pyridyl (2-Pyridyl, 3-Pyridyl, 4-Pyridyl), Pyrrolyl, Pyridazinyl, Pyrimidinyl, Pyrazinyl, Purinyl, Phenazinyl, Thienyl (Thiophenyl), Triazolyl, Tetrazolyl, Thiazolyl, Thiadiazolyl oder Triazinyl umfasst. Besonders bevorzugt sind Furyl, Pyridyl und Thienyl.

Die Ausdrücke "über $C_{1-4}$-Alkyl oder $C_{1-8}$-Alkyl verbrücktes Aryl, Heteroaryl, Heterocyclyl oder Cycloalkyl" bedeuten im Sinne der Erfindung, dass $C_{1-4}$-Alkyl oder $C_{1-8}$-Alkyl und Aryl bzw. Heteroaryl bzw. Heterocyclyl bzw. Cycloalkyl die oben definierten Bedeutungen haben und der Aryl- bzw. Heteroaryl- bzw. Heterocyclyl-bzw. Cycloalkyl-Rest über eine $C_{1-4}$-Alkyl- oder eine $C_{1-8}$-Alkyl-Gruppe an die jeweilige übergeordnete allgemeine Struktur gebunden ist. Die Alkylkette kann in allen Fällen gesättigt oder ungesättigt, verzweigt oder unverzweigt, unsubstituiert oder einfach oder mehrfach substituiert sein. Vorzugsweise sind $C_{1-4}$-Alkyl oder $C_{1-8}$-Alkyl ausgewählt aus der Gruppe umfassend $-CH_2-$, $-CH_2-CH_2-$, $-CH(CH_3)-$, $-CH_2-CH_2-CH_2-$, $-CH(CH_3)-CH_2-$, $-CH(CH_2CH_3)-$, $-CH_2-(CH_2)_2-CH_2-$, $-CH(CH_3)-CH_2-CH_2-$, $-CH_2-CH(CH_3)-CH_2-$, $-CH(CH_3)-CH(CH_3)-$, $-CH(CH_2CH_3)-CH_2-$, $-C(CH_3)_2-CH_2-$, $-CH(CH_2CH_2CH_3)-$, $-C(CH_3)(CH_2CH_3)-$, $-CH_2-(CH_2)_3-CH_2-$, $-CH(CH_3)-CH_2-CH_2-CH_2-$, $-CH_2-CH(CH_3)-CH_2-CH_2-$, $-CH(CH_3)-CH_2-CH(CH_3)-$, $-CH(CH_3)-CH(CH_3)-CH_2-$, $-C(CH_3)_2-CH_2-CH_2-$, $-CH_2-C(CH_3)_2-CH_2-$, $-CH(CH_2CH_3)-CH_2-CH_2-$, $-CH_2-CH(CH_2CH_3)-CH_2-$, $-C(CH_3)_2-CH(CH_3)-$, $-CH(CH_2CH_3)-CH(CH_3)-$, $-C(CH_3)(CH_2CH_3)-CH_2-$, $-CH(CH_2CH_2CH_3)-CH_2-$, $-C(CH_2CH_2CH_3)-CH_2-$, $-CH(CH_2CH_2CH_2CH_3)-$, $-C(CH_3)(CH_2CH_2CH_3)-$, $-C(CH_2CH_3)_2-$, $-CH_2-(CH_2)_4-CH_2-$, $-CH=CH-$, $-CH=CH-CH_2-$, $-C(CH_3)=CH_2-$, $-CH=CH-CH_2-CH_2-$, $-CH_2-CH=CH-CH_2-$, $-CH=CH-CH=CH-$, $-C(CH_3)=CH-CH_2-$, $-CH=C(CH_3)-CH_2-$, $-C(CH_3)=C(CH_3)-$, $-C(CH_2CH_3)=CH-$, $-CH=CH-CH_2-CH_2-CH_2-$, $-CH_2-CH=CH_2-CH_2-CH_2-$, $-CH=CH=CH-CH_2-CH_2-$,$-$ $CH=CH_2-CH-CH=CH_2-$, $-C\equiv C-$, $-C\equiv C-CH_2-$, $-C\equiv C-CH_2-CH_2-$, $-C\equiv C-CH(CH_3)-$, $-CH_2-C\equiv C-CH_2-$, $-C=C-C=C-$, $-C\equiv C-C(CH_3)_2-$, $-C\equiv C-CH_2-CH_2-CH_2-$, $-CH_2-C\equiv C-CH_2-CH_2-$, $-C\equiv C-C\equiv C-CH_2-$ und $-C\equiv C-CH_2-C\equiv C-$.

Die Ausdrücke "über $C_{2-8}$-Heteroalkyl verbrücktes Aryl, Heteroaryl, Heterocyclyl oder Cycloalkyl" bedeuten im Sinne der Erfindung, dass $C_{2-8}$-Heteroalkyl und Aryl bzw. Heteroaryl bzw. Heterocyclyl bzw. Cycloalkyl die oben definierten Bedeutungen haben und der Aryl- bzw. Heteroaryl- bzw. Heterocyclyl- bzw. Cycloalkyl-Rest über eine $C_{2-8}$-Heteroalkyl-Gruppe an die jeweilige übergeordnete allgemeine Struktur gebunden ist. Die Heteroalkylkette kann in allen Fällen gesättigt oder ungesättigt, verzweigt oder unverzweigt, unsubstituiert oder einfach oder mehrfach substituiert sein. Ist ein endständiges Kohlenstoffatom der $C_{2-8}$-Heteroalkyl-Gruppe durch ein Heteroatom oder eine Heteroatomgruppe ersetzt, so erfolgt die Bindung eines Heteroaryls oder eines Heterocyclyls an das Heteroatom oder die Heteroatomgruppe des $C_{2-8}$-Heteroalkyls stets über ein Kohlenstoffatom des Heteroaryls oder Heterocyclyls. Unter dem endständigen Kohlenstoffatom wird das Kohlenstoffatom innerhalb des $C_{2-8}$-Heteroalkyls verstanden, welches innerhalb der Kette am weitesten von der jeweiligen allgemeinen übergeordneten Struktur entfernt ist. Ist das endständige Kohlenstoffatom eines $C_{2-8}$-Heteroalkyls bspw. durch eine $N(CH_3)$-Gruppe ersetzt, ist diese innerhalb des $C_{2-8}$-Heteroalkyls am weitesten von der allgemeinen übergeordneten Struktur entfernt und an den Aryl- bzw. Heteroaryl- bzw. Heterocyclyl- bzw. Cycloalkyl-Rest gebunden. Vorzugsweise ist $C_{2-8}$-Heteroalkyl ausgewählt aus der Gruppe umfassend $-CH_2-NH-$, $-CH_2-N(CH_3)-$, $-CH_2-O-$, $-CH_2-CH_2-NH-$, $-CH_2-CH_2-N(CH_3)-$, $-CH_2-CH_2-O-$, $-CH_2-CH_2-CH_2-NH-$, $-CH_2-CH_2-CH_2-N(CH_3)-$, $-CH_Z-CH_2-CH_2-O-$, $-CH_2-O-CH_2-$, $-CH_2-CH_2-O-CH_2-$, $-CH_2-CH_2-O-CH_2-CH_2-$, $-CH_2-CH_2-O-CH_2-CH_2-O-CH_2-$, $-CH=CH-O-CH_2-$, $-CH=CH-O-CH_2-CH_2-$, $=CH-O-CH_2-$, $=CH-O-CH_2-CH_2-$, $=CH-CH_2-O-CH_2-CH_2-$, $=CH-CH_2-O-CH_2-$, $-CH_2-NH-CH_2-$, $-CH_2-CH_2-NH-CH_2-$, $-CH_2-CH_2-NH-CH_2-CH_2-$, $-CH_2-CH_2-NH-CH_2-CH_2-NH-CH_2$, $-CH=CH-NH-CH_2-$, $-CH=CH-NH-CH_2-CH_2-$, $-CH=CH-N(CH_3)-CH_2-CH_2-$, $=CH-NH-CH_2-$, $=CH-NH-CH_2-CH_2-$, $=CH-CH_2-NH-CH_2-CH_2-$, $=CH-CH_2-NH-CH_2-$, $-CH_2-N(CH_3)-CH_2-$,$-CH_2-CH_2-N(CH_3)-CH_2-$, $-CH_2-CH_2-N(CH_3)-CH_2-CH_2-$, $-CH_2-CH_2-N(CH_3)-CH_2-CH_2-N(CH_3)-CH_2-$, $CH_2-CH_2-NH-CH_2-CH_2-O-CH_2-$, $CH_2-CH_2-O-CH_2-CH_2-NH-CH_2-$, $CH_2-CH_2-N(CH_3)-CH_2-CH_2-O-CH_2-$, $CH_2-CH_2-O-CH_2-CH_2-N(CH_3)-CH_2-$, $CH_2-NH-CH_2-O-CH_2-$, $CH_2$ $-O-CH_2-NH-CH_2-$, $CH_2-N(CH_3)-CH_2-O-CH_2-$, $CH_2-O-CH_2-N(CH_3)-CH_2-$,$-CH=CH-N(CH_3)-CH_2-$, $=CH-N(CH_3)-CH_2-$, $=CH-N(CH_3)-CH_2-CH_2-$, $=CH-CH_2-N(CH_3)-CH_2-CH_2-$ und $=CH-CH_2-N(CH_3)-CH_2-$.

Im Zusammenhang mit "Alkyl", "Heteroalkyl", "Heterocyclyl" und "Cycloalkyl" versteht man unter dem Begriff "ein- oder mehrfach substituiert" im Sinne dieser Erfindung die ein- oder mehrfache, z.B. zwei-, drei- oder vierfache

Substitution eines oder mehrerer Wasserstoffatome jeweils unabhängig voneinander durch Substituenten ausgewählt aus der Gruppe aus F; Cl; Br; I; $NO_2$; $CF_3$; CN; =O; $C_{1-8}$-Alkyl; $C_{2-8}$-Heteroalkyl; Aryl; Heteroaryl; $C_{3-10}$-Cycloalkyl; Heterocyclyl; über $C_{1-8}$-Alkyl oder $C_{2-8}$-Heteroalkyl verbrücktes Aryl, Heteroaryl, $C_{3-10}$-Cycloalkyl oder Heterocyclyl; CHO; C(=O)$C_{1-8}$-Alkyl; C(=O)Aryl; C(=O)Heteroaryl; $CO_2H$; C(=O)O-$C_{1-8}$-Alkyl; C(=O)O-Aryl; C(=O)O-Heteroaryl; $CONH_2$; C(=O)NH-$C_{1-8}$-Alkyl; C(=O)N($C_{1-8}$-Alkyl)$_2$; C(=O)NH-Aryl; C(=O)N(Aryl)$_2$; C(=O)NH-Heteroaryl; C(=O)N(Heteroaryl)$_2$; C(=O)N($C_{1-8}$-Alkyl)(Aryl); C(=O)N($C_{1-8}$-Alkyl)(Heteroaryl); C(=O)N(Heteroaryl)(Aryl); OH; O-$C_{1-8}$-Alkyl; $OCF_3$; O-($C_{1-8}$-Alkyl)-OH; O-($C_{1-8}$-Alkyl)-O-$C_{1-8}$-Alkyl; O-Benzyl; O-Aryl; O-Heteroaryl; O-C(=O)$C_{1-8}$-Alkyl; O-C(=O)Aryl; O-C(=O)Heteroaryl; $NH_2$; NH-$C_{1-8}$-Alkyl; N($C_{1-8}$-Alkyl)$_2$; NH-C(=O)$C_{1-8}$-Alkyl; N($C_{1-8}$-Alkyl)-C(=O)$C_{1-8}$-Alkyl; N(C(=O)$C_{1-8}$-Alkyl)$_2$; NH-C(=O)-Aryl; NH-C(=O)-Heteroaryl; SH; S-$C_{1-8}$-Alkyl; $SCF_3$; S-Benzyl; S-Aryl; S-Heteroaryl; S(=O)$_2$$C_{1-8}$-Alkyl; S(=O)$_2$Aryl; S(=O)$_2$Heteroaryl; S(=O)$_2$OH; S(=O)$_2$O-$C_{1-8}$-Alkyl; S(=O)$_2$O-Aryl; S(=O)$_2$O-Heteroaryl; S(=O)$_2$-NH-$C_{1-8}$-Alkyl; S(=O)$_2$-NH-Aryl; und S(=O)$_2$-NH-$C_{1-8}$-Heteroaryl; wobei unter mehrfach substituierten Resten solche Reste zu verstehen sind, die entweder an verschiedenen oder an gleichen Atomen mehrfach, z.B. zwei-, drei- oder vierfach substituiert sind, beispielsweise dreifach am gleichen C-Atom wie im Falle von $CF_3$ oder $CH_2CF_3$ oder an verschiedenen Stellen wie im Falle von CH(OH)-CH=CH-$CHCl_2$. Ein Substituent kann gegebenenfalls seinerseits wiederum einfach oder mehrfach substituiert sein. Die Mehrfachsubstitution kann mit dem gleichen oder mit verschiedenen Substituenten erfolgen.

Bevorzugte "Alkyl-", "Heteroalkyl-", "Heterocyclyl-" und "Cycloalkyl-" Substituenten sind ausgewählt aus der Gruppe aus F; Cl; Br; I; $NO_2$; $CH_2CF_3$; $CF_3$; CN; $C_{1-8}$-Alkyl; $C_{2-8}$-Heteroalkyl; Phenyl; Naphthyl; Pyridyl; Thienyl; Furyl; $C_{3-10}$-Cycloalkyl; Heterocyclyl; $C_{1-8}$-Alkyl oder $C_{2-8}$-Heteroalkyl verbrücktes Phenyl, Naphthyl, Pyridyl, Thienyl, Furyl, $C_{3-10}$-Cycloalkyl oder Heterocyclyl; CHO; C(=O)$C_{1-8}$-Alkyl; $CO_2H$; C(=O)O-$C_{1-8}$-Alkyl; $CONH_2$; C(=O)NH-$C_{1-8}$-Alkyl; C(=O)N($C_{1-8}$-Alkyl)$_2$; OH; =O; O-$C_{1-8}$-Alkyl; $OCF_3$; O-($C_{1-8}$-Alkyl)-OH; O-($C_{1-8}$-Alkyl)-O-$C_{1-8}$-Alkyl; O-Benzyl; O-Phenyl; O-Heteroaryl; O-C(=O)$C_{1-8}$-Alkyl; $NH_2$ ; NH-$C_{1-8}$-Alkyl; N($C_{1-8}$-Alkyl)$_2$; NH-C(=O)$C_{1-8}$-Alkyl; N($C_{1-8}$-Alkyl)-C(=O)$C_{1-8}$-Alkyl; N(C(=O)$C_{1-8}$-Alkyl)$_2$; SH; S-$C_{1-8}$-Alkyl; $SCF_3$; S-Benzyl; S-Phenyl; S-Heteroaryl; S(=O)$_2$$C_{1-8}$-Alkyl;; S(=O)$_2$OH; S(=O)$_2$O-$C_{1-8}$-Alkyl; S(=O)$_2$-NH-$C_{1-8}$-Alkyl.

Im Zusammenhang mit "Aryl" und "Heteroaryl" versteht man im Sinne dieser Erfindung unter "ein- oder mehrfach substituiert" die ein- oder mehrfache, z.B. zwei-, drei- oder vierfache Substitution eines oder mehrerer Wasserstoffatome des Ringsystems jeweils unabhängig voneinander durch Substituenten ausgewählt aus der Gruppe aus F; Cl; Br; I; $NO_2$; $CF_3$; CN; $C_{1-8}$-Alkyl; $C_{2-8}$-Heteroalkyl; Aryl; Heteroaryl; $C_{3-10}$-Cycloalkyl; Heterocyclyl; $C_{1-8}$-Alkyl oder $C_{2-8}$-Heteroalkyl verbrücktes Aryl, Heteroaryl, $C_{3-10}$-Cycloalkyl oder Heterocyclyl; CHO; C(=O)$C_{1-8}$-Alkyl; C(=O)Aryl; C(=O)Heteroaryl; $CO_2H$; C(=O)O-$C_{1-8}$-Alkyl; C(=O)O-Aryl; C(=O)O-Heteroaryl; $CONH_2$; C(=O)NH-$C_{1-8}$-Alkyl; C(=O)N($C_{1-8}$-Alkyl)$_2$; C(=O)NH-Aryl; C(=O)N(Aryl)$_2$; C(=O)NH-Heteroaryl; C(=O)N(Heteroaryl)$_2$; C(=O)N($C_{1-8}$-Alkyl)(Aryl); C(=O)N($C_{1-8}$-Alkyl)(Heteroaryl); C(=O)N(Heteroaryl)(Aryl); OH; O-$C_{1-8}$-Alkyl; $OCF_3$; O-($C_{1-8}$-Alkyl)-OH; O-($C_{1-8}$-Alkyl)-O-$C_{1-8}$-Alkyl; O-Benzyl; O-Aryl; O-Heteroaryl; O-C(=O)$C_{1-8}$-Alkyl; O-C(=O)Aryl; O-C(=O)Heteroaryl; $NH_2$; NH-$C_{1-8}$-Alkyl; N($C_{1-8}$-Alkyl)$_2$; NH-C(=O)$C_{1-8}$-Alkyl; N($C_{1-8}$-Alkyl)-C(=O)$C_{1-8}$-Alkyl; N(C(=O)$C_{1-8}$-Alkyl)$_2$; NH-C(=O)-Aryl; NH-C(=O)-Heteroaryl; SH; S-$C_{1-8}$-Alkyl; $SCF_3$; S-Benzyl; S-Aryl; S-Heteroaryl; S(=O)$_2$$C_{1-8}$-Alkyl; S(=O)$_2$Aryl; S(=O)$_2$Heteroaryl; S(=O)$_2$OH; S(=O)$_2$O-$C_{1-8}$-Alkyl; S(=O)$_2$O-Aryl; S(=O)$_2$O-Heteroaryl; S(=O)$_2$-NH-$C_{1-8}$-Alkyl; S(=O)$_2$-NH-Aryl; S(=O)$_2$-NH-$C_{1-8}$-Heteroaryl; an einem oder ggf. verschiedenen Atomen, wobei ein Substituent ggf. seinerseits wiederum einfach oder mehrfach substituiert sein kann. Die Mehrfachsubstitution erfolgt mit dem gleichen oder mit unterschiedlichen Substituenten.

Bevorzugte "Aryl-" und "Heteroaryl-" Substituenten sind F; Cl; Br; I; $NO_2$; $CH_2CF_3$; $CF_3$; CN; $C_{1-8}$-Alkyl; $C_{2-8}$-Heteroalkyl; Phenyl; Naphthyl; Pyridyl; Thienyl; Furyl; $C_{3-10}$-Cycloalkyl; Heterocyclyl; $C_{1-8}$-Alkyl oder $C_{2-8}$-Heteroalkyl verbrücktes Phenyl, Naphthyl, Pyridyl, Thienyl, Furyl, $C_{3-10}$-Cycloalkyl oder Heterocyclyl; CHO; C(=O)$C_{1-8}$-Alkyl; $CO_2H$; C(=O)O-$C_{1-8}$-Alkyl; $CONH_2$; C(=O)NH-$C_{1-8}$-Alkyl; C(=O)N($C_{1-8}$-Alkyl)$_2$; OH; O-$C_{1-8}$-Alkyl; $OCF_3$; O-($C_{1-8}$-Alkyl)-OH; O-($C_{1-8}$-Alkyl)-O-$C_{1-8}$-Alkyl; O-Benzyl; O-Phenyl; O-Heteroaryl; O-C(=O)$C_{1-8}$-Alkyl; $NH_2$; NH-$C_{1-8}$-Alkyl; N($C_{1-8}$-Alkyl)$_2$; NH-C(=O)$C_{1-8}$-Alkyl; N($C_{1-8}$-Alkyl)-C(=O)$C_{1-8}$-Alkyl; N(C(=O)$C_{1-8}$-Alkyl)$_2$; SH; S-$C_{1-8}$-Alkyl; $SCF_3$; S-Benzyl; S-Phenyl; S-Heteroaryl; S(=O)$_2$$C_{1-8}$-Alkyl;; S(=O)$_2$OH; S(=O)$_2$O-$C_{1-8}$-Alkyl; S(=O)$_2$-NH-$C_{1-8}$-Alkyl.

Die erfindungsgemäßen Verbindungen sind durch Substituenten definiert, beispielsweise durch $R^1$, $R^2$ und $R^3$ (Substituenten der 1. Generation), welche ihrerseits ggf. substituiert sind (Substituenten der 2. Generation). Je nach Definition können diese Substituenten der Substituenten ihrerseits erneut substituiert sein (Substituenten der 3. Generation). Ist beispielsweise $R^1$ = Aryl (Substituent der 1. Generation), so kann Aryl seinerseits substituiert sein, z.B. mit $C_{1-8}$-Alkyl (Substituent der 2. Generation). Es ergibt sich daraus die funktionelle Gruppe Aryl-$C_{1-8}$-Alkyl. $C_{1-8}$-Alkyl kann dann seinerseits erneut substituiert sein, z.B. mit Cl (Substituent der 3. Generation). Es ergibt sich daraus dann insgesamt die funktionelle Gruppe Aryl-$C_{1-8}$-Alkyl-Cl.

In einer bevorzugten Ausführungsform können die Substituenten der 3. Generation jedoch nicht erneut substituiert sein, d.h. es gibt dann keine Substituenten der 4. Generation.

In einer anderen bevorzugten Ausführungsform können die Substituenten der 2. Generation nicht erneut substituiert sein, d.h. es gibt dann bereits keine Substituenten der 3. Generation. Mit anderen Worten können in dieser Ausführungsform bspw. im Fall der allgemeinen Formel (1) die funktionellen Gruppen für $R^1$ bis $R^{13}$ jeweils ggf. substituiert sein, die jeweiligen Substituenten können dann ihrerseits jedoch nicht erneut substituiert sein.

In einigen Fällen sind die erfindungsgemäßen Verbindungen durch Substituenten definiert, die einen Aryl- oder Heteroaryl-Rest darstellen oder tragen, jeweils unsubstituiert oder einfach oder mehrfach substituiert oder die zusammen mit dem oder den sie verbindenden Kohlenstoff- oder Heteroatom(en) als Ringglied oder als Ringgliedern einen Ring bilden, beispielsweise ein Aryl oder Heteroaryl, jeweils unsubstituiert oder einfach oder mehrfach substituiert. Sowohl diese Aryl- oder Heteroaryl-Reste als auch die so gebildeten aromatischen Ringsysteme können ggf. mit $C_{3-10}$-Cycloalkyl oder Heterocyclyl, jeweils gesättigt oder ungesättigt, kondensiert sein, d.h. mit einem $C_{3-10}$-Cycloalkyl wie Cyclopentyl oder einem Heterocyclyl wie Morpholinyl, wobei die so kondensierten $C_{3-10}$-Cycloalkyl- oder Heterocyclyl-Reste ihrerseits jeweils unsubstituiert oder einfach oder mehrfach substituiert sein können.

In einigen Fällen sind die erfindungsgemäßen Verbindungen durch Substituenten definiert, die einen $C_{3-10}$-Cycloalkyl- oder Heterocyclyl-Rest darstellen oder tragen, jeweils unsubstituiert oder einfach oder mehrfach substituiert oder die zusammen mit dem oder den sie verbindenden Kohlenstoff- oder Heteroatom(en) als Ringglied oder als Ringgliedern einen Ring bilden, beispielsweise ein $C_{3-10}$-Cycloalkyl oder Heterocyclyl, jeweils unsubstituiert oder einfach oder mehrfach substituiert. Sowohl diese $C_{3-10}$-Cycloalkyl oder Heterocyclyl-Reste als auch die gebildeten aliphatischen Ringsysteme können ggf. mit Aryl oder Heteroaryl kondensiert sein, d.h. mit einem Aryl wie Phenyl oder einem Heteroaryl wie Pyridyl, wobei die so kondensierten Aryl- oder Heteroaryl-Reste ihrerseits jeweils unsubstituiert oder einfach oder mehrfach substituiert sein können.

Im Rahmen der vorliegenden Erfindung bezeichnet das in Formeln verwendete Symbol

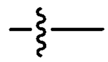

eine Verknüpfung eines entsprechenden Restes an die jeweilige übergeordnete allgemeine Struktur.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Maleinsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, $\alpha$-Liponsäure, Acetylglycin, Hippursäure, Phosphorsäure und/oder Asparaginsäure. Besonders bevorzugt sind die Zitronensäure und die Salzsäure.

Physiologisch verträgliche Salze mit Kationen oder Basen sind Salze der jeweiligen Verbindung - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calcium-Salze.

In bevorzugten Ausführungsformen der erfindungsgemäßen Verbindungen der allgemeinen Formel (1) steht

$A^1$ für S und

$A^2$ für $CR^{12}R^{13}$, O, S oder $S(=O)_2$, vorzugsweise für $CR^{12}R^{13}$, S oder $S(=O)_2$, besonders bevorzugt $CR^{12}R^{13}$.

Weitere bevorzugte Ausführungsformen der erfindungsgemäßen Verbindungen der allgemeinen Formel (1) haben die allgemeine Formel (1 a), (1 b), (1 c), (1 d), (1 e) oder (1f):

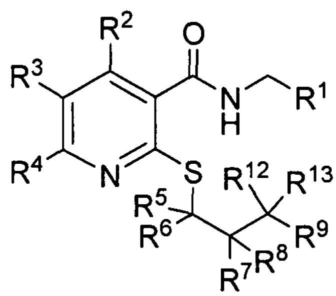

(1a)

,

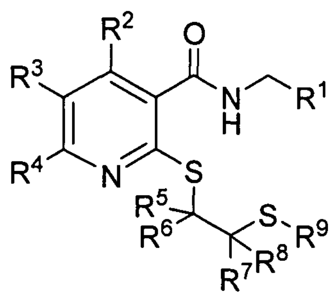

(1b)

,

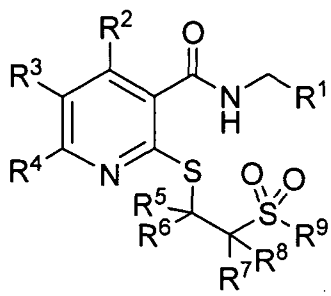

(1c)

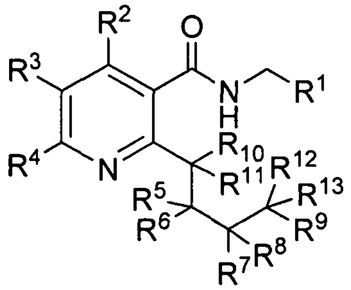

(1d)

,

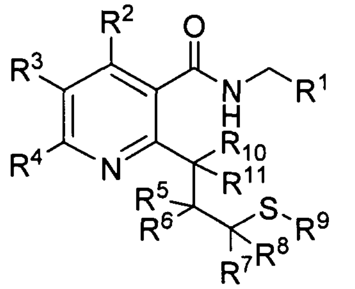

(1e)

,

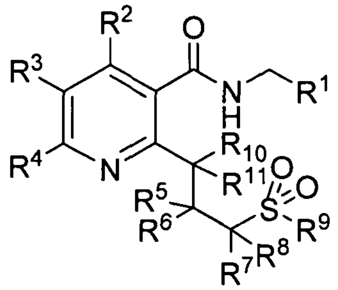

(1f)

.

Ganz besonders bevorzugt sind Verbindungen der allgemeinen Formel (1 a), (1 b) und (1c).

Insbesondere bevorzugt sind Verbindungen der allgemeinen Formel (1 a).

In einer weiteren bevorzugten Ausführungsform steht der Rest $R^1$ für

$C_{1-10}$-Alkyl oder $C_{2-10}$-Heteroalkyl, jeweils gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus F, Cl, Br, I, $NO_2$, $CF_3$, CN, OH, =O, C(=O)-OH, $OCF_3$, $NH_2$, $S(=O)_2OH$, SH, $SCF_3$, $C_{1-8}$-Alkyl, O-$C_{1-8}$-Alkyl, S-$C_{1-8}$-Alkyl, NH-$C_{1-8}$-Alkyl, N($C_{1-8}$-Alkyl)$_2$, $C_{3-10}$-Cycloalkyl und Heterocyclyl, wobei die vorstehenden Alkyl-Reste jeweils gesättigt oder ungesättigt, verzweigt oder unverzweigt, unsubstituiert oder einfach oder mehrfach substituiert sein können mit einem oder mehreren Substituenten jeweils unabhängig voneinander ausgewählt aus der Gruppe bestehend aus F, Cl, Br, I, O-$C_{1-8}$-Alkyl, OH und $OCF_3$, und wobei $C_{3-10}$-Cycloalkyl oder Heterocyclyl, jeweils gesättigt oder ungesättigt, unsubstituiert oder einfach oder mehrfach substituiert sein können mit einem oder mehreren Substituenten jeweils unabhängig voneinander ausgewählt aus der Gruppe bestehend aus F, Cl, Br, I, $C_{1-8}$-Alkyl, OH, =O, O-$C_{1-8}$-Alkyl, $OCF_3$, $NH_2$, NH-$C_{1-8}$-Alkyl und N($C_{1-8}$-Alkyl)$_2$;

$C_{3-10}$-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus F, Cl, Br, I, $NO_2$, CN, OH, O-$C_{1-8}$-Alkyl, $OCF_3$, $C_{1-8}$-Alkyl, C(=O)-OH, $CF_3$, $NH_2$, NH($C_{1-8}$-Alkyl), N($C_{1-8}$-Alkyl)$_2$, SH, S-$C_{1-8}$-Alkyl, $SCF_3$, $S(=O)_2OH$, Benzyl, Phenyl, Pyridyl und Thienyl, wobei Benzyl, Phenyl, Pyridyl, Thienyl jeweils unsubstituiert oder einfach oder mehrfach substituiert sein können mit einem oder mehreren Substituenten unabhängig voneinander ausgewählt aus der Gruppe bestehend aus F, Cl, Br, I, $NO_2$, CN, OH, O-$C_{1-8}$-Alkyl, $OCF_3$, $C_{1-8}$-Alkyl, C(=O)-OH, $CF_3$, $NH_2$, NH($C_{1-8}$-Alkyl), N($C_{1-8}$-Alkyl)$_2$, SH, S-$C_{1-8}$-Alkyl, $SCF_3$ und $S(=O)_2OH$;

Aryl oder Heteroaryl, jeweils unsubstituiert oder einfach oder mehrfach substituiert mit einem oder mehreren Substituenten jeweils unabhängig voneinander ausgewählt aus der Gruppe bestehend aus F, Cl, Br, I, $NO_2$, CN, OH, O-$C_{1-8}$-Alkyl, $OCF_3$, $C_{1-8}$-Alkyl, C(=O)-OH, $CF_3$, $NH_2$, NH($C_{1-8}$-Alkyl), N($C_{1-8}$-Alkyl)$_2$, SH, S-$C_{1-8}$-Alkyl, $SCF_3$, $S(=O)_2OH$, Benzyl, Phenyl, Pyridyl und Thienyl, wobei Benzyl, Phenyl, Pyridyl, Thienyl jeweils unsubstituiert oder einfach oder mehrfach substituiert sein können mit einem oder mehreren Substituenten unabhängig voneinander ausgewählt aus der Gruppe bestehend aus F, Cl, Br, I, $NO_2$, CN, OH, O-$C_{1-8}$-Alkyl, $OCF_3$, $C_{1-8}$-Alkyl, C(=O)-OH, $CF_3$, $NH_2$, NH($C_{1-8}$-Alkyl), N($C_{1-8}$-Alkyl)$_2$, SH, S-$C_{1-8}$-Alkyl, $SCF_3$ und $S(=O)_2OH$,

über $C_{1-8}$-Alkyl oder $C_{2-8}$-Heteroalkyl verbrücktes $C_{3-10}$-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus F, Cl, Br, I, $NO_2$, CN, OH, O-$C_{1-8}$-Alkyl, $OCF_3$, $C_{1-8}$-Alkyl, C(=O)-OH, $CF_3$, $NH_2$, NH($C_{1-8}$-Alkyl), N($C_{1-8}$-Alkyl)$_2$, SH, S-$C_{1-8}$-Alkyl, $SCF_3$, $S(=O)_2OH$, Benzyl, Phenyl, Pyridyl und Thienyl, wobei Benzyl, Phenyl, Pyridyl, Thienyl jeweils unsubstituiert oder einfach oder mehrfach substituiert sein können mit einem oder mehreren Substituenten unabhängig voneinander ausgewählt aus der Gruppe bestehend aus F, Cl, Br, I, $NO_2$, CN, OH, O-$C_{1-8}$-Alkyl, $OCF_3$, $C_{1-8}$-Alkyl, C(=O)-OH, $CF_3$, $NH_2$, NH($C_{1-8}$-Alkyl), N($C_{1-8}$-Alkyl)$_2$, SH, S-$C_{1-8}$-Alkyl, $SCF_3$ und $S(=O)_2OH$;

wobei die Alkyl- oder Heteroalkylkette jeweils verzweigt oder unverzweigt, gesättigt oder ungesättigt, unsubstituiert, einfach oder mehrfach substituiert sein kann mit einem oder mehreren Substituenten jeweils unabhängig voneinander ausgewählt aus der Gruppe bestehend aus F, Cl, Br, I, $NO_2$, $CF_3$, CN, OH, =O, C(=O)-OH, $OCF_3$, $NH_2$, $S(=O)_2OH$, SH, $SCF_3$, $C_{1-8}$-Alkyl, O-$C_{1-8}$ Alkyl, S-$C_{1-8}$-Alkyl, NH-$C_{1-8}$-Alkyl, N($C_{1-8}$-Alkyl)$_2$, $C_{3-10}$-Cycloalkyl und Heterocyclyl, wobei die vorstehenden Alkyl-Reste jeweils gesättigt oder ungesättigt, verzweigt oder unverzweigt, unsubstituiert oder einfach oder mehrfach substituiert sein können mit einem oder mehreren Substituenten jeweils unabhängig voneinander ausgewählt aus der Gruppe bestehend aus F, Cl, Br, I, O-$C_{1-8}$-Alkyl, OH und $OCF_3$, und wobei $C_{3-10}$-Cycloalkyl oder Heterocyclyl, jeweils gesättigt oder ungesättigt, unsubstituiert oder einfach oder mehrfach substituiert sein können mit einem oder mehreren Substituenten jeweils unabhängig voneinander ausgewählt aus der Gruppe bestehend aus F, Cl,

Br, I, $C_{1-8}$-Alkyl, OH, =O, O-$C_{1-8}$-Alkyl, $OCF_3$, $NH_2$, NH-$C_{1-8}$-Alkyl, und N($C_{1-8}$-Alkyl)$_2$;
oder über $C_{1-8}$-Alkyl oder $C_{2-8}$-Heteroalkyl verbrücktes Aryl oder Heteroaryl, jeweils unsubstituiert oder einfach oder mehrfach substituiert mit einem oder mehreren Substituenten jeweils unabhängig voneinander ausgewählt aus der Gruppe bestehend aus F, Cl, Br, I, $NO_2$, CN, OH, O-$C_{1-8}$-Alkyl, $OCF_3$, $C_{1-8}$-Alkyl, C(=O)-OH, $CF_3$, $NH_2$, NH($C_{1-8}$-Alkyl), N($C_{1-8}$-Alkyl)$_2$, SH, S-$C_{1-8}$-Alkyl, $SCF_3$, S(=O)$_2$OH, Benzyl, Phenyl, Pyridyl und Thienyl, wobei Benzyl, Phenyl, Pyridyl, Thienyl jeweils unsubstituiert oder einfach oder mehrfach substituiert sein können mit einem oder mehreren Substituenten unabhängig voneinander ausgewählt aus der Gruppe bestehend aus F, Cl, Br, I, $NO_2$, CN, OH, O-$C_{1-8}$-Alkyl, $OCF_3$, $C_{1-8}$-Alkyl, C(=)-OH, $CF_3$, $NH_2$, NH($C_{1-8}$-Alkyl), N($C_{1-8}$-Alkyl)$_2$, SH, S-$C_{1-8}$-Alkyl, $SCF_3$ und S(=O)$_2$OH;
wobei die Alkyl- oder Heteroalkylkette jeweils verzweigt oder unverzweigt, gesättigt oder ungesättigt, unsubstituiert, einfach oder mehrfach substituiert sein kann mit einem oder mehreren Substituenten jeweils unabhängig voneinander ausgewählt aus der Gruppe bestehend aus F, Cl, Br, I, $NO_2$, $CF_3$, CN, OH, =O, C(=O)-OH, $OCF_3$, $NH_2$, S(=O)$_2$OH, SH, $SCF_3$, $C_{1-8}$-Alkyl, O-$C_{1-8}$-Alkyl, S-$C_{1-8}$-Alkyl, NH-$C_{1-8}$-Alkyl, N($C_{1-8}$-Alkyl)$_2$, $C_{3-10}$-Cycloalkyl und Heterocyclyl, wobei die vorstehenden Alkyl-Reste jeweils gesättigt oder ungesättigt, verzweigt oder unverzweigt, unsubstituiert oder einfach oder mehrfach substituiert sein können mit einem oder mehreren Substituenten jeweils unabhängig voneinander ausgewählt aus der Gruppe bestehend aus F, Cl, Br, I, O-$C_{1-8}$-Alkyl, OH und $OCF_3$, und wobei $C_{3-10}$-Cycloalkyl oder Heterocyclyl jeweils gesättigt oder ungesättigt, unsubstituiert oder einfach oder mehrfach substituiert sein können mit einem oder mehreren Substituenten jeweils unabhängig voneinander ausgewählt aus der Gruppe bestehend aus F, Cl, Br, I, $C_{1-8}$-Alkyl, OH, =O, O-$C_{1-8}$-Alkyl, $OCF_3$, $NH_2$, NH-$C_{1-8}$-Alkyl, und N($C_{1-8}$-Alkyl)$_2$; steht.

In einer weiteren bevorzugten Ausführungsform steht der Substituent $R^1$ für nachfolgende Teilstruktur (T1)

$$-\S-(CR^{14a}R^{14b})_m-Y_n-B$$

(T1)

,

worin

$R^{14a}$ und $R^{14b}$ jeweils unabhängig voneinander für H; F; Cl; Br; I; $NO_2$; $CF_3$; CN; OH; $OCF_3$; $NH_2$; $C_{1-4}$-Alkyl, O-$C_{1-4}$-Alkyl, NH-$C_{1-4}$-Alkyl, N($C_{1-4}$-Alkyl)$_2$, jeweils gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus F, Cl, Br, I, O-$C_{1-4}$-Alkyl, OH und $OCF_3$; $C_{3-10}$-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus F, Cl, Br, I, $C_{1-4}$-Alkyl, OH, =O, O-$C_{1-4}$-Alkyl, $OCF_3$, $NH_2$, NH-$C_{1-4}$-Alkyl, und N($C_{1-4}$-Alkyl)$_2$; stehen;

m für 0, 1, 2 oder 3 steht;

Y für O oder $NR^{15}$ steht, wobei $R^{15}$ für H; $C_{1-4}$-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus F, Cl, Br, I, $C_{1-4}$-Alkyl, OH, 0-$C_{1-4}$-Alkyl, $OCF_3$, $NH_2$, NH-$C_{1-4}$-Alkyl, und N($C_{1-4}$-Alkyl)$_2$; steht; oder für $C_{3-10}$-Cycloalkyl, gesättigt oder ungesättigt, unsubstituiert oder einfach oder mehrfach substituiert mit einem oder mehreren Substituenten jeweils unabhängig voneinander ausgewählt aus der Gruppe bestehend aus F, Cl, Br, I, $C_{1-4}$-Alkyl, OH, O-$C_{1-4}$-Alkyl, $OCF_3$, $NH_2$, NH-$C_{1-4}$-Alkyl, und N($C_{1-4}$-Alkyl)$_2$; steht;

n für 0 oder 1 steht,

B für $C_{1-8}$-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus F, Cl, Br, I, $NO_2$, CN, OH, =O, O-$C_{1-4}$-Alkyl, $OCF_3$, C(=O)-OH, $CF_3$, $NH_2$, Nhi($C_{1-4}$-Alkyl), N($C_{1-4}$-Alkyl)$_2$, SH, S-$C_{1-4}$-Alkyl, $SCF_3$ und S(=O)$_2$OH; $C_{3-10}$-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus F, Cl, Br, I, $NO_2$, CN, OH, O-$C_{1-8}$-Alkyl, $OCF_3$, $C_{1-8}$-Alkyl, C(=O)-OH, $CF_3$, $NH_2$, NH($C_{1-8}$-Alkyl), N($C_{1-8}$-Alkyl)$_2$, SH, S-$C_{1-8}$-Alkyl, $SCF_3$, S(=O)$_2$OH, Benzyl, Phenyl, Pyridyl und Thienyl, wobei Benzyl, Phenyl, Pyridyl, Thienyl jeweils unsubstituiert oder einfach oder mehrfach substituiert sein können mit einem oder mehreren Substituenten unabhängig voneinander ausgewählt aus der Gruppe bestehend aus F, Cl, Br, I, $NO_2$, CN, OH, O-$C_{1-8}$-Alkyl, $OCF_3$, $C_{1-8}$-Alkyl, C(=O)-OH, $CF_3$, $NH_2$, NH($C_{1-8}$-Alkyl), N($C_{1-8}$-Al-

kyl)$_2$, SH, S-$C_{1-8}$-Alkyl, $SCF_3$ und $S(=O)_2OH$; Aryl oder Heteroaryl, jeweils unsubstituiert oder einfach oder mehrfach substituiert mit einem oder mehreren Substituenten jeweils unabhängig voneinander ausgewählt aus der Gruppe bestehend aus F, Cl, Br, I, $NO_2$, CN, OH, O-$C_{1-8}$-Alkyl, $OCF_3$, $C_{1-8}$-Alkyl, C(=O)-OH, $CF_3$, $NH_2$, NH($C_{1-8}$-Alkyl), N($C_{1-8}$-Alkyl)$_2$, SH, S-$C_{1-8}$-Alkyl, $SCF_3$, $S(=O)_2OH$, Benzyl, Phenyl, Pyridyl und Thienyl, wobei Benzyl, Phenyl, Pyridyl, Thienyl jeweils unsubstituiert oder einfach oder mehrfach substituiert sein können mit einem oder mehreren Substituenten unabhängig voneinander ausgewählt aus der Gruppe bestehend aus F, Cl, Br, I, $NO_2$, CN, OH, O-$C_{1-8}$-Alkyl, $OCF_3$, $C_{1-8}$-Alkyl, C(=O)-OH, $CF_3$, $NH_2$, NH($C_{1-8}$-Alkyl), N($C_{1-8}$-Alkyl)$_2$, SH, S-$C_{1-8}$-Alkyl, $SCF_3$ und $S(=O)_2OH$; steht.

Vorzugsweise stehen

$R^{14a}$ und $R^{14b}$ jeweils unabhängig voneinander für H; F; Cl; Br; I; $NO_2$; $CF_3$; $CH_2CF_3$; CN; OH; $OCF_3$, $NH_2$; $C_{1-4}$-Alkyl, O-$C_{1-4}$-Alkyl, O-$C_{1-4}$-Alkyl-OH, O-$C_{1-4}$-Alkyl-$OCH_3$, NH-$C_{1-4}$-Alkyl, N($C_{1-4}$-Alkyl)$_2$, jeweils gesättigt oder ungesättigt, verzweigt oder unverzweigt, unsubstituiert; $C_{3-10}$-Cycloalkyl, gesättigt oder ungesättigt, unsubstituiert oder einfach oder mehrfach substituiert mit einem oder mehreren Substituenten jeweils unabhängig voneinander ausgewählt aus der Gruppe bestehend aus F, Cl, Br, I, $C_{1-4}$-Alkyl, OH, O-$C_{1-4}$-Alkyl;

m für 0, 1, 2 oder 3;

Y für O oder $NR^{15}$;
wobei $R^{15}$ für H; $C_{1-4}$-Alkyl, gesättigt oder ungesättigt, unsubstituiert; oder für $C_{3-10}$-Cycloalkyl, gesättigt oder ungesättigt, unsubstituiert, steht;

n für 0 oder 1;

B für $C_{1-8}$-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus F, Cl, Br, I, $NO_2$, CN, OH, O-$C_{1-4}$-Alkyl, $OCF_3$, $CF_3$, $NH_2$, NH($C_{1-4}$-Alkyl), N($C_{1-4}$-Alkyl)$_2$, $SCF_3$; $C_{3-10}$-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus F, Cl, Br, I, $NO_2$, CN, OH, O-$C_{1-4}$-Alkyl, $OCF_3$, $C_{1-4}$-Alkyl, $CF_3$, $NH_2$, NH($C_{1-4}$-Alkyl), N($C_{1-4}$-Alkyl)$_2$, $SCF_3$; Aryl oder Heteroaryl, jeweils unsubstituiert oder einfach oder mehrfach substituiert mit einem oder mehreren Substituenten jeweils unabhängig voneinander ausgewählt aus der Gruppe bestehend aus F, Cl, Br, I, $NO_2$, CN, OH, O-$C_{1-4}$-Alkyl, $OCF_3$, $C_{1-4}$-Alkyl, C(=O)-OH, $CF_3$, $NH_2$, NH($C_{1-4}$-Alkyl), N($C_{1-4}$-Alkyl)$_2$, SH, S-$C_{1-4}$-Alkyl, $SCF_3$, $S(=O)_2OH$, Benzyl, Phenyl und Pyridyl, wobei Benzyl, Phenyl oder Pyridyl jeweils unsubstituiert oder einfach oder mehrfach substituiert sind mit einem oder mehreren Substituenten jeweils unabhängig voneinander ausgewählt aus der Gruppe bestehend aus F, Cl, Br, I, $NO_2$, CN, OH, O-$C_{1-4}$-Alkyl, $OCF_3$, $C_{1-4}$-Alkyl, C(=O)-OH, $CF_3$, $NH_2$, NH($C_{1-4}$-Alkyl), N($C_{1-4}$-Alkyl)$_2$, SH, S-$C_{1-4}$-Alkyl, $SCF_3$ und $S(=O)_2OH$.

Besonders bevorzugt stehen $R^{14a}$ und $R^{14b}$ jeweils unabhängig voneinander für H; F; Cl; Br; I; $NO_2$; $CF_3$; CN; Methyl; Ethyl; n-Propyl; iso-Propyl; Cyclopropyl; n-Butyl; sec.-Butyl; tert.-Butyl; $CH_2CF_3$; OH; O-Methyl; O-Ethyl; O-$(CH_2)_2$-O-$CH_3$; O-$(CH_2)_2$-OH; $OCF_3$; $NH_2$; NH-Methyl; N(Methyl)$_2$; NH-Ethyl; N(Ethyl)$_2$; oder N(Methyl)(Ethyl);

m für 0, 1 oder 2;

n für 0; und

B für $C_{1-4}$-Alkyl, gesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus F, Cl, Br, I, OH, O-$C_{1-4}$-Alkyl, $OCF_3$ und $CF_3$; $C_{3-10}$-Cycloalkyl, gesättigt, unsubstituiert; Phenyl, Naphthyl, Pyridyl, Thienyl, jeweils unsubstituiert oder einfach oder zweifach oder dreifach substituiert mit einem, zwei oder drei Substituenten jeweils unabhängig voneinander ausgewählt aus der Gruppe bestehend aus F, Cl, Br, I, $NO_2$, CN, OH, O-$C_{1-4}$-Alkyl, $OCF_3$, $C_{1-4}$-Alkyl, C(=O)-OH, $CF_3$, $NH_2$, NH($C_{1-4}$-Alkyl), N($C_{1-4}$-Alkyl)$_2$, SH, S-$C_{1-4}$-Alkyl, $SCF_3$, $S(=O)_2OH$.

Ganz besonders bevorzugt stehen $R^{14a}$ und $R^{14b}$ jeweils unabhängig voneinander für H; F; Cl; Br; I; Methyl; Ethyl; n-Propyl; iso-Propyl; n-Butyl; sec.-Butyl; tert.-Butyl; OH; O-Methyl; O-Ethyl; O-$(CH_2)_2$-O-$CH_3$; oder O-$(CH_2)_2$-OH;

m für 0, 1 oder 2;

n für 0; und

B für Methyl; Ethyl; n-Propyl; iso-Propyl; n-Butyl; sec.-Butyl; tert.-Butyl; Cyclopropyl; Cyclobutyl; Cyclopentyl; Cyclohexyl; Cycloheptyl; Adamantyl; Bicyclo[2.2.1]heptyl; Bicyclo[2.2.2]octyl; Phenyl, Pyridyl, Thienyl, jeweils unsubstituiert oder einfach, zweifach oder dreifach substituiert mit einem, zwei oder drei Substituenten jeweils unabhängig voneinander ausgewählt aus der Gruppe bestehend aus F, Cl, Br, I, $NO_2$, CN, OH, O-$C_{1-4}$-Alkyl, $OCF_3$, $C_{1-4}$-Alkyl, C(=O)-OH, $CF_3$, $NH_2$, NH($C_{1-4}$-Alkyl), N($C_{1-4}$-Alkyl)$_2$, SH, S-$C_{1-4}$-Alkyl, $SCF_3$ und S(=O)$_2$OH.

Für n = 0 ergibt sich aus der Teilstruktur (T-1) für $R^1$ die Teilstruktur (T1-1):

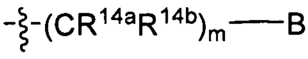

(T1-1)

.

In einer bevorzugten Ausführungsform für n = 0 stehen $R^{14a}$ und $R^{14b}$ jeweils unabhängig voneinander für H; F; Cl; Br; I; Methyl; Ethyl; n-Propyl; iso-Propyl; n-Butyl; sec.-Butyl; tert.-Butyl; OH; O-Methyl; O-Ethyl; O-($CH_2$)$_2$-O-$CH_3$; oder O-($CH_2$)$_2$-OH.

In einer bevorzugten Ausführungsform für m = 0 steht B für Phenyl, Pyridyl oder Thienyl, einfach oder zweifach oder dreifach substituiert mit einem, zwei oder drei Substituenten jeweils unabhängig voneinander ausgewählt aus der Gruppe bestehend aus F, Cl, Br, I, $NO_2$, CN, OH, O-$C_{1-4}$-Alkyl, $OCF_3$, $C_{1-4}$-Alkyl, C(=O)-OH, $CF_3$, $NH_2$, NH($C_{1-4}$-Alkyl), N($C_{1-4}$-Alkyl)$_2$, SH, S-$C_{1-4}$-Alkyl, $SCF_3$, S(=O)$_2$OH

In einer bevorzugten Ausführungsform für m = 1 oder 2 steht B für Cyclopropyl; Cyclobutyl; Cyclopentyl; Cyclohexyl; Cycloheptyl; Adamantyl; Bicyclo[2.2.1]heptyl; Bicyclo[2.2.2]octyl.

In einer bevorzugten Ausführungsform für m = 0, 1 oder 2 steht B für Methyl; Ethyl; n-Propyl; iso-Propyl; n-Butyl; sec.-Butyl oder tert.-Butyl.

Eine weitere besonders bevorzugte Ausführungsform der erfindungsgemäßen Verbindungen der allgemeinen Formel (1) hat die allgemeine Formel (2):

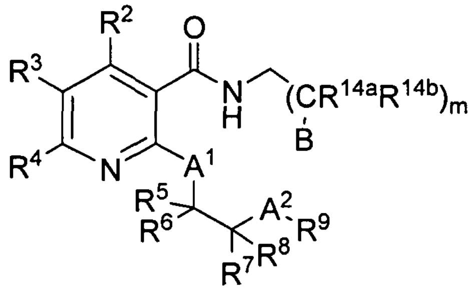

(2)

.

In einer weiteren bevorzugten Ausführungsform stehen die Reste $R^2$, $R^3$ und $R^4$ jeweils unabhängig voneinander für H; F; Cl; Br; I; $NO_2$; $CF_3$; CN; OH; $OCF_3$; SH; $SCF_3$; Methyl; $CH_2$-O-Methyl; $CH_2$-OH; $C_{2-6}$-Alkyl, O-$C_{1-6}$-Alkyl oder S-$C_{1-6}$-Alkyl, jeweils gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus F, Cl, Br, I, OH, =O und O-$C_{1-4}$-Alkyl; $C_{3-7}$-Cycloalkyl, gesättigt oder ungesättigt, verzweigt oder unverzweigt, unsubstituiert; $NR^aR^b$, wobei $R^a$ und $R^b$ jeweils unabhängig voneinander für H oder $C_{1-4}$-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus OH, =O und O-$C_{1-4}$-Alkyl, stehen oder $R^a$ und $R^b$ gemeinsam mit dem sie verbindenden Stickstoffatom ein Heterocyclyl, gesättigt oder ungesättigt, unsubstituiert oder einfach oder mehrfach substituiert mit $C_{1-4}$-Alkyl; bilden.

Vorzugsweise stehen die Reste $R^2$, $R^3$ und $R^4$ jeweils unabhängig voneinander für H; F; Cl; Br; I; $NO_2$; $CF_3$;

CN; OH; $OCF_3$; SH; $SCF_3$; Methyl; Ethyl; n-Propyl; iso-Propyl; Butyl; sec.-Butyl; tert.-Butyl; $CH_2CF_3$; O-Methyl; O-Ethyl; O-n-Propyl; O-iso-Propyl; O-Butyl; O-sec.-Butyl; O-tert.-Butyl; O-$(CH_2)_2$-O-Methyl; O-$(CH_2)_2$-OH; O-(C=O)-Methyl; O-(C=O)-Ethyl; S-Methyl; S-Ethyl; Cyclopropyl; Cyclobutyl; $NR^aR^b$, wobei $R^a$ und $R^b$ jeweils unabhängig voneinander ausgewählt sind aus der Gruppe bestehend aus H, Methyl, Ethyl, $(CH_2)_2$-O-Methyl, $(CH_2)_2$-OH, (C=O)-Methyl, (C=O)-Ethyl oder $R^a$ und $R^b$ gemeinsam mit dem sie verbindenden Stickstoffatom ein Pyrrolidinyl, Piperidinyl, 4-Methylpiperazinyl oder Morpholinyl bilden.

Besonders bevorzugt stehen die Reste $R^2$, $R^3$ und $R^4$ jeweils unabhängig voneinander für H; F; Cl; Br; I; Methyl; Ethyl; n-Propyl, iso-Propyl; Cyclopropyl; CN; $CF_3$; O-Methyl; $OCF_3$; S-Methyl; $SCF_3$, Pyrrolidinyl, $N(Methyl)_2$.

Ganz besonders bevorzugt stehen die Reste $R^2$, $R^3$ und $R^4$ jeweils unabhängig voneinander für H; F; Cl; Methyl; Ethyl; O-Methyl; $CF_3$; insbesondere für H.

In einer weiteren bevorzugten Ausführungsform stehen die Reste $R^5$, $R^6$, $R^7$, $R^8$, $R^{10}$, $R^{11}$, $R^{12}$ und $R^{13}$ jeweils unabhängig voneinander für H; F; Cl; Br; I; $NO_2$; $CF_3$; CN; OH; $OCF_3$; SH; $SCF_3$; $C_{1-6}$-Alkyl; O-$C_{1-6}$-Alkyl oder S-$C_{1-6}$-Alkyl, jeweils gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus F, Cl, Br, I, OH und O-$C_{1-4}$-Alkyl; $C_{3-7}$-Cycloalkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus F, Cl, Br, I, OH, =O und O-$C_{1-4}$-Alkyl;

oder $R^5$ und $R^6$ oder $R^7$ und $R^8$ oder $R^{10}$ und $R^{11}$ oder $R^{12}$ und $R^{13}$ oder $R^5$ und $R^{11}$ oder $R^5$ und $R^7$ oder $R^5$ und $R^{13}$ oder $R^7$ und $R^{13}$ oder $R^7$ und $R^{11}$ oder $R^{11}$ und $R^{13}$ bilden gemeinsam mit dem oder den sie verbindenden Kohlenstoffatom(en) ein Cyclopropyl, Cyclobutyl, Cyclopentyl, Cyclohexyl, Pyrrolidinyl oder Piperidinyl, jeweils unsubstituiert oder einfach oder mehrfach substituiert mit einem oder mehreren Substituenten jeweils unabhängig voneinander ausgewählt aus der Gruppe bestehend aus F, Cl, Br, I, OH, =O und O-$C_{1-4}$-Alkyl; wobei die jeweiligen verbleibenden Substituenten $R^5$, $R^6$, $R^7$, $R^8$, $R^{10}$, $R^{11}$, $R^{12}$ und $R^{13}$ die vorstehende Bedeutung haben;
oder $R^5$ und $R^7$ bilden gemeinsam mit dem oder den sie verbindenden Kohlenstoffatom(en) ein Cyclopropyl, Cyclobutyl, Cyclopentyl oder Cyclohexyl, jeweils unsubstituiert oder einfach oder mehrfach substituiert mit einem oder mehreren Substituenten jeweils unabhängig voneinander ausgewählt aus der Gruppe bestehend aus F, Cl, Br, I, OH, =O und O-$C_{1-4}$-Alkyl; wobei die jeweiligen verbleibenden Substituenten $R^6$, $R^8$, $R^{10}$, $R^{11}$, $R^{12}$ und $R^{13}$ die vorstehende Bedeutung haben.

Vorzugsweise stehen die Reste $R^5$, $R^6$, $R^7$, $R^8$, $R^{10}$, $R^{11}$, $R^{12}$ und $R^{13}$ jeweils unabhängig voneinander für H; F; Cl; Br; I; $NO_2$; $CF_3$; CN; OH; $OCF_3$; SH; $SCF_3$; Methyl; Ethyl; n-Propyl; iso-Propyl; n-Butyl; sec.-Butyl; tert.-Butyl; O-Methyl; O-Ethyl; O-$(CH_2)_2$-O-$CH_3$; O-$(CH_2)_2$-OH; S-Methyl; S-Ethyl; Cyclopropyl; Cyclobutyl; Cyclopentyl; Cyclohexyl; oder $R^5$ und $R^6$ oder $R^7$ und $R^8$ oder $R^{10}$ und $R^{11}$ oder $R^{12}$ und $R^{13}$ oder $R^5$ und $R^{11}$ oder $R^5$ und $R^7$ oder $R^5$ und $R^{13}$ oder $R^7$ und $R^{13}$ oder $R^7$ und $R^{11}$ oder $R^{11}$ und $R^{13}$ bilden gemeinsam mit dem oder den sie verbindenden Kohlenstoffatom(en) ein Cyclopropyl, Cyclobutyl, Cyclopentyl oder Cyclohexyl, jeweils unsubstituiert; wobei die jeweiligen verbleibenden Substituenten $R^5$, $R^6$, $R^7$, $R^8$, $R^{10}$, $R^{11}$, $R^{12}$ und $R^{13}$ die vorstehende Bedeutung haben.

Besonders bevorzugt stehen die Reste $R^5$, $R^6$, $R^7$, $R^8$, $R^{10}$, $R^{11}$, $R^{12}$ und $R^{13}$ jeweils unabhängig voneinander für H; F; Cl; Br; I; CN; $CF_3$; $OCF_3$; $SCF_3$; Methyl; Ethyl; n-Propyl, iso-Propyl; Cyclopropyl; O-Methyl; S-Methyl; oder $R^5$ und $R^7$ bilden einen Cyclopropyl, Cylclobutyl, Cyclopentyl oder Cyclohexylring beliebig substituiert mit H,F, Cl, Me, Et, OMe, bevorzugt einen unsubstituierten Cyclopentyl- oder Cyclohexylring.

Ganz besonders bevorzugt stehen die Reste $R^5$, $R^6$, $R^7$, $R^8$, $R^{10}$, $R^{11}$, $R^{12}$ und $R^{13}$ jeweils unabhängig voneinander für F; Cl; H; Methyl; Ethyl; n-Propyl, iso-Propyl; Cyclopropyl; insbesondere für F; H; Methyl; oder Ethyl.

Insbesondere stehen die Reste $R^{12}$ und $R^{13}$ jeweils unabhängig voneinander für H, F oder Methyl.

In einer weiteren bevorzugten Ausführungsform steht der Rest $R^9$ für $C_{3-10}$-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus F, Cl, Br, I, $NO_2$, CN, OH, =O, O-$C_{1-4}$-Alkyl, $OCF_3$, $C_{1-4}$-Alkyl, $CF_3$, SH, S-$C_{1-4}$-Alkyl und $SCF_3$; Aryl oder Heteroaryl, jeweils unsubstituiert oder einfach oder mehrfach substituiert mit einem oder mehreren Substituenten jeweils unabhängig voneinander ausgewählt aus der Gruppe bestehend aus F, Cl, Br, I, $NO_2$, CN, OH, O-$C_{1-4}$-Alkyl, $OCF_3$, $C_{1-4}$-Alkyl, $CF_3$, $NH_2$, NH($C_{1-4}$-Alkyl), N($C_{1-4}$-Alkyl$)_2$, SH, S-$C_{1-4}$-Alkyl und $SCF_3$; oder für $CR^cR^d$, wobei $R^c$ und $R^d$ jeweils unabhängig voneinander $C_{1-4}$-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus F, Cl, Br, I, OH, O-$C_{1-4}$-Alkyl, $CF_3$, $OCF_3$ und $SCF_3$.

Vorzugsweise steht $R^9$ für $C_{3-7}$-Cycloalkyl, gesättigt oder ungesättigt, Pyrrolidinyl, Piperazinyl, 4-Methylpiperazinyl, Piperidinyl, Morpholinyl, jeweils unsubstituiert oder einfach oder mehrfach substituiert mit einem oder mehreren Substituenten jeweils unabhängig voneinander ausgewählt aus der Gruppe bestehend aus F, Cl, Br, I, OH, O-$C_{1-4}$-Alkyl, $OCF_3$, $C_{1-4}$-Alkyl, $CF_3$, SH, S-$C_{1-4}$-Alkyl und $SCF_3$; Phenyl, Naphthyl, Pyridyl oder Thienyl, jeweils unsubstituiert oder einfach oder mehrfach substituiert mit einem oder mehreren Substituenten jeweils unabhängig voneinander ausgewählt aus der Gruppe bestehend aus F, Cl, Br, I, $NO_2$, CN, OH, O-$C_{1-4}$-Alkyl, $OCF_3$, $C_{1-4}$-Alkyl, $CF_3$, SH, S-$C_{1-4}$-Alkyl und

$SCF_3$; oder für $CR^cR^d$, wobei $R^c$ und $R^d$ jeweils unabhängig voneinander $C_{1-4}$-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus F, Cl, Br, I, OH und O-$C_{1-4}$-Alkyl.

Besonders bevorzugt ist der Rest $R^9$ ausgewählt aus der Gruppe bestehend aus Phenyl, Pyridyl oder Thienyl, jeweils unsubstituiert oder einfach oder mehrfach substituiert mit einem oder mehreren Substituenten jeweils unabhängig voneinander ausgewählt aus der Gruppe bestehend aus F, Cl, Br, I, CN, OH, O-$C_{1-4}$-Alkyl, $OCF_3$, $C_{1-4}$-Alkyl, $CF_3$, SH, S-$C_{1-4}$-Alkyl und $SCF_3$.

Ganz besonders bevorzugt steht $R^9$ für Phenyl, Pyridyl und Thienyl, unsubstituiert oder einfach oder mehrfach substituiert mit einem oder mehreren Substituenten jeweils unabhängig voneinander ausgewählt aus der Gruppe bestehend aus F, Cl, Br, I, CN, Methyl, Ethyl, n-Propyl, iso-Propyl, n-Butyl, sec.-Butyl, tert.-Butyl, O-Methyl, O-Ethyl, O-n-Propyl, O-iso-Propyl, O-Butyl, O-sec.-Butyl, O-tert.-Butyl, OH, $OCF_3$, $CF_3$, SH, S-$C_{1-4}$-Alkyl und $SCF_3$.

Insbesondere steht $R^9$ für Phenyl, unsubstituiert oder einfach oder mehrfach substituiert mit einem oder mehreren Substituenten jeweils unabhängig voneinander ausgewählt aus der Gruppe bestehend aus F, Cl, Br, I, Methyl, Ethyl, n-Propyl, iso-Propyl, n-Butyl, sec.-Butyl, tert.-Butyl, O-Methyl, O-Ethyl, $OCF_3$, $CF_3$ und $SCF_3$.

In einer weiteren, besonders bevorzugten Ausführungsform steht

$A^1$ für S;

$A^2$ für $CR^{12}R^{13}$;

$R^1$ für die Teilstruktur (T1-1)

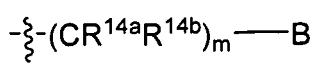

(T1-1)

,

worin

| | |
|---|---|
| $R^{14a}$ und $R^{14b}$ | jeweils unabhängig voneinander für H; F; Cl; Br; I; Methyl; Ethyl; n-Propyl; iso-Propyl; n-Butyl; sec.-Butyl; tert.-Butyl; OH; O-Methyl; O-Ethyl; O-$(CH_2)_2$-O-$CH_3$; oder O-$(CH_2)_2$-OH; stehen; |
| m | für 0, 1 oder 2 steht; |
| B | für Methyl; Ethyl; n-Propyl; iso-Propyl; n-Butyl; sec.-Butyl; tert.-Butyl; Cyclopropyl; Cyclobutyl; Cyclopentyl; Cyclohexyl; Cycloheptyl; Adamantyl; Bicyclo[2.2.1]heptyl; Bicyclo[2.2.2]octyl; Phenyl, Pyridyl, Thienyl, jeweils unsubstituiert oder einfach, zweifach oder dreifach substituiert mit einem, zwei oder drei Substituenten jeweils unabhängig voneinander ausgewählt aus der Gruppe bestehend aus F, Cl, Br, I, $NO_2$, CN, OH, O-$C_{1-4}$-Alkyl, $OCF_3$, $C_{1-4}$-Alkyl, C(=O)-OH, $CF_3$, $NH_2$, NH($C_{1-4}$-Alkyl), N($C_{1-4}$-Alky)$_2$, SH, S-$C_{1-4}$-Alkyl, $SCF_3$ und S(=O)$_2$OH; steht; |

$R^2$, $R^3$ und $R^4$ stehen jeweils unabhängig voneinander für H; F; Cl; Br; I; Methyl; Ethyl; n-Propyl, iso-Propyl; Cyclopropyl; CN; $CF_3$; O-Methyl; $OCF_3$; S-Methyl; $SCF_3$;
$R^5$, $R^6$, $R^7$, $R^8$, $R^{10}$, $R^{11}$, $R^{12}$ und $R^{13}$ stehen jeweils unabhängig voneinander für H; F; Cl; Br; I; CN; $CF_3$; $OCF_3$; $SCF_3$; Methyl; Ethyl; n-Propyl, iso-Propyl; Cyclopropyl; O-Methyl; S-Methyl; und
$R^9$ steht für Cyclopentyl oder Cyclohexyl, jeweils unsubstituiert oder einfach oder mehrfach substituiert mit einem oder mehreren Substituenten jeweils unabhängig voneinander ausgewählt aus der Gruppe bestehend aus F, Cl, Br, I, OH, O-Methyl, $OCF_3$, Methyl, Ethyl und $CF_3$; Phenyl, unsubstituiert oder einfach oder mehrfach substituiert mit einem oder mehreren Substituenten jeweils unabhängig voneinander ausgewählt aus der Gruppe bestehend aus F, Cl, Br, I, CN, Methyl, Ethyl, n-Propyl, iso-Propyl, n-Butyl, sec.-Butyl, tert.-Butyl, O-Methyl, O-Ethyl, O-n-Propyl, O-iso-Propyl, O-Butyl, O-sec.-Butyl, O-tert.-Butyl, OH, $OCF_3$, $CF_3$, SH, S-$C_{1-4}$-Alkyl und $SCF_3$; oder für Methyl, Ethyl, n-Propyl, iso-Propyl.

Insbesondere bevorzugt sind Verbindungen aus der Gruppe

**1** 2-(3-Phenyl-propylsulfanyl)-N-(thiophen-2-yl-methyl)-pyridin-3-carbonsäureamid;

**2** 2-(3-Cyclohexyl-propylsulfanyl)-N-(thiophen-2-yl-methyl)-pyridin-3-carbonsäureamid;

**3** 2-[(3-Oxo-3-phenyl-propyl)sulfanyl]-N-(thiophen-2-yl-methyl)-pyridin-3-carbonsäureamid;

**4** N-(Thiophen-2-yl-methyl)-2-[2-[3-(trifluormethyl)-phenoxy]-ethylsulfanyl]-pyridin-3-carbonsäureamid;
**5** 2-(4-Methyl-pentylsulfanyl)-N-(thiophen-2-yl-methyl)-pyridin-3-carbonsäureamid;
**7** 2-(4-Phenyl-butyl)-N-(thiophen-2-yl-methyl)-pyridin-3-carbonsäureamid;
**8** 2-[3-(Benzolsulfonyl)-propyl]-N-(cyclohexyl-methyl)-pyridin-3-carbonsäureamid;
**9** N-(Cyclohexyl-methyl)-2-(4-phenyl-butyl)-pyridin-3-carbonsäureamid;
**10** 2-[3-(Benzolsulfonyl)-propyl]-N-(thiophen-2-yl-methyl)-pyridin-3-carbonsäureamid;
**12** N-(Thiophen-2-yl-methyl)-2-[3-[[3-(trifluormethyl)phenyl]sulfonyl]-propyl]-pyridin-3-carbonsäureamid;
**13** N-(Thiophen-2-yl-methyl)-2-[3-[[3-(trifluor-methyl)phenyl]sulfanyl]-propyl]-pyridin-3-carbonsäureamid;
**16** 2-(2-Phenylsulfanyl-propylsulfanyl)-N-(thiophen-2-yl-methyl)-pyridin-3-carbonsäureamid;
**17** 2-[3-(4-Fluorphenyl)-propylsulfanyl]-N-(thiophen-2-yl-methyl)-pyridin-3-carbonsäureamid;
**18** N-(Thiophen-2-yl-methyl)-2-[3-[3-(trifluormethyl)phenyl]-propylsulfanyl]-pyridin-3-carbonsäureamid;
**19** 2-[2-[(4-Fluorphenyl)sulfanyl]-propylsulfanyl]-N-(thiophen-2-yl-methyl)-pyridin-3-carbonsäureamid;
**20** N-(Thiophen-2-yl-methyl)-2-[2-[[3-(trifluormethyl)phenyl]sulfanyl]-propylsulfanyl]-pyridin-3-carbonsäureamid;
**21** N-(Thiophen-2-yl-methyl)-2-[4-[3-(trifluormethyl)-phenyl]-butyl]-pyridin-3-carbonsäureamid;
**22** 2-[4-(4-Fluorphenyl)-butyl]-N-(thiophen-2-yl-methyl)-pyridin-3-carbonsäureamid;
**23** 2-(3-Phenylsulfanyl-propyl)-N-(thiophen-2-yl-methyl)-pyridin-3-carbonsäureamid;
**24** 2-[(1-Methyl-2-phenylsulfanyl-ethyl)sulfanyl]-N-(thiophen-2-yl-methyl)-pyridin-3-carbonsäureamid;
**25** N-(Cycloheptyl-methyl)-2-[4-[3-(trifluormethyl)phenyl]-butyl]-pyridin-3-carbonsäure amid
**26** N-(5-Bicyclo[2.2.1]heptanyl-methyl)-2-[4-[3-(trifluormethyl)phenyl]-butyl]-pyridin-3-carbonsäure amid
**27** N-(5-Bicyclo[2.2.1]heptanyl-methyl)-2-[3-[3-(trifluormethyl)phenyl]-propylsulfanyl]-pyridin-3-carbonsäure amid
**28** N-(5-Bicyclo[2.2.1]heptanyl-methyl)-2-(4-methyl-pentylsulfanyl)-pyridin-3-carbonsäure amid
**29** N-(Cycloheptyl-methyl)-2-[3-[(4-fluorphenyl)sulfanyl]-propyl]-pyridin-3-carbonsäure amid
**30** N-(5-Bicyclo[2.2.1]heptanyl-methyl)-2-[3-[(4-fluorphenyl)sulfanyl]-propyl]-pyridin-3-carbonsäure amid
**31** N-(Cycloheptyl-methyl)-2-[3-[[3-(trifluormethyl)phenyl]sulfonyl]-propyl]-pyridin-3-carbonsäure amid
**32** N-(5-Bicyclo[2.2.1]heptanyl-methyl)-2-[3-[[3-(trifluormethyl)phenyl]sulfonyl]-propyl]-pyridin-3-carbonsäure amid
**33** N-(5-Bicyclo[2.2.1]heptanyl-methyl)-2-(3-phenyl-propylsulfanyl)-pyridin-3-carbonsäure amid
**34** N-(5-Bicyclo[2.2.1]heptanyl-methyl)-2-[3-[4-(trifluormethyl)-phenyl]-propylsulfanyl]-pyridin-3-carbonsäure amid
**35** N-(5-Bicyclo[2.2.1]heptanyl-methyl)-2-[3-(4-chlorphenyl)-propylsulfanyl]-pyridin-3-carbonsäure amid
**36** N-[(3,5-Difluor-phenyl)-methyl]-2-[3-(4-fluorphenyl)-propylsulfanyl]-pyridin-3-carbonsäure amid
**37** N-[(5-Chlor-thiophen-2-yl)-methyl]-2-[3-(4-fluorphenyl)-propylsulfanyl]-pyridin-3-carbonsäure amid
**38** N-[(2,2-Dimethyl-cyclopropyl)-methyl]-2-[3-(4-fluorphenyl)-propylsulfanyl]-pyridin-3-carbonsäure amid
**39** N-(5-Bicyclo[2.2.1]heptanyl-methyl)-2-[3-(3-fluorphenyl)-propylsulfanyl]-pyridin-3-carbonsäure amid
**40** N-(Cyclohexyl-methyl)-2-[3-(4-fluorphenyl)-propylsulfanyl]-pyridin-3-carbonsäure amid
**41** N-(Cycloheptyl-methyl)-2-[3-(4-fluorphenyl)-propylsulfanyl]-pyridin-3-carbonsäure amid
**43** N-(5-Bicyclo[2.2.1]heptanyl-methyl)-2-[[3-(4-fluorphenyl)-3-oxo-propyl]sulfanyl]-pyridin-3-carbonsäure amid
**44** N-(5-Bicyclo[2.2.1]heptanyl-methyl)-2-[(3-oxo-3-phenyl-propyl)sulfanyl]-pyridin-3-carbonsäure amid
**45** N-(5-Bicyclo[2.2.1]heptanyl-methyl)-2-(hexylsulfanyl)-pyridin-3-carbonsäure amid
**46** N-(5-Bicyclo[2.2.1]heptanyl-methyl)-2-(2-phenoxy-ethylsulfanyl)-pyridin-3-carbonsäure amid
**47** N-(5-Bicyclo[2.2.1]heptanyl-methyl)-2-[2-[3-(trifluormethyl)-phenoxy]-ethylsulfanyl]-pyridin-3-carbonsäure amid
**48** 2-[3-(4-Fluorphenyl)-propylsulfanyl]-N-(3,3,3-trifluor-propyl)-pyridin-3-carbonsäure amid
**49** N-(5-Bicyclo[2.2.1]heptanyl-methyl)-2-[2-(4-fluor-phenoxy)-ethylsulfanyl]-pyridin-3-carbonsäure amid
**52** N-(5-Bicyclo[2.2.1]heptanyl-methyl)-2-(3-naphthalen-1-yl-propylsulfanyl)-pyridin-3-carbonsäure amid
**53** N-(5-Bicyclo[2.2.1]heptanyl-methyl)-2-[3-[3-fluor-4-(trifluormethyl)-phenyl]-propylsulfanyl]-pyridin-3-carbonsäure amid
**54** N-(5-Bicyclo[2.2.1]heptanyl-methyl)-2-[3-[4-fluor-3-(trifluormethyl)-phenyl]-propylsulfanyl]-pyridin-3-carbonsäure amid
**55** N-(Cyclooctyl-methyl)-2-[3-(4-fluorphenyl)-propylsulfanyl]-pyridin-3-carbonsäure amid
**56** N-(5-Bicyclo[2.2.1]heptanyl-methyl)-2-[3-(4-methoxyphenyl)-propylsulfanyl]-pyridin-3-carbonsäure amid
**57** 2-[3-(4-Fluorphenyl)-propylsulfanyl]-N-[(4-methoxyphenyl)-methyl]-pyridin-3-carbonsäure amid
**59** 2-[3-(4-Fluorphenyl)-propylsulfanyl]-N-[[3-(trifluormethyl)phenyl]-methyl]-pyridin-3-carbonsäure amid
**60** N-(5-Bicyclo[2.2.1]heptanyl-methyl)-2-[3-(3,4-difluor-phenyl)-propylsulfanyl]-pyridin-3-carbonsäure amid
**61** N-(5-Bicyclo[2.2.1]heptanyl-methyl)-2-[3-(3,5-difluor-phenyl)-propylsulfanyl]-pyridin-3-carbonsäure amid
**62** N-(5-Bicyclo[2.2.1]heptanyl-methyl)-2-[3-(2,4-difluor-phenyl)-propylsulfanyl]-pyridin-3-carbonsäure amid
**63** N-[(2-Fluorphenyl)-methyl]-2-[3-(4-fluorphenyl)-propylsulfanyl]-pyridin-3-carbonsäure amid
**64** N-[(3-Fluorphenyl)-methyl]-2-(3-(4-fluorphenyl)-propylsulfanyl]-pyridin-3-carbonsäure amid
**65** N-[(4-Fluorphenyl)-methyl]-2-[3-(4-fluorphenyl)-propylsulfanyl]-pyridin-3-carbonsäure amid
**66** N-Benzyl-2-[3-(4-fluorphenyl)-propylsulfanyl]-pyridin-3-carbonsäure amid
**67** N-(5-Bicyclo[2.2.1]heptanyl-methyl)-2-[(1-methyl-3-phenyl-propyl)sulfanyl]-pyridin-3-carbonsäure amid

**68** N-(5-Bicyclo[2.2.1]heptanyl-methyl)-2-[3-(3,4,5-trifluor-phenyl)-propylsulfanyl]-pyridin-3-carbonsäure amid
**69** N-(5-Bicyclo[2.2.1]heptanyl-methyl)-2-[3-[3-fluor-5-(trifluormethyl)-phenyl]-propylsulfanyl]-pyridin-3-carbonsäure amid
**70** N-(5-Bicyclo[2.2.1]heptanyl-methyl)-2-[3-(3-methoxyphenyl)-propylsulfanyl]-pyridin-3-carbonsäure amid
**71** N-[(3,4-Difluor-phenyl)-methyl]-2-[3-(4-fluorphenyl)-propylsulfanyl]-pyridin-3-carbonsäure amid
**72** N-(2,3-Dihydro-benzofuran-5-yl-methyl)-2-[3-(4-fluorphenyl)-propylsulfanyl]-pyridin-3-carbonsäure amid
**73** N-(5-Bicyclo[2.2.1]heptanyl-methyl)-2-[3-(3-hydroxyphenyl)-propylsulfanyl]-pyridin-3-carbonsäure amid
**74** N-(5-Bicyclo[2.2.1]heptanyl-methyl)-2-[[3-(4-fluorphenyl)-1-methyl-propyl]sulfanyl]-pyridin-3-carbonsäure amid
**75** N-(5-Bicyclo[2.2.1]heptanyl-methyl)-2-[[3-(4-fluorphenyl)-2-methyl-propyl]sulfanyl]-pyridin-3-carbonsäure amid
**76** 2-[3-(4-Fluorphenyl)-propylsulfanyl]-N-[(2-methoxyphenyl)-methyl]-pyridin-3-carbonsäure amid
**77** 2-[3-(4-Fluorphenyl)-propylsulfanyl]-N-[(3-methoxyphenyl)-methyl]-pyridin-3-carbonsäure amid
**78** 2-[3-(4-Fluorphenyl)-propylsulfanyl]-N-phenethyl-pyridin-3-carbonsäure amid
**79** 2-[3-(4-Fluorphenyl)-propylsulfanyl]-N-[[4-(trifluormethyloxy)-phenyl]-methyl]-pyridin-3-carbonsäure amid
**80** 2-[3-(4-Fluorphenyl)-propylsulfanyl]-N-[[4-(trifluormethyl)-phenyl]-methyl]-pyridin-3-carbonsäure amid
**81** 2-[3-(4-Fluorphenyl)-propylsulfanyl]-N-(2-pyridin-3-yl-ethyl)-pyridin-3-carbonsäure amid
**82** 2-[3-(4-Fluorphenyl)-propylsulfanyl]-N-(2-pyridin-2-yl-ethyl)-pyridin-3-carbonsäure amid
**83** 2-[3-(4-Fluorphenyl)-propylsulfanyl]-N-[(2-hydroxyphenyl)-methyl]-pyridin-3-carbonsäure amid
**84** 2-[3-(4-Fluorphenyl)-propylsulfanyl]-N-[(3-hydroxyphenyl)-methyl]-pyridin-3-carbonsäure amid
**85** 2-[3-(4-Fluorphenyl)-propylsulfanyl]-N-[2-(m-tolyl)-ethyl]-pyridin-3-carbonsäure amid
**86** 2-[3-(4-Fluorphenyl)-propylsulfanyl]-N-[2-(o-tolyl)-ethyl]-pyridin-3-carbonsäure amid
**87** 2-[3-(4-Fluorphenyl)-propylsulfanyl]-N-[[3-(trifluormethyloxy)-phenyl]-methyl]-pyridin-3-carbonsäure amid
**88** 2-[3-(4-Fluorphenyl)-propylsulfanyl]-N-[(4-hydroxyphenyl)-methyl]-pyridin-3-carbonsäure amid
**89** 2-[3-(4-Fluorphenyl)-propylsulfanyl]-N-(2-pyridin-4-yl-ethyl)-pyridin-3-carbonsäure amid
**90** 2-[3-(4-Fluorphenyl)-propylsulfanyl]-N-[2-(p-tolyl)-ethyl]-pyridin-3-carbonsäure amid
**91** N-(5-Bicyclo[2.2.1]heptanyl-methyl)-2-[3-(4-fluorphenyl)-butylsulfanyl]-pyridin-3-carbonsäure amid
**92** N-(5-Bicyclo[2.2.1]heptanyl-methyl)-2-[3-(2,4,5-trifluor-phenyl)-propylsulfanyl]-pyridin-3-carbonsäure amid
**93** 2-[3-(4-Fluorphenyl)-propylsulfanyl]-N-(3-pyridin-2-yl-propyl)-pyridin-3-carbonsäure amid
**94** 2-[3-(4-Fluorphenyl)-propylsulfanyl]-N-(3-pyridin-3-yl-propyl)-pyridin-3-carbonsäure amid
**95** 2-[3-(4-Fluorphenyl)-propylsulfanyl]-N-(3-pyridin-4-yl-propyl)-pyridin-3-carbonsäure amid
**96** 2-[3-(4-Fluorphenyl)-propylsulfanyl]-N-propyl-pyridin-3-carbonsäure amid
**97** N-Butyl-2-[3-(4-fluorphenyl)-propylsulfanyl]-pyridin-3-carbonsäure amid
**98** 2-(3-Pyridin-3-yl-propylsulfanyl)-N-(thiophen-2-yl-methyl)-pyridin-3-carbonsäure amid
**99** 2-[3-(p-Tolyl)-propylsulfanyl]-N-(thiophen-2-yl-methyl)-pyridin-3-carbonsäure amid
**100** 2-(4-Phenyl-butylsulfanyl)-N-(thiophen-2-yl-methyl)-pyridin-3-carbonsäure amid
**101** 2-(3-Pyridin-4-yl-propylsulfanyl)-N-(thiophen-2-yl-methyl)-pyridin-3-carbonsäure amid
**102** 2-(3-Naphthalen-2-yl-propylsulfanyl)-N-(thiophen-2-yl-methyl)-pyridin-3-carbonsäure amid
**103** 2-[3-(m-Tolyl)-propylsulfanyl]-N-(thiophen-2-yl-methyl)-pyridin-3-carbonsäure amid
**104** N-(Thiophen-2-yl-methyl)-2-(3-thiophen-2-yl-propylsulfanyl)-pyridin-3-carbonsäure amid
**105** 2-[(1-Methyl-3-phenyl-propyl)sulfanyl]-N-(thiophen-2-yl-methyl)-pyridin-3-carbonsäure amid
**106** N-(Thiophen-2-yl-methyl)-2-(3-thiophen-3-yl-propylsulfanyl)-pyridin-3-carbonsäure amid
**107** 2-[[1-Methyl-3-[3-(trifluormethyl)phenyl]-propyl]sulfanyl]-N-(thiophen-2-yl-methyl)-pyridin-3-carbonsäure amid
**108** 2-[(2-Benzyl-cyclohexyl)sulfanyl]-N-(thiophen-2-yl-methyl)-pyridin-3-carbonsäure amid
**109** 2-[3-[3-Methyl-5-(trifluormethyl)-phenyl]-propylsulfanyl]-N-(thiophen-2-yl-methyl)-pyridin-3-carbonsäure amid
**110** 2-[4-(3,4-Difluor-phenyl)-butylsulfanyl]-N-(thiophen-2-yl-methyl)-pyridin-3-carbonsäure amid
**111** 2-(3-Pyridin-2-yl-propylsulfanyl)-N-(thiophen-2-yl-methyl)-pyridin-3-carbonsäure amid
**113** 2-[3-[4-Methyl-3-(trifluormethyl)-phenyl]-propylsulfanyl]-N-(thiophen-2-yl-methyl)-pyridin-3-carbonsäure amid
**114** 2-[(3-Phenyl-cyclohexyl)sulfanyl]-N-(thiophen-2-yl-methyl)-pyridin-3-carbonsäure amid
**116** 2-[[3,3-Difluor-3-(4-fluorphenyl)-propyl]sulfanyl]-N-(3,3-dimethyl-butyl)-pyridin-3-carbonsäure amid
**117** N-(Cycloheptyl-methyl)-2-[[3,3-difluor-3-(4-fluorphenyl)-propyl]sulfanyl]-pyridin-3-carbonsäure amid
**118** 2-[[3,3-Difluor-3-(4-fluorphenyl)-propyl]sulfanyl]-N-(thiophen-2-yl-methyl)-pyridin-3-carbonsäure amid
**119** 2-[3-(4-Fluorphenyl)-propylsulfanyl]-N-(1,2,3,4-tetrahydro-naphthalen-2-yl-methyl)-pyridin-3-carbonsäure amid
**120** N-(2,3-Dihydro-1H-inden-2-yl-methyl)-2-[3-(4-fluorphenyl)-propylsulfanyl]-pyridin-3-carbonsäure amid
**121** N-(1,3-Benzodioxol-5-yl-methyl)-2-[3-(4-fluorphenyl)-propylsulfanyl]-pyridin-3-carbonsäure amid
**122** 2-[3-(4-Fluorphenyl)-propylsulfanyl]-N-[(3-phenyl-phenyl)-methyl]-pyridin-3-carbonsäure amid
**123** 2-[3-(4-Fluorphenyl)-propylsulfanyl]-N-[(3-methyl-cyclohexyl)-methyl]-pyridin-3-carbonsäure amid
**124** 2-[[3,3-Difluor-3-(4-fluorphenyl)-propyl]sulfanyl]-N-[(4-fluorphenyl)-methyl]-pyridin-3-carbonsäure amid
**125** N-(3,3-Dimethyl-2-oxo-butyl)-2-[3-(4-fluorphenyl)-propylsulfanyl]-pyridin-3-carbonsäure amid

**127** 2-[3-(4-Fluorphenyl)-propylsulfanyl]-N-(pyridin-3-yl-methyl)-pyridin-3-carbonsäure amid
**128** 2-[3-(4-Fluorphenyl)-propylsulfanyl]-N-(pyridin-4-yl-methyl)-pyridin-3-carbonsäure amid
**129** 3-[[[2-[3-(4-Fluorphenyl)-propylsulfanyl]-pyridin-3-carbonyl]amino]-methyl]-benzoesäure methyl ester
**130** 2-[3-(4-Fluorphenyl)-propylsulfanyl]-N-[3-(2-methoxyphenyl)-propyl]-pyridin-3-carbonsäure amid
**132** N-[(4-Fluorphenyl)-methyl]-2-[[1-methyl-3-[3-(trifluormethyl)phenyl]-propyl]sulfanyl]-pyridin-3-carbonsäure amid
**133** 2-[3-(4-Fluorphenyl)-propylsulfanyl]-N-(3-methyl-butyl)-pyridin-3-carbonsäure amid
**134** 2-[3-(4-Fluorphenyl)-propylsulfanyl]-N-(tetrahydro-pyran-2-yl-methyl)-pyridin-3-carbonsäure amid
**135** 2-[3-(4-Fluorphenyl)-propylsulfanyl]-N-[3-(1 H-pyrazol-1-yl)-propyl]-pyridin-3-carbonsäure amid
**136** 2-[3-(4-Fluorphenyl)-propylsulfanyl]-N-(naphthalen-2-yl-methyl)-pyridin-3-carbonsäure amid
**137** N-(2,3-Dihydro-[1,4]benzodioxin-6-yl-methyl)-2-[3-(4-fluorphenyl)-propylsulfanyl]-pyridin-3-carbonsäure amid
**138** 2-[3-(4-Fluorphenyl)-propylsulfanyl]-N-[(3-morpholin-4-yl-phenyl)-methyl]-pyridin-3-carbonsäure amid
**139** N-(2,3-Dihydro-benzofuran-6-yl-methyl)-2-[3-(4-fluorphenyl)-propylsulfanyl]-pyridin-3-carbonsäure amid
**140** 2-[3-(4-Fluorphenyl)-propylsulfanyl]-N-[[3-(1H-pyrazol-1-yl)-phenyl]-methyl]-pyridin-3-carbonsäure amid
**141** 2-[3-(4-Fluorphenyl)-propylsulfanyl]-N-[3-(1H-[1,2,3]triazol-1-yl)-propyl]-pyridin-3-carbonsäure amid
**142** N-(7-Bicyclo[2.2.1]heptanyl-methyl)-2-[3-(4-fluorphenyl)-propylsulfanyl]-pyridin-3-carbonsäure amid
**144** 2-[3-(4-Fluorphenyl)-propylsulfanyl]-N-(thiazol-2-yl-methyl)-pyridin-3-carbonsäure amid
**145** 2-[3-(4-Fluorphenyl)-propylsulfanyl]-N-(thiazol-5-yl-methyl)-pyridin-3-carbonsäure amid
**146** 2-[3-(4-Fluorphenyl)-propylsulfanyl]-N-(oxazol-2-yl-methyl)-pyridin-3-carbonsäure amid
**148** N-(3,3-Dimethyl-butyl)-2-[3-(4-fluorphenyl)-propylsulfanyl]-4-methyl-pyridin-3-carbonsäure amid
**149** N-(3,3-Dimethyl-butyl)-2-[[1-methyl-3-[3-(trifluormethyl)phenyl]-propyl]sulfanyl]-pyridin-3-carbonsäure amid
**150** 2-[3-(4-Fluorphenyl)-propylsulfanyl]-N-(chinolin-7-yl-methyl)-pyridin-3-carbonsäure amid
**151** 2-[3-(4-Fluorphenyl)-propylsulfanyl]-N-[(3-pyridin-2-yl-phenyl)-methyl]-pyridin-3-carbonsäure amid
**152** 2-[3-(4-Fluorphenyl)-propylsulfanyl]-N-[(3-pyridin-3-yl-phenyl)-methyl]-pyridin-3-carbonsäure amid
**153** N-(3,3-Dimethyl-butyl)-2-[[(1R)-1-methyl-3-phenyl-propyl]sulfanyl]-pyridin-3-carbonsäure amid
**154** N-(3,3-Dimethyl-butyl)-2-[[(1S)-1-methyl-3-phenyl-propyl]sulfanyl]-pyridin-3-carbonsäure amid
**155** 2-[(2-Benzyl-cyclopentyl)sulfanyl]-N-(3,3-dimethyl-butyl)-pyridin-3-carbonsäure amid
**156** N-(7-Bicyclo[2.2.1]heptanyl-methyl)-2-[3-[3-(trifluormethyl)phenyl]-propylsulfanyl]-pyridin-3-carbonsäure amid
**157** 2-[3-(4-Fluorphenyl)-propylsulfanyl]-N-[3-(1H-[1,2,4]triazol-1-yl)-propyl]-pyridin-3-carbonsäure amid
**158** 2-[3-(4-Fluorphenyl)-propylsulfanyl]-N-([1,3,4]oxadiazol-2-yl-methyl)-pyridin-3-carbonsäure amid
**159** 2-[3-(4-Fluorphenyl)-propylsulfanyl]-N-[(3-pyridin-4-yl-phenyl)-methyl]-pyridin-3-carbonsäure amid
**160** N-[[4-(Cyclopropyl-methyl)-3,4-dihydro-2H-[1,4]benzoxazin-6-yl]-methyl]-2-[3-(4-fluorphenyl)-propylsulfanyl]-pyridin-3-carbonsäure amid
**161** N-[(4-Ethyl-3,4-dihydro-2H-[1,4]benzoxazin-6-yl)-methyl]-2-[3-(4-fluorphenyl)-propylsulfanyl]-pyridin-3-carbonsäure amid
**162** 2-[3-(4-Fluorphenyl)-propylsulfanyl]-N-[(4-methyl-3,4-dihydro-2H-[1,4]benzoxazin-6-yl)-methyl]-pyridin-3-carbonsäure amid
**163** 2-[3-(4-Fluorphenyl)-propylsulfanyl]-N-(3-methyl-3-phenyl-butyl)-pyridin-3-carbonsäure amid
**164** N-(7-Bicyclo[2.2.1]heptanyl-methyl)-2-[[3,3-difluor-3-(4-fluorphenyl)-propyl]sulfanyl]-pyridin-3-carbonsäure amid
**165** 2-[[3,3-Difluor-3-[3-(trifluormethyl)phenyl]-propyl]sulfanyl]-N-[(4-fluorphenyl)-methyl]-pyridin-3-carbonsäure amid
**166** 2-[[3,3-Difluor-3-[3-(trifluormethyl)phenyl]-propyl]sulfanyl]-N-(3,3-dimethyl-butyl)-pyridin-3-carbonsäure amid
**168** 2-[[3,3-Difluor-3-(4-fluorphenyl)-propyl]sulfanyl]-N-[(3-fluorphenyl)-methyl]-pyridin-3-carbonsäure amid
**170** N-Butyl-2-[[3,3-difluor-3-(4-fluorphenyl)-propyl]sulfanyl]-pyridin-3-carbonsäure amid
**171** 2-[[3,3-Difluor-3-(4-fluorphenyl)-propyl]sulfanyl]-N-(2-methoxy-ethyl)-pyridin-3-carbonsäure amid
**172** 2-[3-(4-Fluorphenyl)-propylsulfanyl]-N-(2-methoxy-ethyl)-pyridin-3-carbonsäure amid
**173** N-[(4-Fluor-2-hydroxy-phenyl)-methyl]-2-[3-(4-fluorphenyl)-propylsulfanyl]-pyridin-3-carbonsäure amid
**174** 2-[3-(4-Fluorphenyl)-propylsulfanyl]-N-(furan-2-yl-methyl)-pyridin-3-carbonsäure amid
**175** 2-[3-(4-Fluorphenyl)-propylsulfanyl]-N-[(5-methyl-furan-2-yl)-methyl]-pyridin-3-carbonsäure amid
**176** N-[(4-Fluorphenyl)-methyl]-2-[[3-(4-fluorphenyl)-1-methyl-propyl]sulfanyl]-pyridin-3-carbonsäure amid
**177** 2-[[3-(4-Fluorphenyl)-1-methyl-propyl]sulfanyl]-N-(3-methyl-butyl)-pyridin-3-carbonsäure amid
**178** N-(7-Bicyclo[2.2.1]heptanyl-methyl)-2-[[3-(4-fluorphenyl)-1-methyl-propyl]sulfanyl]-pyridin-3-carbonsäure amid
**179** 2-[[3,3-Difluor-3-(4-fluorphenyl)-propyl]sulfanyl]-N-(tetrahydro-furan-2-yl-methyl)-pyridin-3-carbonsäure amid
**180** 2-[[3,3-Difluor-3-(4-fluorphenyl)-propyl]sulfanyl]-N-(tetrahydro-pyran-2-yl-methyl)-pyridin-3-carbonsäure amid
**181** 2-[3-(4-Fluorphenyl)-propylsulfanyl]-N-(tetrahydro-furan-2-yl-methyl)-pyridin-3-carbonsäure amid
**182** 2-[3-(4-Fluorphenyl)-propylsulfanyl]-N-(2-methoxy-3,3-dimethyl-butyl)-pyridin-3-carbonsäure amid
**183** 2-[[3,3-Difluor-3-(4-fluorphenyl)-propyl]sulfanyl]-N-(m-tolyl-methyl)-pyridin-3-carbonsäure amid

**184** 2-[[3,3-Difluor-3-(4-fluorphenyl)-propyl]sulfanyl]-N-[(3,5-dimethyl-phenyl)-methyl]-pyridin-3-carbonsäure amid
**185** 2-[[3,3-Difluor-3-(4-fluorphenyl)-propyl]sulfanyl]-N-propyl-pyridin-3-carbonsäure amid
**186** 2-[[3,3-Difluor-3-(4-fluorphenyl)-propyl]sulfanyl]-N-hexyl-pyridin-3-carbonsäure amid
**187** 2-[[3,3-Difluor-3-(4-fluorphenyl)-propyl]sulfanyl]-N-(tetrahydro-furan-3-yl-methyl)-pyridin-3-carbonsäure amid
**188** 2-[[3,3-Difluor-3-(4-fluorphenyl)-propyl]sulfanyl]-N-(tetrahydro-pyran-3-yl-methyl)-pyridin-3-carbonsäure amid
**189** 2-[[3,3-Difluor-3-(4-fluorphenyl)-propyl]sulfanyl]-N-(tetrahydro-pyran-4-yl-methyl)-pyridin-3-carbonsäure amid
**190** 2-[[3,3-Difluor-3-(4-fluorphenyl)-propyl]sulfanyl]-N-(furan-2-yl-methyl)-pyridin-3-carbonsäure amid
**191** 2-[[3,3-Difluor-3-(4-fluorphenyl)-propyl]sulfanyl]-N-[(5-methyl-furan-2-yl)-methyl]-pyridin-3-carbonsäure amid
**192** 2-[[3,3-Difluor-3-(4-fluorphenyl)-propyl]sulfanyl]-N-pentyl-pyridin-3-carbonsäure amid
**193** 2-[[3,3-Difluor-3-(4-fluorphenyl)-propyl]sulfanyl]-N-(3-methoxy-butyl)-pyridin-3-carbonsäure amid
**194** 2-[[3,3-Difluor-3-(4-fluorphenyl)-propyl]sulfanyl]-N-(2-methoxy-propyl)-pyridin-3-carbonsäure amid
**195** 2-[[3,3-Difluor-3-(4-fluorphenyl)-propyl]sulfanyl]-N-(2-methoxy-butyl)-pyridin-3-carbonsäure amid
**196** 3-[[2-[[3,3-Difluor-3-(4-fluorphenyl)-propyl]sulfanyl]-pyridin-3-carbonyl]amino]-propionsäure methyl ester
**197** 3-[[2-[[3,3-Difluor-3-(4-fluorphenyl)-propyl]sulfanyl]-pyridin-3-carbonyl]amino]-propionsäure
**198** 2-[[3,3-Difluor-3-(4-fluorphenyl)-propyl]sulfanyl]-N-(2-dimethylaminoethyl)-pyridin-3-carbonsäure amid
**199** 2-[[3-(3,4-Difluor-phenyl)-1-methyl-propyl]sulfanyl]-N-[(4-fluorphenyl)-methyl]-pyridin-3-carbonsäure amid
**200** 2-[[3-(3,4-Difluor-phenyl)-1-methyl-propyl]sulfanyl]-N-(3-methyl-butyl)-pyridin-3-carbonsäure amid
**201** N-[(4-Fluorphenyl)-methyl]-2-[[3-(3-fluorphenyl)-1-methyl-propyl]sulfanyl]-pyridin-3-carbonsäure amid
**202** 2-[[3-(3-Fluorphenyl)-1-methyl-propyl]sulfanyl]-N-(3-methyl-butyl)-pyridin-3-carbonsäure amid
**203** N-(3-Methyl-butyl)-2-[[1-methyl-3-[3-(trifluormethyl)phenyl]-propyl]sulfanyl]-pyridin-3-carbonsäure amid
**204** 2-[[3-(3,4-Difluor-phenyl)-3,3-difluor-propyl]sulfanyl]-N-[(4-fluorphenyl)-methyl]-pyridin-3-carbonsäure amid
**205** N-(1-Bicyclo[2.2.1]heptanyl-methyl)-2-[[3,3-difluor-3-(4-fluorphenyl)-propyl]sulfanyl]-pyridin-3-carbonsäure amid
**206** N-(1-Bicyclo[2.2.1]heptanyl-methyl)-2-[3-(4-fluorphenyl)-propylsulfanyl]-pyridin-3-carbonsäure amid
**207** N-[(4-Fluorphenyl)-methyl]-2-[3-[3-(trifluormethyl)phenyl]-propylsulfanyl]-pyridin-3-carbonsäure amid
**208** N-(3-Methyl-butyl)-2-[3-[3-(trifluormethyl)phenyl]-propylsulfanyl]-pyridin-3-carbonsäure amid
**209** 2-[[3-(3,4-Difluor-phenyl)-3,3-difluor-propyl]sulfanyl]-N-(3-methyl-butyl)-pyridin-3-carbonsäure amid
**210** N-(7-Bicyclo[2.2.1]heptanyl-methyl)-2-[[3-(3,4-difluor-phenyl)-3,3-difluor-propyl]sulfanyl]-pyridin-3-carbonsäure amid
**211** 2-[[3,3-Difluor-3-(4-fluorphenyl)-propyl]sulfanyl]-N-(2-hydroxy-ethyl)-pyridin-3-carbonsäure amid
**224** N-[(4-Fluor-2-methoxy-phenyl)-methyl]-2-[3-(4-fluorphenyl)-propylsulfanyl]-pyridin-3-carbonsäure amid

oder deren physiologisch verträgliche Salze.

Die erfindungsgemäßen substituierten Nicotinamide sowie jeweils die entsprechenden Säuren, Basen, Salze und Solvate eignen sich als pharmazeutische Wirkstoffe in Arzneimitteln.

Ein weiterer Gegenstand der Erfindung ist daher ein Arzneimittel enthaltend wenigstens ein erfindungsgemäßes substituiertes Nicotinamid der allgemeinen Formel (1), worin die Reste $R^1$-$R^{13}$ die oben angegebene Bedeutung haben sowie ggf. einen oder mehrere pharmazeutisch verträgliche Hilfsstoffe.

Die erfindungsgemäßen Arzneimittel enthalten neben mindestens einer erfindungsgemäßen Verbindung ggf. geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/ Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen in einem Depot, in gelöster Form oder in einem Pflaster, ggf. unter Zusatz von die Hautpenetration fördernden Mitteln, sind geeignete perkutane Applikationszubereitungen. Oral oder perkutan anwendbare Zubereitungsformen können die erfindungsgemäßen Verbindungen verzögert freisetzen. Die erfindungsgemäßen Verbindungen können auch in parenteralen Langzeitdepotformen wie z.B. Implantaten oder implantierten Pumpen angewendet werden. Prinzipiell können den erfindungsgemäßen Arzneimitteln andere dem Fachmann bekannte weitere Wirkstoffe zugesetzt werden.

Diese erfindungsgemäßen Arzneimittel eignen sich zur Beeinflussung von KCNQ2/3-Kanälen und üben eine agonistische oder antagonistische, insbesondere eine agonistische Wirkung aus.

Bevorzugt eignen sich die erfindungsgemäßen Arzneimittel zur Behandlung von Störungen oder Krankheiten, die zumindest teilweise durch KCNQ2/3-Kanäle vermittelt werden.

Bevorzugt eignet sich die erfindungsgemäßen Arzneimittel zur Behandlung von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz, muskulärem Schmerz und inflammatorischen Schmerz; Epilepsie, Harninkontinenz, Angstzuständen, Abhängigkeit, Manie, bipolaren Störungen, Migräne, kognitiven Erkrankungen, Dystonie-assoziierten Dyskinesien und/oder Harninkontinenz.

Besonders bevorzugt eignen sich die erfindungsgemäßen Arzneimittel zur Behandlung von Schmerz, ganz besonders bevorzugt von chronischem Schmerz, neuropathischem Schmerz, inflammatorischem Schmerz und muskulärem Schmerz. Weiterhin eignen sich die erfindungsgemäßen Arzneimittel besonders bevorzugt zur Behandlung von Epilepsie.

Ein weiterer Gegenstand der Erfindung ist die Verwendung wenigstens eines erfindungsgemäßen substituierten Nicotinamids sowie ggf. eines oder mehrerer pharmazeutisch verträglicher Hilfsstoffe zur Herstellung eines Arzneimittels zur Behandlung von Störungen oder Krankheiten, die zumindest teilweise durch KCNQ2/3-Kanäle vermittelt werden.

Bevorzugt ist die Verwendung wenigstens eines erfindungsgemäßen substituierten Nicotinamids sowie ggf. eines oder mehrerer pharmazeutisch verträglicher Hilfsstoffe zur Herstellung eines Arzneimittels zur Behandlung von Schmerz, vorzugsweise von Schmerz ausgewählt aus der Gruppe bestehend aus akutem Schmerz, chronischem Schmerz, neuropathischem Schmerz, muskulärem Schmerz und inflammatorischen Schmerz; Epilepsie, Harninkontinenz, Angstzuständen, Abhängigkeit, Manie, bipolaren Störungen, Migräne, kognitiven Erkrankungen, Dystonie-assoziierten Dyskinesien und/oder Harninkontinenz.

Besonders bevorzugt ist die Verwendung wenigstens eines erfindungsgemäßen substituierten Nicotinamids sowie ggf. eines oder mehrerer pharmazeutisch verträglicher Hilfsstoffe zur Herstellung eines Arzneimittels zur Behandlung von Schmerz, ganz besonders bevorzugt von chronischem Schmerz, neuropathischem Schmerz, inflammatorischem Schmerz und muskulärem Schmerz.

Weiterhin besonders bevorzugt ist die Verwendung wenigstens eines erfindungsgemäßen substituierten Nicotinamids sowie ggf. eines oder mehrerer pharmazeutisch verträglicher Hilfsstoffe zur Herstellung eines Arzneimittels zur Behandlung von Epilepsie.

Ein weiterer Gegenstand der Erfindung ist wenigstens ein erfindungsgemäßes substituiertes Nicotinamid sowie ggf. ein oder mehrere pharmazeutisch verträgliche Hilfsstoffe zur Behandlung von Störungen oder Krankheiten, die zumindest teilweise durch KCNQ2/3-Kanäle vermittelt werden.

Ein weiterer Gegenstand der Erfindung ist wenigstens ein erfindungsgemäßes substituiertes Nicotinamid sowie ggf. ein oder mehrere pharmazeutisch verträgliche Hilfsstoffe zur Behandlung von Schmerz, vorzugsweise von Schmerz ausgewählt aus der Gruppe bestehend aus akutem Schmerz, chronischem Schmerz, neuropathischem Schmerz, muskulärem Schmerz und inflammatorischen Schmerz; Epilepsie, Harninkontinenz, Angstzuständen, Abhängigkeit, Manie, bipolaren Störungen, Migräne, kognitiven Erkrankungen, Dystonie-assoziierten Dyskinesien und/oder Harninkontinenz.

Besonders bevorzugt ist wenigstens ein erfindungsgemäßes substituiertes Nicotinamid sowie ggf. ein oder mehrere pharmazeutisch verträgliche Hilfsstoffe zur Behandlung von Schmerz, ganz besonders bevorzugt von chronischem Schmerz, neuropathischem Schmerz, inflammatorischem Schmerz und muskulärem Schmerz.

Besonders bevorzugt ist auch wenigstens ein erfindungsgemäßes substituiertes Nicotinamid sowie ggf. ein oder mehrere pharmazeutisch verträgliche Hilfsstoffe zur Behandlung von Epilepsie.

Die Wirksamkeit gegen Schmerz kann beispielsweise im Bennett- bzw. Chung-Modell gezeigt werden (Bennett, G.J. and Xie, Y.K., A peripheral mononeuropathy in rat that produces disorders of pain sensation like those seen in man, Pain 1988, 33(1), 87-107; Kim, S.H. and Chung, J.M., An experimental model for peripheral neuropathy produced by segmental spinal nerve ligation in the rat, Pain 1992, 50(3), 355-363). Die Wirksamkeit gegen Epilepsie kann beispielsweise im DBA/2 Maus Modell (De Sarro et al., Naunyn-Schmiedeberg's Arch. Pharmacol. 2001, 363, 330-336) nachgewiesen werden.

Bevorzugt weisen die erfindungsgemäßen substituierten Nicotinamide einen $EC_{50}$-Wert von höchstens 5 μM oder höchstens 3 μM auf, bevorzugter höchstens 2 μM oder höchstens 1 μM, noch bevorzugter höchstens 0,9 μM oder höchstens 0,6 μM, am bevorzugtesten höchstens 0,5 μM oder höchstens 0,3 μM und insbesondere höchstens 0,2 μM oder höchstens 0,1 μM. Methoden zur Bestimmung des $EC_{50}$-Wertes sind dem Fachmann bekannt. Bevorzugt erfolgt die Bestimmung des $EC_{50}$-Wertes fluorimetrisch, besonders bevorzugt wie unter "Pharmakologische Experimente" beschrieben.

Ein weiterer Gegenstand der Erfindung sind Verfahren zur Herstellung der erfindungsgemäßen substituierten Nicotinamide.

Die in den nachstehend beschriebenen Umsetzungen zum Einsatz kommenden Chemikalien und Reaktionskomponenten sind käuflich erhältlich oder können jeweils nach üblichen, dem Fachmann bekannten Methoden hergestellt werden.

__Allgemeine Reaktionsschemata__

## Schema 1:

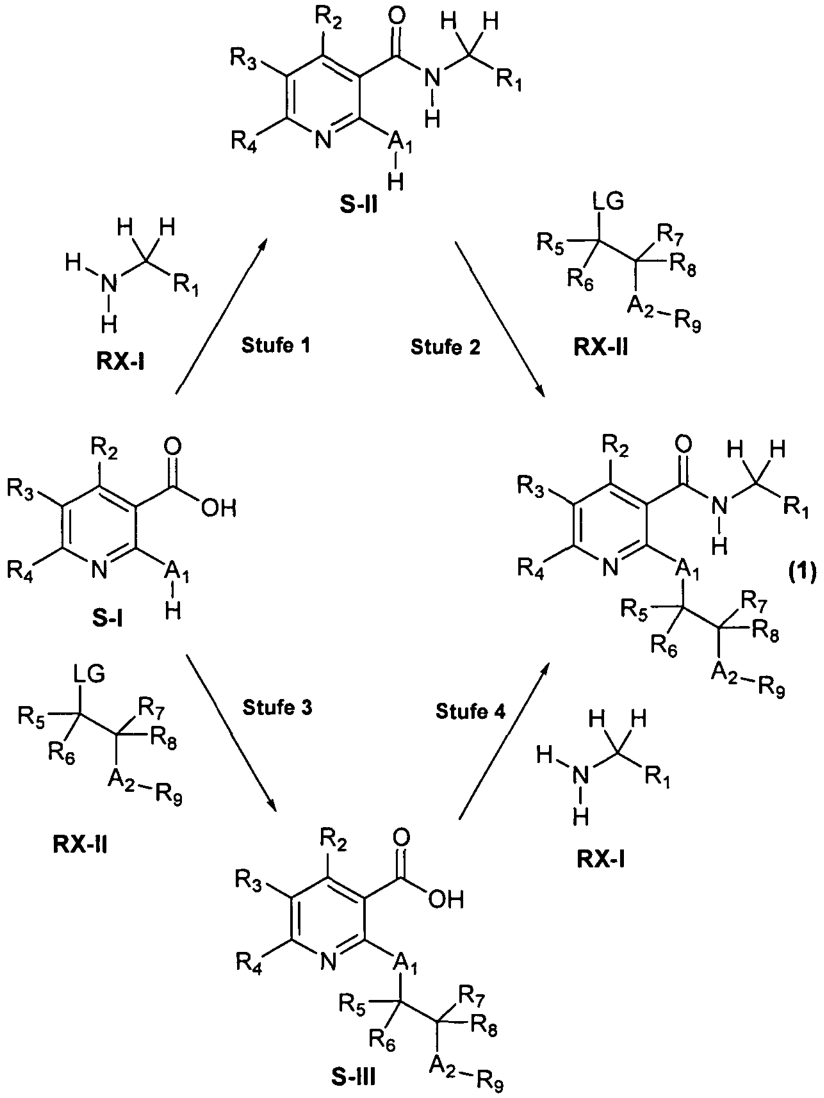


In **Stufe 1** werden Säuren der allgemeinen Formel **S-I** mit Aminen der allgemeinen Formel **RX-I** in einem Reaktionsmedium, vorzugsweise ausgewählt aus der Gruppe bestehend aus Ether, THF, MeCN, MeOH, EtOH, DMF und DCM ggf. in Gegenwart wenigstens eines Kupplungsreagenzes, vorzugsweise ausgewählt aus der Gruppe bestehend aus BOP, DCC, EDC, HATU, HBTU und HOAt, ggf. in Gegenwart wenigstens einer anorganischen Base, vorzugsweise ausgewählt aus der Gruppe bestehend aus Kaliumcarbonat und Cäsiumcarbonat, oder einer organischen Base, vorzugsweise ausgewählt aus der Gruppe bestehend aus $NEt_3$ , Pyridin, DMAP und DIPEA vorzugsweise bei Temperaturen von -70°C bis 100°C, ggf. in Gegenwart von Mikrowellenstrahlung zu Amiden der allgemeinen Formel **S-II** umgesetzt.

Alternativ werden gemäß **Stufe 1** die Säuren allgemeinen Formel **S-I** zunächst mit Reagenzien beispielsweise ausgewählt aus der Gruppe $(COCl)_2$, $PCl_3$, $POCl_3$ oder $SOCl_2$ in einem Reaktionsmedium, vorzugsweise ausgewählt aus der Gruppe bestehend aus Toluol, DMF, DCM, oder Pyridin bei Temperaturen von -70°C bis 100°C, ggf. in Gegenwart von Mikrowellenstrahlung in die entsprechenden Säurechloride überführt und dann mit Aminen der allgemeinen Formel RX-I in einem Reaktionsmedium, vorzugsweise ausgewählt aus der Gruppe bestehend aus Ether, THF, MeCN, MeOH, EtOH, DMF und DCM, mit oder ohne Zusatz wenigstens einer organischen oder anorganischen Base, beispielsweise $NEt_3$, DMAP, Pyridin oder DIPEA, ggf. in Gegenwart wenigstens einer organischen Base, vorzugsweise ausgewählt aus der Gruppe bestehend aus $NEt_3$, DMAP, Pyridin und DIPEA, oder einer anorganischen Base bei Temperaturen von vorzugsweise -70°C bis 100°C, ggf. in Gegenwart von Mikrowellenstrahlung zu Amiden der allgemeinen Formel **S-II** umgesetzt.

In **Stufe 2** werden Verbindungen der allgemeinen Formel **S-II** mit Verbindungen der allgemeinen Formel **RX-II**, worin LG für eine Abgangsgruppe, vorzugweise für Chlor, Brom oder lod steht, in einem Reaktionsmedium, vorzugsweise ausgewählt aus der Gruppe bestehend aus Aceton, Ethanol, Ether, Methanol, THF, MeCN, Toluol und DMF, ggf. in Gegenwart wenigstens einer anorganischen Base, vorzugsweise ausgewählt aus der Gruppe bestehend aus Kaliumcarbonat, Butyllithium, Natriumhydrid, Natriummethylat oder Kalium-tert.butylat, oder einer organischen Base, vorzugsweise ausgewählt aus der Gruppe bestehend aus $NEt_3$, Pyridin, DMAP und DIPEA vorzugsweise bei Temperaturen von -70°C bis 100°C, ggf. in Gegenwart von Mikrowellenstrahlung zu Verbindungen der allgemeinen Formel **(1)** umgesetzt.

In **Stufe 3** werden Säuren der allgemeinen Formel **S-I** mit Verbindungen der allgemeinen Formel **RX-II**, worin LG für eine Abgangsgruppe, vorzugweise für Chlor, Brom oder lod steht, nach dem in **Stufe 2** beschriebenen Verfahren zu Verbindungen der allgemeinen Formel **S-III** umgesetzt.

In **Stufe 4** werden Säuren der allgemeinen Formel **S-III** mit Aminen der allgemeinen Formel **RX-I** nach dem in **Stufe 1** beschriebenen Verfahren zu Verbindungen der allgemeinen Formel **(1)** umgesetzt.

**Schema 2 (nur für $A_1$ = S oder O):**

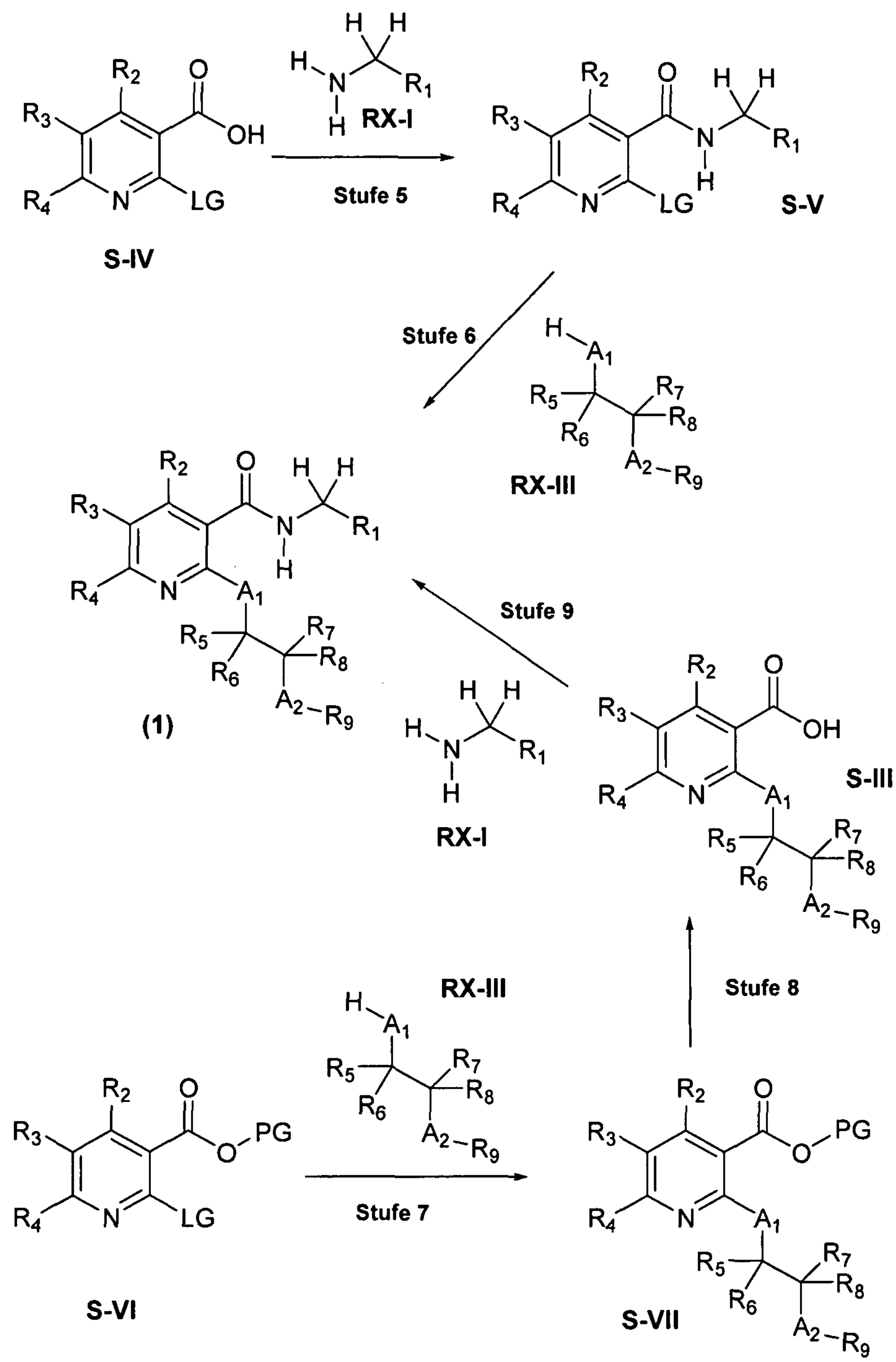


In **Stufe 5** werden Säuren der allgemeinen Formel **S-IV**, worin LG für eine Abgangsgruppe, vorzugsweise für ein Halogenid oder Methansulfonat, besonders bevorzugt für Chlor steht, mit Aminen der allgemeinen Formel **RX-I** nach den in **Stufe 1** beschriebenen Verfahren zu Verbindungen der allgemeinen Formel **S-V** umgesetzt.

In **Stufe 6** werden Verbindungen der allgemeinen Formel **S-V** mit Verbindungen der allgemeinen Formel **RX-III** worin $A_1$ für O oder S steht, in einem Reaktionsmedium, vorzugsweise Ether, THF, MeCN, Toluol und DMF, ggf. in Gegenwart wenigstens einer anorganischen Base, vorzugsweise ausgewählt aus der Gruppe bestehend aus Kaliumcarbonat, Natriumhydrid, Natriummethylat oder Kalium-tert.butylat, bei Temperaturen von -70°C bis 100°C, ggf. in Gegenwart von Mikrowellenstrahlung zu Verbindungen der allgemeinen Formel **(1)** umgesetzt.

In **Stufe 7** werden Verbindungen der allgemeinen Formel **S-VI**, worin LG für eine Abgangsgruppe, vorzugsweise für ein Halogenid oder Methansulfonat, besonders bevorzugt für Chlor, und PG für eine Schutzgruppe, vorzugsweise für Methyl oder Ethyl, stehen, mit Verbindungen der allgemeinen Formel **RX-III**, worin $A_1$ für S oder O steht, nach den in **Stufe 6** beschriebenen Verfahren zu Verbindungen der Formel **S-VII** umgesetzt.

In **Stufe 8** werden Ester der allgemeinen Formel **S-VII**, worin PG für eine Schutzgruppe, vorzugsweise für Methyl oder Ethyl steht, in einem Reaktionsmedium, vorzugsweise ausgewählt aus der Gruppe bestehend aus Ethanol, Methanol, MeCN, THF oder Wasser oder beliebigen Mischungen daraus, ggf. unter Zusatz einer anorganischen Base, vorzugsweise Kaliumhydroxid, Natriumhydroxid oder Lithiumhydroxid bei Temperatur von 0 °C bis 120 °C zu Säuren der allgemeinen Formel **S-III** gespalten.

Die Reaktion auf **Stufe 9** entspricht **Stufe 1** in Schema 1.

**Beschreibung der Synthesen**

**Abkürzungen**

| | |
|---|---|
| AcOH | Essigsäure |
| aq. | wässrig |
| d | Tage |
| BOP | 1-Benzotriazolyloxy-tris-(dimethylamino)-phosphonium hexafluorophosphat |
| Brine | gesättigte Natriumchloridlösung |
| DCC | N,N'-Dicyclohexylcarbodiimid |
| DCM | Dichlormethan |
| DIPEA | N,N-Diisopropylethylamin |
| DMF | N,N-Dimethylformamid |
| DMAP | 4-Dimethylamino-pyridin |
| EDC | N-(3-Dimethylaminopropyl)-N'-ethyl-carbodiimid |
| EE | Ethylacetat |
| Ether | Diethylether |
| EtOH | Ethanol |
| ges. | gesättigt |
| h | Stunde(n) |
| HATU | O-(7-Aza-Benzotriazol-1-yl)-N,N,N',N'-tetramethyluroniumhexafluorphosphat |
| HBTU | O-(Benzotriazol-1-yl)-N,N,N',N'-tetramethyluroniom hexafluorophosphat |
| HOAt | 1-Hydroxy-7-azabenzotriazol |
| Lsg. | Lösung |
| LG | Abgangsgruppe |
| m/z | Verhältnis Masse zur Ladung |
| MeCN | Acetonitril |
| MeOH | Methanol |
| min | Minuten |
| MS | Massenspektrometrie |
| N/A | nicht verfügbar |
| $NEt_3$ | Triethylamin |
| RG | Retigabine |
| RT | Raumtemperatur 23 ± 7 °C |
| SC | Säulenchromatographie auf Kieselgel |
| TBTU | O-(Benzotriazol-1-yl)-N,N,N',N'-tetramethyluroniom tertrafluoroborat |
| THF | Tetrahydrofuran |
| vv | Volumenverhältnis |

Alle nicht explizit beschriebenen Ausgangsstoffe waren entweder kommerziell verfügbar (Anbieter können beispielsweise in der Symyx® Available Chemicals Database der Firma MDL, San Ramon, US recherchiert werden) oder deren Synthese ist in der Fachliteratur bereits exakt beschrieben (Experimentelle Vorschriften können beispielweise in der Reaxys® Datenbank der Firma Elsevier, Amsterdam, NL recherchiert werden) oder können nach den dem Fachmann bekannten Methoden hergestellt werden.

Als stationäre Phase für die Säulenchromathographie (SC) wurde Kieselgel 60 (0.040 - 0.063 mm) eingesetzt.
Die analytische Charakterisierung aller Zwischenprodukte und Beispielverbindungen erfolgte mittels $^1H$-NMR-Spektroskopie. Zusätzlich wurden für alle Beispielverbindungen und ausgewählte Zwischenprodukte massenspektrometrische Untersuchungen (MS, Angabe m/z für $[M+H]^+$) durchgeführt.

<u>**Synthese der Zwischenprodukte**</u>

**Synthese des Zwischenprodukts VB006: 2-(4-(4-Fluorphenyl)butyl)-nicotinsäure**

Zu einer Lösung von 1,54 ml (11,0 mmol) Diisopropylamin in THF (10 ml) wurden bei -10 °C 4,4 ml (11,0 mmol, 2,5M in Hexan) BuLi-Lösung getropft. Anschließend wurde noch 30 min bei -10 °C gerührt. Diese Lösung wurde bei -75 °C in eine Lösung aus 685 mg (5,0 mmol) 2-Methyl-nicotinsäure in THF (2 ml) getropft. Anschließend wurde 30 min bei -75 °C und 60 min bei 0 °C nachgerührt und Dann auf -55 °C abgekühlt. Bei dieser Temperatur wurden 1,62 g (7,5 mmol) 1-(3-Brompropyl)-4-fluorbenzol zugegeben und die Reaktionslösung wurde 1 h bei -55 °C gerührt. Nach Erwärmen auf RT und weiteren 16 h Rühren bei RT wurde mit Wasser (30 ml) und konz. Salzsäure (3 ml) versetzt. Danach wurde mit EE extrahiert (2 x 30 ml). Die vereinten organischen Phasen wurden über $MgSO_4$ getrocknet, filtriert und im Vakuum eingeengt. Der Rückstand wurde mittels SC (EE) aufgereinigt, wobei 1,28 g (4,7 mmol, 93%) 2-(4-(4-Fluorphenyl)butyl)-nicotinsäure erhalten wurden.

**Synthese des Zwischenprodukts VB007: 2-(4-Phenylbutyl)nicotinsäure**

Ausgehend von 2,1 g (15,0 mmol) 2-Methyl-nicotinsäure und 5,5 g (22,5 mmol) 1-lod-3-phenyl-propan wurden nach dem bei Vorstufe VB006 beschriebenen Verfahren 2,9 g (11,3 mmol, 75%) 2-(4-Phenylbutyl)nicotinsäure erhalten.

**Synthese des Zwischenprodukts VB008: 2-(3-(Phenylsulfonyl)propyl)-nicotinsäure**

Ausgehend von 2,1 g (15,0 mmol) 2-Methyl-nicotinsäure und 4,6 g (22,5 mmol) (2-Chlorethyl)-phenylsulfon wurden nach dem bei Vorstufe VB006 beschriebenen Verfahren 0,7 g (2,4 mmol, 16%) 2-(3-(Phenylsulfonyl)propyl)-nicotinsäure erhalten.

**Synthese des Zwischenprodukts VC001: (2-Chlor-propan-1-yl)-(phenyl)sulfan / (1-Chloropropan-2-yl)(phenyl)sulfan (5:4 Mischung)**

a) Synthese von 1-(Phenylthio)propan-2-on

Eine Lösung von 5,1 ml (50,0 mmol) Thiophenol und 4,22 ml (52,5 mmol)
Chloraceton in DMF (30 ml) wurde mit 13,8 g (100,0 mmol) $K_2CO_3$ versetzt und 2 h bei RT gerührt. Anschließend wurde im Vakuum eingeengt. Der Rückstand wurde mittels SC (EE / Hex 15:85) aufgereinigt, wobei 7,7 g (46,3 mmol, 93%) 1-(Phenylthio)propan-2-on erhalten wurden.

b) Synthese von 1-(Phenylthio)propan-2-ol

Eine Lösung von 2,46 g (14,8 mmol) 1-(Phenylthio)propan-2-on in Methanol (18 ml) wurde unter Kühlung (Eisbad) portionsweise mit 732 mg (19,2 mmol) $NaBH_4$ versetzt. Danach wurde noch 2 h unter Kühlung (Eisbad) nachgerührt. Dann wurde mit AcOH (17 ml) gequencht und anschließend wurde im Vakuum eingeengt. Der Rückstand wurde mit einem Ether/Wassergemisch aufgenommen und mit $NaHCO_3$ neutralisiert. Die Phasen wurden getrennt und die organische Phase wurde über $MgSO_4$ getrocknet, filtriert und im Vakuum eingeengt. Der Rückstand wurde mittels SC (EE / Hex 15:85) aufgereinigt, wobei 1,3 g (7,7 mmol, 52%) 1-(Phenylthio)propan-2-ol erhalten wurden.

c) Synthese einer (2-Chlor-propan-1-yl)-(phenyl)sulfan / (1-Chloropropan-2-yl)(phenyl)sulfan 5:4 Mischung

Zu einer Lösung von 1,29 g (7,7 mmol) 1-(Phenylthio)propan-2-ol und 92 $\mu$l (1,2 mmol) Pyridin in Toluol (30 ml) wurde unter Kühlung (Eisbad) eine Lösung von 1,7 ml (23,0 mmol) Thionylchlorid in Toluol (20 ml) getropft. Danach wurde 3 h unter Rückfluss erhitzt. Nach Abkühlen auf RT wurde mit einem Eis/Wassergemisch gequencht. Die Phasen wurden getrennt und die wässrige Phase nochmals mit Toluol extrahiert. Die vereinten organischen Phasen wurden über $MgSO_4$ getrocknet, filtriert und im Vakuum eingeengt. Der Rückstand wurde mittels SC (EE / Hex 1:9) aufgereinigt, wobei 1,13 g (6,1 mmol, 79%) einer (2-Chlor-propan-1-yl)-(phenyl)sulfan / (1-Chloropropan-2-yl)(phenyl)sulfan (5:4)-Mischung erhalten wurden. Diese Mischung wurde ohne weitere Aufreinigung in nachfolgenden Reaktionen eingesetzt.

**Synthese des Zwischenprodukts VC005: 1-(3-Brom-1,1-difluorpropyl)-4-fluorbenzol**

a) Synthese von 2-(2-(4-Fluorphenyl)-1,3-dithiolan-2-yl)ethyl acetat

Eine Lösung von 2,0 g (9,5 mmol) 3-(4-Fluorphenyl)-3-oxopropyl acetat in DCM (40 ml) wurde bei 0 °C nacheinander mit 1,79 g (19,0 mol) Ethan-1,2-dithiol und 680 mg (4,76 mmol) Bortrifluorid-etherat versetzt. Anschließend wurde 16 h bei RT gerührt und danach mit einer 10M aq. NaOH-Lsg neutralisiert. Dann wurde mit DCM (3 x 100 ml) etrahiert und die vereinten organischen Phasen wurden mit Wasser und Brine gewaschen, über $Na_2SO_4$ getrocknet, filtriert und im Vakuum eingeengt. Nach SC (Hexan / EE 24:1) des Rückstands wurden 1,8 g (6,3 mmol, 66%) 2-(2-(4-Fluor-phenyl)-1,3-dithiolan-2-yl)ethyl acetat erhalten.

b) Synthese von 3,3-Difluor-3-(4-fluorphenyl)propyl acetat

Eine Lösung von 2,0 g (7,0 mmol) 1,3-Dibromo-5,5-dimethylhydantoin in DCM (4 ml) in einem Plastikreaktionsgefäß wurde bei -78 °C nacheinander mit einer 30% HFpyridin Lösung (7 ml) und einer Lösung von 500mg (1,75 mmol) 2-(2-(4-Fluorphenyl)-1,3-dithiolan-2-yl)ethyl acetat in DCM (2 ml) versetzt. Unter Rühren wurde innerhalb von 2 h auf 0 °C erwärmt und dann mit wurde mit einer ges. aq. $NaHCO_3$-Lsg neutralisiert. Anschließend wurde mit DCM (3 x 60 ml) extrahiert. Die vereinten organischen Phasen wurden mit Wasser und Brine gewaschen, über $Na_2SO_4$ getrocknet, filtriert und im Vakuum eingeengt. Als Rückstand wurden 250 mg (1,08 mmol, 62%) 3,3-Difluor-3-(4-fluorphenyl)propyl acetat erhalten, das ohne zusätzliche Aufreinigung weiter umgesetzt wurde.

c) Synthese von 3,3-Difluor-3-(4-fluorphenyl)propan-1-ol

Eine Lösung von 1,9 g (8,2 mmol) 3,3-Difluor-3-(4-fluorphenyl)propyl acetat in EtOH (25 ml) wurde mit einer 35% aq. NaOH-Lsg (6 ml) versetzt und 2 h bei RT gerührt. Anschließend wurde im Vakuum eingeengt und der Rückstand wurde mit Wasser (80 ml) aufgenommen. Die Lösung wurde mit EE (3 x 80 ml) extrahiert und die vereinten organischen Phasen wurden mit Wasser und Brine gewaschen, über $Na_2SO_4$ getrocknet, filtriert und im Vakuum eingeengt. Nach SC (Hexan / EE 9:1) des Rückstands wurden 1,1 g (5,8 mmol, 71%) 3,3-Difluor-3-(4-fluorphenyl)propan-1-ol erhalten.

d) Synthese von 1-(3-Brom-1,1-difluorpropyl)-4-fluorbenzol

Eine Lösung von 1,0 g (5,3 mmol) 3,3-Difluor-3-(4-fluorphenyl)propan-1-ol in DCM (20 ml) wurde mit 3,1 g (9,5 mmol) Tetrabrommethan versetzt und auf 0 °C abgekühlt. Bei dieser Temperatur wurden 2,48 g (9,5 mmol) Triphenylphosphin portionsweise zugegeben. Anschließend wurde 3 h bei RT gerührt. Dann wurde mit Wasser (50 ml) verdünnt und mit EE (3 x 60 ml) extrahiert. Die vereinten organischen Phasen wurden mit Wasser und Brine gewaschen, über $Na_2SO_4$ getrocknet, filtriert und im Vakuum eingeengt. Nach SC (Hexan / EE 24:1) des Rückstands wurden 1,25 g (5,0 mmol, 94%) 1-(3-Brom-1,1-difluorpropyl)-4-fluorbenzol erhalten.

**Synthese des Zwischenprodukts VC006: 1-(3-Brombutyl)-3-(trifluormethyl)benzol**

a) Synthese von 4-(3-(Trifluormethyl)phenyl)butan-2-ol

Eine Lösung von 2,2 g (10,3 mmol) 4-(3-(Trifluormethyl)phenyl)but-3-en-2-on in THF (20 ml) wurde bei 0 °C portionsweise mit 590mg (15,4 mmol) $LiAlH_4$ versetzt und anschließend 1 h bei RT gerührt. Dann wurde mit einer ges. aq. $Na_2SO_4$-Lsg bei 0 °C gequencht und die Reaktionslösung wurde durch Kieselgur filtriert. Das Filtrat wurde mit EE (3 x 60 ml) extrahiert und die vereinten organischen Phasen wurden mit Wasser gewaschen, über $Na_2SO_4$ getrocknet, filtriert und im Vakuum eingeengt. Als Rückstand wurden 2,0 g (9,2 mmol, 89%) 4-(3-(Trifluormethyl)phenyl)butan-2-ol erhalten, das ohne zusätzliche Aufreinigung weiter umgesetzt wurde.

b) Synthese von 1-(3-Brombutyl)-3-(trifluormethyl)benzol

Eine Lösung 2,0 g (9,2 mmol) 4-(3-(Trifluormethyl)phenyl)butan-2-ol in DCM (10 ml) wurde mit 750 mg (11,0 mmol) Imidazol und 2,88 g (11,0 mml) Triphenylphosphin versetzt. Bei 0 °C wurden 570 μl (11,0 mmol) Brom zugetropft und es wurde 1 h bei RT gerührt. Dann wurde im Vakuum eingeengt. Nach SC (Hexan) des Rückstands wurden 2,0 g (7,1 mmol, 78%) 1-(3-Brombutyl)-3-(trifluormethyl)benzol erhalten.

**Synthese weiterer Zwischenprodukte**

Die Synthese weiterer Zwischenprodukte erfolgte nach den bereits beschriebenen Verfahren. In Tabelle 1 ist angegeben, welche Verbindung nach welchem Verfahren hergestellt wurde. Dem Fachmann ist dabei ersichtlich, welche Ausgangsstoffe und Reagenzien jeweils eingesetzt wurden.

Tabelle 1:

| Zwischenprodukt | Chemische Bezeichnung | Herstellung analog Zwischenprodukt |
|---|---|---|
| **VB003** | 2-(3-(Phenylthio)propyl)nicotinsäure | VB006 |
| **VB004** | 2-(3-(3-(Trifluormethyl)phenyl-thio)propyl)nicotinsäure | VB006 |
| **VB005** | 2-(4-(3-Trifluormethyl-phenyl)butyl)nicotinsäure | VB006 |
| **VB009** | 2-(3-(3-(Trifluormethyl)-phenylsulfonyl)propyl)nicotinsäure | VB006 |
| **VC002** | (2-Chlor-propan-1-yl)(4-fluorphenyl)sulfan / (1-Chloropropan-2-yl)(4-fluor-phenyl)sulfan (3:2 Mischung) | VC001 |
| **VC003** | (2-Chlor-propan-1-yl)(3-trifluormethylphenyl)sulfan / (1-Chloropropan-2-yl)(3-trifluormethyl-phenyl)sulfan (5:4 Mischung) | VC001 |
| **VC004** | (2-Chlor-propan-1-yl)(phenyl)sulfan / (1-Chloropropan-2-yl)(phenyl)sulfan (5:13 Mischung) | siehe Beispiel **16** |

## Synthese der Beispielverbindungen

**Synthese der Beispielverbindung 1: 2-(3-Phenyl-propylsulfanyl)-N-(thiophen-2-yl-methyl)-pyridin-3-carbonsäureamid**

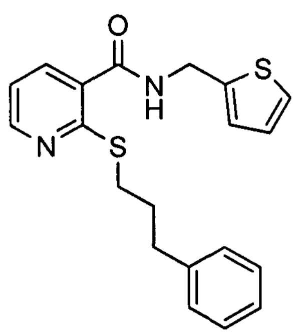

Eine Lösung von 375 mg (1,5 mmol) 2-Mercapto-N-(thiophen-2-ylmethyl)nicotinamid in DMF (3,5 ml) wurde mit 227 mg (1,65 mmol) $K_2CO_3$ versetzt und 30 min bei RT gerührt. Anschließend wurden 369 mg (1,5 mmol) 1-Iod-3-phenylpropan zugegeben und es wurde weitere 3 d bei RT gerührt. Danach wurde im Vakuum eingeengt und der Rückstand wurde mit einem EE/Wassergemisch aufgenommen. Die organische Phase wurde abgetrennt und die wässrige Phase nochmals mit EE extrahiert. Die vereinten organischen Phasen wurden über $MgSO_4$ getrocknet, filtriert und im Vakuum eingeengt. Der Rückstand wurde mittels SC (EE / Hex 1:1) aufgereinigt, wobei 382 mg (1,0 mmol, 69%) 2-(3-Phenyl-propylsulfanyl)-N-(thiophen-2-yl-methyl)-pyridin-3-carbonsäureamid erhalten wurden. MS: m/z 369,1 $[M+H]^+$.

**Synthese der Beispielverbindung 3: 2-[(3-Oxo-3-phenyl-propyl)sulfanyl]-N-(thiophen-2-yl-methyl)-pyridin-3-carbonsäureamid**

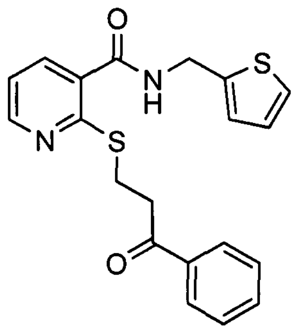

Eine Lösung von 375 mg (1,5 mmol) 2-Mercapto-N-(thiophen-2-ylmethyl)nicotinamid in DMF (3,5 ml) wurde mit 227 mg (1,65 mmol) $K_2CO_3$ versetzt und 30 min bei RT gerührt. Anschließend wurden 252 mg (1,5 mmol) 3-Chlor-1-phenylpropan-1-on zugegeben und es wurde weitere 2 d bei RT gerührt. Danach wurde im Vakuum eingeengt und der Rückstand wurde mit einem Gemisch aus EE und einer 1 N aq. $NaHCO_3$-Lsg aufgenommen. Die organische Phase wurde abgetrennt und die wässrige Phase nochmals mit EE extrahiert. Die vereinten organischen Phasen wurden über $MgSO_4$ getrocknet, filtriert und im Vakuum eingeengt. Der Rückstand wurde mittels SC (EE / Hex 1:1) aufgereinigt und durch nachfolgende Kristallisation (EE / Hexan) wurden 73 mg (0,2 mmol, 13%) 2-[(3-Oxo-3-phenyl-propyl)sulfanyl]-N-(thiophen-2-yl-methyl)-pyridin-3-carbonsäureamid erhalten. MS: m/z 383,1 $[M+H]^+$.

**Synthese der Beispielverbindung 13: N-(Thiophen-2-yl-methyl)-2-[3-[[3-(trifluor-methyl)phenyl]sulfanyl]-propyl]-pyridin-3-carbonsäureamid**

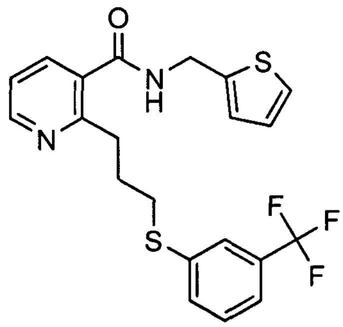

Eine Lösung von 240 mg (0,7 mmol) 2-(3-(3-(Trifluormethyl)phenylthio)-propyl)nicotinsäure (VB004) in DCM (7 ml) wurde mit 91 mg (0,6 mmol) CDI versetzt und 30 min bei RT gerührt. Dann wurden 79 mg (0,7 mmol) 2-(Aminomethyl)-thiophen zugegeben und es wurde weitere 5 d bei RT gerührt. Anschließend wurde mit einer 4M aq. $NH_4Cl$-Lsg (2 x 10 ml) und einer 1 M $NaHCO_3$-Lsg (2 x 10 ml) gewaschen. Die organische Phase wurde über $MgSO_4$ getrocknet, filtriert und im Vakuum eingeengt. Der Rückstand wurde mittels SC (EE) aufgereinigt wobei 134 mg (0,3 mmol, 44%) erhalten wurden. MS: m/z 437,1 $[M+H]^+$.

**Synthese der Beispielverbindung 16: 2-(2-Phenylsulfanyl-propylsulfanyl)-N-(thiophen-2-yl-methyl)-pyridin-3-carbonsäureamid**

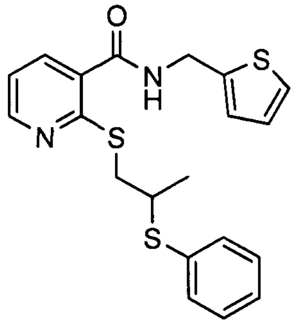

Eine Lösung von 1,25 g (5,0 mmol) 2-Mercapto-N-(thiophen-2-ylmethyl)nicotinamid in DMF (12 ml) wurde mit 1,51 g (11,0 mmol) $K_2CO_3$ versetzt und 1 h bei RT gerührt. Anschließend wurden 930 mg (5,0 mmol) einer (2-Chlor-propan-1-yl)-(phenyl)sulfan / (1-Chloropropan-2-yl)(phenyl)sulfan 5:4 Mischung (VC001) zugegeben und es wurde weitere 18 h bei RT gerührt. Dann wurde im Vakuum eingeengt. Der Rückstand wurde mit einem EE/Wassergemisch aufgenommen und die Phasen wurden getrennt. Die wässrige Phase wurde nochmals mit EE extrahiert und die vereinten organischen Phasen wurden über $MgSO_4$ getrocknet, filtriert und im Vakuum eingeengt. Durch SC (EE / Hex 3:7 $\rightarrow$ 1:1) wurden 400 mg (2,1 mmol, 43%) einer (2-Chlor-propan-1-yl)-(phenyl)sulfan / (1-Chloropropan-2-yl)(phenyl)sulfan 5:13 Mischung zurückgewonnen (VC004) und eine 2-(2-Phenylsulfanyl-propylsulfanyl)-N-(thiophen-2-yl-methyl)-pyridin-3-carbonsäureamid enthaltende Mischfraktion. Aus Letzterer wurden durch Kristallisation (EE) 255 mg (0,6 mmol, 13%) 2-(2-Phenylsulfanyl-propylsulfanyl)-N-(thiophen-2-yl-methyl)-pyridin-3-carbon-säureamid isoliert. MS: m/z 401,1 $[M+H]^+$.

**Synthese der Beispielverbindung 17: 2-[3-(4-Fluorphenyl)-propylsulfanyl]-N-(thiophen-2-yl-methyl)-pyridin-3-carbonsäureamid**

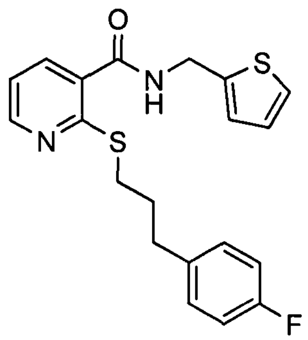

Eine Lösung von 375 mg (1,5 mmol) 2-Mercapto-N-(thiophen-2-ylmethyl)nicotinamid in DMF (6 ml) wurde mit 227 mg (1,65 mmol) $K_2CO_3$ versetzt und 1 h bei RT gerührt. Anschließend wurden 325 mg (1,5 mmol) 1-(3-Brompropyl)-4-fluorbenzol zugegeben und es wurde weitere 16 h bei RT gerührt. Danach wurde im Vakuum eingeengt und der Rückstand wurde mit einem EE/Wassergemisch aufgenommen. Die organische Phase wurde abgetrennt und die wässrige Phase nochmals mit EE extrahiert. Die vereinten organischen Phasen wurden über $MgSO_4$ getrocknet, filtriert und im Vakuum eingeengt. Der Rückstand wurde mittels SC (EE / Hex 1:2) aufgereinigt wobei 428 mg (1,1 mmol, 74%) 2-[3-(4-Fluorphenyl)-propylsulfanyl]-N-(thiophen-2-yl-methyl)-pyridin-3-carbonsäureamid erhalten wurden. MS: m/z 3871 $[M+H]^+$.

**Synthese der Beispielverbindung 21: N-(Thiophen-2-yl-methyl)-2-[4-[3-(trifluormethyl)-phenyl]-butyl]-pyridin-3-carbonsäureamid**

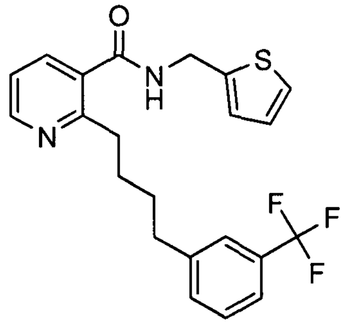

Eine Lösung von 323 mg (1,0 mmol) 2-(4-(3-Trifluormethyl-phenyl)butyl)nicotinsäure (VB005), 113 mg (1,0 mmol) 2-(Aminomethyl)-thiophen und 381 mg (1,0 mmol) HATU in THF (8 ml) wurde mit $NEt_3$ versetzt und 4 d bei RT gerührt. Dann wurde im Vakuum eingeengt. Der Rückstand wurde mit EE aufgenommen und mit es wurde mit einer 1 M aq. $NH_4Cl$-Lsg und einer 1 M aq. $NaHCO_3$-Lsg gewaschen. Die organische Phase wurde über $MgSO_4$ getrocknet, filtriert und im Vakuum eingeengt. Der Rückstand wurde mittels SC (EE / Hex 3:1) aufgereinigt wobei 221 mg (0,5 mmol, 53%) erhalten wurden. MS: m/z 419,1 $[M+H]^+$.

**Synthese der Beispielverbindung 42: N-(3,3-Dimethyl-butyl)-2-[3-(4-fluorphenyl)-propylsulfanyl]-pyridin-3-carbonsäure amid**

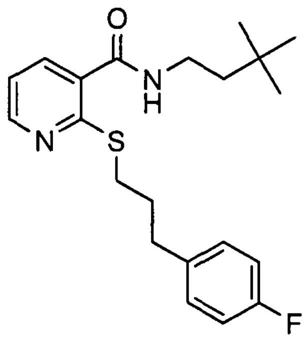

a) Synthese von 2-(3-(4-Fluorphenyl)propylthio)nicotinsäure

Eine Lösung von 2,5 g (16,3 mmol) 2-Mercapto-nicotinsäure in DMF (40 ml) wurde mit 4,9 g (35,8 mmol) $K_2CO_3$ versetzt und 30 min bei RT gerührt. Anschließend wurden 3,5 g (16,3 mmol) 1-(3-Brompropyl)-4-fluorbenzol zugegeben und es wurde weitere 72 h bei RT gerührt. Dann wurde mit Wasser und EE verdünnt und mit einer 5M Essigsäure auf pH 5-6 eingestellt. Die organische Phase wurde abgetrennt, mit Wasser gewaschen, über $MgSO_4$ getrocknet, filtriert und im Vakuum eingeengt. Nach SC (EE / Hexan 1:2 -> 5:1) des Rückstands wurden 2,2 g (7,6 mmol, 47%) 2-(3-(4-Fluorphenyl)propylthio)nicotinsäure erhalten.

b) Synthese von N-(3,3-Dimethyl-butyl)-2-[3-(4-fluorphenyl)-propylsulfanyl]-pyridin-3-carbonsäure amid

Eine Lösung von 200 mg (0,69 mmol) 2-(3-(4-Fluorphenyl)propylthio)nicotinsäure und 92 µl (0,69 mmol) 3,3-Dimethyl-butylamin in THF (11 ml) wurde mit 274 mg (0,72 mmol) HATU und 100 µl (0,72 mmol) $NEt_3$ versetzt und 16 h bei RT gerührt. Anschließend wurde mit EE verdünnt. Die organische Phase wurde abgetrennt, mit einer 1 M aq. $Na_2CO_3$-Lsg und einer 4M aq. $NH_4Cl$-Lsg gewaschen, über $MgSO_4$ getrocknet, filtriert und im Vakuum eingeengt. Nach SC (Hexan / EE 2:1) des Rückstands wurden 173 mg (0,46 mmol, 67%) N-(3,3-Dimethyl-butyl)-2-[3-(4-fluorphenyl)-propylsulfanyl]-pyridin-3-carbonsäure amid erhalten. MS: m/z 375,2 $[M+H]^+$.

**Synthese der Beispielverbindung 50: N-(5-Bicyclo[2.2.1]heptanyl-methyl)-2-[[3-(4-fluorphenyl)-3-hydroxy-propyl]sulfanyl]-pyridin-3-carbonsäure amid**

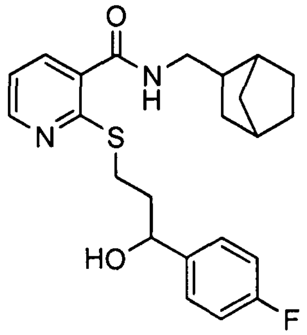

Eine Lösung von 400 mg (0,97 mmol) N-(5-Bicyclo[2.2.1]heptanyl-mthyl)-2-[[3-(4-fluorphenyl)-3-oxo-propyl]sulfanyl]-pyridin-3-carbonsäure amid (Beispielverbindung 43) in MeOH (10 ml) wurde auf 0 °C abgekühlt und portionsweise mit 18 mg (0,49 mmol) $NaBH_4$ versetzt. Anschließend wurde 2 h bei 0 °C und 1 h bei RT gerührt. Dann wurde die Reaktionslösung auf Eiswasser gegossen und es wurde mit DCM extrahiert. Die organische Phase wurde über $MgSO_4$ getrocknet, filtriert und im Vakuum eingeengt. Nach SC (Hexan / EE 2:1 -> 1:1) des Rückstands wurden 180 mg (0,43 mmol, 45%) N-(5-Bicyclo[2.2.1]heptanyl-methyl)-2-[[3-(4-fluorphenyl)-3-hydroxy-propyl]sulfanyl]-pyridin-3-carbonsäure amid erhalten. MS: m/z 415,2 $[M+H]^+$.

**Synthese der Beispielverbindung 51: N-(5-Bicyclo[2.2.1]heptanyl-methyl)-2-[3-(4-fluorphenyl)-propylsulfanyl]-pyridin-3-carbonsäure amid**

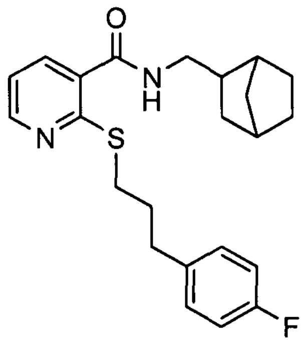

a) Synthese von N-(bicyclo[2.2.1]heptan-2-ylmethyl)-2-mercapto-nicotinamid

Eine Lösung von 6,2 g (40,0 mmol) 2-Mercapto-nicotinsäure in THF (320 ml) wurde nacheinander mit 5,0 g (40,0 mmol) Bicyclo[2.2.1]heptan-2-ylmethanamin, 16,0 g (42,0 mmol) HATU und 5,8 ml (42,0 mmol) $NEt_3$ versetzt und 4d bei RT gerührt. Anschließend wurde mit EE verdünnt. Die organische Phase wurde abgetrennt, mit einer 1 M aq. $Na_2CO_3$-Lsg und einer 4M aq. $NH_4Cl$-Lsg gewaschen, über $MgSO_4$ getrocknet, filtriert und im Vakuum eingeengt. Mit dem Rückstand wurde eine SC (Hexan / EE 7:3 -> 1:1 -> 3:7) durchgeführt. Das erhaltene vorgereinigte Produkt wurde in 1 N aq. NaOH (100 ml) gelöst und die Lösung wurde mit Ether gewaschen. Die wässrige Phase wurde mit einer 2M Salzsäure auf pH ~2 gestellt. Der entstandene Niederschlag wurde abfiltriert, mit Pentan nachgewaschen und im Vakuum getrocknet. Dabei wurden 5,3 g (20,2 mmol, 50%) N-(bicyclo[2.2.1]heptan-2-ylmethyl)-2-mercapto-nicotinamid erhalten.

b) Synthese von N-(5-Bicyclo[2.2.1]heptanyl-methyl)-2-[3-(4-fluorphenyl)-propyl-sulfanyl]-pyridin-3-carbonsäure amid

Eine Lösung von 308 mg (1,18 mmol) N-(bicyclo[2.2.1]heptan-2-ylmethyl)-2-mercapto-nicotinamid in DMF (5 ml) wurde mit 178 mg (1,29 mmol) $K_2CO_3$ versetzt und 60 min bei RT gerührt. Dann wurden 255 mg (1,18 mmol) 1-(3-Brompropyl)-4-fluorbenzol zugegeben und es wurde weitere 16 h bei RT gerührt. Dann wurde mit Wasser verdünnt und mit EE extrahiert. Die organische Phase wurde mit Wasser gewaschen, über $MgSO_4$ getrocknet, filtriert und im Vakuum eingeengt. Nach SC (Hexan / EE 2:1) des Rückstands wurden 333 mg (0,83 mmol, 71%) N-(5-Bicyclo[2.2.1]heptanyl-methyl)-2-[3-(4-fluorphenyl)-propyl-sulfanyl]-pyridin-3-carbonsäure amid erhalten. MS: m/z 399,2 $[M+H]^+$.

**Synthese der Beispielverbindung 58: N-(5-Bicyclo[2.2.1]heptanyl-methyl)-2-[3-(4-hydroxyphenyl)-propylsulfanyl]-pyridin-3-carbonsäure amid**

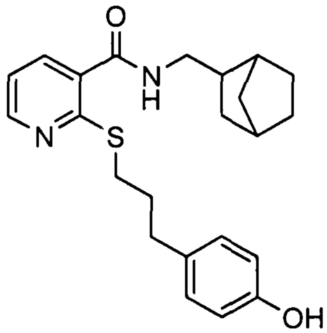

Eine Lösung 483 mg (1,18 mmol) N-(5-Bicyclo[2.2.1]heptanyl-methyl)-2-[3-(4-methoxyphenyl)-propylsulfanyl]-pyridin-3-carbonsäure amid (Beispielverbindung 56) in DCM (50 ml) wurde auf -60 °C abgekühlt und bei dieser Temperatur mit 11,8 ml (11,8 mmol, 1M in DCM) Bortribromid versetzt und 90 min bei -60 °C gerührt. Nach Erwärmen auf RT wurde mit einer 1 M aq. $NaHCO_3$-Lsg gequencht und die Phasen wurden getrennt. Die wässrige Phase wurde mit EE extrahiert und die organischen Phasen wurden vereint, mit Wasser gewaschen, über $MgSO_4$ getrocknet, filtriert und im Vakuum eingeengt. Nach Kristallisation (Hexan / EE 2:1) des Rückstands wurden 393 mg (0,99 mmol, 84%) N-(5-Bicyclo[2.2.1]heptanyl-methyl)-2-[3-(4-hydroxyphenyl)-propylsulfanyl]-pyridin-3-carbonsäure amid erhalten. MS: m/z 397,2 $[M+H]^+$.

**Synthese der Beispielverbindung 112: 2-(3-Phenyl-propylsulfanyl)-6-pyrrolidin-1-yl-N-(thiophen-2-yl-methyl)-pyridin-3-carbonsäure amid**

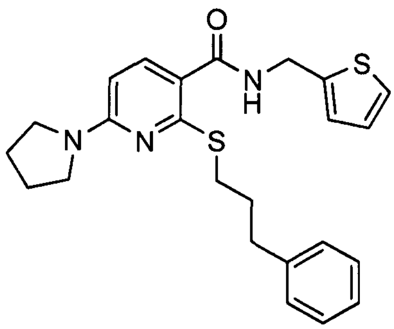

Eine Lösung von 387 mg (1,0 mmol) 6-Fluor-2-(3-phenylpropylthio)-N-(thiophen-2-ylmethyl)pyridin-3-carbonsäure amid (Beispielverbindung 225) in DMF (10ml) wurde mit 153 mg $K_2CO_3$ und 71 mg (1,0 mmol) Pyrrolidin versetzt und 2 h bei RT gerührt. Anschließend wurde mit Wasser verdünnt und mit EE extrahiert. Die organische Phase wurde mit Wasser und Brine gewaschen, über $Na_2SO_4$ getrocknet, filtriert und im Vakuum eingeengt. Nach SC (Hexan / EE 7:3) des Rückstands wurden 308 mg (0,7 mmol, 71%) 2-(3-Phenyl-propylsulfanyl)-6-pyrrolidin-1-yl-N-(thiophen-2-yl-methyl)-pyridin-3-carbonsäure amid erhalten. MS: m/z 438,2 $[M+H]^+$.

**Synthese der Beispielverbindung 115: N-(5-Bicyclo[2.2.1]heptanyl-methyl)-2-[[3,3-difluor-3-(4-fluorphenyl)-propyl]sulfanyl]-pyridin-3-carbonsäure amid**

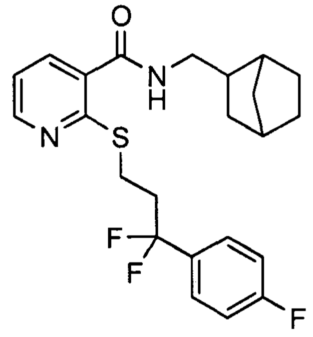

a) Synthese von N-(Bicyclo[2.2.1]heptan-2-ylmethyl)-2-mercapto-nicotinamid

Eine Lösung von 1,55 g (10,0 mmol) 2-Mercapto-nicotinsäure in DMF (30 ml) wurde mit 4,82 g (15,0 mmol) TBTU und 3,54 g (35 mmol) N-Methyl-morpholin versetzt und 30 min bei RT gerührt. Dann wurden 1,25 g (10,0 mmol) Bicyclo[2.2.1]heptan-2-ylmethanamin zugegeben und es wurde 16 h bei RT gerührt. Anschließend wurde mit EE verdünnt und mit ges. aq. $NH_4Cl$-Lsg, Wasser, einer 1 N aq. $NaHCO_3$-Lsg und Brine gewaschen. Die organische Phase wurde über $Na_2SO_4$ getrocknet, filtriert und im Vakuum eingeengt. Nach SC (DCM / MeOH 99:1) des Rückstands wurden 308 mg 1,39 g (5,3 mmol, 53%) N-(Bicyclo[2.2.1]heptan-2-ylmethyl)-2-mercapto-nicotinamid erhalten.

b) Synthese von N-(5-Bicyclo[2.2.1]heptanyl-methyl)-2-[[3,3-difluor-3-(4-fluorphenyl)-propyl]sulfanyl]-pyridin-3-carbonsäure

Eine Lösung von 262 mg (1,0 mmol) N-(Bicyclo[2.2.1]heptan-2-ylmethyl)-2-mercapto-nicotinamid in DMF (3 ml) wurde nacheinander mit 553 mg (4 mmol) $K_2CO_3$ und 268 mg (1,0 mmol) 3,3-Difluor-3-(4-fluorphenyl)propyl methansulfonat versetzt und 3 h auf 60 °C erhitzt. Anschließend wurde mit Wasser verdünnt und mit EE extrahiert. Die organische Phase wurde mit Brine gewaschen, über $Na_2SO_4$ getrocknet, filtriert und im Vakuum eingeengt. Nach SC (Hexan / EE 7:3) des Rückstands wurden 229 mg (0,5 mmol, 53%) N-(5-Bicyclo[2.2.1]heptanyl-methyl)-2-[[3,3-difluor-3-(4-fluorphenyl)-propyl]sulfanyl]-pyridin-3-carbonsäure amid erhalten. MS: m/z 435,2 $[M+H]^+$.

**Synthese der Beispielverbindung 126: 2-[3-(4-Fluorphenyl)-propylsulfanyl]-N-(2-hydroxy-3,3-dimethyl-butyl)-pyridin-3-carbonsäure amid**

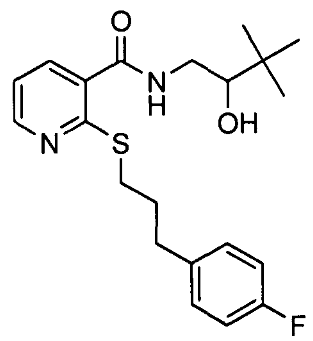

Eine Lösung von 350 mg (0,9 mmol) N-(3,3-Dimethyl-2-oxo-butyl)-2-[3-(4-fluorphenyl)-propylsulfanyl]-pyridin-3-carbonsäure amid (Beispielverbindung 125) in EtOH (7 ml) wurde bei 0 °C portionsweise mit 70 mg (1,8 mmol) $NaBH_4$ versetzt. Anschließend wurde 2 h bei RT gerührt. Dann wurde auf 0 °C abgekühlt, mit einer ges. aq. $NH_4Cl$-Lsg (20 ml) gequencht und im Vakuum aufkonzentriert. Der Rückstand wurde mit EE aufgenommen und die Lösung wurde mit einer 1 M aq. $NaHCO_3$-Lsg, Wasser und Brine gewaschen, über $Na_2SO_4$ getrocknet, filtriert und im Vakuum eingeengt. Nach SC (Hexan / EE 3:2) des Rückstands wurden 320 mg (0,82 mmol, 91 %) 2-[3-(4-Fluorphenyl)-propylsulfanyl]-N-(2-hydroxy-3,3-dimethyl-butyl)-pyridin-3-carbonsäure amid erhalten. MS: m/z 391,2 $[M+H]^+$.

**Synthese der Beispielverbindung 131: 3-[[[2-[3-(4-Fluorphenyl)-propylsulfanyl]-pyridin-3-carbonyl]amino]-methyl]-benzoesäure**

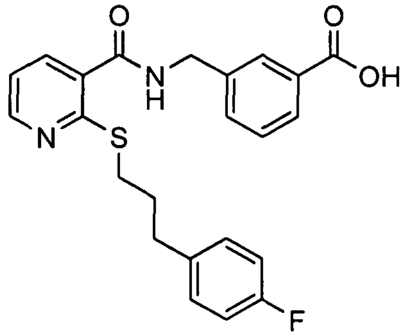

Eine Lösung von 320 mg (0,73 mmol) 3-[[[2-[3-(4-Fluorphenyl)-propylsulfanyl]-pyridin-3-carbonyl]amino]-methyl]-benzoesäure methylester (Beispielverbindung 129) in einem MeOH/THF-Gemisch (2:1 vv, 9 ml) wurde mit einer Lösung von 120 mg (2,92 mmol) Lithiumhydroxidmonohydrat in Wasser (4 ml) versetzt und 16 h bei RT gerührt. Anschließend wurde im Vakuum eingeengt. Der Rückstand wurde mit Wasser aufgenommen und mit EE gewaschen. Dann wurde die wässrige Phase mit einer 2M Salzsäure bei 0 °C auf pH ~2 eingestellt. Der entstandene Niederschlag wurde abfiltriert, mit Toluol aufgenommen und im Vakuum eingeengt. Der Rückstand wurde mit einem Ether/Pentan-Gemisch (1:4 vv) gewaschen wobei 170 mg (0,4 mmol, 55%) 3-[[[2-[3-(4-Fluorphenyl)-propylsulfanyl]-pyridin-3-carbonyl]amino]-methyl]-benzoesäure erhalten wurden. MS: m/z 425,1 $[M+H]^+$.

**Synthese der Beispielverbindung 143: 2-[3-(4-Fluorphenyl)-propylsulfanyl]-N-(pyridin-2-yl-methyl)-pyridin-3-carbonsäure amid**

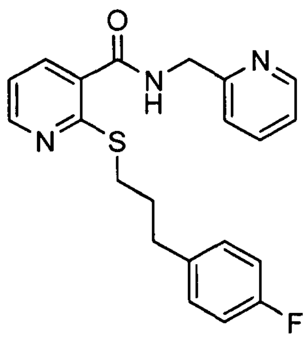

a) Synthese von 2-(3-(4-Fluorphenyl)propylthio)nicotinsäure

2-(3-(4-Fluorphenyl)propylthio)nicotinsäure wurde nach dem bei Beispielverbindung 169 Abschnitt a) beschriebenen Verfahren hergestellt.

b) Synthese von 2-[3-(4-Fluorphenyl)-propylsulfanyl]-N-(pyridin-2-yl-methyl)-pyridin-3-carbonsäure amid

Eine Lösung von 400 mg (1,37 mmol) 2-(3-(4-Fluorphenyl)propylthio)nicotinsäure in DCM (6ml) wurde nacheinander mit 520 mg (2,74 mmol) EDC Hydrochlorid, 250 mg (1,64 mmol) HOBT, 0,9 ml (5,48 mmol) DIPEA und 180 mg (1,64 mmol) Pyridin-2-yl-methanamin versetzt und 4 h bei RT gerührt. Anschließend wurde mit Wasser (40 ml) verdünnt und mit DCM (3 x 50 ml) extrahiert. Die vereinten organischen Phasen wurde mit einer ges. $NH_4Cl$-Lsg und Wasser gewaschen, über $Na_2SO_4$ getrocknet, filtriert und im Vakuum eingeengt. Nach SC (Hexan / EE 22:3) des Rückstands wurden 240 mg (0,63 mmol, 46%) 2-[3-(4-Fluorphenyl)-propylsulfanyl]-N-(pyridin-2-yl-methyl)-pyridin-3-carbonsäure amid erhalten. MS: m/z 382,1 $[M+H]^+$.

**Synthese der Beispielverbindung 147: 2-[3-(4-Fluorphenyl)-propylsulfanyl]-4-methyl-N-(thiophen-2-yl-methyl)-pyridin-3-carbonsäure amid**

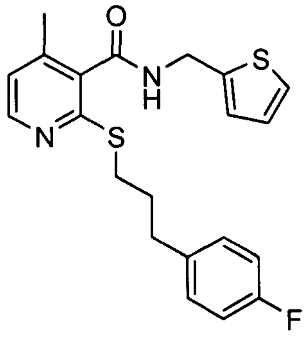

a) Synthese von 2-(3-(4-Fluorphenyl)propylthio)-4-methylnicotinnitril

Eine Lösung von 3,0 g (20 mmol) 2-Mercapto-4-methylnicotinnitril in Aceton (60 ml) wurde mit 4,14 g (30 mmol) $K_2CO_3$ und 5,2 g (30 mmol) 1-(3-Chlorpropyl)-4-fluorbenzol versetzt und dann für 16 h auf 60 °C erhitzt. Anschließend wurde durch Kiesekgur filtriert und das Filtrat wurde mit Wasser (50 ml) verdünnt. Dann wurde mit EE (200 ml) gewaschen. Die wässrige Phase wurde mit einer 6M Salzsäure (50 ml) sauer gestellt und dann mit EE (3 x 100 ml) extrahiert. Die vereinten organischen Phasen wurden mit Wasser und Brine gewaschen, über $Na_2SO_4$ getrocknet, filtriert und im Vakuum eingeengt. Nach SC (Hexan / EE 9:1) des Rückstands wurden 3,5 g (12 mmol, 61%) 2-(3-(4-Fluorphenyl)propylthio)-4-methylnicotinnitril erhalten.

b) Synthese von 2-(3-(4-Fluorphenyl)propylthio)-4-methylnicotinsäure

500 mg (1,74 mmol) 2-(3-(4-Fluorphenyl)propylthio)-4-methylnicotinnitril wurden mit 50%-iger Schwefelsäure versetzt (8 ml) und die Reaktionlösung wurde 6 d auf 140 °C erhitzt. Anschließend wurde auf Eiswasser gegossen und mit EE (3 x 100 ml) extrahiert. Die vereinten organischen Phasen wurden mit Wasser und Brine gewaschen, über $Na_2SO_4$ getrocknet, filtriert und im Vakuum eingeengt. Als Rückstand wurden 400 mg (1,31 mmol, 75%) 2-(3-(4-Fluorphenyl)propylthio)-4-methylnicotinsäure erhalten, die ohne zusätzliche Aufreinigung weiter umgesetzt wurden.

c) Synthese von 2-[3-(4-Fluorphenyl)-propylsulfanyl]-4-methyl-N-(thiophen-2-yl-methyl)-pyridin-3-carbonsäure amid

Aus 200 mg (0,66 mmol) 2-(3-(4-Fluorphenyl)propylthio)-4-methylnicotinsäure und 80 $\mu$l (0,79 mmol) Thiophen-2-yl-methanamin wurden nach dem für Beispielverbindung 169 Abschnitt b) beschriebenen Verfahren 120 mg (0,30 mmol, 45%) 2-[3-(4-Fluorphenyl)-propylsulfanyl]-4-methyl-N-(thiophen-2-yl-methyl)-pyridin-3-carbonsäure amid hergestellt. MS: m/z 401,1 $[M+H]^+$.

**Synthese der Beispielverbindung 167: N-(3,3-Dimethyl-butyl)-2-[(2-oxo-3-phenyl-propyl)sulfanyl]-pyridin-3-carbonsäure amid**

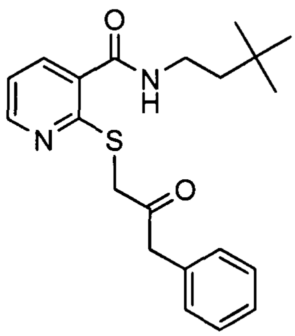

a) Synthese von N-(3,3-Dimethylbutyl)-2-mercapto-nicotinamid

Eine Lösung von 1,50 g (9,7 mmol) 2-Mercapto-nicotinsäure in DMF (30 ml) wurde bei 0 °C mit 5,84 ml (33,8 mmol) DIPEA und 4,66 g (14,5 mmol) TBTU versetzt. Nach 30 min Rühren bei 0 °C wurden 1,6 ml (11,6 mmol) 3,3-Dimethylbutan-1-amin zugegeben und anschließend wurde 16 h bei RT gerührt. Dann wurde mit Wasser (100 ml) verdünnt und mit EE (2 x 100 ml) extrahiert. Die vereinten organischen Phasen wurden mit Wasser und Brine gewaschen, über $Na_2SO_4$ getrocknet, filtriert und im Vakuum eingeengt. Nach SC (Hexan / Aceton 1:1) des Rückstands wurden 1,4 g (5,9 mmol, 61%) N-(3,3-Dimethylbutyl)-2-mercapto-nicotinamid erhalten.

b) Synthese von N-(3,3-Dimethyl-butyl)-2-[(2-oxo-3-phenyl-propyl)sulfanyl]-pyridin-3-carbonsäure

Eine Lösung von 450 mg (2,0 mmol) N-(3,3-Dimethylbutyl)-2-mercapto-nicotinamid in Aceton (10 ml) wurde mit 840 mg (6,1 mmol) $K_2CO_3$ und 340 mg (2,0 mmol) 1-Chlor-3-phenylpropan-2-on versetzt und anschließend 1 h auf 50 °C erhitzt. Dann wurde mit Wasser (20 ml) verdünnt und mit EE (2 x 30 ml) extrahiert. Die vereinten organischen Phasen wurden mit Wasser und Brine gewaschen, über $Na_2SO_4$ getrocknet, filtriert und im Vakuum eingeengt. Nach SC (Hexan / EE 4:1) des Rückstands wurden 420 mg (1,1 mmol, 56%)N-(3,3-Dimethyl-butyl)-2-[(2-oxo-3-phenyl-propyl)sulfanyl]-pyridin-3-carbonsäure amid erhalten. MS: m/z 371,2 $[M+H]^+$.

**Synthese der Beispielverbindung 169: 2-[[3,3-Difluor-3-(4-fluorphenyl)-propyl]sulfanyl]-N-(3-methyl-butyl)-pyridin-3-carbonsäure amid**

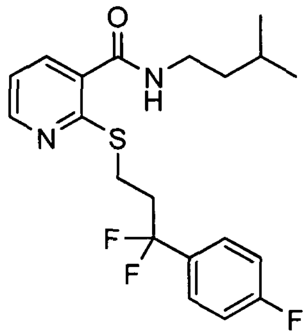

a) Synthese von 2-(3,3-Difluor-3-(4-fluorphenyl)propylthio)nicotinsäure

Eine Lösung von 620 mg (4,0 mmol) 2-Mercapto-nicotinsäure in DMF (10 ml) wurde mit 1,02 g (4,0 mmol) 1-(3-Brom-1,1-Difluorpropyl)-4-fluorbenzol (Vorstufe VC005) und 1,67 g (12,0 mmol) $K_2CO_3$ versetzt und 2 h auf 90 °C erhitzt. Anschließend wurde mit Wasser (20 ml) verdünnt und mit einer 6M Salzsäure auf pH ~2 gestellt. Dann wurde mit EE (3 x 40 ml) extrahiert. Die vereinten organischen Phasen wurden mit Wasser und Brine gewaschen, über $Na_2SO_4$ getrocknet, filtriert und im Vakuum eingeengt. Nach SC (Hexan / EE 1:4) des Rückstands wurden 400 mg (1,2 mmol, 31 %) 2-(3,3-Difluor-3-(4-fluorphenyl)propylthio)nicotinsäure erhalten.

b) Synthese von 2-[[3,3-Difluor-3-(4-fluorphenyl)-propyl]sulfanyl]-N-(3-methyl-butyl)-pyridin-3-carbonsäure amid

Eine Lösung von 150 mg (0,46 mmol) 2-(3,3-Difluor-3-(4-fluorphenyl)propylthio)-nicotinsäure in DCM (10 ml) wurde bei 0 °C mit 320 μl (1,83 mmol) DIPEA und 350 mg (0,91 mmol) HATU versetzt. Nach 30 min Rühren bei 0 °C wurden 63 μl (0,55 mmol) 3-Methylbutan-1-amin zugegeben und anschließend wurde 16 h bei RT gerührt. Dann wurde mit Wasser (20 ml) verdünnt und mit DCM (2 x 20 ml) extrahiert. Die vereinten organischen Phasen wurden mit Wasser und Brine gewaschen, über $Na_2SO_4$ getrocknet, filtriert und im Vakuum eingeengt. Nach SC (EE / Hexan 7:3) des Rückstands wurden 100 mg (0,25 mmol, 55%) 2-[[3,3-Difluor-3-(4-fluorphenyl)-propyl]sulfanyl]-N-(3-methyl-butyl)-pyridin-3-carbonsäure amid erhalten. MS: m/z 397,1 $[M+H]^+$.

**Synthese der Beispielverbindung 225: 6-Fluor-2-(3-phenylpropylthio)-N-(thiophen-2-ylmethyl)pyridin-3-carbonsäure amid**

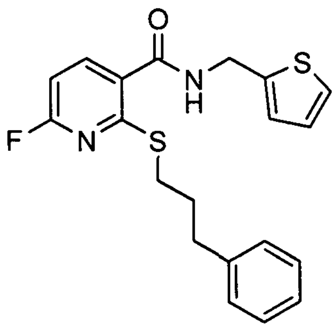

a) Synthese von 2,6-Difluor-N-(thiophen-2-ylmethyl)nicotinamid

795 mg (5,0 mmol) 2,6-Difluor-nicotinsäure wurden in Thionylchlorid (15 ml) gelöst und die Lösung wurde 2 h auf 80 °C erhitzt. Anschließend wurde im Vakuum eingeengt und der Rückstand wurde mit Dioxan (15 ml) aufgenommen. Dann wurden 566 mg (5,0 mmol) Thiophen-2yl-methanamin zugegeben und es wurde 1 h bei RT gerührt. Anschließend wurde im Vakuum eingeengt und der Rückstand wurde mit EE aufgenommen, mit Wasser, einer ges. aq. $Na_2CO_3$-Lsg, wieder Wasser und Brine gewaschen, über $Na_2SO_4$ getrocknet, filtriert und im Vakuum eingeengt. Nach SC (Hexan / EE 7:3) des Rückstands wurden 605 mg (2,4 mmol, 48%) 2,6-Difluor-N-(thiophen-2-ylmethyl)nicotinamid erhalten.

b) Synthese von 6-Fluor-2-(3-phenylpropylthio)-N-(thiophen-2-ylmethyl)pyridin-3-carbonsäure amid

Eine Lösung von 509 mg (2,0 mmol) 2,6-Difluor-N-(thiophen-2-ylmethyl)nicotinamid in DMF (10 ml) wurde mit 919 mg (6,0 mmol) $K_2CO_3$ und 305 mg (2,0 mmol) 3-Phenylpropan-1-thiol versetzt und 1 h bei RT gerührt. Anschließend wurde mit Wasser verdünnt und mit EE extrahiert. Die organische Phase wurde mit Wasser und Brine gewaschen, über $Na_2SO_4$ getrocknet, filtriert und im Vakuum eingeengt. Nach SC (Hexan / EE 4:1) des Rückstands wurden 336 mg (0,9 mmol, 43%) 6-Fluor-2-(3-phenylpropylthio)-N-(thiophen-2-ylmethyl)pyridin-3-carbonsäure amid erhalten. MS: m/z 387,1 $[M+H]^+$.

**Synthese weiterer Beispielverbindungen**

Die Synthese weiterer Beispielverbindungen erfolgte nach den bereits beschriebenen Verfahren. In Tabelle 2 ist angegeben, welche Verbindung nach welchem Verfahren hergestellt wurde. Dem Fachmann ist dabei ersichtlich, welche Ausgangsstoffe und Reagenzien jeweils eingesetzt wurden.

Tabelle 2:

| Beispiel-verbindung | Chemische Bezeichnung | Synthese analog Beispiel-verbindung | MS m/z $[M+H]^+$ |
|---|---|---|---|
| **2** | 2-(3-Cyclohexyl-propylsulfanyl)-N-(thiophen-2-yl-methyl)-pyridin-3-carbonsäure amid | 17 | 375,1 |
| **4** | N-(Thiophen-2-yl-methyl)-2-[2-[3-(trifluormethyl)-phenoxy]-ethylsulfanyl]-pyridin-3-carbonsäure amid | 17 | 439,1 |
| **5** | 2-(4-Methyl-pentylsulfanyl)-N-(thiophen-2-yl-methyl)-pyridin-3-carbonsäure amid | 17 | 335,1 |
| **7** | 2-(4-Phenyl-butyl)-N-(thiophen-2-yl-methyl)-pyridin-3-carbonsäure amid | 13 | 351,1 |
| **8** | 2-[3-(Benzolsulfonyl)-propyl]-N-(cyclohexyl-methyl)-pyridin-3-carbonsäure amid | 13 | 401,2 |
| **9** | N-(Cyclohexyl-methyl)-2-(4-phenyl-butyl)-pyridin-3-carbonsäure amid | 13 | 351,2 |
| **10** | 2-[3-(Benzolsulfonyl)-propyl]-N-(thiophen-2-yl-methyl)-pyridin-3-carbonsäure amid | 13 | 401,1 |
| **12** | N-(Thiophen-2-yl-methyl)-2-[3-[[3-(trifluormethyl)phenyl]sulfonyl]-propyl]-pyridin-3-carbonsäure amid | 13 | 469,1 |
| **18** | N-(Thiophen-2-yl-methyl)-2-[3-[3-(trifluormethyl)phenyl]-propylsulfanyl]-pyridin-3-carbonsäure amid | 17 | 437,1 |
| **19** | 2-[2-[(4-Fluorphenyl)sulfanyl]-propylsulfanyl]-N-(thiophen-2-yl-methyl)-pyridin-3-carbonsäure amid | 16 (aus VC002) | 419,1 |
| **20** | N-(Thiophen-2-yl-methyl)-2-[2-[[3-(trifluormethyl)phenyl]sulfanyl]-propylsulfanyl]-pyridin-3-carbonsäure amid | 16 (aus VC003) | 469,1 |
| **22** | 2-[4-(4-Fluorphenyl)-butyl]-N-(thiophen-2-yl-methyl)-pyridin-3-carbonsäure amid | 21 | 369,1 |
| **23** | 2-(3-Phenylsulfanyl-propyl)-N-(thiophen-2-yl-methyl)-pyridin-3-carbonsäure amid | 21 | 369,1 |
| **24** | 2-[(1-Methyl-2-phenylsulfanyl-ethyl)sulfanyl]-N-(thiophen-2-yl-methyl)-pyridin-3-carbonsäure amid | 16 (aus VC004) | 401,1 |
| **25** | N-(Cycloheptyl-methyl)-2-[4-[3-(trifluormethyl)phenyl]-butyl]-pyridin-3-carbonsäure amid | 17 | 433,2 |
| **26** | N-(5-Bicyclo[2.2.1]heptanyl-methyl)-2-[4-[3-(trifluormethyl)phenyl]-butyl]-pyridin-3-carbonsäure amid | 17 | 431,2 |
| **27** | N-(5-Bicyclo[2.2.1]heptanyl-methyl)-2-[3-[3-(trifluormethyl)phenyl]-propylsulfanyl]-pyridin-3-carbonsäure amid | 51 | 449,2 |
| **28** | N-(5-Bicyclo[2.2.1]heptanyl-methyl)-2-(4-methyl-pentylsulfanyl)-pyridin-3-carbonsäure amid | 51 | 347,2 |
| **29** | N-(Cycloheptyl-methyl)-2-[3-[(4-fluorphenyl)sulfanyl]-propyl]-pyridin-3-carbonsäure amid | 21 | 401,2 |
| **30** | N-(5-Bicyclo[2.2.1]heptanyl-methyl)-2-[3-[(4-fluorphenyl)sulfanyl]-propyl]-pyridin-3-carbonsäure amid | 21 | 399,2 |

(fortgesetzt)

| Beispiel-verbindung | Chemische Bezeichnung | Synthese analog Beispiel-verbindung | MS m/z $[M+H]^+$ |
|---|---|---|---|
| **31** | N-(Cycloheptyl-methyl)-2-[3-[[3-(trifluormethyl)phenyl]sulfonyl]-propyl]-pyridin-3-carbonsäure amid | 21 | 483,2 |
| **32** | N-(5-Bicyclo[2.2.1]heptanyl-methyl)-2-[3-[[3-(trifluormethyl)phenyl]sulfonyl]-propyl]-pyridin-3-carbonsäure amid | 21 | 481,2 |
| **33** | N-(5-Bicyclo[2.2.1]heptanyl-methyl)-2-(3-phenyl-propylsulfanyl)-pyridin-3-carbonsäure amid | 51 | 381,2 |
| **34** | N-(5-Bicyclo[2.2.1]heptanyl-methyl)-2-[3-[4-(trifluormethyl)-phenyl]-propylsulfanyl]-pyridin-3-carbonsäure amid | 51 | 449,2 |
| **35** | N-(5-Bicyclo[2.2.1]heptanyl-methyl)-2-[3-(4-chlorphenyl)-propylsulfanyl]-pyridin-3-carbonsäure amid | 51 | 415,2 |
| **36** | N-[(3,5-Difluor-phenyl)-methyl]-2-[3-(4-fluorphenyl)-propylsulfanyl]-pyridin-3-carbonsäure amid | 42 | 417,1 |
| **37** | N-[(5-Chlor-thiophen-2-yl)-methyl]-2-[3-(4-fluorphenyl)-propylsulfanyl]-pyridin-3-carbonsäure amid | 42 | 421,1 |
| **38** | N-[(2,2-Dimethyl-cyclopropyl)-methyl]-2-[3-(4-fluorphenyl)-propylsulfanyl]-pyridin-3-carbonsäure amid | 42 | 373,2 |
| **39** | N-(5-Bicyclo[2.2.1]heptanyl-methyl)-2-[3-(3-fluorphenyl)-propylsulfanyl]-pyridin-3-carbonsäure amid | 51 | 399,2 |
| **40** | N-(Cyclohexyl-methyl)-2-[3-(4-fluorphenyl)-propylsulfanyl]-pyridin-3-carbonsäure amid | 42 | 387,2 |
| **41** | N-(Cycloheptyl-methyl)-2-[3-(4-fluorphenyl)-propylsulfanyl]-pyridin-3-carbonsäure amid | 42 | 401,2 |
| **43** | N-(5-Bicyclo[2.2.1]heptanyl-methyl)-2-[[3-(4-fluorphenyl)-3-oxo-propyl]sulfanyl]-pyridin-3-carbonsäure amid | 51 | 413,2 |
| **44** | N-(5-Bicyclo[2.2.1]heptanyl-methyl)-2-[(3-oxo-3-phenyl-propyl)sulfanyl]-pyridin-3-carbonsäure amid | 51 | 395,2 |
| **45** | N-(5-Bicyclo[2.2.1]heptanyl-methyl)-2-(hexylsulfanyl)-pyridin-3-carbonsäure amid | 51 | 347,2 |
| **46** | N-(5-Bicyclo[2.2.1]heptanyl-methyl)-2-(2-phenoxy-ethylsulfanyl)-pyridin-3-carbonsäure amid | 51 | 383,2 |
| **47** | N-(5-Bicyclo[2.2.1]heptanyl-methyl)-2-[2-[3-(trifluormethyl)-phenoxy]-ethylsulfanyl]-pyridin-3-carbonsäure amid | 51 | 451,2 |
| **48** | 2-[3-(4-Fluorphenyl)-propylsulfanyl]-N-(3,3,3-trifluor-propyl)-pyridin-3-carbonsäure amid | 51 | 387,1 |
| **49** | N-(5-Bicyclo[2.2.1]heptanyl-methyl)-2-[2-(4-fluor-phenoxy)-ethylsulfanyl]-pyridin-3-carbonsäure amid | 51 | 401,2 |

(fortgesetzt)

| Beispiel-verbindung | Chemische Bezeichnung | Synthese analog Beispiel-verbindung | MS m/z $[M+H]^+$ |
|---|---|---|---|
| **52** | N-(5-Bicyclo[2.2.1]heptanyl-methyl)-2-(3-naphthalen-1-yl-propylsulfanyl)-pyridin-3-carbonsäure amid | 51 | 431,2 |
| **53** | N-(5-Bicyclo[2.2.1]heptanyl-methyl)-2-[3-[3-fluor-4-(trifluormethyl)-phenyl]-propylsulfanyl]-pyridin-3-carbonsäure amid | 51 | 467,2 |
| **54** | N-(5-Bicyclo[2.2.1]heptanyl-methyl)-2-[3-[4-fluor-3-(trifluormethyl)-phenyl]-propylsulfanyl]-pyridin-3-carbonsäure amid | 51 | 467,2 |
| **55** | N-(Cyclooctyl-methyl)-2-[3-(4-fluorphenyl)-propylsulfanyl]-pyridin-3-carbonsäure amid | 42 | 415,2 |
| **56** | N-(5-Bicyclo[2.2.1]heptanyl-methyl)-2-[3-(4-methoxyphenyl)-propylsulfanyl]-pyridin-3-carbonsäure amid | 51 | 411,2 |
| **57** | 2-[3-(4-Fluorphenyl)-propylsulfanyl]-N-[(4-methoxyphenyl)-methyl]-pyridin-3-carbonsäure amid | 42 | 411,1 |
| **59** | 2-[3-(4-Fluorphenyl)-propylsulfanyl]-N-[[3-(trifluormethyl)phenyl]-methyl]-pyridin-3-carbonsäure amid | 42 | 449,1 |
| **60** | N-(5-Bicyclo[2.2.1]heptanyl-methyl)-2-[3-(3,4-difluor-phenyl)-propylsulfanyl]-pyridin-3-carbonsäure amid | 51 | 417,2 |
| **61** | N-(5-Bicyclo[2.2.1]heptanyl-methyl)-2-[3-(3,5-difluor-phenyl)-propylsulfanyl]-pyridin-3-carbonsäure amid | 51 | 417,2 |
| **62** | N-(5-Bicyclo[2.2.1]heptanyl-methyl)-2-[3-(2,4-difluor-phenyl)-propylsulfanyl]-pyridin-3-carbonsäure amid | 51 | 417,2 |
| **63** | N-[(2-Fluorphenyl)-methyl]-2-[3-(4-fluorphenyl)-propylsulfanyl]-pyridin-3-carbonsäure amid | 42 | 399,1 |
| **64** | N-[(3-Fluorphenyl)-methyl]-2-[3-(4-fluorphenyl)-propylsulfanyl]-pyridin-3-carbonsäure amid | 42 | 399,1 |
| **65** | N-[(4-Fluorphenyl)-methyl]-2-[3-(4-fluorphenyl)-propylsulfanyl]-pyridin-3-carbonsäure amid | 42 | 399,1 |
| **66** | N-Benzyl-2-[3-(4-fluorphenyl)-propylsulfanyl]-pyridin-3-carbonsäure amid | 42 | 381,1 |
| **67** | N-(5-Bicyclo[2.2.1]heptanyl-methyl)-2-[(1-methyl-3-phenyl-propyl)sulfanyl]-pyridin-3-carbonsäure amid | 51 | 395,2 |
| **68** | N-(5-Bicyclo[2.2.1]heptanyl-methyl)-2-[3-(3,4,5-trifluor-phenyl)-propylsulfanyl]-pyridin-3-carbonsäure amid | 51 | 435,2 |
| **69** | N-(5-Bicyclo[2.2.1]heptanyl-methyl)-2-[3-[3-fluor-5-(trifluormethyl)-phenyl]-propylsulfanyl]-pyridin-3-carbonsäure amid | 51 | 467,2 |

(fortgesetzt)

| Beispiel-verbindung | Chemische Bezeichnung | Synthese analog Beispiel-verbindung | MS m/z $[M+H]^+$ |
|---|---|---|---|
| **70** | N-(5-Bicyclo[2.2.1]heptanyl-methyl)-2-[3-(3-methoxyphenyl)-propylsulfanyl]-pyridin-3-carbonsäure amid | 51 | 411,2 |
| **71** | N-[(3,4-Difluor-phenyl)-methyl]-2-[3-(4-fluorphenyl)-propylsulfanyl]-pyridin-3-carbonsäure amid | 42 | 417,1 |
| **72** | N-(2,3-Dihydro-benzofuran-5-yl-methyl)-2-[3-(4-fluorphenyl)-propylsulfanyl]-pyridin-3-carbonsäure amid | 42 | 423,1 |
| **73** | N-(5-Bicyclo[2.2.1]heptanyl-methyl)-2-[3-(3-hydroxyphenyl)-propylsulfanyl]-pyridin-3-carbonsäure amid | 58 | 397,2 |
| **74** | N-(5-Bicyclo[2.2.1]heptanyl-methyl)-2-[[3-(4-fluorphenyl)-1-methyl-propyl]sulfanyl]-pyridin-3-carbonsäure amid | 51 | 413,2 |
| **75** | N-(5-Bicyclo[2.2.1]heptanyl-methyl)-2-[[3-(4-fluorphenyl)-2-methylpropyl]sulfanyl]-pyridin-3-carbonsäure amid | 51 | 413,2 |
| **76** | 2-[3-(4-Fluorphenyl)-propylsulfanyl]-N-[(2-methoxyphenyl)-methyl]-pyridin-3-carbonsäure amid | 42 | 411,1 |
| **77** | 2-[3-(4-Fluorphenyl)-propylsulfanyl]-N-[(3-methoxyphenyl)-methyl]-pyridin-3-carbonsäure amid | 42 | 411,1 |
| **78** | 2-[3-(4-Fluorphenyl)-propylsulfanyl]-N-phenethyl-pyridin-3-carbonsäure amid | 42 | 395,2 |
| **79** | 2-[3-(4-Fluorphenyl)-propylsulfanyl]-N-[[4-(trifluormethyloxy)-phenyl]-methyl]-pyridin-3-carbonsäure amid | 42 | 465,1 |
| **80** | 2-[3-(4-Fluorphenyl)-propylsulfanyl]-N-[[4-(trifluormethyl)-phenyl]-methyl]-pyridin-3-carbonsäure amid | 42 | 449,1 |
| **81** | 2-[3-(4-Fluorphenyl)-propylsulfanyl]-N-(2-pyridin-3-yl-ethyl)-pyridin-3-carbonsäure amid | 42 | 396,1 |
| **82** | 2-[3-(4-Fluorphenyl)-propylsulfanyl]-N-(2-pyridin-2-yl-ethyl)-pyridin-3-carbonsäure amid | 42 | 396,1 |
| **83** | 2-[3-(4-Fluorphenyl)-propylsulfanyl]-N-[(2-hydroxyphenyl)-methyl]-pyridin-3-carbonsäure amid | 58 | 397,1 |
| **84** | 2-[3-(4-Fluorphenyl)-propylsulfanyl]-N-[(3-hydroxyphenyl)-methyl]-pyridin-3-carbonsäure amid | 58 | 397,1 |
| **85** | 2-[3-(4-Fluorphenyl)-propylsulfanyl]-N-[2-(m-tolyl)-ethyl]-pyridin-3-carbonsäure amid | 42 | 409,2 |
| **86** | 2-[3-(4-Fluorphenyl)-propylsulfanyl]-N-[2-(o-tolyl)-ethyl]-pyridin-3-carbonsäure amid | 42 | 409,2 |
| **87** | 2-[3-(4-Fluorphenyl)-propylsulfanyl]-N-[[3-(trifluormethyloxy)-phenyl]-methyl]-pyridin-3-carbonsäure amid | 42 | 465,1 |
| **88** | 2-[3-(4-Fluorphenyl)-propylsulfanyl]-N-[(4-hydroxyphenyl)-methyl]-pyridin-3-carbonsäure amid | 42 | 397,1 |

(fortgesetzt)

| Beispiel-verbindung | Chemische Bezeichnung | Synthese analog Beispiel-verbindung | MS m/z $[M+H]^+$ |
|---|---|---|---|
| **89** | 2-[3-(4-Fluorphenyl)-propylsulfanyl]-N-(2-pyridin-4-yl-ethyl)-pyridin-3-carbonsäure amid | 42 | 396,1 |
| **90** | 2-[3-(4-Fluorphenyl)-propylsulfanyl]-N-[2-(p-tolyl)-ethyl]-pyridin-3-carbonsäure amid | 42 | 409,2 |
| **91** | N-(5-Bicyclo[2.2.1]heptanyl-methyl)-2-[3-(4-fluorphenyl)-butylsulfanyl]-pyridin-3-carbonsäure amid | 51 | 413,2 |
| **92** | N-(5-Bicyclo[2.2.1]heptanyl-methyl)-2-[3-(2,4,5-trifluor-phenyl)-propylsulfanyl]-pyridin-3-carbonsäure amid | 51 | 435,2 |
| **93** | 2-[3-(4-Fluorphenyl)-propylsulfanyl]-N-(3-pyridin-2-yl-propyl)-pyridin-3-carbonsäure amid | 42 | 410,2 |
| **94** | 2-[3-(4-Fluorphenyl)-propylsulfanyl]-N-(3-pyridin-3-yl-propyl)-pyridin-3-carbonsäure amid | 42 | 410,2 |
| **95** | 2-[3-(4-Fluorphenyl)-propylsulfanyl]-N-(3-pyridin-4-yl-propyl)-pyridin-3-carbonsäure amid | 42 | 410,2 |
| **96** | 2-[3-(4-Fluorphenyl)-propylsulfanyl]-N-propyl-pyridin-3-carbonsäure amid | 42 | 333,1 |
| **97** | N-Butyl-2-[3-(4-fluorphenyl)-propylsulfanyl]-pyridin-3-carbonsäure amid | 42 | 347,2 |
| **98** | 2-(3-Pyridin-3-yl-propylsulfanyl)-N-(thiophen-2-yl-methyl)-pyridin-3-carbonsäure amid | 167 | 370,1 |
| **99** | 2-[3-(p-Tolyl)-propylsulfanyl]-N-(thiophen-2-yl-methyl)-pyridin-3-carbonsäure amid | 167 | 383,1 |
| **100** | 2-(4-Phenyl-butylsulfanyl)-N-(thiophen-2-yl-methyl)-pyridin-3-carbonsäure amid | 51 | 383,1 |
| **101** | 2-(3-Pyridin-4-yl-propylsulfanyl)-N-(thiophen-2-yl-methyl)-pyridin-3-carbonsäure amid | 51 | 370,1 |
| **102** | 2-(3-Naphthalen-2-yl-propylsulfanyl)-N-(thiophen-2-yl-methyl)-pyridin-3-carbonsäure amid | 51 | 419,1 |
| **103** | 2-[3-(m-Tolyl)-propylsulfanyl]-N-(thiophen-2-yl-methyl)-pyridin-3-carbonsäure amid | 167 | 383,1 |
| **104** | N-(Thiophen-2-yl-methyl)-2-(3-thiophen-2-yl-propylsulfanyl)-pyridin-3-carbonsäure amid | 167 | 375,1 |
| **105** | 2-[(1-Methyl-3-phenyl-propyl)sulfanyl]-N-(thiophen-2-yl-methyl)-pyridin-3-carbonsäure amid | 51 | 383,1 |
| **106** | N-(Thiophen-2-yl-methyl)-2-(3-thiophen-3-yl-propylsulfanyl)-pyridin-3-carbonsäure amid | 167 | 375,1 |
| **107** | 2-[[1-Methyl-3-[3-(trifluormethyl)phenyl]-propyl]sulfanyl]-N-(thiophen-2-yl-methyl)-pyridin-3-carbonsäure amid | 167 | 451,1 |
| **108** | 2-[(2-Benzyl-cyclohexyl)sulfanyl]-N-(thiophen-2-yl-methyl)-pyridin-3-carbonsäure amid | 115 | 423,1 |
| **109** | 2-[3-[3-Methyl-5-(trifluormethyl)-phenyl]-propylsulfanyl]-N-(thiophen-2-yl-methyl)-pyridin-3-carbonsäure amid | 115 | 451,1 |
| **110** | 2-[4-(3,4-Difluor-phenyl)-butylsulfanyl]-N-(thiophen-2-yl-methyl)-pyridin-3-carbonsäure amid | 115 | 419,1 |

(fortgesetzt)

| Beispiel-verbindung | Chemische Bezeichnung | Synthese analog Beispiel-verbindung | MS m/z $[M+H]^+$ |
|---|---|---|---|
| **111** | 2-(3-Pyridin-2-yl-propylsulfanyl)-N-(thiophen-2-yl-methyl)-pyridin-3-carbonsäure amid | 225 | 370,1 |
| **113** | 2-[3-[4-Methyl-3-(trifluormethyl)-phenyl]-propylsulfanyl]-N-(thiophen-2-yl-methyl)-pyridin-3-carbonsäure amid | 115 | 451,1 |
| **114** | 2-[(3-Phenyl-cyclohexyl)sulfanyl]-N-(thiophen-2-yl-methyl)-pyridin-3-carbonsäure amid | 115 | 409,1 |
| **116** | 2-[[3,3-Difluor-3-(4-fluorphenyl)-propyl]sulfanyl]-N-(3,3-dimethyl-butyl)-pyridin-3-carbonsäure amid | 115 | 411,2 |
| **117** | N-(Cycloheptyl-methyl)-2-[[3,3-difluor-3-(4-fluorphenyl)-propyl]sulfanyl]-pyridin-3-carbonsäure amid | 115 | 437,2 |
| **118** | 2-[[3,3-Difluor-3-(4-fluorphenyl)-propyl]sulfanyl]-N-(thiophen-2-yl-methyl)-pyridin-3-carbonsäure amid | 115 | 423,1 |
| **119** | 2-[3-(4-Fluorphenyl)-propylsulfanyl]-N-(1,2,3,4-tetrahydro-naphthalen-2-yl-methyl)-pyridin-3-carbonsäure amid | 169 | 435,2 |
| **120** | N-(2,3-Dihydro-1H-inden-2-yl-methyl)-2-[3-(4-fluorphenyl)-propylsulfanyl]-pyridin-3-carbonsäure amid | 169 | 421,2 |
| **121** | N-(1,3-Benzodioxol-5-yl-methyl)-2-[3-(4-fluorphenyl)-propylsulfanyl]-pyridin-3-carbonsäure amid | 169 | 425,1 |
| **122** | 2-[3-(4-Fluorphenyl)-propylsulfanyl]-N-[(3-phenyl-phenyl)-methyl]-pyridin-3-carbonsäure amid | 169 | 457,2 |
| **123** | 2-[3-(4-Fluorphenyl)-propylsulfanyl]-N-[(3-methyl-cyclohexyl)-methyl]-pyridin-3-carbonsäure amid | 169 | 401,2 |
| **124** | 2-[[3,3-Difluor-3-(4-fluorphenyl)-propyl]sulfanyl]-N-[(4-fluorphenyl)-methyl]-pyridin-3-carbonsäure amid | 115 | 435,1 |
| **125** | N-(3,3-Dimethyl-2-oxo-butyl)-2-[3-(4-fluorphenyl)-propylsulfanyl]-pyridin-3-carbonsäure amid | 169 | 389,2 |
| **127** | 2-[3-(4-Fluorphenyl)-propylsulfanyl]-N-(pyridin-3-yl-methyl)-pyridin-3-carbonsäure amid | 169 | 382,1 |
| **128** | 2-[3-(4-Fluorphenyl)-propylsulfanyl]-N-(pyridin-4-yl-methyl)-pyridin-3-carbonsäure amid | 169 | 382,1 |
| **129** | 3-[[[2-[3-(4-Fluorphenyl)-propylsulfanyl]-pyridin-3-carbonyl]amino]-methyl]-benzoesäure methyl ester | 169 | 439,1 |
| **130** | 2-[3-(4-Fluorphenyl)-propylsulfanyl]-N-[3-(2-methoxyphenyl)-propyl]-pyridin-3-carbonsäure amid | 169 | 439,2 |
| **132** | N-[(4-Fluorphenyl)-methyl]-2-[[1-methyl-3-[3-(trifluormethyl)phenyl]-propyl]sulfanyl]-pyridin-3-carbonsäure amid | 51 | 463,1 |
| **133** | 2-[3-(4-Fluorphenyl)-propylsulfanyl]-N-(3-methyl-butyl)-pyridin-3-carbonsäure amid | 169 | 361,2 |

(fortgesetzt)

| Beispiel-verbindung | Chemische Bezeichnung | Synthese analog Beispiel-verbindung | MS m/z $[M+H]^+$ |
|---|---|---|---|
| **134** | 2-[3-(4-Fluorphenyl)-propylsulfanyl]-N-(tetrahydro-pyran-2-yl-methyl)-pyridin-3-carbonsäure amid | 169 | 389,2 |
| **135** | 2-[3-(4-Fluorphenyl)-propylsulfanyl]-N-[3-(1 H-pyrazol-1-yl)-propyl]-pyridin-3-carbonsäure amid | 169 | 399,2 |
| **136** | 2-[3-(4-Fluorphenyl)-propylsulfanyl]-N-(naphthalen-2-yl-methyl)-pyridin-3-carbonsäure amid | 169 | 431,2 |
| **137** | N-(2,3-Dihydro-[1,4]benzodioxin-6-yl-methyl)-2-[3-(4-fluorphenyl)-propylsulfanyl]-pyridin-3-carbonsäure amid | 169 | 439,1 |
| **138** | 2-[3-(4-Fluorphenyl)-propylsulfanyl]-N-[(3-morpholin-4-yl-phenyl)-methyl]-pyridin-3-carbonsäure amid | 169 | 466,2 |
| **139** | N-(2,3-Dihydro-benzofuran-6-yl-methyl)-2-[3-(4-fluorphenyl)-propylsulfanyl]-pyridin-3-carbonsäure amid | 169 | 423,1 |
| **140** | 2-[3-(4-Fluorphenyl)-propylsulfanyl]-N-[[3-(1H-pyrazol-1-yl)-phenyl]-methyl]-pyridin-3-carbonsäure amid | 169 | 447,2 |
| **141** | 2-[3-(4-Fluorphenyl)-propylsulfanyl]-N-[3-(1 H-[1,2,3]triazol-1-yl)-propyl]-pyridin-3-carbonsäure amid | 169 | 400,2 |
| **142** | N-(7-Bicyclo[2.2.1]heptanyl-methyl)-2-[3-(4-fluorphenyl)-propylsulfanyl]-pyridin-3-carbonsäure amid | 169 | 399,2 |
| **144** | 2-[3-(4-Fluorphenyl)-propylsulfanyl]-N-(thiazol-2-yl-methyl)-pyridin-3-carbonsäure amid | 143 | 388,1 |
| **145** | 2-[3-(4-Fluorphenyl)-propylsulfanyl]-N-(thiazol-5-yl-methyl)-pyridin-3-carbonsäure amid | 143 | 388,1 |
| **146** | 2-[3-(4-Fluorphenyl)-propylsulfanyl]-N-(oxazol-2-yl-methyl)-pyridin-3-carbonsäure amid | 143 | 372,1 |
| **148** | N-(3,3-Dimethyl-butyl)-2-[3-(4-fluorphenyl)-propylsulfanyl]-4-methyl-pyridin-3-carbonsäure amid | 147 | 389,2 |
| **149** | N-(3,3-Dimethyl-butyl)-2-[[1-methyl-3-[3-(trifluormethyl)phenyl]-propyl]sulfanyl]-pyridin-3-carbonsäure amid | 115 | 439,2 |
| **150** | 2-[3-(4-Fluorphenyl)-propylsulfanyl]-N-(chinolin-7-yl-methyl)-pyridin-3-carbonsäure amid | 143 | 432,1 |
| **151** | 2-[3-(4-Fluorphenyl)-propylsulfanyl]-N-[(3-pyridin-2-yl-phenyl)-methyl]-pyridin-3-carbonsäure amid | 143 | 458,2 |
| **152** | 2-[3-(4-Fluorphenyl)-propylsulfanyl]-N-[(3-pyridin-3-yl-phenyl)-methyl]-pyridin-3-carbonsäure amid | 143 | 458,2 |
| **153** | N-(3,3-Dimethyl-butyl)-2-[[(1R)-1-methyl-3-phenyl-propyl]sulfanyl]-pyridin-3-carbonsäure amid | 115 | 371,2 |
| **154** | N-(3,3-Dimethyl-butyl)-2-[[(1S)-1-methyl-3-phenyl-propyl]sulfanyl]-pyridin-3-carbonsäure amid | 115 | 371,2 |

(fortgesetzt)

| Beispiel-verbindung | Chemische Bezeichnung | Synthese analog Beispiel-verbindung | MS m/z $[M+H]^+$ |
|---|---|---|---|
| **155** | 2-[(2-Benzyl-cyclopentyl)sulfanyl]-N-(3,3-dimethyl-butyl)-pyridin-3-carbonsäure amid | 115 | 397,2 |
| **156** | N-(7-Bicyclo[2.2.1]heptanyl-methyl)-2-[3-[3-(trifluormethyl)phenyl]-propylsulfanyl]-pyridin-3-carbonsäure amid | 169 | 449,2 |
| **157** | 2-[3-(4-Fluorphenyl)-propylsulfanyl]-N-[3-(1H-[1,2,4]triazol-1-yl)-propyl]-pyridin-3-carbonsäure amid | 143 | 400,2 |
| **158** | 2-[3-(4-Fluorphenyl)-propylsulfanyl]-N-([1,3,4]oxadiazol-2-yl-methyl)-pyridin-3-carbonsäure amid | 143 | 373,1 |
| **159** | 2-[3-(4-Fluorphenyl)-propylsulfanyl]-N-[(3-pyridin-4-yl-phenyl)-methyl]-pyridin-3-carbonsäure amid | 143 | 458,2 |
| **160** | N-[[4-(Cyclopropyl-methyl)-3,4-dihydro-2H-[1,4]benzoxazin-6-yl]-methyl]-2-[3-(4-fluorphenyl)-propylsulfanyl]-pyridin-3-carbonsäure amid | 169 | 492,2 |
| **161** | N-[(4-Ethyl-3,4-dihydro-2H-[1,4]benzoxazin-6-yl)-methyl]-2-[3-(4-fluorphenyl)-propylsulfanyl]-pyridin-3-carbonsäure amid | 169 | 466,2 |
| **162** | 2-[3-(4-Fluorphenyl)-propylsulfanyl]-N-[(4-methyl-3,4-dihydro-2H-[1,4]benzoxazin-6-yl)-methyl]-pyridin-3-carbonsäure amid | 169 | 452,2 |
| **163** | 2-[3-(4-Fluorphenyl)-propylsulfanyl]-N-(3-methyl-3-phenyl-butyl)-pyridin-3-carbonsäure amid | 169 | 437,2 |
| **164** | N-(7-Bicyclo[2.2.1]heptanyl-methyl)-2-[[3,3-difluor-3-(4-fluorphenyl)-propyl]sulfanyl]-pyridin-3-carbonsäure amid | 115 | 435,2 |
| **165** | 2-[[3,3-Difluor-3-[3-(trifluormethyl)phenyl]-propyl]sulfanyl]-N-[(4-fluorphenyl)-methyl]-pyridin-3-carbonsäure amid | 115 | 485,1 |
| **166** | 2-[[3,3-Difluor-3-[3-(trifluormethyl)phenyl]-propyl]sulfanyl]-N-(3,3-dimethyl-butyl)-pyridin-3-carbonsäure amid | 169 | 461,2 |
| **168** | 2-[[3,3-Difluor-3-(4-fluorphenyl)-propyl]sulfanyl]-N-[(3-fluorphenyl)-methyl]-pyridin-3-carbonsäure amid | 169 | 435,1 |
| **170** | N-Butyl-2-[[3,3-difluor-3-(4-fluorphenyl)-propyl]sulfanyl]-pyridin-3-carbonsäure amid | 169 | 383,1 |
| **171** | 2-[[3,3-Difluor-3-(4-fluorphenyl)-propyl]sulfanyl]-N-(2-methoxy-ethyl)-pyridin-3-carbonsäure amid | 169 | 385,1 |
| **172** | 2-[3-(4-Fluorphenyl)-propylsulfanyl]-N-(2-methoxy-ethyl)-pyridin-3-carbonsäure amid | 169 | 349,1 |
| **173** | N-[(4-Fluor-2-hydroxy-phenyl)-methyl]-2-[3-(4-fluorphenyl)-propylsulfanyl]-pyridin-3-carbonsäure amid | 58 | 415,1 |

(fortgesetzt)

| Beispiel-verbindung | Chemische Bezeichnung | Synthese analog Beispiel-verbindung | MS m/z $[M+H]^+$ |
|---|---|---|---|
| **174** | 2-[3-(4-Fluorphenyl)-propylsulfanyl]-N-(furan-2-yl-methyl)-pyridin-3-carbonsäure amid | 169 | 371,1 |
| **175** | 2-[3-(4-Fluorphenyl)-propylsulfanyl]-N-[(5-methyl-furan-2-yl)-methyl]-pyridin-3-carbonsäure amid | 169 | 385,1 |
| **176** | N-[(4-Fluorphenyl)-methyl]-2-[[3-(4-fluorphenyl)-1-methyl-propyl]sulfanyl]-pyridin-3-carbonsäure amid | 169 | 413,1 |
| **177** | 2-[[3-(4-Fluorphenyl)-1-methylpropyl]sulfanyl]-N-(3-methyl-butyl)-pyridin-3-carbonsäure amid | 169 | 375,2 |
| **178** | N-(7-Bicyclo[2.2.1]heptanyl-methyl)-2-[[3-(4-fluorphenyl)-1-methyl-propyl]sulfanyl]-pyridin-3-carbonsäure amid | 169 | 413,2 |
| **179** | 2-[[3,3-Difluor-3-(4-fluorphenyl)-propyl]sulfanyl]-N-(tetrahydro-furan-2-yl-methyl)-pyridin-3-carbonsäure amid | 169 | 411,1 |
| **180** | 2-[[3,3-Difluor-3-(4-fluorphenyl)-propyl]sulfanyl]-N-(tetrahydro-pyran-2-yl-methyl)-pyridin-3-carbonsäure amid | 169 | 425,1 |
| **181** | 2-[3-(4-Fluorphenyl)-propylsulfanyl]-N-(tetrahydro-furan-2-yl-methyl)-pyridin-3-carbonsäure amid | 169 | 375,1 |
| **182** | 2-[3-(4-Fluorphenyl)-propylsulfanyl]-N-(2-methoxy-3,3-dimethyl-butyl)-pyridin-3-carbonsäure amid | 169 | 405,2 |
| **183** | 2-[[3,3-Difluor-3-(4-fluorphenyl)-propyl]sulfanyl]-N-(m-tolyl-methyl)-pyridin-3-carbonsäure amid | 169 | 431,1 |
| **184** | 2-[[3,3-Difluor-3-(4-fluorphenyl)-propyl]sulfanyl]-N-[(3,5-dimethyl-phenyl)-methyl]-pyridin-3-carbonsäure amid | 169 | 445,1 |
| **185** | 2-[[3,3-Difluor-3-(4-fluorphenyl)-propyl]sulfanyl]-N-propyl-pyridin-3-carbonsäure amid | 169 | 369,1 |
| **186** | 2-[[3,3-Difluor-3-(4-fluorphenyl)-propyl]sulfanyl]-N-hexyl-pyridin-3-carbonsäure amid | 169 | 411,2 |
| **187** | 2-[[3,3-Difluor-3-(4-fluorphenyl)-propyl]sulfanyl]-N-(tetrahydro-furan-3-yl-methyl)-pyridin-3-carbonsäure amid | 169 | 411,1 |
| **188** | 2-[[3,3-Difluor-3-(4-fluorphenyl)-propyl]sulfanyl]-N-(tetrahydro-pyran-3-yl-methyl)-pyridin-3-carbonsäure amid | 169 | 425,1 |
| **189** | 2-[[3,3-Difluor-3-(4-fluorphenyl)-propyl]sulfanyl]-N-(tetrahydro-pyran-4-yl-methyl)-pyridin-3-carbonsäure amid | 169 | 425,1 |
| **190** | 2-[[3,3-Difluor-3-(4-fluorphenyl)-propyl]sulfanyl]-N-(furan-2-yl-methyl)-pyridin-3-carbonsäure amid | 169 | 407,1 |
| **191** | 2-[[3,3-Difluor-3-(4-fluorphenyl)-propyl]sulfanyl]-N-[(5-methyl-furan-2-yl)-methyl]-pyridin-3-carbonsäure amid | 169 | 421,1 |

(fortgesetzt)

| Beispiel-verbindung | Chemische Bezeichnung | Synthese analog Beispiel-verbindung | MS m/z $[M+H]^+$ |
|---|---|---|---|
| **192** | 2-[[3,3-Difluor-3-(4-fluorphenyl)-propyl]sulfanyl]-N-pentyl-pyridin-3-carbonsäure amid | 169 | 397,1 |
| **193** | 2-[[3,3-Difluor-3-(4-fluorphenyl)-propyl]sulfanyl]-N-(3-methoxy-butyl)-pyridin-3-carbonsäure amid | 169 | 413,1 |
| **194** | 2-[[3,3-Difluor-3-(4-fluorphenyl)-propyl]sulfanyl]-N-(2-methoxy-propyl)-pyridin-3-carbonsäure amid | 169 | 399,1 |
| **195** | 2-[[3,3-Difluor-3-(4-fluorphenyl)-propyl]sulfanyl]-N-(2-methoxy-butyl)-pyridin-3-carbonsäure amid | 169 | 413,1 |
| **196** | 3-[[2-[[3,3-Difluor-3-(4-fluorphenyl)-propyl]sulfanyl]-pyridin-3-carbonyl]amino]-propionsäure methyl ester | 169 | 413,1 |
| **197** | 3-[[2-[[3,3-Difluor-3-(4-fluorphenyl)-propyl]sulfanyl]-pyridin-3-carbonyl]amino]-propionsäure | 131 | 399,1 |
| **198** | 2-[[3,3-Difluor-3-(4-fluorphenyl)-propyl]sulfanyl]-N-(2-dimethylaminoethyl)-pyridin-3-carbonsäure amid | 169 | 398,1 |
| **199** | 2-[[3-(3,4-Difluor-phenyl)-1-methyl-propyl]sulfanyl]-N-[(4-fluorphenyl)-methyl]-pyridin-3-carbonsäure amid | 169 | 431,1 |
| **200** | 2-[[3-(3,4-Difluor-phenyl)-1-methyl-propyl]sulfanyl]-N-(3-methyl-butyl)-pyridin-3-carbonsäure amid | 169 | 393,2 |
| **201** | N-[(4-Fluorphenyl)-methyl]-2-[[3-(3-fluorphenyl)-1-methyl-propyl]sulfanyl]-pyridin-3-carbonsäure amid | 169 | 413,1 |
| **202** | 2-[[3-(3-Fluorphenyl)-1-methylpropyl]sulfanyl]-N-(3-methyl-butyl)-pyridin-3-carbonsäure amid | 169 | 375,2 |
| **203** | N-(3-Methyl-butyl)-2-[[1-methyl-3-[3-(trifluormethyl)phenyl]-propyl]sulfanyl]-pyridin-3-carbonsäure amid | 169 | 425,2 |
| **204** | 2-[[3-(3,4-Difluor-phenyl)-3,3-difluor-propyl]sulfanyl]-N-[(4-fluorphenyl)-methyl]-pyridin-3-carbonsäure amid | 169 | 453,1 |
| **205** | N-(1-Bicyclo[2.2.1]heptanyl-methyl)-2-[[3,3-difluor-3-(4-fluorphenyl)-propyl]sulfanyl]-pyridin-3-carbonsäure amid | 169 | 435,2 |
| **206** | N-(1-Bicyclo[2.2.1]heptanyl-methyl)-2-[3-(4-fluorphenyl)-propylsulfanyl]-pyridin-3-carbonsäure amid | 169 | 399,2 |
| **207** | N-[(4-Fluorphenyl)-methyl]-2-[3-[3-(trifluormethyl)phenyl]-propylsulfanyl]-pyridin-3-carbonsäure amid | 169 | 449,1 |
| **208** | N-(3-Methyl-butyl)-2-[3-[3-(trifluormethyl)phenyl]-propylsulfanyl]-pyridin-3-carbonsäure amid | 169 | 411,2 |

(fortgesetzt)

| Beispiel-verbindung | Chemische Bezeichnung | Synthese analog Beispiel-verbindung | MS m/z $[M+H]^+$ |
|---|---|---|---|
| **209** | 2-[[3-(3,4-Difluor-phenyl)-3,3-difluor-propyl]sulfanyl]-N-(3-methyl-butyl)-pyridin-3-carbonsäure amid | 169 | 415,1 |
| **210** | N-(7-Bicyclo[2.2.1]heptanyl-methyl)-2-[[3-(3,4-difluor-phenyl)-3,3-difluor-propyl]sulfanyl]-pyridin-3-carbonsäure amid | 169 | 453,2 |
| **211** | 2-[[3,3-Difluor-3-(4-fluorphenyl)-propyl]sulfanyl]-N-(2-hydroxy-ethyl)-pyridin-3-carbonsäure amid | 169 | 371,1 |
| **224** | N-[(4-Fluor-2-methoxy-phenyl)-methyl]-2-[3-(4-fluorphenyl)-propylsulfanyl]-pyridin-3-carbonsäure amid | 169 | 429,1 |

**Pharmakologische Experimente**

**Fluoreszenzassay unter Verwendung eines 'voltage sensitive dyes'**

Humane, KCNQ2/3-Kanäle exprimierende CHO-K1-Zellen werden in Zellkultur-flaschen (z.B. 80 $cm^2$ TC flasks, Nunc) mit DMEM-high glucose (Sigma Aldrich, D7777) inklusive 10% FCS (PAN Biotech, z.B. 3302-P270521) oder alternativ MEM Alpha Medium (1x, flüssig, Invitrogen, #22571), 10 % Fetal Calf Serum (FCS) (Invitrogen, #10270-106, hitzeinaktiviert) und den notwendigen Selektionsantibiotika bei 37°C, 5 % $CO_2$ und 95 % Luftfeuchtigkeit adhärent kultiviert.

Vor Aussaat für die Messungen werden die Zellen mit einem 1 x DPBS-Puffer ohne $Ca^{2+}$/$Mg^{2+}$ (z.B. Invitrogen, #14190-094) gewaschen und mittels Accutase (PAA Laboratories, #L11-007) vom Boden des Kulturgefäßes abgelöst (Inkubation mit Accutase für 15 min bei 37°C). Die Bestimmung der dann vorliegenden Zellzahl wird mit einem CASY™ cell counter (Modell TCC, Schärfe System) durchgeführt, um anschließend je nach Dichteoptimierung für die individuelle Zelllinie 20.000 - 30.000 Zellen/well/100 µl des beschriebenen Nährmediums auf die 96 well-Messplatten des Typs Corning™ CellBIND™ (Flat Clear Bottom Black Polystyrene Microplates, #3340) auszubringen. Danach erfolgt eine einstündige Inkubation bei Raumtemperatur ohne Begasung oder Regelung der Luftfeuchtigkeit, gefolgt von einer 24stündigen Inkubation bei 37°C, 5 % $CO_2$ und 95 % Luftfeuchtigkeit.

Der spannungssensitive Fluoreszenzfarbstoff aus dem Membrane Potential Assay Kit (Red™ Bulk format part R8123 for FLIPR, MDS Analytical Technologies™) wird vorbereitet, indem der Inhalt eines Gefäßes *Membrane Potential Assay Kit Red Component A* in 200 ml Extrazellulären Puffers (ES-Puffer, 120 mM NaCl, 1 mM KCl, 10 mM HEPES, 2 mM $CaCl_2$, 2 mM $MgCl_2$, 10 mM Glucose; pH 7,4) gelöst wird. Nach Abnahme des Nährmediums werden die Zellen mit 200 µl ES-Puffer gewaschen, anschließend mit 100 µl der oben angesetzten Farbstofflösung überschichtet und 45 min bei Raumtemperatur unter Lichtverschluss inkubiert.

Die Fluoreszenzmessungen werden mit einem BMG Labtech FLUOstar™-, BMG Labtech NOVOstar™- oder BMG Labtech POLARstar™-Instrument durchgeführt (525 nm Exication, 560 nm Emission, Bottom Read mode). Nach der Farbstoff-Inkubation werden 50 µl der zu testenden Substanzen in den gewünschten Konzentrationen oder 50 µl ES-Puffer zwecks Kontrolle in separate Cavitäten der Messplatte gegeben und 30 min bei Raumtemperatur unter Abschirmung von Licht inkubiert. Anschließend misst man für 5 min die Fluoreszenzintensität des Farbstoffs und ermittelt so zu einem festgelegten und gleichbleibenden Zeitpunkt den Fluoreszenzwert $F_1$ eines jeden wells. Daraufhin erfolgt Zugabe von 15 µl einer 100 mM KCl-Lösung (Endkonzentration 92 mM) in jedes well. Die Veränderung der Fluoreszenz wird anschließend so lange gemessen, bis alle relevanten Messwerte erhalten sind (vornehmlich 5-30 min). Zu einem festgelegten Zeitpunkt nach KCl-Applikation wird ein Fluoreszenzwert $F_2$ ermittelt, in diesem Falle zum Zeitpunkt des Fluoreszenzpeaks.

Zur Berechnung wird die Fluoreszenzintensität $F_2$ mit der Fluoreszenzintensität $F_1$ verglichen und daraus die agonistische Aktivität der Zielverbindung auf den Kaliumkanal ermittelt. $F_2$ und $F_1$ werden hierfür wie folgt verrechnet:

$$\left(\frac{F_2 - F_1}{F_1}\right) \times 100 = \frac{\Delta F}{F} (\%)$$

Um zu ermitteln, ob eine Substanz eine agonistische Aktivität besitzt, kann z.B. $\frac{\Delta F}{F}$ mit $\left(\frac{\Delta F}{F}\right)_K$ von Kontrollzellen verglichen werden. $\left(\frac{\Delta F}{F}\right)_K$ wird ermittelt, indem man dem Reaktionsansatz anstatt der zu testenden Substanz lediglich die Pufferlösung zusetzt, den Wert $F_{1K}$ der Fluoreszenzintensität bestimmt, die Kaliumionen wie oben beschrieben zugibt und einen Wert $F_{2K}$ der Fluoreszenzintensität misst. Dann werden $F_{2K}$ und $F_{1K}$ wie folgt verrechnet:

$$\left(\frac{F_{2K} - F_{1K}}{F_{1K}}\right) \times 100 = \left(\frac{\Delta F}{F}\right)_K (\%)$$

Eine Substanz besitzt eine agonistische Aktivität auf den Kaliumkanal, wenn $\frac{\Delta F}{F}$ größer als $\left(\frac{\Delta F}{F}\right)_K$ ist:

$$\frac{\Delta F}{F} \rangle \left(\frac{\Delta F}{F}\right)_K$$

Unabhängig vom Vergleich von $\frac{\Delta F}{F}$ mit $\left(\frac{\Delta F}{F}\right)_K$ lässt sich auch auf eine agonistische Aktivität einer Zielverbindung schließen, wenn mit steigender Dosierung der Zielverbindung eine Zunahme von $\frac{\Delta F}{F}$ zu beobachten ist. Kalkulationen von $EC_{50}$-Werten werden mit Hilfe der Software 'Prism v4.0' (GraphPad Software™) durchgeführt.

**Low-intensity tail flick an der Ratte**

Die antinociceptive Wirksamkeit der Testsubstanz gegenüber eines akuten, noxischen thermischen Reizes wurde im Brennstrahl (Tail flick) -Test an der Ratte nach der Methode von D'Amour und Smith (J. Pharm. Exp. Ther. 72, 74 79 (1941)) untersucht. Dazu wurden männliche Sprague-Dawley Ratten (Züchter: Janvier, Le Genest St. Isle, Frankreich) mit einem Gewicht zwischen 200 - 250 g verwendet. Die Tiere wurden einzeln in spezielle Testkompartimente verbracht und die Schwanzbasis einem fokussierten Brennstrahl eines Analgesiemeters (Modell 2011, Rhema Labortechnik, Hofheim, Deutschland) ausgesetzt. Es wurden 10 Tiere pro Gruppe verwendet. Vor Gabe einer erfindungsgemäßen Substanz wurde im Abstand von fünf Minuten zweimal die Wegziehlatenz (Zeit vom Einschalten des Brennstrahls bis zum plötzlichen Wegziehen des Schwanzes) bestimmt und der Mittelwert als Kontroll-Latenzzeit definiert. Die Brennstrahlintensität war dabei so gewählt, dass die Kontroll-Latenzzeit 7 - 9 Sekunden betrug. Die Messung der Wegziehlatenz wurde dann 10, 20, 30 und 60 Minuten nach peroraler Substanzgabe wiederholt. Die antinociceptive Wirkung der Testsubstanz wurde als Zunahme der Wegziehlatenzzeit nach folgender Formel bestimmt:

$$MPE\ [\%] = [(T_1 - T_0)/(T_2 - T_0)] \times 100$$

Dabei sind: $T_0$: Kontroll-Latenzzeit vor Substanzapplikation, $T_1$: Latenzzeit nach Substanzapplikation, $T_2$: maximale Expositionszeit des Brennstrahls (30 Sekunden), MPE: maximum possible effect (maximal möglicher Effekt).
Mittels Varianzanalyse (repeated measures ANOVA) wurde auf statistisch signifikante Unterschiede zwischen Substanz- und Vehikelgruppe getestet. Das Signifikanzniveau wurde auf ≤ 0.05 gesetzt.

**Pharmakologische Daten**

In Tabelle 3 sind die Ergebnisse aus den zuvor beschriebenen pharmakologischen Modellen zusammengefasst.

Tabelle 3:

| Beispiel-verbindung | Fluorimetrie $EC_{50}$ [nM] | Fluorimetrie % Efficacy (Retigabine 50 µM = 100%) | low intensity tail flick Ratte p.o. % Effekt (Dosis [mg/kg]) |
|---|---|---|---|
| **1** | 170 | 132 | |
| **2** | 1349 | 97 | |
| **3** | | 53 | |
| **4** | 772 | 126 | |
| **5** | 435 | 161 | |
| **7** | | 91 | |
| **8** | | 37 | |
| **9** | 1354 | 157 | |

(fortgesetzt)

| Beispiel-verbindung | Fluorimetrie $EC_{50}$ [nM] | Fluorimetrie % Efficacy (Retigabine 50 $\mu$M = 100%) | low intensity tail flick Ratte p.o. % Effekt (Dosis [mg/kg]) |
|---|---|---|---|
| **10** | | 13 | |
| **12** | 2919 | 170 | |
| **13** | 302 | 200 | |
| **16** | 74 | 176 | |
| **17** | 128 | 176 | |
| **18** | 111 | 160 | |
| **19** | 37 | 171 | |
| **20** | 49 | 174 | |
| **21** | 364 | 126 | |
| **22** | 2350 | 150 | |
| **23** | 3766 | 163 | |
| **24** | 11 | 122 | |
| **25** | 1016 | 216 | |
| **26** | 126 | 244 | |
| **27** | 85 | 210 | 18 (10,0) |
| **28** | 1571 | 192 | |
| **29** | 275 | 159 | |
| **30** | 630 | 261 | |
| **31** | 437 | 98 | |
| **32** | 3125 | 191 | |
| **33** | 451 | 242 | |
| **34** | 463 | 204 | |
| **35** | 242 | 221 | |
| **36** | 45 | 78 | |
| **37** | 74 | 89 | |
| **38** | 144 | 176 | |
| **39** | 250 | 250 | |
| **40** | 129 | 144 | 26 (21,5) |
| **41** | 157 | 159 | |
| **42** | 128 | 164 | |
| **43** | 896 | 46 | |
| **44** | 2962 | 167 | |
| **45** | 1606 | 224 | |
| **46** | 672 | 238 | |
| **47** | 218 | 183 | |
| **48** | 148 | 183 | |
| **49** | 231 | 174 | |

(fortgesetzt)

| Beispiel-verbindung | Fluorimetrie $EC_{50}$ [nM] | Fluorimetrie % Efficacy (Retigabine 50 $\mu$M = 100%) | low intensity tail flick Ratte p.o. % Effekt (Dosis [mg/kg]) |
|---|---|---|---|
| **50** | 1491 | 169 | |
| **51** | 198 | 192 | 49 (21,5) |
| **52** | 496 | 190 | |
| **53** | 302 | 220 | 0 (10,0) |
| **54** | 133 | 240 | 20 (10,0) |
| **55** | 227 | 149 | |
| **56** | 1014 | 251 | |
| **57** | 252 | 83 | |
| **58** | 3875 | 174 | |
| **59** | 210 | 83 | |
| **60** | 263 | 236 | |
| **61** | 221 | 283 | |
| **62** | 182 | 274 | |
| **63** | 34 | 72 | |
| **64** | 49 | 100 | |
| **65** | 40 | 97 | 49 (10,0) |
| **66** | 50 | 121 | |
| **67** | 113 | 290 | |
| **68** | 65 | 280 | 32 (10,0) |
| **69** | 140 | 259 | |
| **70** | 244 | 308 | |
| **71** | 72 | 107 | |
| **72** | 85 | 85 | |
| **73** | 377 | 268 | |
| **74** | 62 | 260 | |
| **75** | 37 | 261 | |
| **76** | | 33 | |
| **77** | 233 | 118 | |
| **78** | 319 | 53 | |
| **79** | 101 | 103 | |
| **80** | 77 | 104 | |
| **81** | 8023 | 89 | |
| **82** | 6457 | 96 | |
| **83** | 139 | 171 | |
| **84** | 3512 | 55 | |
| **85** | 394 | 59 | |
| **86** | | 26 | |

(fortgesetzt)

| Beispiel-verbindung | Fluorimetrie $EC_{50}$ [nM] | Fluorimetrie % Efficacy (Retigabine 50 $\mu$M = 100%) | low intensity tail flick Ratte p.o. % Effekt (Dosis [mg/kg]) |
|---|---|---|---|
| **87** | 101 | 116 | |
| **88** | 3759 | 53 | |
| **89** | 5447 | 72 | |
| **90** | | 37 | |
| **91** | 184 | 241 | |
| **92** | 138 | 270 | |
| **93** | 2809 | 154 | |
| **94** | 2215 | 181 | |
| **95** | 2061 | 147 | |
| **96** | 660 | 194 | |
| **97** | 134 | 196 | |
| **98** | | 29 | |
| **99** | 178 | 70 | |
| **100** | 1592 | 48 | |
| **101** | | 38 | |
| **102** | 456 | 111 | |
| **103** | 111 | 129 | |
| **104** | 361 | 87 | |
| **105** | 479 | 166 | |
| **106** | 191 | 95 | |
| **107** | 57 | 119 | 20 (21,5) |
| **108** | 165 | 143 | |
| **109** | 180 | 129 | |
| **110** | 462 | 110 | |
| **111** | 11061 | 61 | |
| **112** | 494 | 104 | |
| **113** | 5199 | 90 | |
| **114** | 553 | 128 | |
| **115** | 42 | 213 | |
| **116** | 62 | 186 | $ED_{50}$ 5,4 |
| **117** | 35 | 160 | |
| **118** | 37 | 159 | |
| **119** | | 30 | |
| **120** | 307 | 61 | |
| **121** | 256 | 99 | |
| **122** | 153 | 94 | |
| **123** | 155 | 168 | |

(fortgesetzt)

| Beispiel-verbindung | Fluorimetrie $EC_{50}$ [nM] | Fluorimetrie % Efficacy (Retigabine 50 μM = 100%) | low intensity tail flick Ratte p.o. % Effekt (Dosis [mg/kg]) |
|---|---|---|---|
| **124** | 17 | 114 | $ED_{50}$ 3,5 |
| **125** | 1404 | 99 | |
| **126** | 627 | 177 | |
| **127** | 1857 | 147 | |
| **128** | 1846 | 122 | |
| **129** | 325 | 122 | |
| **130** | 465 | 164 | |
| **131** | | 25 | |
| **132** | 82 | 120 | |
| **133** | 106 | 185 | 61 (10,0) |
| **134** | 268 | 224 | 40 (21,5) |
| **135** | 3143 | 154 | |
| **136** | | 28 | |
| **137** | 366 | 93 | |
| **138** | 417 | 92 | |
| **139** | 101 | 81 | |
| **140** | 312 | 88 | |
| **141** | 12773 | 86 | |
| **142** | 97 | 260 | |
| **143** | 1513 | 175 | |
| **144** | 815 | 215 | |
| **145** | 1493 | 174 | |
| **146** | 2551 | 193 | |
| **147** | | 34 | |
| **148** | 1958 | 85 | |
| **149** | 93 | 181 | |
| **150** | 1492 | 96 | |
| **151** | 667 | 95 | |
| **152** | 1292 | 89 | |
| **153** | 1251 | 155 | |
| **154** | 237 | 207 | |
| **155** | 657 | 159 | |
| **156** | 110 | 249 | |
| **157** | | 88 | |
| **158** | | 30 | |
| **159** | 670 | 113 | |
| **160** | 228 | 74 | |

(fortgesetzt)

| Beispiel-verbindung | Fluorimetrie $EC_{50}$ [nM] | Fluorimetrie % Efficacy (Retigabine 50 $\mu$M = 100%) | low intensity tail flick Ratte p.o. % Effekt (Dosis [mg/kg]) |
|---|---|---|---|
| **161** | 145 | 67 | |
| **162** | 166 | 83 | |
| **163** | 537 | 89 | |
| **164** | 32 | 287 | 20 (4,64) |
| **165** | 30 | 119 | |
| **166** | 46 | 206 | |
| **167** | 2714 | 77 | |
| **168** | 17 | 110 | |
| **169** | 19 | 224 | $ED_{50}$ 2,7 |
| **170** | 24 | 229 | 20 (4,64) |
| **171** | 432 | 181 | |
| **172** | 1046 | 196 | |
| **173** | 156 | 185 | |
| **174** | 134 | 213 | |
| **175** | 126 | 173 | |
| **176** | 40 | 108 | |
| **177** | 76 | 192 | |
| **178** | 96 | 270 | |
| **179** | 378 | 234 | |
| **180** | 47 | 224 | 64(10,0) |
| **181** | 1552 | 254 | |
| **182** | 366 | 110 | |
| **183** | 43 | 109 | |
| **184** | | 23 | |
| **185** | 167 | 212 | |
| **186** | 236 | 191 | |
| **187** | 805 | 231 | |
| **188** | 315 | 210 | |
| **189** | 287 | 161 | |
| **190** | 26 | 214 | |
| **191** | 37 | 202 | |
| **192** | 32 | 195 | |
| **193** | 371 | 206 | |
| **194** | 232 | 203 | |
| **195** | 48 | 122 | |
| **196** | 3710 | 190 | |
| **197** | 14717 | 128 | |

(fortgesetzt)

| Beispiel-verbindung | Fluorimetrie $EC_{50}$ [nM] | Fluorimetrie % Efficacy (Retigabine 50 μM = 100%) | low intensity tail flick Ratte p.o. % Effekt (Dosis [mg/kg]) |
|---|---|---|---|
| **198** | 8840 | 110 | |
| **199** | 20 | 124 | |
| **200** | 40 | 230 | |
| **201** | 40 | 128 | |
| **202** | 71 | 235 | |
| **203** | 20 | 233 | |
| **204** | 11 | 125 | |
| **205** | 28 | 294 | |
| **206** | 40 | 293 | |
| **207** | 34 | 119 | |
| **208** | 27 | 207 | |
| **209** | 9 | 228 | |
| **210** | 14 | 276 | |
| **211** | 8828 | 87 | |
| **212** | 427 | 184 | |

**Patentansprüche**

1. Substituierte Nicotinamide der allgemeinen Formel (1),

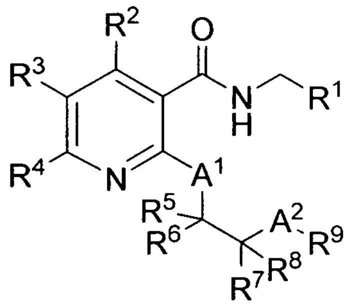

(1)

,

worin

$A^1$ für $CR^{10}R^{11}$ oder S steht;
$A^2$ für $CR^{12}R^{13}$, C(=O), O, S, S(=O) oder $S(=O)_2$ steht;
$R^1$ für $C_{1-10}$-Alkyl oder $C_{2-10}$-Heteroalkyl, jeweils gesättigt oder ungesättigt, verzweigt oder unverzweigt, unsubstituiert oder einfach oder mehrfach substituiert; $C_{3-10}$-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; über $C_{1-8}$-Alkyl oder $C_{2-8}$-Heteroalkyl verbrücktes $C_{3-10}$-Cycloalkyl oder Heterocyclyl, jeweils gesättigt oder ungesättigt, unsubstituiert oder einfach oder mehrfach substituiert, wobei die Alkyl- oder Heteroalkylkette jeweils verzweigt oder unverzweigt, gesättigt oder ungesättigt, unsubstituiert, einfach oder mehrfach substituiert sein kann; oder über $C_{1-8}$-Alkyl oder $C_{2-8}$-Heteroalkyl verbrücktes Aryl oder

Heteroaryl, jeweils unsubstituiert oder einfach oder mehrfach substituiert, wobei die Alkyl- oder Heteroalkylkette jeweils verzweigt oder unverzweigt, gesättigt oder ungesättigt, unsubstituiert, einfach oder mehrfach substituiert sein kann; steht;

$R^2$, $R^3$ und $R^4$ jeweils unabhängig voneinander für H; F; Cl; Br; I; $NO_2$; $CF_3$; CN; OH; $OCF_3$; SH; $SCF_3$; Methyl; $CH_2$-O-Methyl; $CH_2$-OH; $C_{2-6}$-Alkyl, O-$C_{1-6}$-Alkyl, S-$C_{1-6}$-Alkyl, jeweils gesättigt oder ungesättigt, verzweigt oder unverzweigt, unsubstituiert oder einfach oder mehrfach substituiert; $C_{3-7}$-Cycloalkyl, gesättigt oder ungesättigt, verzweigt oder unverzweigt, unsubstituiert oder einfach oder mehrfach substituiert; $NR^aR^b$, wobei $R^a$ und $R^b$ jeweils unabhängig voneinander für H oder $C_{1-4}$-Alkyl, gesättigt oder ungesättigt, verzweigt oder unverzweigt, unsubstituiert oder einfach oder mehrfach substituiert, stehen oder $R^a$ und $R^b$ gemeinsam mit dem sie verbindenden Stickstoffatom ein Heterocyclyl, gesättigt oder ungesättigt, verzweigt oder unverzweigt, unsubstituiert oder einfach oder mehrfach substituiert; bilden.

$R^5$, $R^6$, $R^7$, $R^8$, $R^{10}$, $R^{11}$, $R^{12}$ und $R^{13}$ jeweils unabhängig voneinander für H; F; Cl; Br; I; $NO_2$; $CF_3$; CN; OH; $OCF_3$; SH; $SCF_3$; $C_{1-10}$-Alkyl, $C_{2-10}$-Heteroalkyl, O-$C_{1-10}$-Alkyl oder S-$C_{1-10}$-Alkyl, jeweils gesättigt oder ungesättigt, verzweigt oder unverzweigt, unsubstituiert oder einfach oder mehrfach substituiert; $C_{3-10}$-Cycloalkyl, gesättigt oder ungesättigt, verzweigt oder unverzweigt, unsubstituiert oder einfach oder mehrfach substituiert; stehen;

mit der Maßgabe, dass, wenn $R^5$, $R^6$, $R^7$ und $R^8$ jeweils H bedeuten und $A^1$ für S steht, $A^2$ nicht S, S(=O) oder $S(=O)_2$ bedeuten kann;

oder $R^5$ und $R^6$ oder $R^7$ und $R^8$ oder $R^{10}$ und $R^{11}$ oder $R^{12}$ und $R^{13}$ oder $R^5$ und $R^{11}$ oder $R^5$ und $R^{13}$ oder $R^7$ und $R^{13}$ oder $R^7$ und $R^{11}$ oder $R^{11}$ und $R^{13}$ gemeinsam mit dem oder den sie verbindenden Kohlenstoffatom(en) ein $C_{3-8}$-Cycloalkyl oder ein Heterocyclyl mit drei bis acht Ringgliedern bilden, jeweils gesättigt oder ungesättigt, unsubstituiert oder einfach oder mehrfach substituiert; wobei die jeweiligen verbleibenden Substituenten $R^5$, $R^6$, $R^7$, $R^8$, $R^{10}$, $R^{11}$, $R^{12}$ und $R^{13}$ die vorstehende Bedeutung haben;

oder $R^5$ und $R^7$ gemeinsam mit dem oder den sie verbindenden Kohlenstoffatom(en) ein $C_{3-8}$-Cycloalkyl, gesättigt oder ungesättigt, unsubstituiert oder einfach oder mehrfach substituiert; oder ein Heterocyclyl mit drei bis acht Ringgliedern, ungesättigt, unsubstituiert oder einfach oder mehrfach substituiert, bilden; wobei die jeweiligen verbleibenden Substituenten $R^6$, $R^8$, $R^{10}$, $R^{11}$, $R^{12}$ und $R^{13}$ die vorstehende Bedeutung haben;

$R^9$ für $C_{3-10}$-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; oder für $CR^cR^d$ steht, wobei $R^c$ und $R^d$ jeweils unabhängig voneinander $C_{1-4}$-Alkyl, gesättigt oder ungesättigt, verzweigt oder unverzweigt, unsubstituiert oder einfach oder mehrfach substituiert; bedeuten;

mit der Maßgabe, dass, wenn $A^2$ für O oder S steht und $R^9$ Heterocyclyl, gesättigt oder ungesättigt, unsubstituiert oder einfach oder mehrfach substituiert; oder Heteroaryl, unsubstituiert oder einfach oder mehrfach substituiert; bedeutet, die Bindung des Heteroaryls oder Heterocyclyls über ein Kohlenstoffatom des Heteroaryls oder Heterocyclyls erfolgt;

worin "Alkyl substituiert", "Heteroalkyl substituiert", "Heterocyclyl substituiert" und "Cycloalkyl substituiert" für die Substitution eines oder mehrerer Wasserstoffatome jeweils unabhängig voneinander durch F; Cl; Br; I; $NO_2$; $CF_3$; CN; =O; $C_{1-8}$-Alkyl; $C_{2-8}$-Heteroalkyl; Aryl; Heteroaryl; $C_{3-10}$-Cycloalkyl; Heterocyclyl; über $C_{1-8}$-Alkyl oder $C_{2-8}$-Heteroalkyl verbrücktes Aryl, Heteroaryl, $C_{3-10}$-Cycloalkyl oder Heterocyclyl; CHO; C(=O)$C_{1-8}$-Alkyl; C(=O)Aryl; C(=O)Heteroaryl; $CO_2H$; C(=O)O-$C_{1-8}$-Alkyl; C(=O)O-Aryl; C(=O)O-Heteroaryl, $CONH_2$; C(=O)NH-$C_{1-8}$-Alkyl, C(=O)N($C_{1-8}$-Alkyl$)_2$; C(=O)NH-Aryl; C(=O)N(Aryl$)_2$; C(=O)NH-Heteroaryl; C(=O)N(Heteroaryl$)_2$; C(=O)N($C_{1-8}$-Alkyl)(Aryl); C(=O)N($C_{1-8}$-Alkyl)(Heteroaryl); C(=O)N(Heteroaryl)(Aryl); OH; O-$C_{1-8}$-Alkyl; $OCF_3$; O-($C_{1-8}$-Alkyl)-OH; O-($C_{1-8}$-Alkyl)-O-$C_{1-8}$-Alkyl; O-Benzyl; O-Aryl; O-Heteroaryl; O-C(=O)$C_{1-8}$-Alkyl; O-C(=O)Aryl; O-C(=O)Heteroaryl; $NH_2$ ; NH-$C_{1-8}$-Alkyl; N($C_{1-8}$-Alkyl$)_2$; NH-C(=O)$C_{1-8}$-Alkyl; N($C_{1-8}$-Alkyl)-C(=O)$C_{1-8}$-Alkyl; N(C(=O)$C_{1-8}$-Alkyl$)_2$; NH-C(=O)-Aryl; NH-C(=O)-Heteroaryl; SH; S-$C_{1-8}$-Alkyl; $SCF_3$; S-Benzyl; S-Aryl; S-Heteroaryl; $S(=O)_2C_{1-8}$-Alkyl; $S(=O)_2$Aryl; $S(=O)_2$Heteroaryl; $S(=O)_2$OH-1 $S(=O)_2$O-$C_{1-8}$-Alkyl; $S(=O)_2$O-Aryl; $S(=O)_2$O-Heteroaryl; $S(=O)_2$-NH-$C_{1-8}$-Alkyl; $S(=O)_2$-NH-Aryl; und $S(=O)_2$-NH-$C_{1-8}$-Heteroaryl; steht;

worin "Aryl substituiert" und "Heteroaryl substituiert" für die Substitution eines oder mehrerer Wasserstoffatome jeweils unabhängig voneinander durch F; Cl; Br; I; $NO_2$; $CF_3$; CN; $C_{1-8}$-Alkyl; $C_{2-8}$-Heteroalkyl-, Aryl; Heteroaryl; $C_{3-10}$-Cycloalkyl; Heterocyclyl; $C_{1-8}$-Alkyl oder $C_{2-8}$-Heteroalkyl verbrücktes Aryl, Heteroaryl, $C_{3-10}$-Cycloalkyl oder Heterocyclyl; CHO; C(=O)$C_{1-8}$-Alkyl; C(=O)Aryl; C(=O)Heteroaryl; C(=O)O-$C_{1-8}$-Alkyl; C(=O)O-Aryl; C(=O)O-Heteroaryl; $CONH_2$; C(=O)NH-$C_{1-8}$-Alkyl; C(=O)N($C_{1-8}$-Alkyl$)_2$; C(=O)NH-Aryl; C(=O)N(Aryl$)_2$; C(=O)NH-Heteroaryl; C(=O)N(Heteroaryl$)_2$; C(=O)N($C_{1-8}$-Alkyl)(Aryl); C(=O)N($C_{1-8}$-Alkyl)(Heteroaryl); C(=O)N(Heteroaryl)(Aryl); OH; O-$C_{1-8}$-Alkyl; $OCF_3$; O-($C_{1-8}$-Alkyl)-OH; O-($C_{1-8}$-Alkyl)-O-$C_{1-8}$-Alkyl; O-Benzyl; O-Aryl; O-Heteroaryl; O-C(=O)$C_{1-8}$-Alkyl-, O-C(=O)Aryl; O-C(=O)Heteroaryl, $NH_2$ ; NH-$C_{1-8}$-Alkyl; N($C_{1-8}$-Alkyl$)_2$; NH-C(=O)$C_{1-8}$-Alkyl; N($C_{1-8}$-Alkyl)-C(=O)$C_{1-8}$-Alkyl; N(C(=O)$C_{1-8}$-Alkyl$)_2$; NH-C(=O)-Aryl- NH-C(=O)-Heteroaryl; SH; S-$C_{1-8}$-Alkyl; $SCF_3$; S-Benzyl; S-Aryl; S-Heteroaryl; $S(=O)_2C_{1-8}$-Alkyl; $S(=O)_2$Aryl; $S(=O)_2$Heteroaryl; $S(=O)_2$OH; $S(=O)_2$O-$C_{1-8}$-Alkyl; $S(=O)_2$O-Aryl; $S(=O)_2$O-Heteroaryl; $S(=O)_2$-NH-$C_{1-8}$-Alkyl; $S(=O)_2$-NH-Aryl; $S(=O)_2$-NH-$C_{1-8}$-Heteroaryl; steht;

in Form der freien Verbindungen oder Salze physiologisch verträglicher Säuren oder Basen.

**2.** Substituierte Nicotinamide nach Anspruch 1, **dadurch gekennzeichnet, dass**

$A^1$ für S; und
$A^2$ für $CR^{12}R^{13}$, O, S oder $S(=O)_2$ steht.

**3.** Substituierte Nicotinamide nach Anspruch 1 oder 2, **dadurch gekennzeichnet, dass** $R^1$ für die Teilstruktur (T1) steht

$$-(CR^{14a}R^{14b})_m-Y_n-B$$

(T1)

,

worin
$R^{14a}$ und $R^{14b}$ jeweils unabhängig voneinander für H; F; Cl; Br; I; $NO_2$; $CF_3$; CN; OH; $OCF_3$; $NH_2$; $C_{1-4}$-Alkyl, O-$C_{1-4}$-Alkyl, NH-$C_{1-4}$-Alkyl, N($C_{1-4}$-Alkyl)$_2$, jeweils gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus F, Cl, Br, I, O-$C_{1-4}$-Alkyl, OH und $OCF_3$; $C_{3-10}$-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus F, Cl, Br, I, $C_{1-4}$-Alkyl, OH, =O, O-$C_{1-4}$-Alkyl, $OCF_3$, $NH_2$, NH-$C_{1-4}$-Alkyl, und N($C_{1-4}$-Alkyl)$_2$; stehen;

m für 0, 1, 2 oder 3 steht;
Y für O oder $NR^{15}$ steht,

wobei $R^{15}$ für H; $C_{1-4}$-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus F, Cl, Br, I, $C_{1-4}$-Alkyl, OH, O-$C_{1-4}$-Alkyl, $OCF_3$, $NH_2$, NH-$C_{1-4}$-Alkyl, und N($C_{1-4}$-Alkyl)$_2$; steht; oder für $C_{3-10}$-Cycloalkyl, gesättigt oder ungesättigt, unsubstituiert oder einfach oder mehrfach substituiert mit einem oder mehreren Substituenten jeweils unabhängig voneinander ausgewählt aus der Gruppe bestehend aus F, Cl, Br, I, $C_{1-4}$-Alkyl, OH, O-$C_{1-4}$-Alkyl, $OCF_3$, $NH_2$, NH-$C_{1-4}$-Alkyl, und N($C_{1-4}$-Alkyl)$_2$; steht;

n für 0 oder 1 steht,
B für $C_{1-8}$-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus F, Cl, Br, I, $NO_2$, CN, OH, =O, O-$C_{1-4}$-Alkyl, $OCF_3$, C(=O)-OH, $CF_3$, $NH_2$, NH($C_{1-4}$-Alkyl), N($C_{1-4}$-Alkyl)$_2$, SH, S-$C_{1-4}$-Alkyl, $SCF_3$ und $S(=O)_2OH$; $C_{3-10}$-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus F, Cl, Br, I, $NO_2$, CN, OH, O-$C_{1-8}$-Alkyl, $OCF_3$, $C_{1-8}$-Alkyl, C(=O)-OH, $CF_3$, $NH_2$, NH($C_{1-8}$-Alkyl), N($C_{1-8}$-Alkyl)$_2$, SH, S-$C_{1-8}$-Alkyl, $SCF_3$, $S(=O)_2OH$, Benzyl, Phenyl, Pyridyl und Thienyl, wobei Benzyl, Phenyl, Pyridyl, Thienyl jeweils unsubstituiert oder einfach oder mehrfach substituiert sein können mit einem oder mehreren Substituenten unabhängig voneinander ausgewählt aus der Gruppe bestehend aus F, Cl, Br, I, $NO_2$, CN, OH, O-$C_{1-8}$-Alkyl, $OCF_3$, $C_{1-8}$-Alkyl, C(=O)-OH, $CF_3$, $NH_2$, NH($C_{1-8}$-Alkyl), N($C_{1-8}$-Alkyl)$_2$, SH, S-$C_{1-8}$-Alkyl, $SCF_3$ und $S(=O)_2OH$; Aryl oder Heteroaryl, jeweils unsubstituiert oder einfach oder mehrfach substituiert mit einem oder mehreren Substituenten jeweils unabhängig voneinander ausgewählt aus der Gruppe bestehend aus F, Cl, Br, I, $NO_2$, CN, OH, O-$C_{1-8}$-Alkyl, $OCF_3$, $C_{1-8}$-Alkyl, C(=O)-OH, $CF_3$, $NH_2$, NH($C_{1-8}$-Alkyl), N($C_{1-8}$-Alkyl)$_2$, SH, S-$C_{1-8}$-Alkyl, $SCF_3$, $S(=O)_2OH$, Benzyl, Phenyl, Pyridyl und Thienyl, wobei Benzyl, Phenyl, Pyridyl, Thienyl jeweils unsubstituiert oder einfach oder mehrfach substituiert sein können mit einem oder mehreren Substituenten unabhängig voneinander ausgewählt aus der Gruppe bestehend aus F, Cl, Br, I, $NO_2$, CN, OH, O-$C_{1-8}$-Alkyl, $OCF_3$, $C_{1-8}$-Alkyl, C(=O)-OH, $CF_3$, $NH_2$, NH($C_{1-8}$-Alkyl), N($C_{1-8}$-Alkyl)$_2$, SH, S-$C_{1-8}$-Alkyl, $SCF_3$ und $S(=O)_2OH$; steht.

**4.** Substituierte Nicotinamide nach Anspruch 3, **dadurch gekennzeichnet, dass**

$R^{14a}$ und $R^{14b}$ jeweils unabhängig voneinander für H; F; Cl; Br; I; $NO_2$; $CF_3$; CN; Methyl; Ethyl; n-Propyl; iso-Propyl; Cyclopropyl; n-Butyl; sec.-Butyl; tert.-Butyl; $CH_2CF_3$; OH; O-Methyl; O-Ethyl- O-$(CH_2)_2$-O-$CH_3$; O-$(CH_2)_2$-OH; $OCF_3$; $NH_2$; NH-Methyl; N$(Methyl)_2$; NH-Ethyl; N$(Ethyl)_2$; oder N(Methyl)(Ethyl); stehen;

m für 0, 1 oder 2 steht;
n für 0 steht; und
B für $C_{1-4}$-Alkyl, gesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus F, Cl, Br, I, OH, O-$C_{1-4}$-Alkyl, $OCF_3$ und $CF_3$; $C_{3-10}$-Cycloalkyl, gesättigt, unsubstituiert; Phenyl, Naphthyl, Pyridyl, Thienyl, jeweils unsubstituiert oder einfach oder zweifach oder dreifach substituiert mit einem, zwei oder drei Substituenten jeweils unabhängig voneinander ausgewählt aus der Gruppe bestehend aus F, Cl, Br, I, $NO_2$, CN, OH, O-$C_{1-4}$-Alkyl, $OCF_3$, $C_{1-4}$-Alkyl, C(=O)-OH, $CF_3$, $NH_2$, NH($C_{1-4}$-Alkyl), N$(C_{1-4}\text{-Alkyl})_2$, SH, S-$C_{1-4}$-Alkyl, $SCF_3$, S$(=O)_2$OH; steht.

**5.** Substituierte Nicotinamide nach einem der Ansprüche 1-4, **dadurch gekennzeichnet, dass** $R^2$, $R^3$ und $R^4$ jeweils unabhängig voneinander für H; F; Cl; Br; I; $NO_2$; $CF_3$; CN; OH; $OCF_3$; SH; $SCF_3$; Methyl; $CH_2$-O-Methyl; $CH_2$-OH; $C_{2-6}$-Alkyl, O-$C_{1-6}$-Alkyl, S-$C_{1-6}$-Alkyl, jeweils gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus F, Cl, Br, I, OH, =O und O-$C_{1-4}$-Alkyl; $C_{3-7}$-Cycloalkyl, gesättigt oder ungesättigt, verzweigt oder unverzweigt, unsubstituiert; $NR^aR^b$, wobei $R^a$ und $R^b$ jeweils unabhängig voneinander für H oder $C_{1-4}$-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus OH, =O und O-$C_{1-4}$-Alkyl, stehen oder $R^a$ und $R^b$ gemeinsam mit dem sie verbindenden Stickstoffatom ein Heterocyclyl, gesättigt oder ungesättigt, unsubstituiert oder einfach oder mehrfach substituiert mit $C_{1-4}$-Alkyl, bilden; stehen.

**6.** Substituierte Nicotinamide nach Anspruche 5, **dadurch gekennzeichnet, dass** $R^2$, $R^3$ und $R^4$ jeweils unabhängig voneinander für H; F; Cl; Br; I; Methyl; Ethyl; n-Propyl, iso-Propyl; Cyclopropyl; CN; $CF_3$; O-Methyl; $OCF_3$; S-Methyl; $SCF_3$; stehen.

**7.** Substituierte Nicotinamide nach einem der Ansprüche 1-6, **dadurch gekennzeichnet, dass** $R^5$, $R^6$, $R^7$, $R^8$, $R^{10}$, $R^{11}$, $R^{12}$ und $R^{13}$ jeweils unabhängig voneinander für H; F; Cl; Br; I; $NO_2$; $CF_3$; CN; OH; $OCF_3$; SH; $SCF_3$; Methyl; Ethyl; n-Propyl; iso-Propyl; n-Butyl; sec.-Butyl; tert.-Butyl; O-Methyl; O-Ethyl; O-$(CH_2)_2$-O-$CH_3$; O-$(CH_2)_2$-OH; S-Methyl; S-Ethyl; Cyclopropyl; Cyclobutyl; Cyclopentyl; Cyclohexyl; stehen;
oder $R^5$ und $R^6$ oder $R^7$ und $R^8$ oder $R^{10}$ und $R^{11}$ oder $R^{12}$ und $R^{13}$ oder $R^5$ und $R^{11}$ oder $R^5$ und $R^7$ oder $R^5$ und $R^{13}$ oder $R^7$ und $R^{13}$ oder $R^7$ und $R^{11}$ oder $R^{11}$ und $R^{13}$ bilden gemeinsam mit dem oder den sie verbindenden Kohlenstoffatom(en) ein Cyclopropyl, Cyclobutyl, Cyclopentyl oder Cyclohexyl, jeweils unsubstituiert, bilden; wobei die jeweiligen verbleibenden Substituenten $R^5$, $R^6$, $R^7$, $R^8$, $R^{10}$, $R^{11}$, $R^{12}$ und $R^{13}$ die vorstehende Bedeutung haben.

**8.** Substituierte Nicotinamide nach einem der Ansprüche 1-7, **dadurch gekennzeichnet, dass** $R^9$ für $C_{3-10}$-Cycloalkyl oder Heterocyclyl, jeweils gesättigt oder ungesättigt, unsubstituiert oder einfach oder mehrfach substituiert mit einem oder mehreren Substituenten jeweils unabhängig voneinander ausgewählt aus der Gruppe bestehend aus F, Cl, Br, I, $NO_2$, CN, OH, =O, O-$C_{1-4}$-Alkyl, $OCF_3$, $C_{1-4}$-Alkyl, $CF_3$, SH, S-$C_{1-4}$-Alkyl und $SCF_3$; Aryl oder Heteroaryl, jeweils unsubstituiert oder einfach oder mehrfach substituiert mit einem oder mehreren Substituenten jeweils unabhängig voneinander ausgewählt aus der Gruppe bestehend aus F, Cl, Br, I, $NO_2$, CN, OH, O-$C_{1-4}$-Alkyl, $OCF_3$, $C_{1-4}$-Alkyl, $CF_3$, $NH_2$, NH($C_{1-4}$-Alkyl), N$(C_{1-4}\text{-Alkyl})_2$, SH, S-$C_{1-4}$-Alkyl und $SCF_3$; oder für $CR^cR^d$, wobei $R^c$ und $R^d$ jeweils unabhängig voneinander $C_{1-4}$-Alkyl, gesättigt oder ungesättigt, verzweigt oder unverzweigt, unsubstituiert oder einfach oder mehrfach substituiert mit einem oder mehreren Substituenten jeweils unabhängig voneinander ausgewählt aus der Gruppe bestehend aus F, Cl, Br, I, OH, O-$C_{1-4}$-Alkyl, $CF_3$, $OCF_3$ und $SCF_3$; steht.

**9.** Substituierte Nicotinamide nach einem der Ansprüche 1-8, **dadurch gekennzeichnet, dass** $R^9$ für Phenyl, Pyridyl oder Thienyl, jeweils unsubstituiert oder einfach oder mehrfach substituiert mit einem oder mehreren Substituenten jeweils unabhängig voneinander ausgewählt aus der Gruppe bestehend aus F, Cl, Br, I, CN, OH, O-$C_{1-4}$-Alkyl, $OCF_3$, $C_{1-4}$-Alkyl, $CF_3$, SH, S-$C_{1-4}$-Alkyl und $SCF_3$ steht.

**10.** Substituierte Nicotinamide nach einem der Ansprüche 1-9, **dadurch gekennzeichnet, dass**

$A^1$ für S und

$A^2$ für $CR^{12}R^{13}$ steht;

$R^1$ für die Teilstruktur (T1-1) steht

$$-\!\wr\!-(CR^{14a}R^{14b})_m—B$$

(T1-1)

,

worin

$R^{14a}$ und $R^{14b}$ jeweils unabhängig voneinander für H; F; Cl; Br; I; Methyl; Ethyl; n-Propyl; iso-Propyl; n-Butyl; sec.-Butyl; tert.-Butyl; OH; O-Methyl; O-Ethyl; O-$(CH_2)_2$-O-$CH_3$; oder O-$(CH_2)_2$-OH; stehen;
m für 0, 1 oder 2 steht;
B für Methyl; Ethyl; n-Propyl; iso-Propyl; n-Butyl; sec.-Butyl; tert.-Butyl; Cyclopropyl; Cyclobutyl; Cyclopentyl; Cyclohexyl; Cycloheptyl; Adamantyl; Bicyclo[2.2.1]heptyl; Bicyclo[2.2.2]octyl; Phenyl, Pyridyl, Thienyl, jeweils unsubstituiert oder einfach, zweifach oder dreifach substituiert mit einem, zwei oder drei Substituenten jeweils unabhängig voneinander ausgewählt aus der Gruppe bestehend aus F, Cl, Br, I, $NO_2$, CN, OH, O-$C_{1-4}$-Alkyl, $OCF_3$, $C_{1-4}$-Alkyl, C(=O)-OH, $CF_3$, $NH_2$, NH($C_{1-4}$-Alkyl), N($C_{1-4}$-Alkyl$)_2$, SH, S-$C_{1-4}$-Alkyl, $SCF_3$ und $S(=O)_2OH$; steht;

$R^2$, $R^3$ und $R^4$ jeweils unabhängig voneinander für H; F; Cl; Methyl; Ethyl; $CF_3$ oder O-Methyl stehen;
$R^5$, $R^6$, $R^7$, $R^8$, $R^{10}$, $R^{11}$, $R^{12}$ und $R^{13}$ jeweils unabhängig voneinander für H; F; Cl; Methyl; Ethyl; n-Propyl, iso-Propyl; Cyclopropyl stehen;
$R^9$ für Phenyl, Pyridyl oder Thienyl unsubstituiert oder einfach oder mehrfach substituiert mit einem oder mehreren Substituenten jeweils unabhängig voneinander ausgewählt aus der Gruppe bestehend aus F, Cl, Br, I, CN, Methyl, Ethyl, n-Propyl, iso-Propyl, n-Butyl, sec.-Butyl, tert.-Butyl, O-Methyl, O-Ethyl, O-n-Propyl, O-iso-Propyl, O-Butyl, O-sec.-Butyl, O-tert.-Butyl, OH, $OCF_3$, $CF_3$, SH, S-$C_{1-4}$-Alkyl und $SCF_3$ steht.

**11.** Substituierte Nicotinamide nach einem der Ansprüche 1-10 ausgewählt aus der Gruppe

**1** 2-(3-Phenyl-propylsulfanyl)-N-(thiophen-2-yl-methyl)-pyridin-3-carbonsäureamid;
**2** 2-(3-Cyclohexyl-propylsulfanyl)-N-(thiophen-2-yl-methyl)-pyridin-3-carbonsäureamid;
**3** 2-[(3-Oxo-3-phenyl-propyl)sulfanyl]-N-(thiophen-2-yl-methyl)-pyridin-3-carbonsäureamid;
**4** N-(Thiophen-2-yl-methyl)-2-[2-[3-(trifluormethyl)-phenoxy]-ethylsulfanyl]-pyridin-3-carbonsäureamid;
**5** 2-(4-Methyl-pentylsulfanyl)-N-(thiophen-2-yl-methyl)-pyridin-3-carbonsäureamid;
**7** 2-(4-Phenyl-butyl)-N-(thiophen-2-yl-methyl)-pyridin-3-carbonsäureamid;
**8** 2-[3-(Benzolsulfonyl)-propyl]-N-(cyclohexyl-methyl)-pyridin-3-carbonsäureamid;
**9** N-(Cyclohexyl-methyl)-2-(4-phenyl-butyl)-pyridin-3-carbonsäureamid;
**10** 2-[3-(Benzolsulfonyl)-propyl]-N-(thiophen-2-yl-methyl)-pyridin-3-carbonsäureamid;
**12** N-(Thiophen-2-yl-methyl)-2-[3-[[3-(trifluormethyl)phenyl]sulfonyl]-propyl]-pyridin-3-carbonsäureamid;
**13** N-(Thiophen-2-yl-methyl)-2-[3-[[3-(trifluor-methyl)phenyl]sulfanyl]-propyl]-pyridin-3-carbonsäureamid;
**16** 2-(2-Phenylsulfanyl-propylsulfanyl)-N-(thiophen-2-yl-methyl)-pyridin-3-carbonsäureamid;
**17** 2-[3-(4-Fluorphenyl)-propylsulfanyl]-N-(thiophen-2-yl-methyl)-pyridin-3-carbonsäureamid;
**18** N-(Thiophen-2-yl-methyl)-2-[3-[3-(trifluormethyl)phenyl]-propylsulfanyl]-pyridin-3-carbonsäureamid;
**19** 2-[2-[(4-Fluorphenyl)sulfanyl]-propylsulfanyl]-N-(thiophen-2-yl-methyl)-pyridin-3-carbonsäureamid;
**20** N-(Thiophen-2-yl-methyl)-2-[2-[[3-(trifluormethyl)phenyl]sulfanyl]-propylsulfanyl]-pyridin-3-carbonsäureamid;
**21** N-(Thiophen-2-yl-methyl)-2-[4-[3-(trifluormethyl)-phenyl]-butyl]-pyridin-3-carbonsäureamid;
**22** 2-[4-(4-Fluorphenyl)-butyl]-N-(thiophen-2-yl-methyl)-pyridin-3-carbonsäureamid;
**23** 2-(3-Phenylsulfanyl-propyl)-N-(thiophen-2-yl-methyl)-pyridin-3-carbonsäureamid;
**24** 2-[(1-Methyl-2-phenylsulfanyl-ethyl)sulfanyl]-N-(thiophen-2-yl-methyl)-pyridin-3-carbonsäureamid;
**25** N-(Cycloheptyl-methyl)-2-[4-[3-(trifluormethyl)phenyl]-butyl]-pyridin-3-carbonsäure amid
**26** N-(5-Bicyclo[2.2.1]heptanyl-methyl)-2-[4-[3-(trifluormethyl)phenyl]-butyl]-pyridin-3-carbonsäure amid
**27** N-(5-Bicyclo[2.2.1]heptanyl-methyl)-2-[3-[3-(trifluormethyl)phenyl]-propylsulfanyl]-pyridin-3-carbonsäure

amid

**28** N-(5-Bicyclo[2.2.1]heptanyl-methyl)-2-(4-methyl-pentylsulfanyl)-pyridin-3-carbonsäure amid

**29** N-(Cycloheptyl-methyl)-2-[3-[(4-fluorphenyl)sulfanyl]-propyl]-pyridin-3-carbonsäure amid

**30** N-(5-Bicyclo[2.2.1]heptanyl-methyl)-2-[3-[(4-fluorphenyl)sulfanyl]-propyl]-pyridin-3-carbonsäure amid

**31** N-(Cycloheptyl-methyl)-2-[3-[[3-(trifluormethyl)phenyl]sulfonyl]-propyl]-pyridin-3-carbonsäure amid

**32** N-(5-Bicyclo[2.2.1]heptanyl-methyl)-2-[3-[[3-(trifluormethyl)phenyl]sulfonyl]-propyl]-pyridin-3-carbonsäure amid

**33** N-(5-Bicyclo[2.2.1]heptanyl-methyl)-2-(3-phenyl-propylsulfanyl)-pyridin-3-carbonsäure amid

**34** N-(5-Bicyclo[2.2.1]heptanyl-methyl)-2-[3-[4-(trifluormethyl)-phenyl]-propylsulfanyl]-pyridin-3-carbonsäure amid

**35** N-(5-Bicyclo[2.2.1]heptanyl-methyl)-2-[3-(4-chlorphenyl)-propylsulfanyl]-pyridin-3-carbonsäure amid

**36** N-[(3,5-Difluor-phenyl)-methyl]-2-[3-(4-fluorphenyl)-propylsulfanyl]-pyridin-3-carbonsäure amid

**37** N-[(5-Chlor-thiophen-2-yl)-methyl]-2-[3-(4-fluorphenyl)-propylsulfanyl]-pyridin-3-arbonsäure amid

**38** N-[(2,2-Dimethyl-cyclopropyl)-methyl]-2-[3-(4-fluorphenyl)-propylsulfanyl]-pyridin-3-carbonsäure amid

**39** N-(5-Bicyclo[2.2.1]heptanyl-methyl)-2-[3-(3-fluorphenyl)-propylsulfanyl]-pyridin-3-carbonsäure amid

**40** N-(Cyclohexyl-methyl)-2-[3-(4-fluorphenyl)-propylsulfanyl]-pyridin-3-carbonsäure amid

**41** N-(Cycloheptyl-methyl)-2-[3-(4-fluorphenyl)-propylsulfanyl]-pyridin-3-carbonsäure amid

**43** N-(5-Bicyclo[2.2.1]heptanyl-methyl)-2-[[3-(4-fluorphenyl)-3-oxo-propyl]sulfanyl]-pyridin-3-carbonsäure amid

**44** N-(5-Bicyclo[2.2.1]heptanyl-methyl)-2-[(3-oxo-3-phenyl-propyl)sulfanyl]-pyridin-3-carbonsäure amid

**45** N-(5-Bicyclo[2.2.1]heptanyl-methyl)-2-(hexylsulfanyl)-pyridin-3-carbonsäure amid

**46** N-(5-Bicyclo[2.2.1]heptanyl-methyl)-2-(2-phenoxy-ethylsulfanyl)-pyridin-3-carbonsäure amid

**47** N-(5-Bicyclo[2.2.1]heptanyl-methyl)-2-[2-[3-(trifluormethyl)-phenoxy]-ethylsulfanyl]-pyridin-3-carbonsäure amid

**48** 2-[3-(4-Fluorphenyl)-propylsulfanyl]-N-(3,3,3-trifluor-propyl)-pyridin-3-carbonsäure amid

**49** N-(5-Bicyclo[2.2.1]heptanyl-methyl)-2-[2-(4-fluor-phenoxy)-ethylsulfanyl]-pyridin-3-carbonsäure amid

**52** N-(5-Bicyclo[2.2.1]heptanyl-methyl)-2-(3-naphthalen-1-yl-propylsulfanyl)-pyridin-3-carbonsäure amid

**53** N-(5-Bicyclo[2.2.1]heptanyl-methyl)-2-[3-[3-fluor-4-(trifluormethyl)-phenyl]-propylsulfanyl]-pyridin-3-carbonsäure amid

**54** N-(5-Bicyclo[2.2.1]heptanyl-methyl)-2-[3-[4-fluor-3-(trifluormethyl)-phenyl]-propylsulfanyl]-pyridin-3-carbonsäure amid

**55** N-(Cyclooctyl-methyl)-2-[3-(4-fluorphenyl)-propylsulfanyl]-pyridin-3-carbonsäure amid

**56** N-(5-Bicyclo[2.2.1]heptanyl-methyl)-2-[3-(4-methoxyphenyl)-propylsulfanyl]-pyridin-3-carbonsäure amid

**57** 2-[3-(4-Fluorphenyl)-propylsulfanyl]-N-[(4-methoxyphenyl)-methyl]-pyridin-3-carbonsäure amid

**59** 2-[3-(4-Fluorphenyl)-propylsulfanyl]-N-[[3-(trifluormethyl)phenyl]-methyl]-pyridin-3-carbonsäure amid

**60** N-(5-Bicyclo[2.2.1]heptanyl-methyl)-2-[3-(3,4-difluor-phenyl)-propylsulfanyl]-pyridin-3-carbonsäure amid

**61** N-(5-Bicyclo[2.2.1]heptanyl-methyl)-2-[3-(3,5-difluor-phenyl)-propylsulfanyl]-pyridin-3-carbonsäure amid

**62** N-(5-Bicyclo[2.2.1]heptanyl-methyl)-2-[3-(2,4-difluor-phenyl)-propylsulfanyl]-pyridin-3-carbonsäure amid

**63** N-[(2-Fluorphenyl)-methyl]-2-[3-(4-fluorphenyl)-propylsulfanyl]-pyridin-3-carbonsäure amid

**64** N-[(3-Fluorphenyl)-methyl]-2-[3-(4-fluorphenyl)-propylsulfanyl]-pyridin-3-carbonsäure amid

**65** N-[(4-Fluorphenyl)-methyl]-2-[3-(4-fluorphenyl)-propylsulfanyl]-pyridin-3-carbonsäure amid

**66** N-Benzyl-2-[3-(4-fluorphenyl)-propylsulfanyl]-pyridin-3-carbonsäure amid

**67** N-(5-Bicyclo[2.2.1]heptanyl-methyl)-2-[(1-methyl-3-phenyl-propyl)sulfanyl]-pyridin-3-carbonsäure amid

**68** N-(5-Bicyclo[2.2.1]heptanyl-methyl)-2-[3-(3,4,5-trifluor-phenyl)-propylsulfanyl]-pyridin-3-carbonsäure amid

**69** N-(5-Bicyclo[2.2.1]heptanyl-methyl)-2-[3-[3-fluor-5-(trifluormethyl)-phenyl]-propylsulfanyl]-pyridin-3-carbonsäure amid

**70** N-(5-Bicyclo[2.2.1]heptanyl-methyl)-2-[3-(3-methoxyphenyl)-propylsulfanyl]-pyridin-3-carbonsäure amid

**71** N-[(3,4-Difluor-phenyl)-methyl]-2-[3-(4-fluorphenyl)-propylsulfanyl]-pyridin-3-carbonsäure amid

**72** N-(2,3-Dihydro-benzofuran-5-yl-methyl)-2-[3-(4-fluorphenyl)-propylsulfanyl]-pyridin-3-carbonsäure amid

**73** N-(5-Bicyclo[2.2.1]heptanyl-methyl)-2-[3-(3-hydroxyphenyl)-propylsulfanyl]-pyridin-3-carbonsäure amid

**74** N-(5-Bicyclo[2.2.1]heptanyl-methyl)-2-[[3-(4-fluorphenyl)-1-methyl-propyl]sulfanyl]-pyridin-3-carbonsäure amid

**75** N-(5-Bicyclo[2.2.1]heptanyl-methyl)-2-[[3-(4-fluorphenyl)-2-methylpropyl]sulfanyl]-pyridin-3-carbonsäure amid

**76** 2-[3-(4-Fluorphenyl)-propylsulfanyl]-N-[(2-methoxyphenyl)-methyl]-pyridin-3-carbonsäure amid

**77** 2-[3-(4-Fluorphenyl)-propylsulfanyl]-N-[(3-methoxyphenyl)-methyl]-pyridin-3-carbonsäure amid

**78** 2-[3-(4-Fluorphenyl)-propylsulfanyl]-N-phenethyl-pyridin-3-carbonsäure amid

**79** 2-[3-(4-Fluorphenyl)-propylsulfanyl]-N-[[4-(trifluormethyloxy)-phenyl]-methyl]-pyridin-3-carbonsäure amid

**80** 2-[3-(4-Fluorphenyl)-propylsulfanyl]-N-[[4-(trifluormethyl)-phenyl]-methyl]-pyridin-3-carbonsäure amid

**81** 2-[3-(4-Fluorphenyl)-propylsulfanyl]-N-(2-pyridin-3-yl-ethyl)-pyridin-3-carbonsäure amid
**82** 2-[3-(4-Fluorphenyl)-propylsulfanyl]-N-(2-pyridin-2-yl-ethyl)-pyridin-3-carbonsäure amid
**83** 2-[3-(4-Fluorphenyl)-propylsulfanyl]-N-[(2-hydroxyphenyl)-methyl]-pyridin-3-carbonsäure amid
**84** 2-[3-(4-Fluorphenyl)-propylsulfanyl]-N-[(3-hydroxyphenyl)-methyl]-pyridin-3-carbonsäure amid
**85** 2-[3-(4-Fluorphenyl)-propylsulfanyl]-N-[2-(m-tolyl)-ethyl]-pyridin-3-carbonsäure amid
**86** 2-[3-(4-Fluorphenyl)-propylsulfanyl]-N-[2-(o-tolyl)-ethyl]-pyridin-3-carbonsäure amid
**87** 2-[3-(4-Fluorphenyl)-propylsulfanyl]-N-[[3-(trifluormethyloxy)-phenyl]-methyl]-pyridin-3-carbonsäure amid
**88** 2-[3-(4-Fluorphenyl)-propylsulfanyl]-N-[(4-hydroxyphenyl)-methyl]-pyridin-3-carbonsäure amid
**89** 2-[3-(4-Fluorphenyl)-propylsulfanyl]-N-(2-pyridin-4-yl-ethyl)-pyridin-3-carbonsäure amid
**90** 2-[3-(4-Fluorphenyl)-propylsulfanyl]-N-[2-(p-tolyl)-ethyl]-pyridin-3-carbonsäure amid
**91** N-(5-Bicyclo[2.2.1]heptanyl-methyl)-2-[3-(4-fluorphenyl)-butylsulfanyl]-pyridin-3-carbonsäure amid
**92** N-(5-Bicyclo[2.2.1]heptanyl-methyl)-2-[3-(2,4,5-trifluor-phenyl)-propylsulfanyl]-pyridin-3-carbonsäure amid
**93** 2-[3-(4-Fluorphenyl)-propylsulfanyl]-N-(3-pyridin-2-yl-propyl)-pyridin-3-carbonsäure amid
**94** 2-[3-(4-Fluorphenyl)-propylsulfanyl]-N-(3-pyridin-3-yl-propyl)-pyridin-3-carbonsäure amid
**95** 2-[3-(4-Fluorphenyl)-propylsulfanyl]-N-(3-pyridin-4-yl-propyl)-pyridin-3-carbonsäure amid
**96** 2-[3-(4-Fluorphenyl)-propylsulfanyl]-N-propyl-pyridin-3-carbonsäure amid
**97** N-Butyl-2-[3-(4-fluorphenyl)-propylsulfanyl]-pyridin-3-carbonsäure amid
**98** 2-(3-Pyridin-3-yl-propylsulfanyl)-N-(thiophen-2-yl-methyl)-pyridin-3-carbonsäure amid
**99** 2-[3-(p-Tolyl)-propylsulfanyl]-N-(thiophen-2-yl-methyl)-pyridin-3-carbonsäure amid
**100** 2-(4-Phenyl-butylsulfanyl)-N-(thiophen-2-yl-methyl)-pyridin-3-carbonsäure amid
**101** 2-(3-Pyridin-4-yl-propylsulfanyl)-N-(thiophen-2-yl-methyl)-pyridin-3-carbonsäure amid
**102** 2-(3-Naphthalen-2-yl-propylsulfanyl)-N-(thiophen-2-yl-methyl)-pyridin-3-carbonsäure amid
**103** 2-[3-(m-Tolyl)-propylsulfanyl]-N-(thiophen-2-yl-methyl)-pyridin-3-carbonsäure amid
**104** N-(Thiophen-2-yl-methyl)-2-(3-thiophen-2-yl-propylsulfanyl)-pyridin-3-carbonsäure amid
**105** 2-[(1-Methyl-3-phenyl-propyl)sulfanyl]-N-(thiophen-2-yl-methyl)-pyridin-3-carbonsäure amid
**106** N-(Thiophen-2-yl-methyl)-2-(3-thiophen-3-yl-propylsulfanyl)-pyridin-3-carbonsäure amid
**107** 2-[[1-Methyl-3-[3-(trifluormethyl)phenyl]-propyl]sulfanyl]-N-(thiophen-2-yl-methyl)-pyridin-3-carbonsäure amid
**108** 2-[(2-Benzyl-cyclohexyl)sulfanyl]-N-(thiophen-2-yl-methyl)-pyridin-3-carbonsäure amid
**109** 2-[3-[3-Methyl-5-(trifluormethyl)-phenyl]-propylsulfanyl]-N-(thiophen-2-yl-methyl)-pyridin-3-carbonsäure amid
**110** 2-[4-(3,4-Difluor-phenyl)-butylsulfanyl]-N-(thiophen-2-yl-methyl)-pyridin-3-carbonsäure amid
**111** 2-(3-Pyridin-2-yl-propylsulfanyl)-N-(thiophen-2-yl-methyl)-pyridin-3-carbonsäure amid
**113** 2-[3-[4-Methyl-3-(trifluormethyl)-phenyl]-propylsulfanyl]-N-(thiophen-2-yl-methyl)-pyridin-3-carbonsäure amid
**114** 2-[(3-Phenyl-cyclohexyl)sulfanyl]-N-(thiophen-2-yl-methyl)-pyridin-3-carbonsäure amid
**116** 2-[[3,3-Difluor-3-(4-fluorphenyl)-propyl]sulfanyl]-N-(3,3-dimethyl-butyl)-pyridin-3-carbonsäure amid
**117** N-(Cycloheptyl-methyl)-2-[[3,3-difluor-3-(4-fluorphenyl)-propyl]sulfanyl]-pyridin-3-carbonsäure amid
**118** 2-[[3,3-Difluor-3-(4-fluorphenyl)-propyl]sulfanyl]-N-(thiophen-2-yl-methyl)-pyridin-3-carbonsäure amid
**119** 2-[3-(4-Fluorphenyl)-propylsulfanyl]-N-(1,2,3,4-tetrahydro-naphthalen-2-yl-methyl)-pyridin-3-carbonsäure amid
**120** N-(2,3-Dihydro-1H-inden-2-yl-methyl)-2-[3-(4-fluorphenyl)-propylsulfanyl]-pyridin-3-carbonsäure amid
**121** N-(1,3-Benzodioxol-5-yl-methyl)-2-[3-(4-fluorphenyl)-propylsulfanyl]-pyridin-3-carbonsäure amid
**122** 2-[3-(4-Fluorphenyl)-propylsulfanyl]-N-[(3-phenyl-phenyl)-methyl]-pyridin-3-carbonsäure amid
**123** 2-[3-(4-Fluorphenyl)-propylsulfanyl]-N-[(3-methyl-cyclohexyl)-methyl]-pyridin-3-carbonsäure amid
**124** 2-[[3,3-Difluor-3-(4-fluorphenyl)-propyl]sulfanyl]-N-[(4-fluorphenyl)-methyl]-pyridin-3-carbonsäure amid
**125** N-(3,3-Dimethyl-2-oxo-butyl)-2-[3-(4-fluorphenyl)-propylsulfanyl]-pyridin-3-carbonsäure amid
**127** 2-[3-(4-Fluorphenyl)-propylsulfanyl]-N-(pyridin-3-yl-methyl)-pyridin-3-carbonsäure amid
**128** 2-[3-(4-Fluorphenyl)-propylsulfanyl]-N-(pyridin-4-yl-methyl)-pyridin-3-carbonsäure amid
**129** 3-[[[2-[3-(4-Fluorphenyl)-propylsulfanyl]-pyridin-3-carbonyl]amino]-methyl]-benzoesäure methyl ester
**130** 2-[3-(4-Fluorphenyl)-propylsulfanyl]-N-[3-(2-methoxyphenyl)-propyl]-pyridin-3-carbonsäure amid
**132** N-[(4-Fluorphenyl)-methyl]-2-[[1-methyl-3-[3-(trifluormethyl)phenyl]-propyl]sulfanyl]-pyridin-3-carbonsäure amid
**133** 2-[3-(4-Fluorphenyl)-propylsulfanyl]-N-(3-methyl-butyl)-pyridin-3-carbonsäure amid
**134** 2-[3-(4-Fluorphenyl)-propylsulfanyl]-N-(tetrahydro-pyran-2-yl-methyl)-pyridin-3-carbonsäure amid
**135** 2-[3-(4-Fluorphenyl)-propylsulfanyl]-N-[3-(1H-pyrazol-1-yl)-propyl]-pyridin-3-carbonsäure amid
**136** 2-[3-(4-Fluorphenyl)-propylsulfanyl]-N-(naphthalen-2-yl-methyl)-pyridin-3-carbonsäure amid
**137** N-(2,3-Dihydro-[1,4]benzodioxin-6-yl-methyl)-2-[3-(4-fluorphenyl)-propylsulfanyl]-pyridin-3-carbonsäure

amid
**138** 2-[3-(4-Fluorphenyl)-propylsulfanyl]-N-[(3-morpholin-4-yl-phenyl)-methyl]-pyridin-3-carbonsäure amid
**139** N-(2,3-Dihydro-benzofuran-6-yl-methyl)-2-[3-(4-fluorphenyl)-propylsulfanyl]-pyridin-3-carbonsäure amid
**140** 2-[3-(4-Fluorphenyl)-propylsulfanyl]-N-[[3-(1H-pyrazol-1-yl)-phenyl]-methyl]-pyridin-3-carbonsäure amid
**141** 2-[3-(4-Fluorphenyl)-propylsulfanyl]-N-[3-(1 H-[1,2,3]triazol-1-yl)-propyl]-pyridin-3-carbonsäure amid
**142** N-(7-Bicyclo[2.2.1]heptanyl-methyl)-2-[3-(4-fluorphenyl)-propylsulfanyl]-pyridin-3-carbonsäure amid
**144** 2-[3-(4-Fluorphenyl)-propylsulfanyl]-N-(thiazol-2-yl-methyl)-pyridin-3-carbonsäure amid
**145** 2-[3-(4-Fluorphenyl)-propylsulfanyl]-N-(thiazol-5-yl-methyl)-pyridin-3-carbonsäure amid
**146** 2-[3-(4-Fluorphenyl)-propylsulfanyl]-N-(oxazol-2-yl-methyl)-pyridin-3-carbonsäure amid
**148** N-(3,3-Dimethyl-butyl)-2-[3-(4-fluorphenyl)-propylsulfanyl]-4-methyl-pyridin-3-carbonsäure amid
**149** N-(3,3-Dimethyl-butyl)-2-[[1-methyl-3-[3-(trifluormethyl)phenyl]-propyl]sulfanyl]-pyridin-3-carbonsäure amid
**150** 2-[3-(4-Fluorphenyl)-propylsulfanyl]-N-(chinolin-7-yl-methyl)-pyridin-3-carbonsäure amid
**151** 2-[3-(4-Fluorphenyl)-propylsulfanyl]-N-[(3-pyridin-2-yl-phenyl)-methyl]-pyridin-3-carbonsäure amid
**152** 2-[3-(4-Fluorphenyl)-propylsulfanyl]-N-[(3-pyridin-3-yl-phenyl)-methyl]-pyridin-3-carbonsäure amid
**153** N-(3,3-Dimethyl-butyl)-2-[[(1R)-1-methyl-3-phenyl-propyl]sulfanyl]-pyridin-3-carbonsäure amid
**154** N-(3,3-Dimethyl-butyl)-2-[[(1S)-1-methyl-3-phenyl-propyl]sulfanyl]-pyridin-3-carbonsäure amid
**155** 2-[(2-Benzyl-cyclopentyl)sulfanyl]-N-(3,3-dimethyl-butyl)-pyridin-3-carbonsäure amid
**156** N-(7-Bicyclo[2.2.1]heptanyl-methyl)-2-[3-[3-(trifluormethyl)phenyl]-propylsulfanyl]-pyridin-3-carbonsäure amid
**157** 2-[3-(4-Fluorphenyl)-propylsulfanyl]-N-[3-(1H-[1,2,4]triazol-1-yl)-propyl]-pyridin-3-carbonsäure amid
**158** 2-[3-(4-Fluorphenyl)-propylsulfanyl]-N-([1,3,4]oxadiazol-2-yl-methyl)-pyridin-3-carbonsäure amid
**159** 2-[3-(4-Fluorphenyl)-propylsulfanyl]-N-[(3-pyridin-4-yl-phenyl)-methyl]-pyridin-3-carbonsäure amid
**160** N-[[4-(Cyclopropyl-methyl)-3,4-dihydro-2H-[1,4]benzoxazin-6-yl]-methyl]-2-[3-(4-fluorphenyl)-propylsulfanyl]-pyridin-3-carbonsäure amid
**161** N-[(4-Ethyl-3,4-dihydro-2H-[1,4]benzoxazin-6-yl)-methyl]-2-[3-(4-fluorphenyl)-propylsulfanyl]-pyridin-3-carbonsäure amid
**162** 2-[3-(4-Fluorphenyl)-propylsulfanyl]-N-[(4-methyl-3,4-dihydro-2H-[1,4]benzoxazin-6-yl)-methyl]-pyridin-3-carbonsäure amid
**163** 2-[3-(4-Fluorphenyl)-propylsulfanyl]-N-(3-methyl-3-phenyl-butyl)-pyridin-3-carbonsäure amid
**164** N-(7-Bicyclo[2.2.1]heptanyl-methyl)-2-[[3,3-difluor-3-(4-fluorphenyl)-propyl]sulfanyl]-pyridin-3-carbonsäure amid
**165** 2-[[3,3-Difluor-3-[3-(trifluormethyl)phenyl]-propyl]sulfanyl]-N-[(4-fluorphenyl)-methyl]-pyridin-3-carbonsäure amid
**166** 2-[[3,3-Difluor-3-[3-(trifluormethyl)phenyl]-propyl]sulfanyl]-N-(3,3-dimethylbutyl)-pyridin-3-carbonsäure amid
**168** 2-[[3,3-Difluor-3-(4-fluorphenyl)-propyl]sulfanyl]-N-[(3-fluorphenyl)-methyl]-pyridin-3-carbonsäure amid
**170** N-Butyl-2-[[3,3-difluor-3-(4-fluorphenyl)-propyl]sulfanyl]-pyridin-3-carbonsäure amid
**171** 2-[[3,3-Difluor-3-(4-fluorphenyl)-propyl]sulfanyl]-N-(2-methoxy-ethyl)-pyridin-3-carbonsäure amid
**172** 2-[3-(4-Fluorphenyl)-propylsulfanyl]-N-(2-methoxy-ethyl)-pyridin-3-carbonsäure amid
**173** N-[(4-Fluor-2-hydroxy-phenyl)-methyl]-2-[3-(4-fluorphenyl)-propylsulfanyl]-pyridin-3-carbonsäure amid
**174** 2-[3-(4-Fluorphenyl)-propylsulfanyl]-N-(furan-2-yl-methyl)-pyridin-3-carbonsäure amid
**175** 2-[3-(4-Fluorphenyl)-propylsulfanyl]-N-[(5-methyl-furan-2-yl)-methyl]-pyridin-3-carbonsäure amid
**176** N-[(4-Fluorphenyl)-methyl]-2-[[3-(4-fluorphenyl)-1-methyl-propyl]sulfanyl]-pyridin-3-carbonsäure amid
**177** 2-[[3-(4-Fluorphenyl)-1-methyl-propyl]sulfanyl]-N-(3-methyl-butyl)-pyridin-3-carbonsäure amid
**178** N-(7-Bicyclo[2.2.1]heptanyl-methyl)-2-[[3-(4-fluorphenyl)-1-methylpropyl]sulfanyl]-pyridin-3-carbonsäure amid
**179** 2-[[3,3-Difluor-3-(4-fluorphenyl)-propyl]sulfanyl]-N-(tetrahydro-furan-2-yl-methyl)-pyridin-3-carbonsäure amid
**180** 2-[[3,3-Difluor-3-(4-fluorphenyl)-propyl]sulfanyl]-N-(tetrahydro-pyran-2-yl-methyl)-pyridin-3-carbonsäure amid
**181** 2-[3-(4-Fluorphenyl)-propylsulfanyl]-N-(tetrahydro-furan-2-yl-methyl)-pyridin-3-carbonsäure amid
**182** 2-[3-(4-Fluorphenyl)-propylsulfanyl]-N-(2-methoxy-3,3-dimethyl-butyl)-pyridin-3-carbonsäure amid
**183** 2-[[3,3-Difluor-3-(4-fluorphenyl)-propyl]sulfanyl]-N-(m-tolyl-methyl)-pyridin-3-carbonsäure amid
**184** 2-[[3,3-Difluor-3-(4-fluorphenyl)-propyl]sulfanyl]-N-[(3,5-dimethyl-phenyl)-methyl]-pyridin-3-carbonsäure amid
**185** 2-[[3,3-Difluor-3-(4-fluorphenyl)-propyl]sulfanyl]-N-propyl-pyridin-3-carbonsäure amid
**186** 2-[[3,3-Difluor-3-(4-fluorphenyl)-propyl]sulfanyl]-N-hexyl-pyridin-3-carbonsäure amid

**187** 2-[[3,3-Difluor-3-(4-fluorphenyl)-propyl]sulfanyl]-N-(tetrahydro-furan-3-yl-methyl)-pyridin-3-carbonsäure amid
**188** 2-[[3,3-Difluor-3-(4-fluorphenyl)-propyl]sulfanyl]-N-(tetrahydro-pyran-3-yl-methyl)-pyridin-3-carbonsäure amid
**189** 2-[[3,3-Difluor-3-(4-fluorphenyl)-propyl]sulfanyl]-N-(tetrahydro-pyran-4-yl-methyl)-pyridin-3-carbonsäure amid
**190** 2-[[3,3-Difluor-3-(4-fluorphenyl)-propyl]sulfanyl]-N-(furan-2-yl-methyl)-pyridin-3-carbonsäure amid
**191** 2-[[3,3-Difluor-3-(4-fluorphenyl)-propyl]sulfanyl]-N-[(5-methyl-furan-2-yl)-methyl]-pyridin-3-carbonsäure amid
**192** 2-[[3,3-Difluor-3-(4-fluorphenyl)-propyl]sulfanyl]-N-pentyl-pyridin-3-carbonsäure amid
**193** 2-[[3,3-Difluor-3-(4-fluorphenyl)-propyl]sulfanyl]-N-(3-methoxy-butyl)-pyridin-3-carbonsäure amid
**194** 2-[[3,3-Difluor-3-(4-fluorphenyl)-propyl]sulfanyl]-N-(2-methoxy-propyl)-pyridin-3-carbonsäure amid
**195** 2-[[3,3-Difluor-3-(4-fluorphenyl)-propyl]sulfanyl]-N-(2-methoxy-butyl)-pyridin-3-carbonsäure amid
**196** 3-[[2-[[3,3-Difluor-3-(4-fluorphenyl)-propyl]sulfanyl]-pyridin-3-carbonyl]amino]-propionsäure methyl ester
**197** 3-[[2-[[3,3-Difluor-3-(4-fluorphenyl)-propyl]sulfanyl]-pyridin-3-carbonyl]amino]-propionsäure
**198** 2-[[3,3-Difluor-3-(4-fluorphenyl)-propyl]sulfanyl]-N-(2-dimethylaminoethyl)-pyridin-3-carbonsäure amid
**199** 2-[[3-(3,4-Difluor-phenyl)-1-methyl-propyl]sulfanyl]-N-[(4-fluorphenyl)-methyl]-pyridin-3-carbonsäure amid
**200** 2-[[3-(3,4-Difluor-phenyl)-1-methyl-propyl]sulfanyl]-N-(3-methyl-butyl)-pyridin-3-carbonsäure amid
**201** N-[(4-Fluorphenyl)-methyl]-2-[[3-(3-fluorphenyl)-1-methyl-propyl]sulfanyl]-pyridin-3-carbonsäure amid
**202** 2-[[3-(3-Fluorphenyl)-1-methyl-propyl]sulfanyl]-N-(3-methyl-butyl)-pyridin-3-carbonsäure amid
**203** N-(3-Methyt-butyl)-2-[[1-methyl-3-[3-(trifluormethyl)phenyl]-propyl]sulfanyl]-pyridin-3-carbonsäure amid
**204** 2-[[3-(3,4-Difluor-phenyl)-3,3-difluor-propyl]sulfanyl]-N-[(4-fluorphenyl)-methyl]-pyridin-3-carbonsäure amid
**205** N-(1-Bicyclo[2.2.1]heptanyl-methyl)-2-[[3,3-difluor-3-(4-fluorphenyl)-propyl]sulfanyl]-pyridin-3-carbonsäure amid
**206** N-(1-Bicyclo[2.2.1]heptanyl-methyl)-2-[3-(4-fluorphenyl)-propylsulfanyl]-pyridin-3-carbonsäure amid
**207** N-[(4-Fluorphenyl)-methyl]-2-[3-[3-(trifluormethyl)phenyl]-propylsulfanyl]-pyridin-3-carbonsäure amid
**208** N-(3-Methyl-butyl)-2-[3-[3-(trifluormethyl)phenyl]-propylsulfanyl]-pyridin-3-carbonsäure amid
**209** 2-[[3-(3,4-Difluor-phenyl)-3,3-difluor-propyl]sulfanyl]-N-(3-methyl-butyl)-pyridin-3-carbonsäure amid
**210** N-(7-Bicyclo[2.2.1]heptanyl-methyl)-2-[[3-(3,4-difluor-phenyl)-3,3-difluor-propyl]sulfanyl]-pyridin-3-carbonsäure amid
**211** 2-[[3,3-Difluor-3-(4-fluorphenyl)-propyl]sulfanyl]-N-(2-hydroxy-ethyl)-pyridin-3-carbonsäure amid und
**224** N-[(4-Fluor-2-methoxy-phenyl)-methyl]-2-[3-(4-fluorphenyl)-propylsulfanyl]-pyridin-3-carbonsäure amid

oder deren physiologisch verträgliche Salze.

**12.** Arzneimittel enthaltend wenigstens ein substituiertes Nicotinamid nach einem der Ansprüche 1 bis 11, jeweils in Form eines einzelnen Stereoisomers oder deren Mischung, der freien Verbindungen und/oder ihrer physiologisch verträglichen Salze, sowie ggf. geeignete Zusatz- und/ oder Hilfsstoffe und/oder gegebenenfalls weiterer Wirkstoffe.

**13.** Verwendung wenigstens eines substituierten Nicotinamids nach einem der Ansprüche 1 bis 11. jeweils in Form eines einzelnen Stereoisomers oder deren Mischung, der freien Verbindung und/oder ihrer physiologisch verträglichen Salze, zur Herstellung eines Arzneimittels zur Behandlung von Schmerz, Epilepsie, Angstzuständen, Abhängigkeit, Manie, bipolaren Störungen, Migräne, kognitiven Erkrankungen, Dystonie-assoziierten Dyskinesien und/oder Harninkontinenz.

**14.** Ein substituiertes Nicotinamid nach einem der Ansprüche 1 bis 11, jeweils in Form eines einzelnen Stereoisomers oder deren Mischung, der freien Verbindung und/oder ihrer physiologisch verträglichen Salze zur Behandlung von Schmerz, Epilepsie, Angstzuständen, Abhängigkeit, Manie, bipolaren Störungen, Migräne, kognitiven Erkrankungen, Dystonie-assoziierten Dyskinesien und/oder Harninkontinenz.

## Claims

**1.** Substituted nicotine amides with the general formula (1),

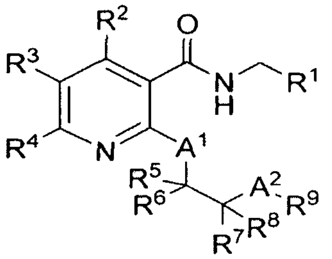

(1)

wherein

$A^1$ stands for $CR^{10}R^{11}$ or S;
$A^2$ stands for $CR^{12}R^{13}$, C(=O), O, S, S(=O) or $S(=O)_2$;

$R^1$ stands for $C_{1-10}$-alkyl or $C_{2-10}$-heteroalkyl, each saturated or unsaturated, branched or unbranched, unsubstituted or mono- or polysubstituted; $C_{3-10}$-cycloalkyl or heterocyclyl, each saturated or unsaturated, unsubstituted or mono- or polysubstituted, aryl or heteroaryl, each unsubstituted or mono- or polysubstituted; $C_{3-10}$-cycloalkyl or heterocyclyl bridged via $C_{1-8}$-alkyl or $C_{2-8}$-heteroalkyl, each saturated or unsaturated, unsubstituted or mono- or polysubstituted, whereby the alkyl or heteroalkyl chain can each be branched or unbranched, saturated or unsaturated, unsubstituted, mono- or polysubstituted; or aryl or heteroaryl bridged via $C_{1-8}$-alkyl or $C_{2-8}$-heteroalkyl, each unsubstituted or mono- or polysubstituted, whereby the alkyl or heteroalkyl chain can each be branched or unbranched, saturated or unsaturated, unsubstituted, mono- or polysubstituted;
$R^2$, $R^3$ and $R^4$ each stand, independent from each other, for H; F; Cl; Br; I; $NO_2$; $CF_3$; CN; OH; $OCF_3$; SH; $SCF_3$; methyl; $CH_2$-methyl; $CH_2$-OH; $C_{2-6}$-alkyl; O-$C_{1-6}$-alkyl, S-$C_{1-6}$-alkyl, each saturated or unsaturated, branched or unbranched, unsubstituted or mono- or polysubstituted; $C_{3-7}$-cycloalkyl, saturated or unsaturated, branched or unbranched, unsubstituted or mono- or polysubstituted; $NR^aR^b$, whereby $R^a$ and $R^b$, each stand, independent from each other, for H or $C_{1-4}$-alkyl, saturated or unsaturated, branched or unbranched, unsubstituted or mono- or polysubstituted, or $R^a$ and $R^b$ together with the nitrogen atom connecting the same form a heterocyclyl, saturated or unsaturated, branched or unbranched, unsubstituted or mono- or polysubstituted.
$R^5$, $R^6$, $R^7$, $R^8$, $R^{10}$, $R^{11}$, $R^{12}$ and $R^{13}$ each stand, independent from each other, for H; F; Cl; Br; I; $NO_2$; $CF_3$; CN; OH; $OCF_3$; SH; $SCF_3$; $C_{1-10}$-alkyl, $C_{2-10}$-heteroalkyl, O-$C_{1-10}$-alkyl or S-$C_{1-10}$-alkyl, each saturated or unsaturated, branched or unbranched, unsubstituted or mono- or polysubstituted; $C_{3-10}$-cycloalkyl, saturated or unsaturated, branched or unbranched, unsubstituted or mono- or polysubstituted;
under the provision that $A^2$ cannot mean S, S(=O) or $S(=O)_2$ if $R^5$, $R^6$, $R^7$ and $R^8$ each mean H, and $A^1$ stands for S; or $R^5$ and $R^6$ or $R^7$ and $R^8$ or $R^{10}$ and $R^{11}$ or $R^{12}$ and $R^{13}$ or $R^5$ and $R^{11}$ or $R^5$ and $R^{13}$ or $R^7$ and $R^{13}$ or $R^7$ and $R^{11}$ or $R^{11}$ and $R^{13}$ have a $C_{3-8}$-cycloalkyl or a heterocyclyl with three to eight ring members, each saturated or unsaturated, unsubstituted or mono- or polysubstituted together with the carbon atom or carbon atoms connecting the same; whereby the relevant remaining substituents $R^5$, $R^6$, $R^7$, $R^8$, $R^{10}$, $R^{11}$, $R^{12}$ and $R^{13}$ have the above meaning; or $R^5$ and $R^7$ form a $C_{3-8}$-cycloalkyl, saturated or unsaturated, unsubstituted or mono- or polysubstituted together with the carbon atom or carbon atoms connecting the same; or a heterocyclyl with three to eight
ring members, unsaturated, unsubstituted or mono- or polysubstituted; whereby the relevant remaining substituents $R^6$, $R^8$, $R^{10}$, $R^{11}$, $R^{12}$ and $R^{13}$ have the above meaning;
$R^9$ stands for $C_{3-10}$-cycloalkyl or heteroalkyl, each saturated or unsaturated, unsubstituted or mono- or polysubstituted; aryl or heteroaryl, each unsubstituted or mono- or polysubstituted; or for $CR^cR^d$, whereby $R^c$ and $R^d$ each mean, independent from each other, $C_{1-4}$-alkyl, each saturated or unsaturated, branched or unbranched, unsubstituted or mono- or polysubstituted;
under the provision that the binding of the heteroaryl or heterocyclyl is realised via a carbon atom of the heteroaryl or heterocyclyl of $A^2$ stands for O or S, and $R^9$ means heterocyclyl, saturated or unsaturated, unsubstituted or mono- or polysubstituted; or heteroaryl, unsubstituted or mono- or polysubstituted;
wherein "alkyl substituted", "heteroalkyl substituted", "heterocyclyl substituted" and "cycloalkyl substituted" stands for the substitution of one or more hydrogen atoms, each independent from each other, with F; Cl; Br; I; $NO_2$; $CF_3$; CN; =O; $C_{1-8}$-alkyl; $C_{2-8}$-heteroalkyl; aryl; heteroaryl, $C_{3-10}$-cycloalkyl; heterocyclyl; aryl, heteroaryl, $C_{3-10}$-cycloalkyl or heterocyclyl bridged via $C_{1-8}$-alkyl or $C_{2-8}$-heteroalkyl; CHO; C(=O)$C_{1-8}$-alkyl; C(=O)aryl; C(=O)heteroaryl; $CO_2H$; C(=O)O-$C_{1-8}$-alkyl; C(=O)O=aryl; C(=O)O-heteroaryl; $CONH_2$; C(=O)NH-$C_{1-8}$-alkyl; C(=O)N($C_{1-8}$-alkyl)$_2$; C(=O)NH-aryl, C(=O)N(aryl)$_2$; C(=O)NH-heteroaryl; C(=O)N(heteroaryl)$_2$; C(=O)N($C_{1-8}$-alkyl)(aryl); C(=O)N($C_{1-8}$-alkyl)(heter-

oaryl); C(=O)N(heteroaryl)(aryl); OH; O-$C_{1-8}$-alkyl; $OCF_3$; O-($C_{1-8}$-alkyl)-OH; O-($C_{1-8}$-alkyl)-O-$C_{1-8}$-alkyl; O-benzyl; O-aryl; O-heteroaryl; O-C(=O)$C_{1-8}$-alkyl; O-C(=O)aryl; O-C(=O)heteroaryl; $NH_2$; NH-$C_{1-8}$-alkyl; N($C_{1-8}$-alkyl)$_2$; NH-C(=O)$C_{1-8}$-alkyl; N($C_{1-8}$-alkyl)-C(=O)$C_{1-8}$-alkyl; N(C(=O)$C_{1-8}$-alkyl$_2$; NH-C(=O)-aryl; NH-C(=O)=heteroaryl; SH; S-$C_{1-8}$-alkyl; $SCF_3$; S-benzyl; S-aryl; S-heteroaryl; S(=O)$_2C_{1-8}$-alkyl; S(=O)$_2$aryl; S(=O)$_2$heteroaryl; S(=O)$_2$OH; S(=O)$_2$O-$C_{1-8}$-alkyl; S(=O)$_2$O-aryl; S(=O)$_2$O-heteroaryl; S(=O)$_2$-NH-$C_{1-8}$-alkyl; S(=O)$_2$-NH-aryl; and S(=O)$_2$-NH-$C_{1-8}$-heteroaryl;
wherein "aryl substituted" and "heteroaryl substituted" stands for the substitution of one or more hydrogen atoms, each independent from each other, with F; Cl; Br; I; $NO_2$; $CF_3$; CN; $C_{1-8}$-alkyl; $C_{2-8}$-heteroalkyl; aryl; heteroaryl, $C_{3-10}$-cycloalkyl; heterocyclyl; aryl, heteroaryl, $C_{3-10}$-cycloalkyl or heterocyclyl bridged via $C_{1-8}$-alkyl or $C_{2-8}$-heteroalkyl; CHO; C(=O)$C_{1-8}$-alkyl; C(=O)aryl; C(=O)heteroaryl; C(=O)O-$C_{1-8}$-alkyl; C(=O)O=aryl; C(=O)O-heteroaryl; $CONH_2$; C(=O)NH-$C_{1-8}$-alkyl; C(=O)N($C_{1-8}$-alkyl)$_2$; C(=O)NH-aryl, C(=O)N(aryl)$_2$; C(=O)NH-heteroaryl; C(=O)N(heteroaryl)$_2$; C(=O)N($C_{1-8}$-alkyl)(aryl); C(=O)N($C_{1-8}$-alkyl)(heteroaryl); C(=O)N(heteroaryl)(aryl); OH; O-$C_{1-8}$-alkyl; $OCF_3$; O-($C_{1-8}$-alkyl)-OH; O-($C_{1-8}$-alkyl)-O-$C_{1-8}$-alkyl; O-benzyl; O-aryl; O-heteroaryl; O-C(=O)$C_{1-8}$-alkyl; O-C(=O)aryl; O-C(=O)heteroaryl; $NH_2$; NH-$C_{1-8}$-alkyl; N($C_{1-8}$-alkyl)$_2$; NH-C(=O)$C_{1-8}$-alkyl; N($C_{1-8}$-alkyl)-C(=O)$C_{1-8}$-alkyl; N(C(=O)$C_{1-8}$-alkyl$_2$; NH-C(=O)-aryl; NH-C(=O)=heteroaryl; SH; S-$C_{1-8}$-alkyl; $SCF_3$; S-benzyl; S-aryl; S-heteroaryl; S(=O)$_2C_{1-8}$-alkyl; S(=O)$_2$aryl; S(=O)$_2$heteroaryl; S(=O)$_2$OH; S(=O)$_2$O-$C_{1-8}$-alkyl; S(=O)$_2$O-aryl; S(=O)$_2$O-heteroaryl; S(=O)$_2$-NH-$C_{1-8}$-alkyl; S(=O)$_2$-NH-aryl; and S(=O)$_2$-NH-$C_{1-8}$-heteroaryl;
in the form of the free compounds or salts of physiologically compatible acids or bases.

2. Substituted nicotine amides according to claim 1, **characterised in that**

$A^1$ stands for S; and
$A^2$ stands for $CR^{12}R^{13}$, O, S or S(=O)$_2$.

3. Substituted nicotine amides according to claim 1 or 2, **characterised in that**
$R^1$ stands for part structure (T1)

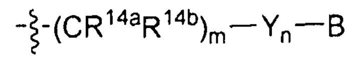

(T1)

wherein
$R^{14a}$ and $R^{14b}$, each independent from each other, stand for H; F; Cl; Br; I; $NO_2$; $CF_3$; CN; OH; $OCF_3$; $NH_2$; $C_{1-4}$-alkyl, O-$C_{1-4}$-alkyl, NH-$C_{1-4}$-alkyl, N($C_{1-4}$-alkyl)$_2$, each saturated or unsaturated, branched or unbranched, unsubstituted or mono- or polysubstituted with one or more substituents, each selected independently from each other from the group standing for and consisting of F, Cl, Br, I, O-$C_{1-4}$-alkyl, OH and $OCF_3$; $C_{3-10}$-cycloalkyl or heterocyclyl, each saturated or unsaturated, unsubstituted or mono- or polysubstituted with one or more substituents, each selected independently from the group consisting of F, Cl, Br, I, $C_{1-4}$-alkyl, OH, =O, O-$C_{1-4}$-alkyl, $OCF_3$, $NH_2$, NH-$C_{1-4}$-alkyl and N($C_{1-4}$-alkyl)$_2$;

m stands for 0, 1, 2 or 3;
Y stands for O or $NR^{15}$,

whereby $R^{15}$ stands for H; $C_{1-4}$-alkyl stands for saturated or unsaturated, branched or unbranched, unsubstituted or mono- or polysubstituted with one or more substituents, each selected independently from the group consisting of F, Cl, Br, I, $C_{1-4}$-alkyl, OH, O-$C_{1-4}$-alkyl, $OCF_3$, $NH_2$, NH-$C_{1-4}$-alkyl and N($C_{1-4}$-alkyl)$_2$; or for $C_{3-10}$-cycloalkyl, saturated or unsaturated, unsubstituted or mono- or polysubstituted with one or more substituents, each selected independently from the group consisting of F, Cl, Br, I, $C_{1-4}$-alkyl, OH, O-$C_{1-4}$-alkyl, $OCF_3$, $NH_2$, NH-$C_{1-4}$-alkyl and N($C_{1-4}$-alkyl)$_2$;

n stands for 0 or 1,
B stands for $C_{1-8}$-alkyl, saturated or unsaturated, branched or unbranched, unsubstituted or mono- or polysubstituted with one or more substituents, each selected independently from the group consisting of F, Cl, Br, I, $NO_2$, CN, OH, =O, O-$C_{1-4}$-alkyl, $OCF_3$, C(=O)-OH, $CF_3$, $NH_2$, NH($C_{1-4}$-alkyl), N($C_{1-4}$-alkyl)$_2$, SH, S-$C_{1-4}$-alkyl, $SCF_3$ and S(=O)$_2$OH; $C_{3-10}$-cycloalkyl or heterocyclyl, each saturated or unsaturated, unsubstituted or mono- or polysubstituted with one or more substituents, each selected independently from the group consisting of F,

Cl, Br, I, $NO_2$, CN, OH, O-$C_{1-8}$-alkyl, $OCF_3$, $C_{1-8}$-alkyl, C(=O)-OH, $CF_3$, $NH_2$, NH($C_{1-8}$-alkyl), N($C_{1-8}$-alkyl)$_2$, SH, S-$C_{1-8}$-alkyl, $SCF_3$, S(=O)$_2$OH, benzyl, phenyl, pyridil and thienyl, whereby benzyl, phenyl, pyridin, thienyl, can each be unsubstituted or mono- or polysubstituted with one or more substituents, each selected independent from each other from the group consisting of F, Cl, Br, I, $NO_2$, CN, OH, O-$C_{1-8}$-alkyl, $OCF_3$, $C_{1-8}$-alkyl, C(=O)-OH, $CF_3$, $NH_2$, NH($C_{1-8}$-alkyl), N($C_{1-8}$-alkyl)$_2$, SH, S-$C_{1-8}$-alkyl, $SCF_3$ and S(=O)$_2$OH; aryl or heteroaryl, each unsubstituted or mono- or polysubstituted with one or more substituents, each selected independent from each other from the group consisting of F, Cl, Br, I, $NO_2$, CN, OH, O-$C_{1-8}$-alkyl, $OCF_3$, $C_{1-8}$-alkyl, C(=O)-OH, $CF_3$, $NH_2$, NH($C_{1-8}$-alkyl), N($C_{1-8}$-alkyl)$_2$, SH, S-$C_{1-8}$-alkyl, $SCF_3$ and S(=O)$_2$OH, benzyl, phenyl, pyridil and thienyl, whereby benzyl, phenyl, pyridil, thienyl can each be unsubstituted or mono- or polysubstituted with one or more substituents, each selected independent from each other from the group consisting of F, Cl, Br, I, $NO_2$, CN, OH, O-$C_{1-8}$-alkyl, $OCF_3$, $C_{1-8}$-alkyl, C(=O)-OH, $CF_3$, $NH_2$, NH($C_{1-8}$-alkyl), N($C_{1-8}$-alkyl)$_2$, SH, S-$C_{1-8}$-alkyl, $SCF_3$ and S(=O)$_2$OH;

4. Substituted nicotine amides according to claim 3, **characterised in that**
$R^{14a}$ and $R^{14b}$, each independent from each other, stand for H; F; Cl; Br; I; $NO_2$; $CF_3$; CN; methyl; ethyl; n-propyl; iso-propyl; cyclopropyl; n-butyl; sec.-butyl; tert.-butyl;
$CH_2CF_3$; OH; O-methyl; O-ethyl; O-($CH_2$)$_2$-O-$CH_3$; O-($CH_2$)$_2$-OH; $OCF_3$; $NH_2$; NH-methyl; N(methyl)$_2$; NH-ethyl; N(ethyl)$_2$; or N(methyl)(ethyl);

m for 0, 1 or 2;
n for 0; and B stands for $C_{1-4}$-alkyl, saturated, branched or unbranched, unsubstituted or mono- or polysubstituted with one or more substituents, each selected independently from the group consisting of F, Cl, Br, I, OH, O-$C_{1-4}$-alkyl, $OCF_3$ and $CF_3$; $C_{3-10}$-cycloalkyl, saturated, unsubstituted; phenyl, naphthyl, pyridil, thienyl, each unsubstituted or mono- or double- or triple-substituted with one, two or three substituents, each selected independently from the group consisting of F, Cl, Br, I, $NO_2$, CN, OH, O-$C_{1-4}$-alkyl, $OCF_3$, $C_{1-4}$-alkyl, C(=O)-OH, $CF_3$, $NH_2$, NH($C_{1-4}$-alkyl), N($C_{1-4}$-alkyl)$_2$, SH, S-$C_{1-4}$-alkyl, $SCF_3$, S(=O)$_2$OH.

5. Substituted nicotine amides according to one of the claims 1-4, **characterised in that** $R^2$, $R^3$ and $R^4$ each stand, independent from each other, for H; F; Cl; Br; I; $NO_2$; $CF_3$; CN; OH; $OCF_3$; SH; $SCF_3$; methyl; $CH_2$-O-methyl; $CH_2$-OH; $C_{2-6}$-alkyl; O-$C_{1-6}$-alkyl, S-$C_{1-6}$-alkyl, each saturated or unsaturated, branched or unbranched, unsubstituted or mono- or polysubstituted with one or more substituents selected, independent from each other, from the group consisting of F, Cl, Br, I, OH, =O and O-$C_{1-4}$-alkyl; $C_{3-7}$-cycloalkyl, saturated or unsaturated, branched or unbranched, unsubstituted; $NR^aR^b$, whereby $R^a$ and $R^b$ each stand, independent from each other, for H or $C_{1-4}$-alkyl, saturated or unsaturated, branched or unbranched, unsubstituted or mono- or polysubstituted with one or more substituents selected, independent from each other, from the group consisting of OH, =O and O-$C_{1-4}$-alkyl, or $R^a$ and $R^b$ together with the nitrogen atom connecting the same form a heterocyclyl,
saturated or unsaturated, branched or unbranched, unsubstituted or mono- or polysubstituted with $C_{1-4}$-alkyl.

6. Substituted nicotine amides according to claim 5, **characterised in that**
$R^2$, $R^3$ and $R^4$ each stand, independent from each other, for H; F; Cl; Br; I; methyl; ethyl; n-propyl; iso-propyl; cyclopropyl; CN; $CF_3$; O-methyl; $OCF_3$; S-methyl; $SCF_3$.

7. Substituted nicotine amides according to one of the claims 1-6, **characterised in that**
$R^5$, $R^6$, $R^7$, $R^8$, $R^{10}$, $R^{11}$, $R^{12}$ and $R^{13}$ preferably each stand, independent from each other, for H; F; Cl; Br; I; $NO_2$; $CF_3$; CN; OH; $OCF_3$; SH; $SCF_3$; methyl; ethyl; n-propyl; iso-propyl; n-butyl, sec.-butyl, tert.-butyl, O-methyl; O-ethyl; O-($CH_2$)$_2$-O-$CH_3$; O-($CH_2$)$_2$-OH; S-methyl; S-ethyl; cyclopropyl, cyclobutyl; cyclopentyl; cyclohexyl;
or $R^5$ and $R^6$ or $R^7$ and $R^8$ or $R^{10}$ and $R^{11}$ or $R^{12}$ and $R^{13}$ or $R^5$ and $R^{11}$ or $R^5$ and $R^7$ or $R^5$ and $R^{13}$ or $R^7$ and $R^{13}$ or $R^7$ and $R^{11}$ or $R^{11}$ and $R^{13}$ form a cyclopropyl, cyclobutyl, cyclopentyl or cyclohexyl together with the carbon atom(s) connecting the same, each unsubstituted or mono- or polysubstituted with one or more substituents, each unsubstituted; whereby the relevant remaining substituents $R^5$, $R^6$, $R^7$, $R^8$, $R^{10}$, $R^{11}$, $R^{12}$ and R1$^3$ have the above meaning.

8. Substituted nicotine amides according to one of the claims 1-7, **characterised in that**
$R^9$ stands for $C_{3-10}$-cycloalkyl or heterocyclyl, each saturated or unsaturated, unsubstituted or mono- or polysubstituted with one or more substituents, each selected independent from each other from the group consisting of F, Cl, Br, I, $NO_2$, CN, OH, =O, O-$C_{1-4}$-alkyl, $OCF_3$, $C_{1-4}$-alkyl, $CF_3$, SH, S-$C_{1-4}$-alkyl and $SCF_3$; aryl or heteroaryl, each unsubstituted or mono- or polysubstituted with one or
more substituents, each selected independent from each other from the group consisting of F, Cl, Br, I, $NO_2$, CN,

OH, O-$C_{1-4}$-alkyl, $OCF_3$, $C_{1-4}$-alkyl, $CF_3$, $NH_2$, NH($C_{1-4}$-alkyl), N($C_{1-4}$-alkyl)$_2$, SH, S-$C_{1-4}$-alkyl and $SCF_3$; or for $CR^cR^d$, whereby $R^c$ and $R^d$, each independent from each other, substitute $C_{1-4}$-alkyl, saturated or unsaturated, branched or unbranched, unsubstituted or mono- or polysubstituted with one or more substituents, each selected independent from each other from the group consisting of F, Cl, Br, I, OH, O-$C_{1-4}$-alkyl, $CF_3$, $OCF_3$ and $SCF_3$.

**9.** Substituted nicotine amides according to one of the claims 1-8, **characterised in that**
$R^9$ stands for phenyl, pyridyl and thienyl, unsubstituted or mono- or polysubstituted with one or more substituents, each selected independent from each other from the group consisting of F, Cl, Br, I, CN, OH, O-$C_{1-4}$-alkyl, $OCF_3$, $C_{1-4}$-alkyl, $CF_3$, SH, S-$C_{1-4}$-alkyl and $SCF_3$.

**10.** Substituted nicotine amides according to one of the claims 1-9, **characterised in that**

$A^1$ stands for S; and
$A^2$ for $CR^{12}R^{13}$;
$R^1$ for the part structure (T1-1)

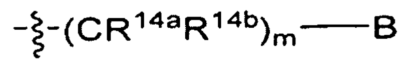

(T1-1)

wherein
$R^{14a}$ and $R^{14b}$, each independent from each other, stand for H; F; Cl; Br; I; methyl; ethyl; n-propyl; iso-propyl; n-butyl; sec.-butyl; tert.-butyl; OH; O-methyl; O-ethyl; O-$(CH_2)_2$-O-$CH_3$; or O-$(CH_2)_2$-OH;

m stands for 0, 1 or 2;
B stands for methyl; ethyl; n-propyl; iso-propyl; n-butyl; sec.-butyl; tert.-butyl; cyclopropyl; cyclobutyl; cyclopentyl; cyclohexyl; cycloheptyl; adamantyl; bicyclo[2.2.1]heptyl; bicyclo[2.2.2]octyl; phenyl, pyridyl, thienyl, each unsubstituted or mono-, double- or triple-substituted with one, two or three substituents, each selected independent from each other from the group consisting of F, Cl, Br, I, $NO_2$, CN, OH, O-$C_{1-4}$-alkyl, $OCF_3$, $C_{1-4}$-alkyl, C(=O)-OH, $CF_3$, $NH_2$, NH($C_{1-4}$-alkyl), N($C_{1-4}$-alkyl)$_2$, SH, S-$C_{1-4}$-alkyl, $SCF_3$, S(=O)$_2$OH;

$R^2$, $R^3$ and $R^4$, each independent from each other, stand for H; F; Cl; methyl; ethyl; $CF_3$ or O-methyl;
$R^5$, $R^6$, $R^7$, $R^8$, $R^{10}$, $R^{11}$, $R^{12}$ and $R^{13}$, each independent from each other, stand for H; F; Cl; methyl; ethyl; n-propyl, iso-propyl; cyclopropyl;
$R^9$ stands for phenyl, pyridyl and thienyl, unsubstituted or mono- or polysubstituted with one or more substituents, each selected independent from each other from the group consisting of F, Cl, Br, I, CN, methyl, ethyl, n-propyl, iso-propyl, n-butyl, sec.-butyl, tert.-butyl, O-methyl, O-ethyl, O-n-propyl, O-iso-propyl, O-butyl, O-sec.-butyl, O-tert.-butyl, OH, $OCF_3$, $CF_3$, SH, S-$C_{1-4}$-alkyl and $SCF_3$.

**11.** Substituted nicotine amides according to one of the claims 1-10, selected from the group

**1** 2-(3-phenyl-propylsulfanyl)-N-(thiophene-2-yl-methyl)-pyridine-3-carboxylic acid amide;
**2** 2-(3-cyclohexyl-propylsulfanyl)-N-(thiophene-2-yl-methyl)-pyridine-3-carboxylic acid amide;
**3** 2-[(3-oxo-phenyl-propyl)sulfanyl]-N-(thiophene-2-yl-methyl)-pyridine-3-carboxylic acid amide;
**4** N-(thiophene-2-yl-methyl)-2-[2-[3-(trifluoromethyl)-phenoxy]-ethylsulfanyl]-pyridine-3-carboxylic acid amide;
**5** 2-(4-methyl-pentylsulfanyl)-N-(thiophene-2-yl-methyl)-pyridine-3-carboxylic acid amide;
**7** 2-(4-phenyl-butyl)-N-(thiophene-2-yl-methyl)-pyridine-3-carboxylic acid amide;
**8** 2-[(3-benzosulfonyl)-propyl]- N-(cyclohexyl-methyl)-pyridine-3-carboxylic acid amide;
**9** N-(cyclohexyl-methyl)-2-(4-phenyl-butyl)- pyridine-3-carboxylic acid amide;
**10** 2-[3-benzosulfonyl)-propyl]-N-(thiophene-2-yl-methyl)-pyridine-3-carboxylic acid amide;
**12** N-(thiophene-2-yl-methyl)-2-[3-[[3-(trifluoromethyl)phenyl]sulfonyl]-propyl]-pyridine-3-carboxylic acid amide;
**13** N-(thiophene-2-yl-methyl)-2-[3-[[3-(trifluoro-methyl)phenyl]sulfanyl]-propyl]-pyridine-3-carboxylic acid amide;
**16** 2-(2-phenylsulfanyl-propylsulfanyl)-N-(thiophene-2-yl-methyl)-pyridine-3-carboxylic acid amide;
**17** 2-[3-(4-fluorophenyl)-propylsulfanyl]-N-(thiophene-2-yl-methyl)-pyridine-3-carboxylic acid amide;
**18** N-(thiophene-2-yl-methyl)-2-[3-[3-(trifluoromethyl)phenyl]-propylsulfanyl]-pyridine-3-carboxylic acid amide;

**19** 2-[2-[(4-fluorophenyl)sulfanyl]-propylsulfanyl]-N-(thiophene-2-yl-methyl)-pyridine-3-carboxylic acid amide;
**20** N-(thiophene-2-yl-methyl)-2-[2-[[3-(trifluoromethyl)phenyl]sulfanyl]-propylsulfanyl]-pyridine-3-carboxylic acid amide;
**21** N-(thiophene-2-yl-methyl)-2-[4-[3-(trifluoromethyl)-phenyl]-butyl]-pyridine-3-carboxylic acid amide;
**22** 2-[4-(4-fluorophenyl)-butyl]-N-(thiophene-2-yl-methyl)-pyridine-3-carboxylic acid amide;
**23** 2-(3-phenylsulfanyl-propyl)-N-(thiophene-2-yl-methyl)-pyridine-3-carboxylic acid amide;
**24** 2-[(1-methyl-phenylsulfanyl-ethyl)sulfanyl]-N-(thiophene-2-yl-methyl)-pyridine-3-carboxylic acid amide;
**25** N-(cycloheptyl-methyl)-2-[4-[3-(trifluoromethyl)phenyl]-butyl]-pyridine-3-carboxylic acid amide;
**26** N-(5-bicyclo[2.2.1]heptanyl-methyl)-2-[4-[3-(trifluoromethyl)phenyl]-butyl]-pyridine-3-carboxylic acid amide;
**27** N-(5-bicyclo[2.2.1]heptanyl-methyl)-2-[3-[3-(trifluoromethyl)phenyl]-propylsulfanyl]-pyridine-3-carboxylic acid amide;
**28** N-(5-bicyclo[2.2.1]heptanyl-methyl)-2-(4-methyl-pentylsulfanyl)-pyridine-3-carboxylic acid amide;
**29** N-(cycloheptyl-methyl)-2-[3-[(4-fluorophenyl)sulphanyl]-propyl]-pyridine-3-carboxylic acid amide;
**30** N-(5-bicyclo[2.2.1]heptanyl-methyl)-2-[3-[(4-fluorophenyl)sulfanyl]-propyl]-pyridine-3-carboxylic acid amide;
**31** N-(cycloheptyl-methyl)-2-[3-[[3-(trifluoromethyl)phenyl]sulphonyl]-propyl]-pyridine-3-carboxylic acid amide;
**32** N-(5-bicyclo[2.2.1]heptanyl-methyl)-2-[3-[[3-(trifluoromethyl)phenyl]sulfonyl]-propyl]-pyridine-3-carboxylic acid amide;
**33** N-(5-bicyclo[2.2.1]heptanyl-methyl)-2-(3-phenyl-propylsulfanyl)-pyridine-3-carboxylic acid amide;
**34** N-(5-bicyclo[2.2.1]heptanyl-methyl)-2-[3-[4-(trifluoromethyl)-phenyl]-propylsulfanyl]-pyridine-3-carboxylic acid amide;
**35** N-(5-bicyclo[2.2.1]heptanyl-methyl)-2-[3-(4-chlorophenyl)-propylsulfanyl]-pyridine-3-carboxylic acid amide;
**36** N-[(3,5-difluoro-phenyl)-methyl]-2-[3-(4-fluorophenyl)-propylsulfanyl]-pyridine-3-carboxylic acid amide;
**37** N-[(5-chloro-thiophene-2-yl)-methyl]-2-[3-(4-fluorophenyl)-propylsulfanyl]-pyridine-3-carboxylic acid amide;
**38** N-[(2,2-dimethyl-cycopropyl)-methyl]-2-[3-(4-fluorophenyl)-propylsulfanyl]-pyridine-3-carboxylic acid amide;
**39** N-(5-bicyclo[2.2.1]heptanyl-methyl)-2-[3-(3-fluorophenyl)-propylsulfanyl]-pyridine-3-carboxylic acid amide;
**40** N-(cyclohexyl-methyl)-2-[3-(4-fluorophenyl]-propylsulfanyl]-pyridine-3-carboxylic acid amide;
**41** N-(cycloheptyl-methyl)-2-[3-(4-fluorophenyl]-propylsulfanyl]-pyridine-3-carboxylic acid amide;
**43** N-(5-bicyclo[2.2.1]heptanyl-methyl)-2-[[3-(4-fluorophenyl)-3-oxo-propyl]sulfanyl]-pyridine-3-carboxylic acid amide;
**44** N-(5-bicyclo[2.2.1]heptanyl-methyl)-2-[(3-oxo-phenyl-propyl)sulfanyl]-pyridine-3-carboxylic acid amide;
**45** N-(5-bicyclo[2.2.1]heptanyl-methyl)-2-(hexylsulfanyl)-pyridine-3-carboxylic acid amide;
**46** N-(5-bicyclo[2.2.1]heptanyl-methyl)-2-(2-phenoxy-ethylsulfanyl)-pyridine-3-carboxylic acid amide;
**47** N-(5-bicyclo[2.2.1]heptanyl-methyl)-2-[2-[3-(trifluoromethyl)-phenoxy]-ethylsulfanyl]-pyridine-3-carboxylic acid amide;
**48** 2-[3-(4-fluorophenyl)-propylsulfanyl]-N-(3,3,3-trifluoro-propyl)-pyridine-3-carboxylic acid amide;
**49** N-(5-bicyclo[2.2.1]heptanyl-methyl)-2-[2-(4-fluoro-phenoxy)-ethylsulfanyl]-pyridine-3-carboxylic acid amide;
**52** N-(5-bicyclo[2.2.1]heptanyl-methyl)-2-(3-naphthalene-1-yl-propylsulfanyl)-pyridine-3-carboxylic acid amide;
**53** N-(5-bicyclo[2.2.1]heptanyl-methyl)-2-[3-[3-fluoro-4-(trifluoromethyl)-phenyl]-propylsulfanyl]-pyridine-3-carboxylic acid amide;
**54** N-(5-bicyclo[2.2.1]heptanyl-methyl)-2-[3-[4-fluoro-3-(trifluoromethyl)-phenyl]-propylsulfanyl]-pyridine-3-carboxylic acid amide;
**55** N-(cyclooctyl-methyl)-2-[3-(4-fluorophenyl)-propylsulfanyl]-pyridine-3-carboxylic acid amide;
**56** N-(5-bicyclo[2.2.1]heptanyl-methyl)-2-[3-(4-methoxyphenyl)-propylsulfanyl]-pyridine-3-carboxylic acid amide;
**57** 2-[3-(4-fluorophenyl)-propylsulfanyl]-N-[(4methoxyphenyl)-methyl]-pyridine-3-carboxylic acid amide;
**59** 2-[3-(4-fluorophenyl)-propylsulfanyl]-N-[[3-(trifluoromethyl)phenyl]-methyl]-pyridine-3-carboxylic acid amide;
**60** N-(5-bicyclo[2.2.1]heptanyl-methyl)-2-[3-(3,4-difluoro-phenyl)-propylsulfanyl]-pyridine-3-carboxylic acid amide;
**61** N-(5-bicyclo[2.2.1]heptanyl-methyl)-2-[3-(3,5-difluoro-phenyl)-propylsulfanyl]-pyridine-3-carboxylic acid amide;
**62** N-(5-bicyclo[2.2.1]heptanyl-methyl)-2-[3-(2,4-difluoro-phenyl)-propylsulfanyl]-pyridine-3-carboxylic acid amide;
**63** N-[(2-fluorophenyl)-methyl]-2-[3-(4-fluorophenyl)-propylsulfanyl]- pyridine-3-carboxylic acid amide;
**64** N-[(3-fluorophenyl)-methyl]-2-[3-(4-fluorophenyl)-propylsulfanyl]- pyridine-3-carboxylic acid amide;
**65** N-[(4-fluorophenyl)-methyl]-2-[3-(4-fluorophenyl)-propylsulfanyl]-pyridine-3-carboxylic acid amide;
**66** N-benzyl-2-[3-(4-fluorophenyl)-propylsulfanyl]-pyridine-3-carboxylic acid amide;
**67** N-(5-bicyclo[2.2.1]heptanyl-methyl)-2-[(1-methyl-3-phenyl-propyl)sulfanyl]-pyridine-3-carboxylic acid amide;

**68** N-(5-bicyclo[2.2.1]heptanyl-methyl)-2-[3-(3,4,5-trifluoro-phenyl)-propylsulfanyl]- pyridine-3-carboxylic acid amide;
**69** N-(5-bicyclo[2.2.1]heptanyl-methyl)-2-[3-[3-fluoro-5-(trifluoromethyl)-phenyl]-propylsulfanyl]- pyridine-3-carboxylic acid amide;
**70** N-(5-bicyclo[2.2.1]heptanyl-methyl)-2-[3-(3-methoxyphenyl)-propylsulfanyl]-pyridine-3-carboxylic acid amide;
**71** N-[(3,4-difluoro-phenyl)-methyl]-2-[3-(4-fluorophenyl)-propylsulfanyl]-pyridine-3-carboxylic acid amide;
**72** N-(2,3-dihydro-benzofuran-5-yl-methyl)-2-[3-(4-fluorophenyl)-propylsulfanyl]-pyridine-3-carboxylic acid amide;
**73** N-(5-bicyclo[2.2.1]heptanyl-methyl)-2-[3-(3-hydroxyphenyl)-propylsulfanyl]-pyridine-3-carboxylic acid amide;
**74** N-(5-bicyclo[2.2.1]heptanyl-methyl)-2-[[3-(4-fluorophenyl)-1-methylpropyl]sulfanyl]-pyridine-3-carboxylic acid amide;
**75** N-(5-bicyclo[2.2.1]heptanyl-methyl)-2-[[3-(4-fluorophenyl)-2-methyl-propyl]sulfanyl]- pyridine-3-carboxylic acid amide;
**76** 2-[3-(4-fluorophenyl)-propylsulfanyl]-N-[(2-methoxyphenyl)-methyl]-pyridine-3-carboxylic acid amide;
**77** 2-[3-(4-fluorophenyl)-propylsulfanyl]-N-[(3-methoxyphenyl)-methyl]-pyridine-3-carboxylic acid amide;
**78** 2-[3-(4-fluorophenyl)-propylsulfanyl]-N-phenethyl-pyridine-3-carboxylic acid amide;
**79** 2-[3-(4-fluorophenyl)-propylsulfanyl]-N-[[4-(trifluoromethyloxy)-phenyl]-methyl]-pyridine-3-carboxylic acid amide;
**80** 2-[3-(4-fluorophenyl)-propylsulfanyl]-N-[[4-(trifluoromethyl)-phenyl]-methyl]-pyridine-3-carboxylic acid amide;
**81** 2-[3-(4-fluorophenyl)-propylsulfanyl]-N-(2-pyridine-3-yl-ethyl)-pyridine-3-carboxylic acid amide;
**82** 2-[3-(4-fluorophenyl)-propylsulfanyl]-N-(2-pyridine-2-yl-ethyl)-pyridine-3-carboxylic acid amide;
**83** 2-[3-(4-fluorophenyl)-propylsulfanyl]-N-[(2-hydroxyphenyl)-methyl]-pyridine-3-carboxylic acid amide;
**84** 2-[3-(4-fluorophenyl)-propylsulfanyl]-N-[(3-hydroxyphenyl)-methyl]-pyridine-3-carboxylic acid amide;
**85** 2-[3-(4-fluorophenyl)-propylsulfanyl]-N-[2-(m-tolyol)-ethyl]-pyridine-3-carboxylic acid amide;
**86** 2-[3-(4-fluorophenyl)-propylsulfanyl]-N-[2-(o-tolyol)-ethyl]-pyridine-3-carboxylic acid amide;
**87** 2-[3-(4-fluorophenyl)-propylsulfanyl]-N-[[3-(trifluoromethyloxy)-phenyl]-methyl]-pyridine-3-carboxylic acid amide;
**88** 2-[3-(4-fluorophenyl)-propylsulfanyl]-N-[(4-hydroxyphenyl)-methyl]-pyridine-3-carboxylic acid amide;
**89** 2-[3-(4-fluorophenyl)-propylsulfanyl]-N-(2-pyridine-4-yl-ethyl)-pyridine-3-carboxylic acid amide;
**90** 2-[3-(4-fluorophenyl)-propylsulfanyl]-N-[2-(p-tolyol)-ethyl]-pyridine-3-carboxylic acid amide;
**91** N-(5-bicyclo[2.2.1]heptanyl-methyl)-2-[3-(4-fluorophenyl)-butylsulfanyl]-pyridine-3-carboxylic acid amide;
**92** N-(5-bicyclo[2.2.1]heptanyl-methyl)-2-[3-(2,4,5-trifluoro-phenyl)-propylsulfanyl]- pyridine-3-carboxylic acid amide;
**93** 2-[3-(4-fluorophenyl)-propylsulfanyl]-N-(3-pyridine-2-yl-propyl)-pyridine-3-carboxylic acid amide;
**94** 2-[3-(4-fluorophenyl)-propylsulfanyl]-N-(3-pyridine-3-yl-propyl)-pyridine-3-carboxylic acid amide;
**95** 2-[3-(4-fluorophenyl)-propylsulfanyl]-N-(3-pyridine-4-yl-propyl)-pyridine-3-carboxylic acid amide;
**96** 2-[3-(4-fluorophenyl)-propylsulfanyl]-N-propyl-pyridine-3-carboxylic acid amide;
**97** N-butyl-2-[3-(4-fluorophenyl)-propylsulfanyl]- pyridine-3-carboxylic acid amide;
**98** 2-(3-pyridine-3-yl-propylsylfanyl)-N-(thiophene-2-yl-methyl)-pyridine-3-carboxylic acid amide;
**99** 2-[3-(p-tolyol)-propylsulfanyl]-N-(thiophene-2-yl-methyl)-pyridine-3-carboxylic acid amide;
**100** 2-(4-phenyl-butylsulfanyl)-N-(thiophene-2-yl-methyl)-pyridine-3-carboxylic acid amide;
**101** 2-(3-pyridine-4-yl-propylsulfanyl)-N-(thiophene-2-yl-methyl)-pyridine-3-carboxylic acid amide;
**102** 2-(3-naphthalene-2-yl-propylsulfanyl)-N-(thiophene-2-yl-methyl)-pyridine-3-carboxylic acid amide;
**103** 2-[3-(m-tolyol)-propylsulfanyl]-N-(thiophene-2-yl-methyl)-pyridine-3-carboxylic acid amide;
**104** N-(thiophene-2-yl-methyl)-2-(3-thiophene-2-yl-propylsulfanyl)-pyridine-3-carboxylic acid amide;
**105** 2-[(1-methyl-3-phenyl-propyl)sulfanyl]-N-(thiophene-2-yl-methyl)-pyridine-3-carboxylic acid amide;
**106** N-(thiophene-2-yl-methyl)-2-(3-thiophene-3-yl-propylsulfanyl)-pyridine-3-carboxylic acid amide;
**107** 2-[[1-methyl-3-[3-(trifluoromethyl)phenyl]-propyl]sulfanyl]-N-(thiophene-2-yl-methyl)-pyridine-3-carboxylic acid amide;
**108** 2-[(2-benzyl-cyclohexyl)sulfanyl]-N-(thiophene-2-yl-methyl)-pyridine-3-carboxylic acid amide;
**109** 2-[3-[3-methyl-5-(trifluoromethyl)-phenyl]-propylsulfanyl]-N-(thiophene-2-yl-methyl)-pyridine-3-carboxylic acid amide;
**110** 2-[4-(3,4-difluoro-phenyl)-butylsulfanyl]-N-(thiophene-2-yl-methyl)-pyridine-3-carboxylic acid amide;
**111** 2-(3-pyridine-2-yl-propylsulfanyl)-N-(thiophene-2-yl-methyl)-pyridine-3-carboxylic acid amide;
**113** 2-[3-[4-methyl-3-(trifluoromethyl)-phenyl]-propylsulfanyl]-N-(thiophene-2-yl-methyl)-pyridine-3-carboxylic

acid amide;

**114** 2-[(3-phenyl-cyclohexyl)sulfanyl]-N-(thiophene-2-yl-methyl)-pyridine-3-carboxylic acid amide;

**116** 2-[[3,3-difluoro-3-(4-fluorophenyl)-propyl]sulfanyl-N-(3,3-dimethyl-butyl)-pyridine-3-carboxylic acid amide;

**117** N-(cycloheptyl-methyl)-2-[[3,3-difluoro-3-(4-fluorophenyl)-propyl]sulfanyl]-pyridine-3-carboxylic acid amide;

**118** 2-[[3,3-difluoro-3-(4-fluorophenyl)-propyl]sulfanyl]-N-(thiophene-2-yl-methyl)-pyridine-3-carboxylic acid amide;

**119** 2-[3-(4-fluorophenyl)-propylsulfanyl]-N-(1,2,3,4-tetrahydro-naphthalene-2-yl-methyl)-pyridine-3-carboxylic acid amide;

**120** N-(2,3-dihydro-1H-inden-2-yl-methyl)-2-[3-(4-fluorophenyl)-propylsulfanyl]-pyridine-3-carboxylic acid amide;

**121** N-(1,3-benzodioxol-5-yl-methyl)-2-[3-(4-fluorophenyl)-propylsulfanyl]-pyridine-3-carboxylic acid amide;

**122** 2-[3-(4-fluorophenyl)-propylsulfanyl]-N-[(3-phenyl-phenyl)-methyl]-pyridine-3-carboxylic acid amide;

**123** 2-[3-(4-fluorophenyl)-propylsulfanyl]-N-[(3-methyl-cyclohexyl)-methyl]-pyridine-3-carboxylic acid amide;

**124** 2-[[3,3-difluoro-3-(4-fluorophenyl)-propyl]sulfanyl]-N-[(4-fluorophenyl)-methyl]-pyridine-3-carboxylic acid amide;

**125** N-(3,3-dimethyl-2-oxo-butyl)-2-[3-(4-fluorophenyl)-propylsulfanyl]-pyridine-3-carboxylic acid amide;

**127** 2-[3-(4-fluorophenyl)-propylsulfanyl]-N-(pyridine-3-yl-methyl)-pyridine-3-carboxylic acid amide;

**128** 2-[3-(4-fluorophenyl)-propylsulfanyl]-N-(pyridine-4-yl-methyl)-pyridine-3-carboxylic acid amide;

**129** 3-[[[2-[3-(4-fluorophenyl)-propylsulfanyl]-pyridine-3-carboxyl]amino]-methyl]-benzoic acid methyl ester;

**130** 2-[3-(4-fluorophenyl)-propylsulfanyl]-N-[3-(2-methoxyphenyl)-propyl]-pyridine-3-carboxylic acid amide;

**132** N-[(4-fluorophenyl)-methyl]-2-[[1-methyl-3-[3-(trilfuoromethyl)phenyl]-propyl]sulfanyl]-pyridine-3-carboxylic acid amide;

**133** 2-[3-(4-fluorophenyl)-propylsulfanyl]-N-(3-metyl-butyl)-pyridine-3-carboxylic acid amide;

**134** 2-[3-(4-fluorophenyl)-propylsulfanyl]-N-(tetrahydro-pyran-2-yl-methyl)-pyridine-3-carboxylic acid amide;

**135** 2-[3-(4-fluorophenyl)-propylsulfanyl]-N-[3-(1H-pyrazol-1-yl)-propyl]-pyridine-3-carboxylic acid amide;

**136** 2-[3-(4-fluorophenyl)-propylsulfanyl]-N-(naphthalene-2-yl-methyl)-pyridine-3-carboxylic acid amide;

**137** N-(2,3-dihydro-[1,4]benzodioxin-6-yl-methyl)-2-[3-(4-fluorophenyl)-propylsulfanyl]-pyridine-3-carboxylic acid amide;

**138** 2-[3-(4-fluorophenyl)-propylsulfanyl]-N-[(3-morpholin-4-yl-phenyl)-methyl]-pyridine-3-carboxylic acid amide;

**139** N-(2,3-dihydro-benzofuran-6-yl-methyl)-2-[3-(4-fluorophenyl)-propylsulfanyl]-pyridine-3-carboxylic acid amide;

**140** 2-[3-(4-fluorophenyl)-propylsulfanyl]-N-[[3-(1H-pyrazol-1-yl)-phenyl]-methyl]-pyridine-3-carboxylic acid amide;

**141** 2-[3-(4-fluorophenyl)-propylsulfanyl]-N-[3-(1H-[1,2,3]triazol-1-yl)-propyl]-pyridine-3-carboxylic acid amide;

**142** N-(7-bicyclo[2.2.1]heptanyl-methyl)-2-[3-(4-fluorophenyl)-propylsulfanyl]-pyridine-3-carboxylic acid amide;

**144** 2-[3-(4-fluorophenyl)-propylsulfanyl]-N-(thiazol-2-yl-methyl)-pyridine-3-carboxylic acid amide;

**145** 2-[3-(4-fluorophenyl)-propylsulfanyl]-N-(thiazol-5-yl-methyl)-pyridine-3-carboxylic acid amide;

**146** 2-[3-(4-fluorophenyl)-propylsulfanyl]-N-(oxazol-2-yl-methyl)-pyridine-3-carboxylic acid amide;

**148** N-(3,3-dimethyl-butyl)-2-[3-(4-fluorophenyl)-propylsulfanyl]-4-methyl-pyridine-3-carboxylic acid amide;

**149** N-(3,3-dimethyl-butyl)-2-[[1-methyl-3-[3-(trifluoromethyl)phenyl]-propyl]sulfanyl]-pyridine-3-carboxylic acid amide;

**150** 2-[3-(4-fluorophenyl)-propylsulfanyl]-N-(chinolin-7-yl-methyl)-pyridine-3-carboxylic acid amide;

**151** 2-[3-(4-fluorophenyl)-propylsulfanyl]-N-[(3-pyridine-2-yl-phenyl)-methyl]-pyridine-3-carboxylic acid amide;

**152** 2-[3-(4-fluorophenyl)-propylsulfanyl]-N-[(3-pyridine-3-yl-phenyl)-methyl]-pyridine-3-carboxylic acid amide;

**153** N-(3,3-dimethyl-butyl)-2-[[(1R)-1-methyl-3-phenyl-propyl]sulfanyl]-pyridine-3-carboxylic acid amide;

**154** N-(3,3-dimethyl-butyl)-2-[[(11S)-1-methyl-3-phenyl-propyl]sulfanyl]-pyridine-3-carboxylic acid amide;

**155** 2-[(2-benzyl-cyclopentyl)sulfanyl]-N-(3,3-dimethyl-butyl)-pyridine-3-carboxylic acid amide;

**156** N-(7-bicyclo[2.2.1]heptanyl-methyl)-2-[3-[3-(trifluoromethyl)phenyl]-propylsulfanyl]- pyridine-3-carboxylic acid amide;

**157** 2-[3-(4-fluorophenyl)-propylsulfanyl]-N-[3-(1H-[1,2,4]triazol-1-yl)-propyl]-pyridine-3-carboxylic acid amide;

**158** 2-[3-(4-fluorophenyl)-propylsulfanyl]-N-([1,3,4]oxadiazol-2-yl-methyl)-pyridine-3-carboxylic acid amide;

**159** 2-[3-(4-fluorophenyl)-propylsulfanyl]-N-[(3-pyridine-4-yl-phenyl)-methyl]-pyridine-3-carboxylic acid amide;

**160** N-[[4-(cyclopropylmethyl)-3,4-dihydro-2H-[1,4]benzoxazin-6-yl]-methyl]-2-[3-(4-fluorophenyl)-propylsulfanyl]-pyridine-3-carboxylic acid amide;

**161** N-[(4-ethyl-3,4-dihydro-2H-[1,4]benzoxazin-6-yl)-methyl]-2-[3-(4-fluorophenyl)-propylsulfanyl]-pyridine-3-carboxylic acid amide;

**162** 2-[3-(4-fluorophenyl)-propylsulfanyl]-N-[(4-methyl-3,4-dihydro-2H-[1,4]benzoxazin-6-yl)-methyl]-pyridine-3-carboxylic acid amide;
**163** 2-[3-(4-fluorophenyl)-propylsulfanyl]-N-(3-methyl-3-phenyl-butyl)-pyridine-3-carboxylic acid amide;
**164** N-(7-bicyclo[2.2.1]heptanyl-methyl)-2-[[3,3-difluoro-3-(4-fluorophenyl)-propyl]sulfanyl]- pyridine-3-carboxylic acid amide;
**165** 2-[[3,3-difluoro-3-[3-(trifluoromethyl)phenyl]-propyl]sulfanyl]-N-[(4-fluorophenyl)-methyl]-pyridine-3-carboxylic acid amide;
**166** 2-[[3,3-difluoro-3-[3-(trifluoromethyl)phenyl]-propyl]sulfanyl]-N-(3,3-dimethyl-butyl)-pyridine-3-carboxylic acid amide
**168** 2-[[3,3-difluoro-3-(4-(fluorophenyl)propyl]sulfanyl]-N-[(3-fluorophenyl)-methyl]-pyridine-3-carboxylic acid amide;
**170** N-butyl-2-[[3,3-(difluoro-3-(4-fluorophenyl)-propyl]sulfanyl]-pyridine-3-carboxylic acid amide;
**171** 2-[[3,3-difluoro-3-(4-(fluorophenyl)propyl]sulfanyl]-N-[(2-methoxy-ethyl)-pyridine-3-carboxylic acid amide;
**172** 2-[3-(4-fluorophenyl)-propylsulfanyl]-N-(2-methoxy-ethyl)-pyridine-3-carboxylic acid amide;
**173** N-[(4-fluoro-2-hydroxy-phenyl)-methyl]-2-[3-(4-fluorophenyl)-propylsulfanyl]-pyridine-3-carboxylic acid amide;
**174** 2-[3-(4-fluorophenyl)-propylsulfanyl]-N-(furan-2-yl-methyl)-pyridine-3-carboxylic acid amide;
**175** 2-[3-(4-fluorophenyl)-propylsulfanyl]-N-[(5-methyl-furan-2-yl)-methyl]-pyridine-3-carboxylic acid amide;
**176** N-[(4-fluorophenyl)-methyl]-2-[[3-(4-fluorophenyl)-1-methyl-propyl]sulfanyl]-pyridine-3-carboxylic acid amide;
**177** 2-[[3-(4-(fluorophenyl)-1-methyl-propyl]sulfanyl]-N-(3-methyl-butyl)-pyridine-3-carboxylic acid amide;
**178** N-(7-bicyclo[2.2.1]heptanyl-methyl)-2-[[3-(4-fluorophenyl)-1-methyl-propyl]sulfanyl]-pyridine-3-carboxylic acid amide;
**179** 2-[[3,3-difluoro-3-(4-(fluorophenyl)propyl]sulfanyl-N-(tetrahydro-furan-2-yl-methyl)-pyridine-3-carboxylic acid amide;
**180** 2-[[3,3-difluoro-3-(4-fluorophenyl)-propyl]sulfanyl]-N-(tetrahydro-pyran-2-yl-methyl)-pyridine-3-carboxylic acid amide;
**181** 2-[3-(4-fluorophenyl)-propylsulfanyl]-N-(tetrahydro-furan-2-yl-methyl)-pyridine-3-carboxylic acid amide;
**182** 2-[3-(4-fluorophenyl)-propylsulfanyl]-N-(2-methoxy-3,3-dimethyl-butyl)-pyridine-3-carboxylic acid amide;
**183** 2-[[3,3-difluoro-3-(4-fluorophenyl)-propyl]sulfanyl]-N-(m-tolyol-methyl)-pyridine-3-carboxylic acid amide;
**184** 2-[[3,3-difluoro-3-(4-fluorophenyl)-propyl]sulfanyl]-N-[(3,5-dimethyl-phenyl)-methyl]-pyridine-3-carboxylic acid amide;
**185** 2-[[3,3-difluoro-3-(4-fluorophenyl)-propyl]sulfanyl]-N-propyl-pyridine-3-carboxylic acid amide;
**186** 2-[[3,3-difluoro-3-(4-fluorophenyl)-propyl]sulfanyl]-N-hexyl-pyridine-3-carboxylic acid amide;
**187** 2-[[3,3-difluoro-3-(4-fluorophenyl)-propyl]sulfanyl]-N-(tetrahydro-furan-3-yl-methyl)-pyridine-3-carboxylic acid amide;
**188** 2-[[3,3-difluoro-3-(4-fluorophenyl)-propyl]sulfanyl]-N-(tetrahydro-pyran-3-yl-methyl)-pyridine-3-carboxylic acid amide;
**189** 2-[[3,3-difluoro-3-(4-fluorophenyl)-propyl]sulfanyl]-N-(tetrahydro-pyran-4-yl-methyl)-pyridine-3-carboxylic acid amide;
**190** 2-[[3,3-difluoro-3-(4-fluorophenyl)-propyl]sulfanyl]-N-(furan-2-yl-methyl)-pyridine-3-carboxylic acid amide;
**191** 2-[[3,3-difluoro-3-(4-fluorophenyl)-propyl]sulfanyl]-N-[(5-methyl-furan-2-yl)-methyl]-pyridine-3-carboxylic acid amide;
**192** 2-[[3,3-difluoro-3-(4-fluorophenyl)-propyl]sulfanyl]-N-pentyl-pyridine-3-carboxylic acid amide;
**193** 2-[[3,3-difluoro-3-(4-fluorophenyl)-propyl]sulfanyl]-N-(3-methoxy-butyl)-pyridine-3-carboxylic acid amide;
**194** 2-[[3,3-difluoro-3-(4-fluorophenyl)-propyl]sulfanyl]-N-(2-methoxy-propyl)-pyridine-3-carboxylic acid amide;
**195** 2-[[3,3-difluoro-3-(4-fluorophenyl)-propyl]sulfanyl]-N-(2-methoxy-butyl)-pyridine-3-carboxylic acid amide;
**196** 3-[[2-[[3,3-difluoro-3-(4-fluorophenyl)-propyl]sulfanyl]-pyridine-3-carbonyl]amino]-propionic acid methyl ester;
**197** 3-[[2-[[3,3-difluoro-3-(4-fluorophenyl)-propyl]sulfanyl]-pyridine-3-carbonyl]amino]-propionic acid;
**198** 2-[[3,3-difluoro-3-(4-fluorophenyl)-propyl]sulfanyl]-N-(2-dimethylaminoethyl)-pyridine-3-carboxylic acid amide;
**199** 2-[[3-(3,4-difluoro-phenyl)-1-methyl-propyl]sulfanyl]-N-[(4-fluorophenyl)-methyl]-pyridine-3-carboxylic acid amide;
**200** 2-[[3-(3,4-difluoro-phenyl)-1-methyl-propyl]sulfanyl]-N-(3-methyl-butyl)-pyridine-3-carboxylic acid amide;
**201** N-[(4-fluorophenyl)-methyl]-2-[[3-(3-fluorophenyl)-1-methyl-propyl]sulfanyl]-pyridine-3-carboxylic acid amide;
**202** 2-[[3-(3-fluorophenyl)-1-methyl-propyl]sulfanyl]-N-(3-methyl-butyl)-pyridine-3-carboxylic acid amide;

**203** N-(3-methyl-butyl)-2-[[1-methyl-3-[3-(trifluoromethyl)phenyl]-propyl]sulfanyl]-pyridine-3-carboxylic acid amide;
**204** 2-[[3-(3,4-difluoro-phenyl)-3,3-difluoro-propyl]sulfanyl]-N-[(4-fluorophenyl)-methyl]-pyridine-3-carboxylic acid amide;
**205** N-(1-bicyclo[2.2.1]heptanyl-methyl)-2-[[3,3-difluoro-3-(4-fluorophenyl)-propyl]sulfanyl]- pyridine-3-carboxylic acid amide;
**206** N-(1-bicyclo[2.2.1]heptanyl-methyl)-2-[3-(4-fluorophenyl)-propylsulfanyl]-pyridine-3-carboxylic acid amide;
**207** N-[(4-fluorophenyl)-methyl]-2-[3-[3-(trifluoromethyl)phenyl]-propylsulfanyl]-pyridine-3-carboxylic acid amide;
**208** N-(3-methyl-butyl)-2-[3-[3-(trifluoromethyl)phenyl]-propylsulfanyl]-pyridine-3-carboxylic acid amide;
**209** 2-[[3-(3,4-difluoro-phenyl)-3,3-difluoro-propyl]sulfanyl]-N-(3-methyl-butyl)-pyridine-3-carboxylic acid amide;
**210** N-(7-bicyclo[2.2.1]heptanyl-methyl)-2-[[3-(3,4-difluoro-phenyl)-3,3-difluoro-propyl]sulfanyl]- pyridine-3-carboxylic acid amide;
**211** 2-[[3,3-difluoro-3-(4-fluorophenyl)-propyl]sulfanyl]-N-(2-hydroxy-ethyl)-pyridine-3-carboxylic acid amide;
**224** N-[(4-fluoro-2-methoxy-phenyl)methyl]-2-[3-(4-fluorophenyl)-propylsulfanyl]-pyridine-3-carboxylic acid amide;
or their physiologically compatible salts.

**12.** Medications containing at least one substituted nicotine amide according to one of the claims 1 to 11, each in the form of a single stereoisomer or its mixture, the free compounds and/or its physiologically compatible salts, as well as possible suitable additives and/or excipients and/or possible further active substances.

**13.** Use of at least one substituted nicotine amide according to one of the claims 1 to 11, each in the form of a single stereoisomer or its mixture, the free compound and/or its physiologically compatible salts, for producing a medication for treating pain, epilepsy, anxiety disorders, dependency, mania, bipolar disorders, migraine, cognitive diseases, dystonia associated dyskinesia and/or urine incontinence.

**14.** A substituted nicotine amide according to one of the claims 1 to 11, each in the form of a single stereoisomer or its mixture, the free compound and/or its physiologically compatible salts, for treating pain, epilepsy, anxiety disorders, dependency, mania, bipolar disorders, migraine, cognitive diseases, dystonia associated dyskinesia and/or urine incontinence.

## Revendications

**1.** Nicotinamides substitués de formule générale (1)

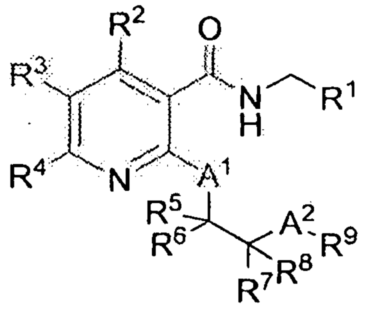

(1)

,

dans laquelle :

$A^1$ représente $CR^{10}R^{11}$ ou S ;
$A^2$ représente $CR^{12}R^{13}$, C(=O), O, S, S(=O) ou $S(=O)_2$ ;
$R^1$ représente un groupe alkyle en $C_1$ à $C_{10}$ ou hétéroalkyle en $C_2$ à $C_{10}$, respectivement saturé ou insaturé,

ramifié ou linéaire, non substitué ou mono- ou polysubstitué ; un groupe cycloalkyle en $C_3$ à $C_{10}$ ou hétérocyclyle, respectivement saturé ou insaturé, non substitué ou mono- ou polysubstitué ; un groupe aryle ou hétéroaryle, respectivement non substitué ou mono- ou polysubstitué ; un groupe cycloalkyle en $C_3$ à $C_{10}$ ou hétérocyclyle ponté par le biais d'un groupe alkyle en $C_1$ à $C_8$ ou d'un groupe hétéroalkyle en $C_2$ à $C_8$, respectivement saturé ou insaturé, non substitué ou mono- ou polysubstitué, la chaîne alkyle ou hétéroalkyle pouvant être ramifiée ou linéaire, saturée ou non saturée, non substituée, mono ou polysubstituée ; ou un groupe aryle ou hétéroaryle ponté par le biais d'un groupe alkyle en $C_1$ à $C_8$ ou hétéroalkyle en $C_2$ à $C_8$ respectivement non substitué ou mono ou polysubstitué, la chaîne alkyle ou hétéroalkyle pouvant être respectivement ramifiée ou linéaire, saturée ou insaturée, non substituée, mono- ou polysubstituée ;

$R^2$, $R^3$ et $R^4$ représentent, respectivement indépendamment les uns des autres, H ; F ; Cl ; Br ; I ; $NO_2$ ; $CF_3$ ; CN ; OH ; $OCF_3$ ; SH, $SCF_3$ ; un groupe méthyle ; $CH_2$-O-méthyle ; $CH_2$-OH ; alkyle en $C_2$ à $C_6$, O-alkyle en $C_1$ à $C_6$, S-alkyle en $C_1$ à $C_6$, respectivement saturé ou insaturé, ramifié ou linéaire, non substitué ou mono- ou polysubstitué ; un groupe cycloalkyle en $C_3$ à $C_7$, saturé ou insaturé, ramifié ou linéaire, non substitué ou mono- ou polysubstitué ; $NR^aR^b$, où $R^a$ et $R^b$ représentent indépendamment l'un de l'autre H ou un groupe alkyle en $C_1$ à $C_4$, saturé ou insaturé, ramifié ou linéaire, non substitué ou mono- ou polysubstitué ou $R^a$ et $R^b$ forment conjointement, avec l'atome d'azote les reliant, un groupe hétérocyclyle, saturé ou insaturé, ramifié ou linéaire, non substitué ou mono- ou polysubstitué ;

$R^5$, $R^6$, $R^7$, $R^8$, $R^{10}$, $R^{11}$, $R^{12}$ et $R^{13}$ représentent indépendamment les uns des autres H ; F ; Cl ; Br ; I ; $NO_2$ ; $CF_3$ ; CN ; OH ; $OCF_3$ ; SH ; $SCF_3$ ; un groupe alkyle en $C_1$ à $C_{10}$, hétéroalkyle en $C_2$ à $C_{10}$, O-alkyle en $C_1$ à $C_{10}$ ou S-alkyle en $C_1$ à $C_{10}$, respectivement saturé ou insaturé, ramifié ou linéaire, non substitué ou mono- ou polysubstitué ; un groupe cycloalkyle en $C_3$ à $C_{10}$, saturé ou insaturé, ramifié ou linéaire, non substitué ou mono- ou polysubstitué ;

à la condition que, si $R^5$, $R^6$, $R^7$ et $R^8$ représentent respectivement H et $A^1$ représente S, $A^2$ ne peut représenter S, S(=O) ou $S(=O)_2$ ;

ou $R^5$ et $R^6$ ou $R^7$ et $R^8$ ou $R^{10}$ et $R^{11}$ ou $R^{12}$ et $R^{13}$ ou $R^5$ et $R^{11}$ ou $R^5$ et $R^{13}$ ou $R^7$ et $R^{13}$ ou $R^7$ et $R^{11}$ ou $R^{11}$ et $R^{13}$ forment conjointement avec le ou les atomes de carbone les reliant un groupe cycloalkyle en $C_3$ à $C_8$ ou hétérocyclyle tri- à octogonal, respectivement saturé ou insaturé, ramifié ou linéaire, non substitué ou mono- ou polysubstitué ; les substituants restants respectifs $R^5$, $R^6$, $R^7$, $R^8$, $R^{10}$, $R^{11}$, $R^{12}$ et $R^{13}$ ayant la signification indiquée précédemment ;

ou $R^5$ et $R^7$ forment conjointement avec le ou les atomes de carbone les reliant un groupe cycloalkyle en $C_3$ à $C_8$, saturé ou insaturé, non substitué ou mono- ou polysubstitué ; ou un groupe hétérocyclyle tri- à octogonal, insaturé, non substitué ou mono- ou polysubstitué ; les substituants restants respectifs $R^6$, $R^8$, $R^{10}$, $R^{11}$, $R^{12}$ et $R^{13}$ ayant la signification indiquée précédemment ;

$R^9$ représente un groupe cycloalkyle en $C_3$ à $C_{10}$ ou hétérocyclyle, respectivement saturé ou insaturé, non substitué ou mono- ou polysubstitué ; un groupe aryle ou hétéroaryle, respectivement non substitué ou mono- ou polysubstitué ; ou $CR^cR^d$, où $R^c$ et $R^d$ représentent respectivement indépendamment l'un de l'autre un groupe alkyle en $C_1$ à $C_4$, saturé ou insaturé, ramifié ou linéaire, non substitué ou mono- ou polysubstitué ;

à la condition que si $A^2$ représente 0 ou S et $R^9$ représente un groupe hétérocyclyle saturé ou insaturé, non substitué ou mono- ou polysubstitué ; ou un groupe hétéroaryle, non substitué ou mono- ou polysubstitué ; la liaison du groupe hétéroaryle ou hétérocyclyle s'effectue par le biais d'un atome de carbone du groupe hétéroaryle ou hétérocyclyle ;

dans lesquels les expressions « alkyle substitué », « hétéroalkyle substitué », « hétérocyclyle substitué » et « cycloalkyle substitué » représentent la substitution d'un ou de plusieurs atomes d'hydrogène, respectivement indépendamment les uns des autres, par F ; Cl ; Br ; $NO_2$ ; $CF_3$ ; CN ; =O ; un groupe alkyle en $C_1$ à $C_8$ ; hétéroalkyle en $C_2$ à $C_8$ ; aryle ; hétéroaryle ; cycloalkyle en $C_3$ à $C_{10}$ ; hétérocyclyle ; aryle, hétéroaryle, cycloalkyle en $C_3$ à $C_{10}$ ou hétérocyclyle pontés par le biais d'un groupe alkyle en $C_1$ à $C_8$ ou hétéroalkyle en $C_2$ à $C_8$ ; CHO ; C(=O) alkyle en $C_1$ à $C_8$ ; C(=O)aryle ; C(=O)hétéroaryle ; $CO_2H$ ; C(=O)O-alkyle en $C_1$ à $C_8$ ; C(=O)O-aryle ; C(=O)O-hétéroaryle ; $CONH_2$ ; C(=O)NH-alkyle en $C_1$ à $C_8$ ; C(=O)N(alkyle en $C_1$ à $C_8)_2$ ; C(=O)NH-aryle ; C(=O)N-$(aryle)_2$ ; C(=O)NH-hétéroaryle ; C (=O) N $(hétéroaryle)_2$ ; C(=O)N (alkyle en $C_1$ à $C_8$)(aryle) ; C(=O)N(alkyle en $C_1$ à $C_8$)(hétéroaryle) ; C(=O)N(hétéroaryle)-(aryle) ; OH ; O-alkyle en $C_1$ à $C_8$ ; $OCF_3$ ; O-(alkyle en $C_1$ à $C_8$)-OH ; O-(alkyl en $C_1$ à $C_8$)-O-alkyle en $C_1$ à $C_8$ ; O-benzyle ; O-aryle ; O-hétéroaryle ; O-C(=O)alkyle en $C_1$ à $C_8$ ; O-C(=O)aryle ; O-C(=O)hétéroaryle ; $NH_2$ ; NH-alkyle en $C_1$ à $C_8$ ; N(alkyle en $C_1$ à $C_8)_2$ ; NH-C(=O)alkyle en $C_1$ à $C_8$ ; N(alkyle en $C_1$ à $C_8$)-C(=O)alkyle en $C_1$ à $C_8$ ; N(C(=O)alkyle en $C_1$ à $C_8)_2$ ; NH-C(=O)aryle ; NH-C(=O)-hétéroaryle ; SH ; S-alkyle en $C_1$ à $C_8$ ; $SCF_3$ ; S-benzyle ; S-aryle ; S-hétéroaryle ; $S(=O)_2$alkyle en $C_1$ à $C_8$ ; $S(=O)_2$aryle ; $S(=O)_2$hétéroaryle ; $S(=O)_2OH$ ; $S=)_2O$-alkyle en $C_1$ à $C_8$ ; $S(=O)_2O$-aryle ; $S(=O)_2O$-hétéroaryle ; $S(=O)_2$-NH-alkyle en $C_1$ à $C_8$ ; $S(=O)_2$-NH-aryle ; et $S(=O)_2$-NH-hétéroaryle en $C_1$ à $C_8$ ;

dans lesquels les expressions « aryle substitué » et « hétéroaryle substitué » représentent la substitution d'un

ou de plusieurs atomes d'hydrogène, respectivement indépendamment les uns des autres, par F ; Cl ; Br ; I ; $NO_2$ ; $CF_3$ ; CN ; un groupe alkyle en $C_1$ à $C_8$ ; hétéroalkyle en $C_2$ à $C_8$ ; aryle ; hétéroaryle ; cycloalkyle en $C_3$ à $C_{10}$ ; hétérocyclyle ; aryle, hétéroaryle, cycloalkyle en $C_3$ à $C_{10}$ ou hétérocyclyle pontés par le biais d'un groupe alkyle en $C_1$ à $C_8$ ou hétéroalkyle en $C_2$ à $C_8$ ; CHO ; C(=O) alkyle en $C_1$ à $C_8$ ; C(=O)aryle ; C(=O)hétéroaryle ; C(=O)O-alkyle en $C_1$ à $C_8$ ; C(=O)O-aryle ; C(=O)O-hétéroaryle ; $CONH_2$ ; C(=O)NH-alkyle en $C_1$ à $C_8$ ; C(=O)N(alkyle en $C_1$ à $C_8)_2$ ; C(=O)NH-aryle ; C(=O)N(aryle)$_2$ ; C(=O)NH-hétéroaryle ; C(=O)N-(hétéroaryle)$_2$ ; C(=O)N(alkyle en $C_1$ à $C_8$)(aryle) ; C(=O)N-(alkyle en $C_1$ à $C_8$)(hétéroaryle) ; C(=O)N(hétéroaryle)-(aryle) ; OH ; O-alkyle en $C_1$ à $C_8$ ; $OCF_3$ ; O-(alkyle en $C_1$ à $C_8$)-OH ; O-(alkyl en $C_1$ à $C_8$)-O-alkyle en $C_1$ à $C_8$ ; O-benzyle ; O-aryle ; O-hétéroaryle ; O-C(=O)alkyle en $C_1$ à $C_8$ ; O-C(=O)aryle ; O-C(=O)hétéroaryle ; $NH_2$ ; NH-alkyle en $C_1$ à $C_8$ ; N (alkyle en $C_1$ à $C_8)_2$ ; NH-C(=O)alkyle en $C_1$ à $C_8$ ; N(alkyle en $C_1$ à $C_8$)-C(=O) alkyle en $C_1$ à $C_8$ ; N(C(=O) alkyle en $C_1$ à $C_8)_2$ ; NH-C(=O)aryle ; NH-C(=O)-hétéroaryle ; SH ; S-alkyle en $C_1$ à $C_8$ ; $SCF_3$ ; S-benzyle ; S-aryle ; S-hétéroaryle ; $S(=O)_2$alkyle en $C_1$ à $C_8$ ; $S(=O)_2$aryle ; $S(=O)_2$hétéroaryle ; $S(=O)_2OH$ ; $S(=O)_2$O-alkyle en $C_1$ à $C_8$ ; $S(=O)_2$O-aryle ; $S(=O)_2$O-hétéroaryle ; $S(=O)_2$-NH-alkyle en $C_1$ à $C_8$ ; $S(=O)_2$-NH-aryle ; $S(=O)_2$-NH-hétéroaryle en $C_1$ à $C_8$ ;
sous la forme de composés libres ou de sels d'acides ou de bases physiologiquement acceptables.

**2.** Nicotinamides substitués selon la revendication 1, **caractérisés en ce que** :

$A^1$ représente S ; et
$A^2$ représente $CR^{12}R^{13}$, O, S ou $S(=O)_2$.

**3.** Nicotinamides substitués selon la revendication 1 ou 2, **caractérisés en ce que** :

$R^1$ représente la structure partielle (T1)

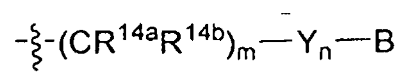

(T1)

,

dans laquelle :

$R^{14a}$ et $R^{14b}$ représentent indépendamment l'un de l'autre H ; F ; Cl ; Br ; I ; $NO_2$ ; $CF_3$ ; CN ; OH ; $OCF_3$ ; $NH_2$ ; un groupe alkyle en $C_1$ à $C_4$, 0-alkyle en $C_1$ à $C_4$, NH-alkyle en $C_1$ à $C_4$, N (alkyle en $C_1$ à $C_4)_2$, respectivement saturé ou insaturé, ramifié ou linéaire, non substitué ou mono- ou polysubstitué par un ou plusieurs substituants choisis, respectivement indépendamment les uns des autres, dans le groupe constitué par F, Cl, Br, I, O-alkyle en $C_1$ à $C_4$, OH et $OCF_3$ ; un groupe cycloalkyle en $C_3$ à $C_{10}$ ou hétérocyclyle, respectivement saturé ou insaturé, non substitué ou mono- ou polysubstitué par un ou plusieurs substituants choisis, respectivement indépendamment les uns des autres, dans le groupe constitué par F, Cl, Br, I, alkyle en $C_1$ à $C_4$, OH, =O, O-alkyle en $C_1$ à $C_4$, $OCF_3$, $NH_2$, NH-alkyle en $C_1$ à $C_4$ et N (alkyle en $C_1$ à $C_4)_2$ ;
m représente 0, 1, 2 ou 3,
Y représente O ou $NR^{15}$,
où $R^{15}$ représente H ; un groupe alkyle en $C_1$ à $C_4$, saturé ou insaturé, ramifié ou linéaire, non substitué ou mono- ou polysubstitué par un ou plusieurs substituants choisis, respectivement indépendamment les uns des autres, dans le groupe constitué par F, Cl, Br, I, alkyle en $C_1$ à $C_4$, OH, O-alkyle en $C_1$ à $C_4$, $OCF_3$, $NH_2$, NH-alkyle en $C_1$ à $C_4$ et N(alkyle en $C_1$ à $C_4)_2$ ; ou un groupe cycloalkyle en $C_3$ à $C_{10}$, respectivement saturé ou insaturé, non substitué ou mono- ou polysubstitué par un ou plusieurs substituants choisis, respectivement indépendamment les uns des autres, dans le groupe constitué par F, Cl, Br, I, alkyle en $C_1$ à $C_4$, OH, O-alkyle en $C_1$ à $C_4$, $OCF_3$, $NH_2$, NH-alkyle en $C_1$ à $C_4$ et N(alkyle en $C_1$ à $C_4)_2$ ;
n représente 0 ou 1,
B représente un groupe alkyle, saturé ou insaturé, ramifié ou linéaire, non substitué ou mono- ou polysubstitué par un ou plusieurs substituants choisis, respectivement indépendamment les uns des autres, dans le groupe constitué par F, Cl, Br, I, $NO_2$, CN, OH, =O, O-alkyle en $C_1$ à $C_4$, $OCF_3$, C(=O)-OH, $CF_3$, $NH_2$, NH (alkyle en $C_1$ à $C_4$), N (alkyle en $C_1$ à $C_4)_2$, SH, S-alkyle en $C_1$ à $C_4$, $SCF_3$ et $S(=O)_2OH$ ; un groupe cycloalkyle en $C_3$ à $C_{10}$ ou hétérocyclyle, respectivement saturé ou insaturé, non substitué ou mono- ou

polysubstitué par un ou plusieurs substituants choisis, respectivement indépendamment les uns des autres, dans le groupe constitué par F, Cl, Br, I, $NO_2$, CN, OH, O-alkyle en $C_1$ à $C_8$, $OCF_3$, alkyle en $C_1$ à $C_8$, C(=O)-OH, $CF_3$, $NH_2$, NH(alkyle en $C_1$ à $C_8$), N(alkyle en C1 à $C_8$)$_2$, SH, S-alkyle en $C_1$ à $C_8$, $SCF_3$, S(=O)$_2$OH, benzyle, phényle, pyridyle et thiényle, où les groupes benzyle, phényle, pyridyle, thiényle peuvent être respectivement non substitués ou mono- ou polysubstitués par un ou plusieurs substituants choisis, respectivement indépendamment les uns des autres, dans le groupe constitué par F, Cl, Br, I, $NO_2$, CN, OH, O-alkyle en $C_1$ à $C_8$, $OCF_3$, alkyle en $C_1$ à $C_8$, C(=O)-OH, $CF_3$, $NH_2$, NH(alkyle en $C_1$ à $C_8$), N (alkyle en $C_1$ à $C_8$)$_2$, SH, S-alkyle en $C_1$ à $C_8$, $SCF_3$ et S(=O)$_2$OH ; un groupe aryle ou hétéroaryle, respectivement non substitué ou mono- ou polysubstitué par un ou plusieurs substituants choisis, respectivement indépendamment les uns des autres, dans le groupe constitué par F, Cl, Br, I, $NO_2$, CN, OH, O-alkyle en $C_1$ à $C_8$, $OCF_3$, alkyle en $C_1$ à $C_8$, C(=O)-OH, $CF_3$, $NH_2$, NH (alkyle en $C_1$ à $C_8$), N (alkyle en $C_1$ à $C_8$)$_2$, SH, S-alkyle en $C_1$ à $C_8$, $SCF_3$, S(=O)$_2$OH, benzyle, phényle, pyridyle et thiényle, où les groupes benzyle, phényle, pyridyle, thiényle peuvent être respectivement non substitués ou mono- ou polysubstitués par un ou plusieurs substituants choisis, respectivement indépendamment les uns des autres, dans le groupe constitué par F, Cl, Br, I, $NO_2$, CN, OH, 0-alkyle en $C_1$ à $C_8$, $OCF_3$, alkyle en $C_1$ à $C_8$, C(=O)-OH, $CF_3$, $NH_2$, NH (alkyle en $C_1$ à $C_8$), N (alkyle en $C_1$ à $C_8$)$_2$, SH, S-alkyle en $C_1$ à $C_8$, $SCF_3$ et S(=O)$_2$OH.

**4.** Nicotinamides substitués selon la revendication 3, **caractérisés en ce que** :

$R^{14a}$ et $R^{14b}$ représentent indépendamment l'un de l'autre H ; F ; Cl ; Br ; I ; $NO_2$ ; $CF_3$ ; CN ; un groupe méthyle ; éthyle ; n-propyle ; isopropyle ; cyclopropyle ; n-butyle ; sec.-butyle ; tertiobutyle ; $CH_2CF_3$ ; OH ; O-méthyle ; O-éthyle ; O-($CH_2$)$_2$-O-$CH_3$ ; O-($CH_2$)$_2$-OH ; $OCF_3$ ; $NH_2$ ; NH-méthyle ; N(méthyle)$_2$ ; NH-éthyle ; N(éthyle)$_2$ ; ou N(méthyle)(éthyle) ;
m représente 0, 1 ou 2 ;
n représente 0 ; et
B représente un groupe alkyle en $C_1$ à $C_4$, saturé, ramifié ou linéaire, non substitué ou mono- ou polysubstitué par un ou plusieurs substituants choisis, respectivement indépendamment les uns des autres, dans le groupe constitué par F, Cl, Br, I, OH, O-alkyle en $C_1$ à $C_4$, $OCF_3$ et $CF_3$ ; un groupe cycloalkyle en $C_3$ à $C_{10}$, saturé, non substitué ; un groupe phényle, naphtyle, pyridyle, thiényle, respectivement non substitués ou mono- ou di- ou trisubstitués par un, deux ou trois substituants choisis, respectivement indépendamment les uns des autres, dans le groupe constitué par F, Cl, Br, I, $NO_2$, CN, OH, O-alkyle en $C_1$ à $C_4$, $OCF_3$, alkyle en $C_1$ à $C_4$, C(=O)-OH , $CF_3$, $NH_2$, NH (alkyle en $C_1$ à $C_4$), N (alkyle en $C_1$ à $C_4$)$_2$, SH, S-alkyle en $C_1$ à $C_4$, $SCF_3$ et S(=O)$_2$OH.

**5.** Nicotinamides substitués selon l'une des revendications 1 à 4, **caractérisés en ce que** :

$R^2$, $R^3$ et $R^4$ représentent, respectivement indépendamment les uns des autres, H ; F ; Cl ; Br ; I ; $NO_2$ ; $CF_3$ ; CN ; OH ; $OCF_3$ ; SH ; $SCF_3$ ; un groupe méthyle ; $CH_2$-O-méthyle ; $CH_2$-OH ; alkyle en Cl à $C_6$, O-alkyle en $C_1$ à $C_6$, S-alkyle en $C_1$ à $C_6$, respectivement saturé ou insaturé, ramifié ou linéaire, non substitué ou mono- ou polysubstitué par un ou plusieurs substituants choisis, respectivement indépendamment les uns des autres, dans le groupe constitué par F, Cl, Br, I, OH, =O et O-alkyle en $C_1$ à $C_4$ ; un groupe cycloalkyle en $C_3$ à $C_7$, saturé ou insaturé, ramifié ou linéaire, non substitué ; $NR^aR^b$, où $R^a$ et $R^b$ représentent indépendamment l'un de l'autre H ou un groupe alkyle en $C_1$ à $C_4$, saturé ou insaturé, ramifié ou linéaire, non substitué ou mono- ou polysubstitué par un ou plusieurs substituants choisis, respectivement indépendamment les uns des autres, dans le groupe constitué par OH, =O et O-alkyle en $C_1$ à $C_4$ ou $R^a$ et $R^b$ forment conjointement, avec l'atome d'azote les reliant, un groupe hétérocyclyle, saturé ou insaturé, non substitué ou mono- ou polysubstitué par un groupe alkyle en $C_1$ à $C_4$.

**6.** Nicotinamides substitués selon la revendication 5, **caractérisés en ce que** :

$R^2$, $R^3$ et $R^4$ représentent, respectivement indépendamment les uns des autres, H ; F ; Cl ; Br ; I ; un groupe méthyle ; éthyle ; n-propyle ; isopropyle ; cyclopropyle ; CN ; $CF_3$ ; O-méthyle ; $OCF_3$ ; S-méthyle ; $SCF_3$.

**7.** Nicotinamides substitués selon l'une des revendications 1 à 6, **caractérisés en ce que** :

$R^5$, $R^6$, $R^7$, $R^8$, $R^{10}$, $R^{11}$, $R^{12}$ et $R^{13}$ représentent indépendamment les uns des autres H ; F ; Cl ; Br ; I ; $NO_2$ ; $CF_3$ ; CN ; OH ; $OCF_3$ ; SH ; $SCF_3$ ; un groupe méthyle ; éthyle ; n-propyle ; isopropyle ; n-butyle ; sec.-butyle ; tertiobutyle ; O-méthyle ; O-éthyle ; O-($CH_2$)$_2$-O-$CH_3$ ; O-($CH_2$)$_2$-OH ; S-méthyle ; S-éthyle ; cyclopropyle ; cyclobutyle ; cyclopentyle ; cyclohexyle ;

ou $R^5$ et $R^6$ ou $R^7$ et $R^8$ ou $R^{10}$ et $R^{11}$ ou $R^{12}$ et $R^{13}$ ou $R^5$ et $R^{11}$ ou $R^5$ et $R^7$ ou $R^5$ et $R^{13}$ ou $R^7$ et $R^{13}$ ou $R^7$ et $R^{11}$ ou $R^{11}$ et $R^{13}$ forment conjointement avec le ou les atomes de carbone les reliant un groupe cyclopropyle, cyclobutyle, cyclopentyle ou cyclohexyle, respectivement non substitué ; les substituants restants respectifs $R^5$, $R^6$, $R^7$, $R^8$, $R^{10}$, $R^{11}$, $R^{12}$ et $R^{13}$ ayant la signification indiquée précédemment.

**8.** Nicotinamides substitués selon l'une des revendications 1 à 7, **caractérisés en ce que** :

$R^9$ représente un groupe cycloalkyle en $C_3$ à $C_{10}$ ou hétérocyclyle, respectivement saturé ou insaturé, non substitué ou mono- ou polysubstitué par un ou plusieurs substituants choisis, respectivement indépendamment les uns des autres, dans le groupe constitué par F, Cl, Br, I, $NO_2$, CN, OH, =O, O-alkyle en $C_1$ à $C_4$ , $OCF_3$ , alkyle en $C_1$ à $C_4$ , $CF_3$, SH, S-alkyle en $C_1$ à $C_4$ et $SCF_3$ ; un groupe aryle ou hétéroaryle, respectivement non substitué ou mono- ou polysubstitué par un ou plusieurs substituants choisis, respectivement indépendamment les uns des autres, dans le groupe constitué par F, Cl, Br, I, $NO_2$, CN, OH, O-alkyle en $C_1$ à $C_4$ , $OCF_3$ , alkyle en $C_1$ à $C_4$ , $CF_3$ , $NH_2$ , NH (alkyle en $C_1$ à $C_4$), N (alkyle en $C_1$ à $C_4)_2$, SH, S-alkyle en $C_1$ à $C_4$ et $SCF_3$ ; ou $CR^cR^d$, où $R^c$ et $R^d$ représentent respectivement indépendamment l'un de l'autre un groupe alkyle en $C_1$ à $C_4$, saturé ou insaturé, ramifié ou linéaire, non substitué ou mono- ou polysubstitué par un ou plusieurs substituants choisis, respectivement indépendamment les uns des autres, dans le groupe constitué par F, Cl, Br, I, OH, O-alkyle en $C_1$ à $C_4$, $CF_3$, $OCF_3$ et $SCF_3$.

**9.** Nicotinamides substitués selon l'une des revendications 1 à 8, **caractérisés en ce que** :

$R^9$ représente un groupe phényle, pyridyle ou thiényle, respectivement non substitué ou mono- ou polysubstitué par un ou plusieurs substituants choisis, respectivement indépendamment les uns des autres, dans le groupe constitué par F, Cl, Br, I, CN, OH, O-alkyle en $C_1$ à $C_4$, $OCF_3$, alkyle en $C_1$ à $C_4$, $CF_3$, SH, S-alkyle en $C_1$ à $C_4$ et $SCF_3$.

**10.** Nicotinamides substitués selon l'une des revendications 1 à 9, **caractérisés en ce que** :

$A^1$ représente S et
$A^2$ représente $CR^{12}R^{13}$ ;
$R^1$ représente la structure partielle (T1-1)

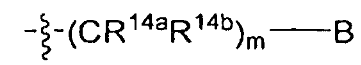

(T1-1)

dans laquelle :

$R^{14a}$ et $R^{14b}$ représentent indépendamment l' un de l'autre H ; F ; Cl ; Br ; I ; un groupe méthyle ; éthyle ; n-propyle ; isopropyle ; n-butyle ; sec.-butyle ; tertio-butyle ; OH ; O-méthyle ; O-éthyle ; O-$(CH_2)_2$-O-$CH_3$ ou O-$(CH_2)_2$-OH ;
m représente 0, 1 ou 2 ;
B représente un groupe méthyle ; éthyle ; n-propyle ; isopropyle ; n-butyle ; sec.-butyle ; tertiobutyle ; cyclopropyle ; cyclobutyle ; cyclopentyle ; cyclohexyle ; cycloheptyle ; adamantyle ; bicyclo[2.2.1]heptyle ; bicyclo-[2.2.2]octyle ; phényle, pyridyle, thiényle, respectivement non substitués ou mono-, di- ou trisubstitués par un, deux ou trois substituants choisis, respectivement indépendamment les uns des autres, dans le groupe constitué par F, Cl, Br, I, $NO_2$, CN, OH, O-alkyle en $C_1$ à $C_4$, $OCF_3$, alkyle en $C_1$ à $C_4$, C(=O)-OH, $CF_3$, $NH_2$, NH(alkyle en $C_1$ à $C_4$), N (alkyle en $C_1$ à $C_4)_2$, SH, S-alkyle en $C_1$ à $C_4$, $SCF_3$ et $S(=O)_2OH$ ;
R2, R3 et R4 représentent, respectivement indépendamment les uns des autres, H ; F ; Cl ; un groupe méthyle ; éthyle ; $CF_3$ ou O-méthyle ;
$R^5$, $R^6$, $R^7$, $R^8$, $R^{10}$, $R^{11}$, $R^{12}$ et $R^{13}$ représentent indépendamment les uns des autres H ; F ; Cl ; un groupe méthyle ; éthyle ; n-propyle ; isopropyle ; cyclopropyle ;
$R^9$ représente un groupe phényle, pyridyle ou thiényle, non substitué ou mono- ou polysubstitué par un ou plusieurs substituants choisis, respectivement indépendamment les uns des autres, dans le groupe constitué par F, Cl, Br, I, CN, un groupe méthyle, éthyle, n-propyle, isopropyle, n-butyle, sec.-butyle, tertiobutyle, OH, O-méthyle, O-éthyle, O-n-propyle, O-isopropyle, O-butyle, O-sec.-butyle, O-tertio-butyle, OH, $OCF_3$, $CF_3$, SH, S-alkyle en $C_1$ à $C_4$ et $SCF_3$.

**11.** Nicotinamides substitués selon l'une des revendications 1 à 10, choisis dans le groupe constitué par :

1 2-(3-phényl-propylsulfanyl)-N-(thiophène-2-yl-méthyl)-pyridine-3-carboxamide ;
2 2-(3-cyclohexyl-propylsulfanyl)-N-(thiophène-2-yl-méthyl)-pyridine-3-carboxamide ;
3 2-[(3-oxo-3-phényl-propyl)sulfanyl]-N-(thiophène-2-yl-méthyl)-pyridine-3-carboxamide ;
4 N-(thiophène-2-yl-méthyl)-2-[2-[3-(trifluorométhyl)-phénoxy]-éthylsulfanyl]-pyridine-3-carboxamide ;
5 2-(4-méthyl-pentylsulfanyl)-N-(thiophène-2-yl-méthyl)-pyridine-3-carboxamide ;
7 2-(4-phényl-butyl)-N-(thiophène-2-yl-méthyl)-pyridine-3-carboxamide ;
8 2-[3-(benzènesulfonyl)-propyl]-N-(cyclohexyl-méthyl)-pyridine-3-carboxamide ;
9 N-(cyclohexyl-méthyl)-2-(4-phényl-butyl)-pyridine-3-carboxamide ;
10 2-[3-(benzènesulfonyl)-propyl]-N-(thiophène-2-yl-méthyl)-pyridine-3-carboxamide ;
12 N-(thiophène-2-yl-méthyl)-2-[3-[[3-(trifluoro-méthyl)-phényl]sulfonyl]-propyl]-pyridine-3-carboxamide ;
13 N-(thiophène-2-yl-méthyl)-2-[3-[[3-(trifluoro-méthyl)-phényl]sulfanyl]-propyl]-pyridine-3-carboxamide ;
16 2-(2-phénylsulfanyl-propylsulfanyl)-N-(thiophène-2-yl-méthyl)-pyridine-3-carboxamide ;
17 2-[3-(4-fluorophényl)-propylsulfanyl]-N-(thiophène-2-yl-méthyl)-pyridine-3-carboxamide ;
18 N-(thiophène-2-yl-méthyl)-2-[3-[3-(trifluorométhyl)-phényl]-propylsulfanyl]-pyridine-3-carboxamide ;
19 2- [2-[(4-fluorophényl)sulfanyl]-propylsulfanyl]-N-(thiophène-2-yl-méthyl)-pyridine-3-carboxamide ;
20 N-(thiophène-2-yl-méthyl)-2-[2-[[3-(trifluoro-méthyl)-phényl]sulfanyl]-propylsulfanyl]-pyridine-3-carboxamide ;
21 N-(thiophène-2-yl-méthyl)-2-[4-[3-(trifluorométhyl)-phényl]-butyl]-pyridine-3-carboxamide ;
22 2-[4-(4-fluorophényl)-butyl]-N-(thiophène-2-yl-méthyl)-pyridine-3-carboxamide ;
23 2-(3-phénylsulfanyl-propyl)-N-(thiophène-2-yl-méthyl)-pyridine-3-carboxamide ;
24 2-[(1-méthyl-2-phénylsulfanyl-éthyl)sulfanyl]-N-(thiophène-2-yl-méthyl)-pyridine-3-carboxamide ;
25 N-(cycloheptyl-méthyl)-2-[4-[3-(trifluorométhyl)-phényl]-butyl]-pyridine-3-carboxamide ;
26 N-(5-bicyclo[2.2.1]heptanyl-méthyl)-2-[4-[3-(trifluoro-méthyl)phényl]-butyl]-pyridine-3-carboxamide ;
27 N-(5-bicyclo[2.2.1]heptanyl-méthyl)-2-[3-[3-(tri-fluorométhyl)phényl]-propylsulfanyl]-pyridine-3-carboxamide ;
28 N-(5-bicyclo[2.2.1]heptanyl-méthyl)-2-(4-méthyl-pentylsulfanyl)-pyridine-3-carboxamide ;
29 N-(cycloheptyl-méthyl)-2-[3-[(4-fluorophényl)-sulfanyl]-propyl]-pyridine-3-carboxamide ;
30 N-(5-bicyclo[2.2.1]heptanyl-méthyl)-2-[3-[(4-fluorophényl)-sulfanyl]-propyl]-pyridine-3-carboxamide ;
31 N-(cycloheptyl-méthyl)-2-[3-[[3-(trifluorométhyl)-phényl]-sulfonyl]-propyl]-pyridine-3-carboxamide ;
32 N-(5-bicyclo[2.2.1]heptanyl-méthyl)-2-[3-[[3-(trifluoro-méthyl)-phényl]-sulfonyl]-propyl]-pyridine-3-carboxamide ;
33 N-(5-bicyclo[2.2.1]heptanyl-méthyl)-2-(3-phényl-propyl-sulfanyl)-pyridine-3-carboxamide ;
34 N-(5-bicyclo[2.2.1]heptanyl-méthyl)-2-[3-[4-(trifluorométhyl)-phényl]-propylsulfanyl]-pyridine-3-carboxamide ;
35 N-(5-bicyclo[2.2.1]heptanyl-méthyl)-2-[3-(4-chlorophényl)-propylsulfanyl]-pyridine-3-carboxamide ;
36 N-[(3,5-difluoro-phényl)-méthyl]-2-[3-(4-fluorophényl)-propylsulfanyl]-pyridine-3-carboxamide ;
37 N-[(5-chloro-thiophène-2-yl)-méthyl]-2-[3-(4-fluorophényl)-propylsulfanyl]-pyridine-3-carboxamide ;
38 N-[(2,2-diméthyl-cyclopropyl)-méthyl]-2-[3-(4-fluorophényl)-propylsulfanyl]-pyridine-3-carboxamide ;
39 N-(5-bicyclo[2.2.1]heptanyl-méthyl)-2-[3-(3-fluorophényl)-propylsulfanyl]-pyridine-3-carboxamide ;
40 N-(cyclohexyl-méthyl)-2-[3-(4-fluorophényl)-propyl-sulfanyl]-pyridine-3-carboxamide ;
41 N-(cycloheptyl-méthyl)-2-[3-(4-fluorophényl)-propyl-sulfanyl]-pyridine-3-carboxamide ;
43 N-(5-bicyclo[2.2.1]heptanyl-méthyl)-2-[[3-(4-fluorophényl)-3-oxopropyl]sulfanyl]-pyridine-3-carboxamide ;
44 N-(5-bicyclo[2.2.1]heptanyl-méthyl)-2-[(3-oxo-3-phényl-propyl)sulfanyl]-pyridine-3-carboxamide ;
45 N-(5-bicyclo[2.2.1]heptanyl-méthyl)-2-(hexylsulfanyl)-pyridine-3-carboxamide ;
46 N-(5-bicyclo[2.2.1]heptanyl-méthyl)-2-(2-phénoxy-éthylsulfanyl)-pyridine-3-carboxamide ;
47 N-(5-bicyclo[2.2.1]heptanyl-méthyl)-2-[2-[3-(trifluoro-méthyl)-phénoxy]-éthylsulfanyl]-pyridine-3-carboxamide ;
48 2-[3-(4-fluorophényl)-propylsulfanyl]-N-(3,3,3-trifluoro-propyl)-pyridine-3-carboxamide ;
49 N-(5-bicyclo[2.2.1]heptanyl-méthyl)-2-[2-(4-fluorophénoxy)-éthylsulfanyl]-pyridine-3-carboxamide ;
52 N-(5-bicyclo[2.2.1]heptanyl-méthyl)-2-(3-naphtalène-1-yl-propylsulfanyl)-pyridine-3-carboxamide ;
53 N-(5-bicyclo[2.2.1]heptanyl-méthyl)-2-[3-[3-fluoro-4-(trifluorométhyl)-phényl]-propylsulfanyl]-pyridine-3-carboxamide ;

54 N-(5-bicyclo[2.2.1]heptanyl-méthyl)-2-[3-[4-fluoro-3-(trifluorométhyl)-phényl]-propylsulfanyl]-pyridine-3-carboxamide ;
55 N-(cyclo-octyl-méthyl)-2-[3-(4-fluorophényl)-propyl-sulfanyl]-pyridine-3-carboxamide ;
56 N-(5-bicyclo[2.2.1]heptanyl-méthyl)-2-[3-(4-méthoxy-phényl)-propylsulfanyl]-pyridine-3-carboxamide ;
57 2-[3-(4-fluorophényl)-propylsulfanyl]-N-[(4-méthoxy-phényl)-méthyl]-pyridine-3-carboxamide ;
59 2-[3-(4-fluorophényl)-propylsulfanyl]-N-[[3-(trifluoro-méthyl)phényl]-méthyl]-pyridine-3-carboxamide ;
60 N-(5-bicyclo[2.2.1]heptanyl-méthyl)-2-[3-(3,4-difluoro-phényl)-propylsulfanyl]-pyridine-3-carboxamide ;
61 N-(5-bicyclo[2.2.1]heptanyl-méthyl)-2-[3-(3,5-difluoro-phényl)-propylsulfanyl]-pyridine-3-carboxamide ;
62 N-(5-bicyclo[2.2.1]heptanyl-méthyl)-2-[3-(2,4-difluoro-phényl)-propylsulfanyl]-pyridine-3-carboxamide ;
63 N-[(2-fluorophényl)-méthyl]-2-[3-(4-fluorophényl)-propylsulfanyl]-pyridine-3-carboxamide ;
64 N-[(3-fluorophényl)-méthyl]-2-[3-(4-fluorophényl)-propylsulfanyl]-pyridine-3-carboxamide ;
65 N-[(4-fluorophényl)-méthyl]-2-[3-(4-fluorophényl)-propylsulfanyl]-pyridine-3-carboxamide ;
66 N-benzyl-2-[3-(4-fluorophényl)-propylsulfanyl]-pyridine-3-carboxamide ;
67 N-(5-bicyclo[2.2.1]heptanyl-méthyl)-2-[(1-méthyl-3-phényl-propyl)sulfanyl]-pyridine-3-carboxamide ;
68 N-(5-bicyclo[2.2.1]heptanyl-méthyl)-2-[3-(3,4,5-tri-fluorophényl)-propylsulfanyl]-pyridine-3-carboxamide ;
69 N-(5-bicyclo[2.2.1]heptanyl-méthyl)-2-[3-[3-fluoro-5-(trifluorométhyl)-phényl]-propylsulfanyl]-pyridine-3-carboxamide ;
70 N-(5-bicyclo[2.2.1]heptanyl-méthyl)-2-[3-(3-méthoxy-phényl)-propylsulfanyl]-pyridine-3-carboxamide ;
71 N-[(3,4-difluoro-phényl)-méthyl]-2-[3-(4-fluorophényl)-propylsulfanyl]-pyridine-3-carboxamide ;
72 N-(2,3-dihydro-benzofuranne-5-yl-méthyl)-2-[3-(4-fluoro-phényl)-propylsulfanyl]-pyridine-3-carboxamide ;
73 N-(5-bicyclo[2.2.1]heptanyl-méthyl)-2-[3-(3-hydroxy-phényl)-propylsulfanyl]-pyridine-3-carboxamide ;
74 N-(5-bicyclo[2.2.1]heptanyl-mêthyl)-2-[[3-(4-fluorophényl)-1-méthylpropyl]sulfanyl]-pyridine-3-carboxamide ;
75 N-(5-bicyclo[2.2.1]heptanyl-méthyl)-2-[[3-(4-fluorophényl)-2-méthylpropyl]sulfanyl]-pyridine-3-carboxamide ;
76 2-[3-(4-fluorophényl)-propylsulfanyl]-N-[(2-méthoxyphényl)-méthyl]-pyridine-3-carboxamide ;
77 2-[3-(4-fluorophényl)-propylsulfanyl]-N-[(3-méthoxyphényl)-méthyl]-pyridine-3-carboxamide ;
78 2-[3-(4-fluorophényl)-propylsulfanyl]-N-phènéthylpyridine-3-carboxamide ;
79 2- [3- (4-fluorophényl)-propylsulfanyl]-N-[[4-(trifluorométhyloxy)-phényl]-méthyl]-pyridine-3-carboxamide ;
80 2- [3- (4-fluorophényl)-propylsulfanyl]-N-[[4-(trifluorométhyl)-phényl]-méthyl]-pyridine-3-carboxamide ;
81 2-[3-(4-fluorophényl)-propylsulfanyl]-N-(2-pyridine-3-yl-éthyl)-pyridine-3-carboxamide ;
82 2-[3-(4-fluorophényl)-propylsulfanyl]-N-(2-pyridine-2-yl-éthyl)-pyridine-3-carboxamide ;
83 2-[3-(4-fluorophényl)-propylsulfanyl]-N-[(2-hydroxyphényl)-méthyl]-pyridine-3-carboxamide ;
84 2-[3-(4-fluorophényl)-propylsulfanyl]-N-[(3-hydroxyphényl)-méthyl]-pyridine-3-carboxamide ;
85 2-[3-(4-fluorophényl)-propylsulfanyl]-N-[2-(m-tolyl)-éthyl]-pyridine-3-carboxamide ;
86 2-[3-(4-fluorophényl)-propylsulfanyl]-N-[2-(o-tolyl)-éthyl]-pyridine-3-carboxamide ;
87 2- [3- (4-fluorophényl) -propylsulfanyl]-N- [[3-(trifluorométhyloxy)-phényl]-méthyl]-pyridine-3-carboxamide ;
88 2-[3-(4-fluorophényl)-propylsulfanyl]-N-[(4-hydroxyphényl)-méthyl]-pyridine-3-carboxamide ;
89 2-[3-(4-fluorophényl)-propylsulfanyl]-N-(2-pyridine-4-yl-éthyl)-pyridine-3-carboxamide ;
90 2-[3-(4-fluorophényl)-propylsulfanyl]-N-[2-(p-tolyl)-éthyl]-pyridine-3-carboxamide ;
91 N-(5-bicyclo[2.2.1]heptanyl-méthyl)-2-[3-(4-fluorophényl)-butylsulfanyl]-pyridine-3-carboxamide ;
92 N-(5-bicyclo[2.2.1]heptanyl-méthyl)-2-[3-(2,4,5-trifluoro-phényl)-propylsulfanyl]-pyridine-3-carboxamide ;
93 2-[3-(4-fluorophényl)-propylsulfanyl]-N-(3-pyridine-2-yl-propyl)-pyridine-3-carboxamide ;
94 2-[3-(4-fluorophényl)-propylsulfanyl]-N-(3-pyridine-3-yl-propyl)-pyridine-3-carboxamide ;
95 2-[3-(4-fluorophényl)-propylsulfanyl]-N-(3-pyridine-4-yl-propyl)-pyridine-3-carboxamide ;
96 2- [3- (4-fluorophényl)-propylsulfanyl]-N-propyl-pyridine-3-carboxamide ;
97 N-butyl-2- [3- (4-fluorophényl)-propylsulfanyl]-pyridine-3-carboxamide ;
98 2-(3-pyridine-3-yl-propylsulfanyl)-N-(thiophène-2-yl-méthyl)-pyridine-3-carboxamide ;
99 2-[3-(p-tolyl)-propylsulfanyl]-N-(thiophène-2-yl-méthyl)-pyridine-3-carboxamide ;
100 2-(4-phényl-butylsulfanyl)-N-(thiophène-2-yl-méthyl)-pyridine-3-carboxamide ;
101 2-(3-pyridine-4-yl-propylsulfanyl)-N-(thiophène-2-yl-méthyl)-pyridine-3-carboxamide ;
102 2-(3-naphtalène-2-yl-propylsulfanyl)-N-(thiophène-2-yl-méthyl)-pyridine-3-carboxamide ;
103 2-[3-(m-tolyl)-propylsulfanyl]-N-(thiophène-2-yl-méthyl)-pyridine-3-carboxamide ;
104 N-(thiophène-2-yl-méthyl)-2-(3-thiophène-2-yl-propylsulfanyl)-pyridine-3-carboxamide ;
105 2-[(1-méthyl-3-phényl-propyl)sulfanyl]-N-(thiophène-2-yl-méthyl)-pyridine-3-carboxamide ;
106 N-(thiophène-2-yl-méthyl)-2-(3-thiophène-3-yl-propylsulfanyl)-pyridine-3-carboxamide ;
107 2-[[1-méthyl-3-[3-(trifluorométhyl)phényl]-propyl]-sulfanyl]-N-(thiophène-2-ylméthyl)-pyridine-3-carboxamide ;

108 2-[(2-benzyl-cyclohexyl)sulfanyl]-N-(thiophène-2-ylméthyl)-pyridine-3-carboxamide ;
109 2-[3-[3-méthyl-5-(trifluorométhyl)-phényl]-propyl-sulfanyl]-N-(thiophène-2-ylméthyl)-pyridine-3-carboxamide ;
110 2-[4-(3,4-difluoro-phényl)-butylsulfanyl]-N-(thiophène-2-yl-méthyl)-pyridine-3-carboxamide ;
111 2-(3-pyridine-2-yl-propylsulfanyl)-N-(thiophène-2-ylméthyl)-pyridine-3-carboxamide ;
113 2-[3-[4-méthyl-3-(trifluorométhyl)-phényl]-propylsulfanyl]-N-(thiophène-2-ylméthyl)-pyridine-3-carboxamide ;
114 2-[(3-phényl-cyclohexyl)sulfanyl]-N-(thiophène-2-ylméthyl)-pyridine-3-carboxamide ;
116 2-[[3,3-difluoro-3-(4-fluorophényl)-propyl]-sulfanyl]-N-(3,3-diméthyl-butyl)-pyridine-3-carboxamide ;
117 N-(cycloheptyl-méthyl)-2-[[3,3-difluoro-3-(4-fluorophényl)-propyl]sulfanyl]-pyridine-3-carboxamide ;
118 2-[[3,3-difluoro-3-(4-fluorophényl)-propyl]-sulfanyl]-N-(thiophène-2-yl-méthyl)-pyridine-3-carboxamide ;
119 2-[3-(4-fluorophényl)-propylsulfanyl]-N-(1,2,3,4-tétrahydronaphtalène-2-ylméthyl)-pyridine-3-carboxamide ;
120 N-(2,3-dihydro-1H-indène-2-yl-méthyl)-2-[3-(4-fluorophényl)-propylsulfanyl]-pyridine-3-carboxamide ;
121 N-(1,3-benzodioxole-5-yl-méthyl)-2-[3-(4-fluorophényl)-propylsulfanyl]-pyridine-3-carboxamide ;
122 2-[3-(4-fluorophényl)-propylsulfanyl]-N-[(3-phénylphényl)-méthyl]-pyridine-3-carboxamide ;
123 2-[3-(4-fluorophényl)-propylsulfanyl]-N-[(3-méthylcyclohexyl)-méthyl]-pyridine-3-carboxamide ;
124 2-[[3,3-difluoro-3-(4-fluorophényl)-propyl]-sulfanyl]-N-[(4-fluorophényl)-méthyl]-pyridine-3-carboxamide ;
125 N-(3,3-diméthyl-2-oxo-butyl)-2-[3-(4-fluorophényl)-propylsulfanyl]-pyridine-3-carboxamide ;
127 2-[3-(4-fluorophényl)-propylsulfanyl]-N-(pyridine-3-yl-méthyl)-pyridine-3-carboxamide ;
128 2-[3-(4-fluorophényl)-propylsulfanyl]-N-(pyridine-4-yl-méthyl)-pyridine-3-carboxamide ;
129 ester méthylique d'acide 3-[[[2-[3-(4-fluoro-phényl)-propylsulfanyl]-pyridine-3-carbonyl]amino]-méthyl]-benzoïque
130 2-[3-(4-fluorophényl)-propylsulfanyl]-N-[3-(2-méthoxyphényl)-propyl]-pyridine-3-carboxamide ;
132 N-[(4-fluorophényl)-méthyl]-2-[[1-méthyl-3-[3-(trifluorométhyl)phényl]-propyl]sulfanyl]-pyridine-3-carboxamide ;
133 2-[3-(4-fluorophényl)-propylsulfanyl]-N-(3-méthyl-butyl)-pyridine-3-carboxamide ;
134 2-[3-(4-fluorophényl)-propylsulfanyl]-N-(tétrahydropyranne-2-yl-méthyl)-pyridine-3-carboxamide ;
135 2-[3-(4-fluorophényl)-propylsulfanyl]-N-[3-(1H-pyrazole-1-yl)-propyl]-pyridine-3-carboxamide ;
136 2-[3-(4-fluorophényl)-propylsulfanyl]-N-(naphtalène-2-yl-méthyl)-pyridine-3-carboxamide ;
137 N-(2,3-dihydro-[1,4]benzodioxine-6-yl-méthyl)-2-[3-(4-fluorophényl)-propylsulfanyl]-pyridine-3-carboxamide ;
138 2- [3-(4-fluorophényl)-propylsulfanyl]-N-[(3-morpholine-4-yl-phényl)-méthyl]-pyridine-3-carboxamide ;
139 N-(2,3-dihydro-benzofuranne-6-yl-méthyl)-2-[3-(4-fluorophényl)-propylsulfanyl]-pyridine-3-carboxamide ;
140 2-[3-(4-fluorophényl)-propylsulfanyl]-N-[[3-(1H-pyrazole-1-yl)-phényl]-méthyl]-pyridine-3-carboxamide ;
141 2-[3-(4-fluorophényl)-propylsulfanyl]-N-[3-(1H-[1,2,3]triazole-1-yl)-propyl]-pyridine-3-carboxamide ;
142 N-(7-bicyclo[2.2.1]heptanyl-méthyl)-2-[3-(4-fluorophényl)-propylsulfanyl]-pyridine-3-carboxamide ;
144 2-[3-(4-fluorophényl)-propylsulfanyl]-N-(thiazole-2-yl-méthyl)-pyridine-3-carboxamide ;
145 2-[3-(4-fluorophényl)-propylsulfanyl]-N-(thiazole-5-yl-méthyl)-pyridine-3-carboxamide ;
146 2-[3-(4-fluorophényl)-propylsulfanyl]-N-(oxazole-2-ylméthyl)-pyridine-3-carboxamide ;
148 N-(3,3-diméthyl-butyl)-2-[3-(4-fluorophényl)-propylsulfanyl]-4-méthylpyridine-3-carboxamide ;
149 N-(3,3-diméthyl-butyl)-2-[[1-méthyl-3-[3-(trifluorométhyl)phényl]-propyl]sulfanyl]-pyridine-3-carboxamide ;
150 2-[3-(4-fluorophényl)-propylsulfanyl]-N-(quinoline-7-yl-méthyl)-pyridine-3-carboxamide ;
151 2-[3-(4-fluorophényl)-propylsulfanyl]-N-[(3-pyridine-2-yl-phényl)-méthyl]-pyridine-3-carboxamide ;
152 2-[3-(4-fluorophényl)-propylsulfanyl]-N-[(3-pyridine-3-yl-phényl)-méthyl]-pyridine-3-carboxamide ;
153 N-(3,3-diméthyl-butyl)-2-[[(1R)-1-méthyl-3-phénylpropyl]sulfanyl]-pyridine-3-carboxamide ;
154 N-(3,3-diméthyl-butyl)-2-[[(1S)-1-méthyl-3-phénylpropyl]sulfanyl]-pyridine-3-carboxamide ;
155 2-[(2-benzyl-cyclopentyl)sulfanyl]-N-(3,3-diméthylbutyl)-pyridine-3-carboxamide ;
156 N-(7-bicyclo[2.2.1]heptanyl-méthyl)-2-[3-[3-(trifluorométhyl)phényl]-propylsulfanyl]-pyridine-3-carboxamide ;
157 2-[3-(4-fluorophényl)-propylsulfanyl]-N-[3-(1H-[1,2,4]triazole-1-yl)-propyl]-pyridine-3-carboxamide ;
158 2-[3-(4-fluorophényl)-propylsulfanyl]-N-([1,3,4]oxadiazole-2-yl-méthyl)-pyridine-3-carboxamide ;
159 2-[3-(4-fluorophényl)-propylsulfanyl]-N-[(3-pyridine-4-yl-phényl)-méthyl]-pyridine-3-carboxamide ;
160 N-[[4-(cyclopropylméthyl)-3,4-dihydro-2H-[1,4]-benzoxazine-6-yl]-méthyl]-2-[3-(4-fluorophényl)-propylsulfanyl]-pyridine-3-carboxamide ;
161 N-[(4-éthyl-3,4-dihydro-2H-[1,4]benzoxazine-6-yl)-méthyl]-2-[3-(4-fluorophényl)-propylsulfanyl]-pyridine-3-carboxamide ;
162 2-[3-(4-fluorophényl)-propylsulfanyl]-N-[(4-méthyl-3,4-dihydro-2H-[1,4]benzoxazine-6-yl]-méthyl]-pyridine-3-carboxamide ;
163 2-[3-(4-fluorophényl)-propylsulfanyl]-N-(3-méthyl-3-phényl-butyl)-pyridine-3-carboxamide ;
164 N-(7-bicyclo[2.2.1]heptanyl-méthyl)-2-[[3,3-difluoro-3-(4-fluorophényl)-propyl]sulfanyl]-pyridine-3-carboxamide ;
165 2-[[3,3-difluoro-3-[3-(trifluorométhyl)phényl]-propyl]-sulfanyl]-N-[(4-fluorophényl)-méthyl]-pyridine-3-carboxamide ;
166 2-[[3,3-difluoro-3-[3-(trifluorométhyl)phényl]-propyl]-sulfanyl]-N-(3,3-diméthylbutyl)-pyridine-3-carboxamide ;
168 2-[[3,3-difluoro-3-(4-fluorophényl)-propyl]-sulfanyl]-N-[(3-fluorophényl)-méthyl]-pyridine-3-carboxamide ;
170 N-butyl-2-[[3,3-difluoro-3-(4-fluorophényl)-propyl]-sulfanyl]-pyridine-3-carboxamide ;
171 2-[[3,3-difluoro-3-(4-fluorophényl)-propyl]-sulfanyl]-N-(2-méthoxy-éthyl)-pyridine-3-carboxamide ;
172 2-[3-(4-fluorophényl)-propylsulfanyl]-N-(2-méthoxyéthyl)-pyridine-3-carboxamide ;
173 N-[(4-fluoro-2-hydroxy-phényl)-méthyl]-2-[3-(4-fluoro-phényl)-propylsulfanyl]-pyridine-3-carboxamide ;
174 2-[3-(4-fluorophényl)-propylsulfanyl]-N-(furanne-2-yl-méthyl)-pyridine-3-carboxamide ;
175 2-[3-(4-fluorophényl)-propylsulfanyl]-N-[(5-méthyl-furanne-2-yl)-méthyl]-pyridine-3-carboxamide ;
176 N-[(4-fluorophényl)-méthyl]-2-[[3-(4-fluoro-phényl)-1-méthyl-propyl]sulfanyl]-pyridine-3-carboxamide ;
177 2-[[3-(4-fluorophényl)-1-méthyl-propyl]sulfanyl]-N-(3-méthyl-butyl)-pyridine-3-carboxamide ;
178 N-(7-bicyclo[2.2.1]heptanyl-méthyl)-2-[[3-(4-fluorophényl)-1-méthylpropyl]-sulfanyl]-pyridine-3-carboxamide ;
179 2-[[3,3-difluoro-3-(4-fluorophényl)-propyl]-sulfanyl]-N-(tétrahydrofuranne-2-ylméthyl)-pyridine-3-carboxamide ;
180 2-[[3,3-difluoro-3-(4-fluorophényl)-propyl]-sulfanyl]-N-(tétrahydropyranne-2-ylméthyl)-pyridine-3-carboxamide ;
181 2-[3-(4-fluorophényl)-propylsulfanyl]-N-(tétrahydrofuranne-2-yl-méthyl)-pyridine-3-carboxamide ;
182 2-[3-(4-fluorophényl)-propylsulfanyl]-N-(2-méthoxy-3,3-diméthylbutyl)-pyridine-3-carboxamide ;
183 2-[[3,3-difluoro-3-(4-fluorophényl)-propyl]-sulfanyl]-N-(m-tolyl-méthyl)-pyridine-3-carboxamide ;
184 2-[[3,3-difluoro-3-(4-fluorophényl)-propyl]-sulfanyl]-N-[(3,5-diméthyl-phényl)-méthyl]-pyridine-3-carboxamide ;
185 2-[[3,3-difluoro-3-(4-fluorophényl)-propyl]-sulfanyl]-N-propyl-pyridine-3-carboxamide ;
186 2-[[3,3-difluoro-3-(4-fluorophényl)-propyl]-sulfanyl]-N-hexyl-pyridine-3-carboxamide ;
187 2-[[3,3-difluoro-3-(4-fluorophényl)-propyl]-sulfanyl]-N-(tétrahydrofuranne-3-ylméthyl)-pyridine-3-carboxamide ;
188 2-[[3,3-difluoro-3-(4-fluorophényl)-propyl]-sulfanyl]-N-(tétrahydropyranne-3-ylméthyl)-pyridine-3-carboxamide ;
189 2-[[3,3-difluoro-3-(4-fluorophényl)-propyl]-sulfanyl]-N-(tétrahydropyranne-4-ylméthyl)-pyridine-3-carboxamide ;
190 2-[[3,3-difluoro-3-(4-fluorophényl)-propyl]-sulfanyl]-N-(furanne-2-yl-méthyl)-pyridine-3-carboxamide ;
191 2-[[3,3-difluoro-3-(4-fluorophényl)-propyl]-sulfanyl]-N-[(5-méthyl-furanne-2-yl)-méthyl]-pyridine-3-carboxamide ;
192 2-[[3,3-difluoro-3-(4-fluorophényl)-propyl]-sulfanyl]-N-pentyl-pyridine-3-carboxamide ;
193 2-[[3,3-difluoro-3-(4-fluorophényl)-propyl]-sulfanyl]-N-(3-méthoxy-butyl)-pyridine-3-carboxamide ;
194 2-[[3,3-difluoro-3-(4-fluorophényl)-propyl]-sulfanyl]-N-(2-méthoxy-propyl)-pyridine-3-carboxamide ;
195 2-[[3,3-difluoro-3-(4-fluorophényl)-propyl]-sulfanyl]-N-(2-méthoxy-butyl)-pyridine-3-carboxamide ;
196 ester méthylique d'acide 3-[[2-[[3,3-difluoro-3-(4-fluorophényl)-propyl]-sulfanyl]-pyridine-3-carbonyl]-amino]-propionique
197 acide 3-[[2-[[3,3-difluoro-3-(4-fluorophényl)-propyl]-sulfanyl]-pyridine-3-carbonyl]amino]-propionique
198 2-[[3,3-difluoro-3-(4-fluorophényl)-propyl]-sulfanyl]-N-(2-diméthylaminoéthyl)-pyridine-3-carboxamide ;
199 2-[[3-(3,4-difluoro-phényl)-1-méthyl-propyl]-sulfanyl]-N-[(4-fluorophényl)-méthyl]-pyridine-3-carboxamide ;
200 2- [[3-(3,4-difluoro-phényl)-1-méthyl-propyl]-sulfanyl]-N-(3-méthyl-butyl)-pyridine-3-carboxamide ;
201 N-[(4-fluorophényl)-méthyl]-2-[[3-(3-fluoro-phényl)-1-méthyl-propyl]sulfanyl]-pyridine-3-carboxamide ;
202 2-[[3-(3-fluorophényl)-1-méthyl-propyl]sulfanyl]-N-(3-méthyl-butyl)-pyridine-3-carboxamide ;
203 N-(3-méthylbutyl)-2-[[1-méthyl-3-[3-(trifluorométhyl)-phényl]-propyl]sulfanyl]-pyridine-3-carboxamide ;
204 2-[[3-(3,4-difluorophényl)-3,3-difluoro-ropyl]-sulfanyl]-N-[(4-fluorophényl)-méthyl]-pyridine-3-carboxamide ;
205 N-(1-bicyclo[2.2.1]heptanyl-méthyl)-2-[[3,3-difluoro-3-(4-fluorophényl)-propyl]sulfanyl]-pyridine-3-carboxamide ;
206 N-(1-bicyclo[2.2.1]heptanyl-méthyl)-2-[3-(4-fluorophényl)-propylsulfanyl]-pyridine-3-carboxamide ;
207 N-[(4-fluorophényl)-méthyl]-2-[3-[3-(trifluoro-méthyl)-phényl]-propylsulfanyl]-pyridine-3-carboxamide ;

208 N-(3-méthyl-butyl)-2-[3-[3-(trifluorométhyl)-phényl]-propylsulfanyl]-pyridine-3-carboxamide ;
209 2-[[3-(3,4-difluoro-phényl)-3,3-difluoro-propyl]-sulfanyl]-N-(3-méthyl-butyl)-pyridine-3-carboxamide ;
210 N-(7-bicyclo[2.2.1]heptanyl-méthyl)-2-[[3-(3,4-difluorophényl)-3,3-difluoropropyl]sulfanyl]-pyridine-3-carboxamide ;
211 2-[[3,3-difluoro-3-(4-fluorophényl)-propyl]-sulfanyl]-N-(2-hydroxy-éthyl)-pyridine-3-carboxamide ; et
224 N-[(4-fluoro-2-méthoxy-phényl)-méthyl]-2-[3-(4-fluorophényl)-propylsulfanyl]-pyridine-3-carboxamide ;

ou leurs sels physiologiquement acceptables.

**12.** Médicaments contenant au moins un nicotinamide substitué selon l'une des revendications 1 à 11, respectivement sous la forme d'un unique stéréoisomère ou de leur mélange, du composé libre et/ou de ses sels physiologiquement acceptables, ainsi que contenant, le cas échéant, des additifs et/ou des auxiliaires et/ou le cas échéant d'autres substances actives.

**13.** Utilisation d'au moins un nicotinamide substitué selon l'une des revendications 1 à 11, respectivement sous la forme d'un unique stéréoisomère ou de leur mélange, du composé libre et/ou de ses sels physiologiquement acceptables, pour la préparation d'un médicament pour le traitement de la douleur, de l'épilepsie, des états d'anxiété, de la dépendance, de la manie, des troubles bipolaires, de la migraine, des maladies cognitives, des dyskinésies associées à une dystonie et/ou de l'incontinence urinaire.

**14.** Nicotinamide substitué selon l'une des revendications 1 à 11, respectivement sous la forme d'un unique stéréoisomère ou de leur mélange, du composé libre et/ou de ses sels physiologiquement acceptables, pour la préparation d'un médicament pour le traitement de la douleur, de l'épilepsie, des états d'anxiété, de la dépendance, de la manie, des troubles bipolaires, de la migraine, des maladies cognitives, des dyskinésies associées à une dystonie et/ou de l'incontinence urinaire.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20020128277 A
- WO 2005105733 A
- WO 2007015767 A
- WO 2008012532 A


### In der Beschreibung aufgeführte Nicht-Patentliteratur


- **PASSMORE et al.** *J Neurosci.,* 2003, vol. 23 (18), 7227-36
- **BLACKBURN-MUNRO ; JENSEN.** *Eur J Pharmacol.,* 2003, vol. 460 (2-3), 109-16
- **DOST et al.** *Naunyn Schmiedebergs Arch Pharmacol,* 2004, vol. 369 (4), 382-390
- **NIELSEN et al.** *Eur J Pharmacol.,* 2004, vol. 487 (1-3), 93-103
- **GRIBKOFF.** *Expert Opin Ther Targets,* 2003, vol. 7 (6), 737-748
- **KORSGAARD et al.** *J Pharmacol Exp Ther.,* 2005, vol. 14 (1), 282-92
- **WICKENDEN et al.** *Expert Opin Ther Pat,* 2004, vol. 14 (4), 457-469
- **GRIBKOFF.** *Expert Opin Ther Targets,* 2008, vol. 12 (5), 565-81
- **MICELI et al.** *Curr Opin Pharmacol,* 2008, vol. 8 (1), 65-74
- **STRENG et al.** *J Urol,* 2004, vol. 172, 2054-2058
- **HANSEN et al.** *Eur J Pharmacol,* 2007, vol. 570 (1-3), 77-88
- **DENCKER et al.** *Epilepsy Behav,* 2008, vol. 12 (1), 49-53
- **RICHTER et al.** *Br J Pharmacol,* 2006, vol. 149 (6), 747-53
- **BENNETT, G.J. ; XIE, Y.K.** A peripheral mononeuropathy in rat that produces disorders of pain sensation like those seen in man. *Pain,* 1988, vol. 33 (1), 87-107
- **KIM, S.H. ; CHUNG, J.M.** An experimental model for peripheral neuropathy produced by segmental spinal nerve ligation in the rat. *Pain,* 1992, vol. 50 (3), 355-363
- **DE SARRO et al.** *Naunyn-Schmiedeberg's Arch. Pharmacol,* 2001, vol. 363, 330-336
- **D'AMOUR ; SMITH.** *J. Pharm. Exp. Ther.,* 1941, vol. 72, 74-79
- **MODELL.** *Rhema Labortechnik, Hofheim, Deutschland,* 2011